# EUROPEAN PATENT APPLICATION

(11) **EP 4 799 608 A2**
(43) Date of publication of application: **02.09.2026**
(21) Application number: 26193064.8
(22) Date of filing: 15.03.2023
(51) Int. Cl.: A61K 9/00

(54) **EXTENDED, HIGH DOSE AFLIBERCEPT REGIMEN FOR TREATMENT OF NEOVASCULAR AGE-RELATED MACULAR DEGENERATION**

(30) Priority: 15.03.2022 US 202263319869 P; 07.09.2022 US 202263404512 P; 08.09.2022 US 202263404893 P; 29.09.2022 US 202263411594 P; 30.09.2022 US 202263412165 P; 01.11.2022 US 202263421298 P; 22.12.2022 US 202263434920 P; 09.02.2023 US 202363444480 P; 22.02.2023 US 202363447582 P; 22.02.2023 CA 3190733
(62) Document number(s) of the earlier application(s) in accordance with Art. 76 EPC: 23162090.7 / 4 245 312
(71) Applicant: Regeneron Pharmaceuticals, Inc., Tarrytown, NY 10591-6707 (US); Bayer Healthcare LLC, Whippany, NJ 07981 (US)
(72) Inventor: VITTI, Robert L, New Jersey, 07675 (US); BERLINER, Alyson J, New York, 10025 (US); CHU, Karen, New York, 10605 (US); ASMUS, Friedrich, 14129 Berlin (DE); DA SILVA LEAL, Sérgio Casimiro, 4153 Reinach (CH); EIßING, Thomas, 40764 Langenfeld (DE); RITTENHOUSE, Kay D, New Jersey, 07869 (US)
(74) Representative: J A Kemp LLP

(57) **Abstract**

The present invention relates to regimens for the treatment of angiogenic eye disorders such as nAMD, characterized by high doses of aflibercept and extended intervals between doses.

## Description

This application is related to U.S. provisional patent application no. 63/319,869, filed on March 15, 2022; U.S. provisional patent application no. 63/404,512, filed on September 7, 2022; U.S. provisional patent application no. 63/404,893, filed on September 8, 2022; U.S. provisional patent application no. 63/411,594, filed on September 29, 2022; U.S. provisional patent application no. 63/412,165, filed on September 30, 2022; U.S. provisional patent application no. 63/421,298, filed on November 1, 2022; U.S. provisional patent application no. 63/434,920, filed on December 22, 2022; U.S. provisional patent application no. 63/444,480, filed on February 9, 2023; and US provisional patent application no. 63/447,582, filed on February 22, 2023 and Canadian Patent Application No. 3,190,733, filed February 22, 2023; each of which is herein incorporated by reference in its entirety.

### REFERENCE TO A SEQUENCE LISTING

This application incorporates by reference a computer readable Sequence Listing in ST.26 XML format, titled 11193WO01_Sequence, created on March 13, 2023, and containing 5,464 bytes.

### FIELD OF THE INVENTION

The field of the present invention relates to methods for treating or preventing angiogenic eye disorders by administering a VEGF antagonist.

### BACKGROUND OF THE INVENTION

Neovascular (wet) AMD (nAMD) is a major health issue in aging populations globally. Vision loss in nAMD results from the abnormal growth and leakage of blood vessels in the macula. In elderly subjects affected by nAMD, vision loss frequently has an even greater impact, as it substantially reduces the visual compensation of functional impairment by other age-related comorbidities, such as arthritis and osteoporosis.

Intravitreally (IVT) administered anti-vascular endothelial growth factor (VEGF) therapies like EYLEA^{®} inhibit neovascular vessel growth and leakage in the retina, and they are currently the standard-of-care for subjects with nAMD. They not only maintain visual function but also provide clinically meaningful visual gains. Treatment of nAMD is chronic and life-long in most subjects to suppress retinal edema and recurrences of choroidal neovascularization (CNV). Although the currently approved IVT anti-VEGF therapies are efficacious and well-tolerated, the need for IVT injections every 4 to 8 weeks, specifically in the initial phase and during maintenance of treatment, represents a significant burden to physicians, subjects, and caregivers. While the procedure is straightforward and relatively easy to perform, capacity issues for ensuring an appropriate injection frequency in order to achieve subject outcomes similar to those seen in the pivotal studies represent an increasing challenge to individual practices and the healthcare system, overall. Moreover, high frequency dosing leads to increased burdens on subjects, *e.g.*, to find transportation and miss work. A secondary effect of this burden is a lower probability of non-compliance with the prescribed treatment regimen.

While the efficacy and safety of currently approved VEGF antagonist therapies have been established for the treatment of nAMD, there remains an unmet medical need for the development of therapies with the potential to reduce treatment burden while providing at least similar or even improved visual outcomes over currently available standard-of-care.

Increasing the molar concentration of VEGF antagonist therapeutic protein in the dosing formulation is a potential way to bring further benefits to subjects with chorioretinal vascular diseases, including nAMD. A higher dose of aflibercept administered IVT has the potential to prolong the drug's therapeutic effects. The resulting extension of treatment intervals early after the initiation of treatment to every 12 weeks or 16 weeks would reduce the number of injections in the first treatment year. A potential decrease in injection-related treatment burden and safety events with fewer injections could be a significant contribution to patient/subject care and healthcare services. EYLEA (2 mg dose, administered at a concentration of 40 mg/mL, also called intravitreal aflibercept injection [IAI]) is currently approved in the United States (US) for the treatment of nAMD, and is also approved for the treatment of macular edema following retinal vein occlusion (RVO), diabetic macular edema (DME), and diabetic retinopathy (DR).

### SUMMARY OF THE INVENTION

The present invention provides methods for treating or preventing neovascular age related macular degeneration comprising administering one or more doses (*e.g.*, of ≥8 mg) of aflibercept such that the clearance of free aflibercept from the ocular compartment is about 0.367-0.458 mL/day (*e.g.*, 0.41 mL/day) after an intravitreal injection of aflibercept and the time for the amount for free aflibercept to reach the lower limit of quantitation (LLOQ) in the ocular compartment of a subject after said intravitreal injection of aflibercept is about 15 weeks; and the time for free aflibercept to reach the lower limit of quantitation (LLOQ) in the plasma (*e.g.*, about 0.0156 mg/L) of a subject after said intravitreal injection of aflibercept is about 3.5 weeks; for example, wherein the aflibercept is administered in an aqueous pharmaceutical formulation wherein the aflibercept has less than about 3.5% high molecular weight species immediately after manufacture and purification and/or less than or equal to about 6% high molecular weight species after storage for about 24 months at about 2-8°C. In an embodiment of the invention, the aqueous pharmaceutical formulation comprises an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C. In an embodiment of the invention, the method comprises administering a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 (preferably 4) weeks after the immediately preceding dose; and wherein each tertiary dose is administered about 12-20 (preferably 12, 16 or 20) weeks after the immediately preceding dose.

The present invention provides a method for slowing the clearance of free aflibercept from the ocular compartment after an intravitreal injection relative to the rate of clearance of aflibercept from the ocular compartment after an intravitreal injection of ≤ 4 mg aflibercept comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose. In an embodiment of the invention, the clearance of free aflibercept from the ocular compartment is about 34% slower than that from the ocular compartment after an intravitreal injection of ≤ 4 mg aflibercept, *e.g.*, wherein the clearance of free aflibercept from the ocular compartment is about 0.367-0.458 mL/day or 0.41 mL/day after an intravitreal injection of > 8 mg aflibercept.

The present invention also provides a method for increasing the time for the amount of free aflibercept to reach the lower limit of quantitation (LLOQ) in the ocular compartment of a subject after an intravitreal injection of aflibercept relative to the time to reach LLOQ of the amount of free aflibercept in the ocular compartment of a subject after an intravitreal injection of about 2 mg aflibercept, *e.g.*, increasing by greater than 1.3 weeks, for example, by about 6 weeks-to more than 10 weeks, for example, to about 15 weeks, comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose.

The present invention also provides a method for increasing the time for free aflibercept to reach the lower limit of quantitation (LLOQ) in the plasma (*e.g.*, about 0.0156 mg/L) of a subject after an intravitreal injection of aflibercept relative to the time to reach LLOQ of free aflibercept in the plasma of a subject after an intravitreal injection of about 2 mg aflibercept, *e.g.*, increased by more than 1.5 weeks, for example by about 2 weeks-to about 3.5 weeks, comprising intravitreally injecting into an eye of a subject in need thereof, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose. In an embodiment of the invention, the >8 mg aflibercept is administered in an aqueous pharmaceutical formulation including aflibercept which includes one or more of histidine-based buffer, arginine (*e.g.*, L-arginine, for example, L-arginine HCl), a sugar or polyol such as sucrose and having a pH of about 5.8. In an embodiment of the invention, the aflibercept has less than about 3.5% high molecular weight species immediately after manufacture and purification and/or less than or equal to about 6% high molecular weight species after storage for about 24 months at about 2-8°C; for example, wherein the ≥8 mg aflibercept is in an aqueous pharmaceutical formulation comprising an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

The present invention provides a method for treating or preventing neovascular age related macular degeneration (nAMD), in a subject in need thereof, for improving best corrected visual acuity in a subject in need thereof with nAMD; or for promoting retinal drying in a subject with nAMD in need thereof; comprising administering to an eye of the subject, one or more doses of about 8 mg or more of VEGF receptor fusion protein once every 12, 13, 14, 15, 16, 17, 18, 19 or 20 or 12-20 or 12-16 or 16-20 weeks.

The present invention provides a method for treating or preventing neovascular age-related macular degeneration (nAMD), in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12-20 weeks after the immediately preceding dose.

The present invention provides a method for treating or preventing neovascular age-related macular degeneration (nAMD), in a subject in need thereof, comprising administering to an eye of the subject, a single initial dose of about 8 mg or more of VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 weeks after the immediately preceding dose.

The present invention provides a method for treating or preventing neovascular age-related macular degeneration (nAMD), in a subject in need thereof, comprising administering to an eye of the subject, a single initial dose of about 8 mg or more of VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 16 weeks after the immediately preceding dose.

The present invention provides a method for treating or preventing neovascular age-related macular degeneration (nAMD), in a subject in need thereof, comprising administering to an eye of the subject, a single initial dose of about 8 mg or more of VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 20 weeks after the immediately preceding dose.

The present invention provides a method for treating or preventing neovascular age-related macular degeneration (nAMD), in a subject in need thereof comprising administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg aflibercept (0.07 mL) via intravitreal injection once every 8 - 16 weeks (2 - 4 months, +/- 7 days).

The present invention provides a method for treating or preventing neovascular age-related macular degeneration (nAMD), in a subject in need thereof comprising administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).

The present invention provides a method for treating or preventing neovascular age-related macular degeneration (nAMD), in a subject in need thereof comprising administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).

The present invention provides a method for treating or preventing neovascular age-related macular degeneration (nAMD), in a subject in need thereof comprising administering 8 mg VEGF receptor fusion protein, preferably aflibercept, (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (+/- 7 days).

The present invention provides a method for treating or preventing neovascular age related macular degeneration (nAMD), in a subject in need thereof, wherein: (1) the subject has received an initial 2 mg dose of VEGF receptor fusion protein then the method comprises, after 1 month, administering to the subject the initial 8 mg dose of VEGF receptor fusion protein and, 1 month thereafter, the 1^{st} 8 mg secondary dose of VEGF receptor fusion protein; and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (2) the subject has received an initial 2 mg dose of VEGF receptor fusion protein, then the method comprises, after 1 month, administering to the subject, the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (3) the subject has received an initial 2 mg dose of VEGF receptor fusion protein, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (4) the subject has received an initial 2 mg dose of VEGF receptor fusion protein, then the method comprises, after 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; (5) the subject has received an initial 2 mg dose of VEGF receptor fusion protein and a 1^{st} 2 mg secondary dose of VEGF receptor fusion protein after 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (6) the subject has received an initial 2 mg dose of VEGF receptor fusion protein and a 1^{st} 2 mg secondary dose of VEGF receptor fusion protein after 1 month, then the method comprises, after another 1 month, administering to the subject a first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (7) the subject has received an initial 2 mg dose of VEGF receptor fusion protein and a 1^{st} 2 mg secondary dose of VEGF receptor fusion protein after 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (8) the subject has received an initial 2 mg dose of VEGF receptor fusion protein and a 1^{st} 2 mg secondary dose of VEGF receptor fusion protein after 1 month, then the method comprises, after another 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; (9) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (10) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (11) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (12) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen; (13) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 1^{st} 2 mg maintenance dose of VEGF receptor fusion protein after 8 weeks, then the method comprises, up to 2 months after the last dose of VEGF receptor fusion protein, administering to the subject the initial 8 mg dose of VEGF receptor fusion protein and 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (14) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the first 8 mg secondary dose of VEGF receptor fusion protein and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (15) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen; (16) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; wherein, (i) said HDq12 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks after the immediately preceding dose; (ii) said HDq16 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks after the immediately preceding dose; and(iii) said HDq20 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 20 weeks after the immediately preceding dose-preferably, wherein the VEGF receptor fusion protein is aflibercept and 2 to 4 weeks is preferably 4 weeks.

The present invention also provides a method for treating or preventing neovascular age related macular degeneration (nAMD), in a subject in need thereof, wherein: (a) the subject has received an initial 8 mg dose of VEGF receptor fusion protein; then the method comprises after 1 month administering to the subject the first 8 mg secondary dose of VEGF receptor fusion protein and 1 month thereafter, administering the 2^{nd} 8 mg secondary dose of VEGF receptor fusion protein; and then, every 12 or 16 or 20 weeks thereafter, administering one or more 8 mg maintenance doses of VEGF receptor fusion protein according to the HDq12 or HDq16 or HDq20 dosing regimen; (b) the subject has received an initial 8 mg dose of VEGF receptor fusion protein & a 1^{st} 8 mg secondary dose of VEGF receptor fusion protein after 1 month, then the method comprises, after another 1 month, administering to the subject the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen; (c) the subject has received an initial 8 mg dose of VEGF receptor fusion protein & a 1^{st} 8 mg secondary dose of VEGF receptor fusion protein after 1 month & a 2^{nd} 8 mg secondary dose of VEGF receptor fusion protein after another month; then the method comprises, after 12 or 16 or 20 weeks, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen; or (d) the subject has received an initial 8 mg dose of VEGF receptor fusion protein & a 1^{st} 8 mg secondary dose of VEGF receptor fusion protein after 1 month & a 2^{nd} 8 mg secondary dose of VEGF receptor fusion protein after another month, then, every 12 or 16 or 20 weeks thereafter, the subject has received one or more 8 mg maintenance doses of VEGF receptor fusion protein; then the method comprises after 12 or 16 or 20 weeks from the last maintenance dose of VEGF receptor fusion protein, administering to the subject one or more 8 mg maintenance doses of VEGF receptor fusion protein and all further 8 mg maintenance doses of VEGF receptor fusion protein every 12 or 16 or 20 weeks according to the HDq12 or HDq16 or HDq20 dosing regimen; wherein, (i) said HDq12 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks after the immediately preceding dose; (ii) said HDq16 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 16 weeks after the immediately preceding dose; and (iii) said HDq20 dosing regimen comprises: a single initial dose of about 8 mg or more of VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 20 weeks after the immediately preceding dose--preferably, wherein the VEGF receptor fusion protein is aflibercept and 2 to 4 weeks is preferably 4 weeks.

The present invention also provides a method for treating or preventing neovascular age related macular degeneration (nAMD), in a subject in need thereof who has been on a dosing regimen for treating or preventing the nAMD calling for a single initial dose of about 2 mg of VEGF receptor fusion protein, preferably aflibercept, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen, comprising administering to an eye of the subject, an 8 mg dose of VEGF receptor fusion protein, evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with 8 mg VEGF receptor fusion protein; or evaluating the subject in about 8 or 10 or 12 weeks after said administering and, if, in the judgment of the treating physician dosing every 12 weeks is appropriate, then administering another 8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.

In an embodiment of the invention, a subject has been on a dosing regimen for treating or preventing neovascular age related macular degeneration of: a single initial dose of about 2 mg of VEGF receptor fusion protein, preferably aflibercept, followed by 2 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose.

The present invention provides a method for treating or preventing neovascular age-related macular degeneration (nAMD), in a subject in need thereof, comprising administering to an eye of the subject, a single initial dose of about 8 mg or more of VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 or 16 weeks after the immediately preceding dose; further comprising, after receiving one or more of said tertiary doses about 12 or 16 after the immediately preceding dose, lengthening the tertiary dose interval from 12 weeks to 16 weeks; 12 weeks to 20 weeks; or 16 weeks to 20 weeks, after the immediately preceding dose; *e.g.*, wherein said tertiary dose interval is adjusted about 48 or 60 weeks after treatment initiation. In an embodiment of the invention, prior to said lengthening, the subject exhibits (a) <5 letter loss in BCVA; and/or (b) CRT <300 or 320 µm. In an embodiment of the invention, the method further comprises evaluating BVCA and/or CRT in the subject and, if the subject exhibits (a) <5 letter loss in BCVA; and/or (b) CRT <300 or 320 µm, lengthening the tertiary dose interval.

The present invention provides a method for treating or preventing nAMD, in a subject in need thereof, comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12 or 16 or 20 weeks after the immediately preceding dose; further comprising, after receiving one or more of said tertiary doses about 12 or 16 or 20 weeks after the immediately preceding dose, shortening the tertiary dose interval from 12 weeks to 8 weeks; 16 weeks to 12 weeks; 16 weeks to 8 weeks, 20 weeks to 8 weeks, 20 weeks to 12 weeks, or 20 weeks to 16 weeks. In an embodiment of the invention, prior to said shortening, the subject exhibits (a) > 10 letter loss in BCVA relative to baseline; and/or (b) > 50 µm increase in CRT relative to baseline. In an embodiment of the invention, the method further comprises evaluating BVCA and/or CRT in the subject and, if the subject exhibits (a) > 10 letter loss in BCVA relative to baseline; and/or (b) > 50 µm increase in CRT relative to baseline, shortening the tertiary dose interval.

In an embodiment of the invention, is (a) greater than 5 letters are lost in BCVA (ETDRS), relative to the BCVA observed at about 12 weeks after treatment initiation; (b) a greater than 25 micrometers increase in CRT is observed relative to the CRT observed at about 12 weeks after treatment initiation; and/or (c) there is a new onset foveal neovascularization or foveal hemorrhage; *e.g.*, at week 16 or week 20 after treatment initiation, then, the interval between tertiary doses is shortened from 12 weeks or 16 weeks to 8 weeks; or if (a) greater than 5 letters are lost in BCVA (ETDRS), relative to the BCVA observed at about 12 weeks after treatment initiation; (b) a greater than 25 micrometers increase in CRT is observed relative to the CRT observed at about 12 weeks after treatment initiation; and/or (c) there is a new onset foveal neovascularization or foveal hemorrhage; *e.g.*, at week 24 after treatment initiation, then, the interval between tertiary doses is shortened from 16 weeks to 12 weeks.

The present invention provides a method for treating or preventing neovascular age related macular degeneration, in a subject in need thereof, comprising administering to an eye of the subject 3 doses of about 8 mg VEGF receptor fusion protein, preferably aflibercept, in a formulation that comprises about 114.3 mg/ml VEGF receptor fusion protein at an interval of once every 4 weeks; and, after said 3 doses, administering one or more doses of the VEGF receptor fusion protein at an interval which is lengthened up to 12, 16 or 20 weeks.

The present invention provides a method for treating or preventing nAMD, in a subject in need thereof, comprising administering to an eye of the subject, a single initial dose of about 8 mg or more of VEGF receptor fusion protein, preferably aflibercept, followed by 2 secondary doses of about 8 mg or more of the VEGF receptor fusion protein, wherein each secondary dose is administered about 2 to 4 (preferably 4) weeks after the immediately preceding dose; and, after said doses, (a) determining if the subject meets at least one criterion for reducing or extending (lengthening) one or more intervals, of 2 weeks, 3 weeks, 4 weeks or 2-4 weeks, between doses of the VEGF receptor fusion protein; and (b) if said determination is made, administering further doses of the VEGF receptor fusion protein at said reduced or extended intervals between doses wherein criteria for reducing the interval include: 1. BCVA loss >5 letters; 2. >25 micrometers increase in central retinal thickness (CRT); 3. new foveal hemorrhage; and/or 4. new foveal neovascularization, wherein criteria for extending the interval include: 1. BCVA loss <5 letters, 2. No fluid at the central subfield; 3. No new onset foveal hemorrhage; and/or 4. No foveal neovascularization. In an embodiment of the invention, criteria for extending the interval include:1. BCVA loss <5 letters from Week 12;2. No fluid at the central subfield on OCT, and3. No new onset foveal hemorrhage or foveal neovascularization; and/or criteria for reducing the interval include both: 1. BCVA loss >5 letters from Week 12, and 2. >25 micrometers increase in central retinal thickness (CRT) from Week 12 or new foveal hemorrhage or new foveal neovascularization. In an embodiment of the invention, if said criteria are met, said interval is extended to 12, 16 or 20 weeks.

The present invention provides a method for treating or preventing neovascular age-related macular degeneration (nAMD), in a subject in need thereof that has been pre-treated with one or more 2 mg doses of VEGF receptor fusion protein, preferably aflibercept, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 12-20 (*e.g.*, 12, 16 or 20) weeks after the immediately preceding dose.

The present invention provides a method for treating or preventing an angiogenic eye disorder (preferably nAMD), in a subject in need thereof, comprising administering to an eye of the subject, (1) a single initial dose of about 8 mg or more of a VEGF receptor fusion protein, preferably aflibercept, followed by one or more (*e.g.*, 2, 3 or 4) secondary doses of about 8 mg or more of the VEGF receptor fusion protein, followed by one or more tertiary doses of about 8 mg or more of the VEGF receptor fusion protein; wherein each secondary dose is administered about 2 to 4 (preferably 4) weeks after the immediately preceding dose; and wherein each tertiary dose is administered about 8 weeks after the immediately preceding dose; or (2) one or more doses of 8 mg or more of VEGF receptor fusion protein about every 4 weeks.

In an embodiment of the invention, a subject having any one or more of ocular or periocular infection; active intraocular inflammation; and/or hypersensitivity; is excluded from treatment or prevention. Subjects lacking such criteria need not be excluded. In an embodiment of the invention, the methods herein further comprise a step of evaluating the subject for: ocular or periocular infection; active intraocular inflammation; and/or hypersensitivity; and excluding the subject treatment or prevention if any one or more if found in the subject.

In an embodiment of the invention, subjects are monitored for adverse events, such as conjunctival hemorrhage, cataract, vitreous detachment, vitreous floaters, corneal epithelium defect and/or increased intraocular pressure. If such AEs are identified, the identified AE may be treated and/or such treatment or prevention may be ceased.

In an embodiment of the invention, a method includes preparation prior to administration of a VEGF receptor fusion protein, preferably aflibercept. For example, wherein the method comprises, prior to each administration, providing or having available- one single-dose glass vial having a protective plastic cap and a stopper containing an aqueous formulation comprising 8 mg VEGF receptor fusion protein in about 70 microliters; a filter needle, *e.g.*, one 18-gauge x 1½-inch, 5-micron, filter needle that includes a tip and a bevel; an invention needle, *e.g.*, one 30-gauge x ½-inch injection needle; and a syringe, *e.g.*, one 1-mL Luer lock syringe having a graduation line marking for 70 microliters of volume; packaged together; then (1) visually inspecting the aqueous formulation in the vial and, if particulates, cloudiness, or discoloration are visible, then using another vial of aqueous formulation containing the VEGF receptor fusion protein; (2) removing the protective plastic cap from the vial; and (3) cleaning the top of the vial with an alcohol wipe; then using aseptic technique: (4) removing the 18-gauge x 1½-inch, 5-micron, filter needle and the 1 mL syringe from their packaging; (5) attaching the filter needle to the syringe by twisting it onto the Luer lock syringe tip; (6) pushing the filter needle into the center of the vial stopper until the needle is completely inserted into the vial and the tip touches the bottom or a bottom edge of the vial; (7) withdrawing all of the VEGF receptor fusion protein vial contents into the syringe, keeping the vial in an upright position, slightly inclined, while ensuring the bevel of the filter needle is submerged into the liquid; (8) continuing to tilt the vial during withdrawal keeping the bevel of the filter needle submerged in the formulation; (9) drawing the plunger rod sufficiently back when emptying the vial in order to completely empty the filter needle; (10) removing the filter needle from the syringe and disposing of the filter needle; (11) removing the 30-gauge x ½-inch injection needle from its packaging and attaching the injection needle to the syringe by firmly twisting the injection needle onto the Luer lock syringe tip; (12) holding the syringe with the needle pointing up, and checking the syringe for bubbles, wherein if there are bubbles, gently tapping the syringe with a finger until the bubbles rise to the top; and (13) slowly depressing the plunger so that the plunger tip aligns with the graduation line that marks 70 microliters on the syringe. In an embodiment of the invention, injection of VEGF receptor fusion protein is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.

In an embodiment of the invention, subject has been receiving a dosing regimen for treating or preventing nAMD calling for: a single initial dose of about 2 mg of VEGF receptor fusion protein, preferably aflibercept, followed by 2 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; wherein the subject is at any phase (initial dose, secondary dose or tertiary dose) of the 2 mg VEGF receptor fusion protein dosing regimen.

In an embodiment of the invention, one or more secondary doses is 2 secondary doses; 2 to 4 weeks is about 4 weeks; 12-20 weeks is about 12 weeks; 12-20 weeks is about 16 weeks; 12-20 weeks is about 20 weeks; 12-20 weeks is about 12-16 weeks; 8-16 weeks is about 12 weeks; 8-16 weeks is about 16 weeks; 8-16 weeks is about 12-16 weeks; 2 to 4 weeks is about 4 weeks and one or more secondary doses is 2 secondary doses; 12-20 weeks is about 12 weeks and one or more secondary doses is 2 secondary doses; 12-20 weeks is about 16 weeks and one or more secondary doses is 2 secondary doses; 12-20 weeks is about 20 weeks and one or more secondary doses is 2 secondary doses; 12-20 weeks is about 12-16 weeks and one or more secondary doses is 2 secondary doses; 2 to 4 weeks is about 4 weeks and one or more secondary doses is 2 secondary doses and a VEGF receptor fusion protein is aflibercept; 12-20 weeks is about 12 weeks and one or more secondary doses is 2 secondary doses and a VEGF receptor fusion protein is aflibercept; 12-20 weeks is about 16 weeks and one or more secondary doses is 2 secondary doses and a VEGF receptor fusion protein is aflibercept; 12-20 weeks is about 20 weeks and one or more secondary doses is 2 secondary doses and a VEGF receptor fusion protein is aflibercept; and/or 12-20 weeks is about 12-16 weeks and one or more secondary doses is 2 secondary doses and a VEGF receptor fusion protein is aflibercept.

In an embodiment of the invention, the VEGF receptor fusion protein: (i) comprises two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2; (ii) comprises two polypeptides that comprise an immunoglobin-like (Ig) domain 2 of VEGFR1, an Ig domain 3 of a VEGFR2, and a multimerizing component; (iii) comprises two polypeptides that comprise an immunoglobin-like (Ig) domain 2 of VEGFR1, an Ig domain 3 of VEGFR2, an Ig domain 4 of VEGFR2 and a multimerizing component; (iv) comprises two VEGFR1R2-FcΔC1(a) polypeptides encoded by the nucleic acid sequence of SEQ ID NO: 1; or (v) is selected from the group consisting of: aflibercept and conbercept. In an embodiment of the invention, the VEGF receptor fusion protein comprises or consists of amino acids 27-457 of the amino acid sequence set forth in SEQ ID NO: 2.

In an embodiment of the invention, the 8 mg of VEGF receptor fusion protein, preferably aflibercept, is in an aqueous pharmaceutical formulation selected from the group consisting of A-KKKK.

In an embodiment of the invention, 8 mg of VEGF receptor fusion protein, preferably aflibercept, is administered in an aqueous pharmaceutical formulation comprising about 114.3 mg/ml VEGF receptor fusion protein.

In an embodiment of the invention, the VEGF receptor fusion protein, preferably aflibercept, is intravitreally administered from a syringe or pre-filled syringe, for example, which is glass or plastic, and/or sterile. In an embodiment of the invention, the VEGF receptor fusion protein is intravitreally injected with a 30 gauge × ½-inch sterile injection needle.

In an embodiment of the invention, a subject has previously received one or more doses of 2 mg VEGF receptor fusion protein, preferably aflibercept-*e.g.*, Eylea.

In an embodiment of the invention, a method set forth herein further includes one or more further doses that are administered.

In an embodiment of the invention, 2 mg VEGF receptor fusion protein, preferably aflibercept, is in an aqueous pharmaceutical formulation comprising 40 mg/ml VEGF receptor fusion protein, *e.g.*, comprising 40 mg/ml VEGF receptor fusion protein, 10 mM sodium phosphate, 40 mM NaCl, 0.03% polysorbate 20 and 5% sucrose, with a pH of 6.2.

In an embodiment of the invention, 8 mg of VEGF receptor fusion protein is in an aqueous pharmaceutical formulation comprising ≥100 mg/ml VEGF receptor fusion protein, preferably aflibercept, histidine-based buffer and arginine (*e.g*., L-arginine). In an embodiment of the invention, 8 mg of VEGF receptor fusion protein is in an aqueous pharmaceutical formulation that comprises a sugar or polyol, for example, sucrose. In an embodiment of the invention, 8 mg of a VEGF receptor fusion protein is in an aqueous pharmaceutical formulation that has a pH of about 5.8. In an embodiment of the invention, 8 mg of a VEGF receptor fusion protein is in an aqueous pharmaceutical formulation comprising about 103-126 mg/ml VEGF receptor fusion protein, histidine-based buffer and arginine, *e.g*., including about 114.3 mg/ml VEGF receptor fusion protein, histidine-based buffer and arginine.

In an embodiment of the invention, the 8 mg of VEGF receptor fusion protein is administered in about 100 µl or less, about 75 µl or less; about 70 µl or less; or about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl; *e.g*., about 70 + 4 or 5 microliters.

In an embodiment of the invention, the methods herein include the step of administering the VEGF receptor fusion protein, preferably aflibercept, to both eyes of the subject, *e*.*g*., intravitreally.

In an embodiment of the invention, a subject achieves and/or maintains one or more of: Increase in best corrected visual acuity (BCVA) by ≥5, ≥10, ≥15, or ≥20 letters; No decrease in best corrected visual acuity (BCVA); Elimination of retinal fluid; Elimination of intraretinal fluid (IRF) and/or subretinal fluid; Decrease in total lesion choroidal neovascularization (CNV) area; Loss of or decrease in intraretinal fluid; Loss of or decrease in subretinal fluid; Decrease in central subfield retinal thickness (CST); Increase in vision-related quality of life; Lack of treatment-emergent adverse events (AEs) and/or serious AEs (SAEs); ETDRS letter score of at least 69 (approximate 20/40 Snellen equivalent); Increase in BCVA as measured by the Early Treatment Diabetic Retinopathy Study (ETDRS) visual acuity chart by ≥5, ≥10, ≥15, or ≥20 letters, or lack of loss thereof during the course of treatment; Increase in BCVA as averaged over a period of 12 weeks; No intraretinal fluid (IRF) and no subretinal fluid; Decrease in choroidal neovascularization (CNV) size; Decrease in total lesion CNV area from baseline; Loss of IRF and/or SRF; Decrease in central subfield retinal thickness (CST); Increase in vision-related quality of life as measured by the National Eye Institute Visual Functioning Questionnaire-25 (NEI-VFQ-25); Lack of treatment-emergent adverse events (AEs) and/or serious AEs (SAEs); Efficacy and/or safety, in a subject suffering from nAMD, similar to that of aflibercept which is intravitreally dosed at 2 mg approximately every 4 weeks for the first 3 months, followed by 2 mg approximately once every 8 weeks or once every 2 months wherein efficacy is measured as increase in BCVA and/or reduction in central retinal thickness, and wherein safety is as measured as the incidence of adverse events such as blood pressure increase, intraocular pressure increase, visual impairment, vitreous floaters, vitreous detachment, iris neovascularization and/or vitreous hemorrhage; No detectable anti-drug antibody during receipt of treatment; Improvement in best corrected visual acuity (BVCA) by week 4, week 8, week 12, week 16, week 20, week 24, week 28, week 32, week 36, week 40, week 44, week 48, week 52, week 56 or week 60 from start of treatment (baseline); Increase in BCVA as measured by the Early Treatment Diabetic Retinopathy Study (ETDRS) visual acuity chart or Snellen equivalent by ≥2 letters, ≥3 letters, ≥4 letters, ≥5 letters, ≥6 letters or ≥7 letters; Improvement in BCVA, by 4 weeks after initiation of treatment, of about 2 or 3 letters (ETDRS or Snellen equivalent) when on HDq12 regimen; or of about 3 letters (ETDRS or Snellen equivalent) when on HDq16 regimen; Improvement in BCVA, by 8 weeks after initiation of treatment, of about 5 letters (ETDRS or Snellen equivalent) when on HDq12 regimen; or of about 4 or 5 letters (ETDRS or Snellen equivalent) when on HDq16 regimen; Improvement in BCVA, by 12 weeks after initiation of treatment, of about 5 or 6 letters (ETDRS or Snellen equivalent) when on HDq12 regimen; or of about 5 or 6 letters (ETDRS or Snellen equivalent) when on HDq16 regimen; Improvement in BCVA, by 16 weeks after initiation of treatment, of about 6 letters (ETDRS or Snellen equivalent) when on HDq12 regimen; or of about 6 or 7 letters (ETDRS or Snellen equivalent) when on HDq16 regimen; Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen; Improvement in BCVA, by 24 weeks after initiation of treatment, of about 5 or 6 letters (ETDRS or Snellen equivalent) when on HDq12 regimen; or of about 5 or 6 letters (ETDRS or Snellen equivalent) when on HDq16 regimen; Improvement in BCVA, by 28 weeks after initiation of treatment, of about 7 letters (ETDRS or Snellen equivalent) when on HDq12 regimen; or of about 6 letters (ETDRS or Snellen equivalent) when on HDq16 regimen; Improvement in BCVA, by 32 weeks after initiation of treatment, of about 6 or 7 letters (ETDRS or Snellen equivalent) when on HDq12 regimen; or of about 7 letters (ETDRS or Snellen equivalent) when on HDq16 regimen; Improvement in BCVA, by 36 weeks after initiation of treatment, of about 6 or 7 letters (ETDRS or Snellen equivalent) when on HDq12 regimen; or of about 6 letters (ETDRS or Snellen equivalent) when on HDq16 regimen; Improvement in BCVA, by 40 weeks after initiation of treatment, of about 6 or 7 letters (ETDRS or Snellen equivalent) when on HDq12 regimen; or of about 5 or 6 letters (ETDRS or Snellen equivalent) when on HDq16 regimen; Improvement in BCVA, by 44 weeks after initiation of treatment, of about 7 letters (ETDRS or Snellen equivalent) when on HDq12 regimen; or of about 5 or 6 letters (ETDRS or Snellen equivalent) when on HDq16 regimen; Improvement in BCVA, by 48 weeks after initiation of treatment, of about 6 or 7 letters (ETDRS or Snellen equivalent) when on HDq12 regimen; or of about 6 letters (ETDRS or Snellen equivalent) when on HDq16 regimen; Improvement in BCVA, by 52 weeks after initiation of treatment, of about 7 or 8 letters (ETDRS or Snellen equivalent) when on HDq12 regimen; or of about 6 or 7 letters (ETDRS or Snellen equivalent) when on HDq16 regimen; Improvement in BCVA, by 56 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; Improvement in BCVA, by 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; Improvement in BCVA, from about 48 to about 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; Improvement in BCVA, by 60 weeks after initiation of treatment, of about 5, 10 or 15 letters (ETDRS or Snellen equivalent) when on the HDq12 regimen; or the HDq16 regimen; An improvement in BCVA by about week 8, 9, 10, 11 or 12 after initiation of treatment which is maintained (within about ±1 or ±2 ETDRS letters or Snellen equivalent) thereafter during the treatment regimen; A BCVA by 4 weeks after initiation of treatment of about 63 letters (ETDRS or Snellen equivalent) when on the HDq12 regimen; or a BCVA of about 63 letters (ETDRS or Snellen equivalent) when on the HDq16 regimen; A BCVA by 8 weeks after initiation of treatment of about 65 letters (ETDRS or Snellen equivalent) when on the HDq12 regimen; or a BCVA of about 65 letters (ETDRS or Snellen equivalent) when on the HDq16 regimen; A BCVA by 12 weeks after initiation of treatment of about 66 letters (ETDRS or Snellen equivalent) when on the HDq12 regimen; or a BCVA of about 66 letters (ETDRS or Snellen equivalent) when on the HDq16 regimen; A BCVA by 16 weeks after initiation of treatment of about 66 letters (ETDRS or Snellen equivalent) when on the HDq12 regimen; or a BCVA of about 66 letters (ETDRS or Snellen equivalent) when on the HDq16 regimen; A BCVA by 20 weeks after initiation of treatment of about 66 letters (ETDRS or Snellen equivalent) when on the HDq12 regimen; or a BCVA of about 66 letters (ETDRS or Snellen equivalent) when on the HDq16 regimen; A BCVA by 24 weeks after initiation of treatment of about 66 letters (ETDRS or Snellen equivalent) when on the HDq12 regimen; or a BCVA of about 66 letters (ETDRS or Snellen equivalent) when on the HDq16 regimen; A BCVA by 28 weeks after initiation of treatment of about 67 letters (ETDRS or Snellen equivalent) when on the HDq12 regimen; or a BCVA of about 66letters (ETDRS or Snellen equivalent) when on the HDq16 regimen; A BCVA by 32 weeks after initiation of treatment of about 67 letters (ETDRS or Snellen equivalent) when on the HDq12 regimen; or a BCVA of about 67 letters (ETDRS or Snellen equivalent) when on the HDq16 regimen; A BCVA by 36 weeks after initiation of treatment of about 67 letters (ETDRS or Snellen equivalent) when on the HDq12 regimen; or a BCVA of about 66 letters (ETDRS or Snellen equivalent) when on the HDq16 regimen; A BCVA by 40 weeks after initiation of treatment of about 67 letters (ETDRS or Snellen equivalent) when on the HDq12 regimen; or a BCVA of about 66 letters (ETDRS or Snellen equivalent) when on the HDq16 regimen; A BCVA by 44 weeks after initiation of treatment of about 68 letters (ETDRS or Snellen equivalent) when on the HDq12 regimen; or a BCVA of about 66 letters (ETDRS or Snellen equivalent) when on the HDq16 regimen; A BCVA by 48 weeks after initiation of treatment of about 67 letters (ETDRS or Snellen equivalent) when on the HDq12 regimen; or a BCVA of about 66 letters (ETDRS or Snellen equivalent) when on the HDq16 regimen; A BCVA by 52 weeks after initiation of treatment of about 67 or 68 letters (ETDRS or Snellen equivalent) when on the HDq12 regimen; or a BCVA of about 66 or 67 letters (ETDRS or Snellen equivalent) when on the HDq16 regimen; A BCVA by 56 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen; A BCVA by 60 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen; A BCVA between about week 48 and about 60 week after initiation of treatment of about 66 to about 72 letters (ETDRS or Snellen equivalent) when on the HDq12 regimen; or a BCVA of about 66 to about 70 letters (ETDRS or Snellen equivalent) when on the HDq16 regimen; Retina without fluid (total fluid, intraretinal fluid [IRF] and/or subretinal fluid [SRF]) in center subfield; No subretinal pigment epithelium fluid; Lack of fluid leakage on fluorescein angiography (FA); Decrease in central retinal thickness (CRT) by at least about 100, 125, 130, 135, 140, 141, 142, 143, 144, 145, 146, 147, 148, 149 or 150 micrometers; A change in central retinal thickness, by 4 weeks after initiation of treatment of about -120 or -121 or -122 or -122.4 or -120.2 micrometers (± about 10, 11 or 12 micrometers) when on the HDq12 regimen; or of about -126 or -127 or-126.6 or -126.3 micrometers (± about 10, 11 or 12 micrometers) when on the HDq16 regimen; A change in central retinal thickness, by 8 weeks after initiation of treatment of about -132, -133, -134, -135 or -136 or -136.2 or -132.8 micrometers (± about 10, 11 or 12 micrometers) when on the HDq12 regimen; or of about -139 or -140 or -139.5 or -139.6 micrometers (± about 10, 11 or 12 micrometers) when on the HDq16 regimen; A change in central retinal thickness, by 12 weeks after initiation of treatment of about -136, -137, -138, -139, -140 or -141 or -140.9 or -136.6 micrometers (± about 10, 11 or 12 micrometers) when on the HDq12 regimen; or of about -144 or -143 or -143.5 micrometers (± about 10, 11 or 12 micrometers) when on the HDq16 regimen A change in central retinal thickness, by 16 weeks after initiation of treatment of about -120 or - 121 or -122 or -123 or -124 or -123.4 or -120.1 micrometers (± about 10, 11 or 12 micrometers) when on the HDq12 regimen; or of about -132 or -133 or -132.1 or -133.1 micrometers (± about 10, 11 or 12 micrometers) when on the HDq16 regimen; A change in central retinal thickness, by 20 weeks after initiation of treatment of about -110 or -111 or -112 or -113 or -114 or -113.6 or -110.9 micrometers (± about 10, 11 or 12 micrometers) when on the HDq12 regimen; or of about -115 or -116 or -117 or -118 or -115.8 or -117.7 micrometers (± about 10, 11 or 12 micrometers) when on the HDq16 regimen; A change in central retinal thickness, by 24 weeks after initiation of treatment of about -134, -135, -136 or -137 or -138 or -137.6 or -134.9 micrometers (± about 10, 11 or 12 micrometers) when on the HDq12 regimen; or of about -105 or -106 or -107 or -108 or -105.3 or -107.8 micrometers (± about 10, 11 or 12 micrometers) when on the HDq16 regimen; A change in central retinal thickness, by 28 weeks after initiation of treatment of about -130, -131 or -132 or -133 or -134 or -133.7 or -130.7 micrometers (± about 10, 11 or 12 micrometers) when on the HDq12 regimen; or of about -144 or -145 or -146 or -147 or -148 or -144.7 or -147.2 micrometers (± about 10, 11 or 12 micrometers) when on the HDq16 regimen; A change in central retinal thickness, by 32 weeks after initiation of treatment of about -118 or -19 or -120 or -121 or -120.4 or -118.1 micrometers (± about 10, 11 or 12 micrometers) when on the HDq12 regimen; or of about -141 or -142 or -143 or -144 or -141.5 or -144 micrometers (± about 10, 11 or 12 micrometers) when on the HDq16 regimen; A change in central retinal thickness, by 36 weeks after initiation of treatment of about -142 or -143 or -144 or -144.2 or -142.2 micrometers (± about 10, 11 or 12 micrometers) when on the HDq12 regimen; or of about -126 or -127, -128 or -129 or -130 or -131 or -126.4 or -130.5 micrometers (± about 10, 11 or 12 micrometers) when on the HDq16 regimen; A change in central retinal thickness, by 40 weeks after initiation of treatment of about -131, -132, -133 or -134 or -133.8 or -131.2 micrometers (± about 10, 11 or 12 micrometers) when on the HDq12 regimen; or of about -127, -128 or -127.5 micrometers (± about 10, 11 or 12 micrometers) when on the HDq16 regimen; A change in central retinal thickness, by 44 weeks after initiation of treatment of about -120 or -121 or -122 or -123 or -124 or -125 or -124.7 or -120.3 micrometers (± about 10, 11 or 12 micrometers) when on the HDq12 regimen; or of about -143, -144 or -145 or -144.8 micrometers (± about 10, 11 or 12 micrometers) when on the HDq16 regimen; A change in central retinal thickness, by 48 weeks after initiation of treatment of about -142 or -143 or -144 or -144.4 or -142.3 micrometers (± about 10, 11 or 12 micrometers) when on the HDq12 regimen; or of about -143 or -144 or -145 or -146 or -147 or -148 or -143.8 or -147.1 micrometers (± about 10, 11 or 12 micrometers) when on the HDq16 regimen; A change in central retinal thickness, by 52 weeks after initiation of treatment of about -143.2 micrometers (± about 10, 11 or 12 micrometers) when on the HDq12 regimen; or of about -139.6 micrometers (± about 10, 11 or 12 micrometers) when on the HDq16 regimen; A change in central retinal thickness, by 56 weeks after initiation of treatment of about -136.3 micrometers (± about 10, 11 or 12 micrometers) when on the HDq12 regimen; or of about -137.5 micrometers (± about 10, 11 or 12 micrometers) when on the HDq16 regimen; A change in central retinal thickness, by 60 weeks after initiation of treatment of about -151.8 micrometers (± about 10, 11 or 12 micrometers) when on the HDq12 regimen; or of about -148.8 micrometers (± about 10, 11 or 12 micrometers) when on the HDq16 regimen; A central retinal thickness by week 4 after initiation of treatment of about 248.2 micrometers when on the HDq12 regimen; or of about 244.1 micrometers when on the HDq16 regimen; A central retinal thickness by week 8 after initiation of treatment of about 234.4 micrometers when on the HDq12 regimen; or of about 231.2 micrometers when on the HDq16 regimen; A central retinal thickness by week 12 after initiation of treatment of about 229.7 micrometers when on the HDq12 regimen; or of about 226.7 micrometers when on the HDq16 regimen; A central retinal thickness by week 16 after initiation of treatment of about 247.2 micrometers when on the HDq12 regimen; or of about 238.6 micrometers when on the HDq16 regimen; A central retinal thickness by week 20 after initiation of treatment of about 257 micrometers when on the HDq12 regimen; or of about 254.9 micrometers when on the HDq16 regimen; A central retinal thickness by week 24 after initiation of treatment of about 233 micrometers when on the HDq12 regimen; or of about 265.4 micrometers when on the HDq16 regimen; A central retinal thickness by week 28 after initiation of treatment of about 236.9 micrometers when on the HDq12 regimen; or of about 226 micrometers when on the HDq16 regimen; A central retinal thickness by week 32 after initiation of treatment of about 250.2 micrometers when on the HDq12 regimen; or of about 229.2 micrometers when on the HDq16 regimen; A central retinal thickness by week 36 after initiation of treatment of about 226.4 micrometers when on the HDq12 regimen; or of about 244.3 micrometers when on the HDq16 regimen; A central retinal thickness by week 40 after initiation of treatment of about 236.8 micrometers when on the HDq12 regimen; or of about 243.7 micrometers when on the HDq16 regimen; A central retinal thickness by week 44 after initiation of treatment of about 245.9 micrometers when on the HDq12 regimen; or of about 227.7 micrometers when on the HDq16 regimen; A central retinal thickness by week 48 after initiation of treatment of about 226.2 micrometers when on the HDq12 regimen; or of about 226.9 micrometers when on the HDq16 regimen; A central retinal thickness by week 52 after initiation of treatment of about 227.4 micrometers when on the HDq12 regimen; or of about 231.1 micrometers when on the HDq16 regimen; A central retinal thickness by week 56 after initiation of treatment of about 234.3 micrometers when on the HDq12 regimen; or of about 233.2 micrometers when on the HDq16 regimen; A central retinal thickness by week 60 after initiation of treatment of about 218.8 micrometers when on the HDq12 regimen; or of about 221.9 micrometers when on the HDq16 regimen; A CRT by about week 4, 5, 6, 7 or 8 after initiation of treatment or a reduction in CRT by week 4, 5, 6, 7 or 8 after initiation of treatment which is maintained (within about ±10, ±11 or ±12 micrometers) thereafter during the treatment regimen; At about 4 hours after treatment initiation of HDq12 or HDq16, a free aflibercept concentration in plasma of about 0.0409 (±0.0605) or 0 mg/L (or <0.0156 mg/L); At about 8 hours after treatment initiation of HDq12 or HDq16, a free aflibercept concentration in plasma of about 0.05 (±3.78), 0.0973 (±0.102) or 0.0672 mg/L; At about day 2 after treatment initiation of HDq12 or HDq16, a free aflibercept concentration in plasma of about 0.11 (±2.21), 0.146 (±0.110) or 0.0903 mg/L; At about day 3 after treatment initiation of HDq12 or HDq16, a free aflibercept concentration in plasma of about 0.11 (±2.06), 0.137 (±0.0947) or 0.112 mg/L; At about day 5 after treatment initiation of HDq12 or HDq16, a free aflibercept concentration in plasma of about 0.08 (±1.86), 0.0933 (±0.0481) or 0.0854 mg/L; At about day 8 after treatment initiation of HDq12 or HDq16, a free aflibercept concentration in plasma of about 0.07 (±1.75), 0.0794 (±0.0413) or 0.0682 mg/L; At about day 15 after treatment initiation of HDq12 or HDq16, a free aflibercept concentration in plasma of about 0.04 (±1.76), 0.0435 (±0.0199) or 0.0385 mg/L; At about day 22 after treatment initiation of HDq12 or HDq16, a free aflibercept concentration in plasma of about 0.02 (±1.76), 0.0213 (±0.0148) or 0.0232 mg/L; At about day 29 after treatment initiation of HDq12 or HDq16, a free aflibercept concentration in plasma of about 0.00766 (±0.00958) or 0 mg/L (or <0.0156 mg/L); At about 4 hours post-dose after treatment initiation of HDq12 or HDq16, an adjusted bound aflibercept concentration in plasma of about 0.00 mg/L; At about 8 hours post-dose after treatment initiation of HDq12 or HDq16, an adjusted bound aflibercept concentration in plasma of about 0.00 mg/L; At about day 2 after treatment initiation of HDq12 or HDq16, an adjusted bound aflibercept concentration in plasma of about 0.06 (±3.50) or 0.124 (±0.186) mg/L; At about day 3 after treatment initiation of HDq12 or HDq16, an adjusted bound aflibercept concentration in plasma of about 0.13 (±2.07) or 0.173 (±0.155) mg/L; At about day 5 after treatment initiation of HDq12 or HDq16, an adjusted bound aflibercept concentration in plasma of about 0.18 (±1.88) or 0.223 (±0.157) mg/L; At about day 8 after treatment initiation of HDq12 or HDq16, an adjusted bound aflibercept concentration in plasma of about 0.31 (±1.56) or 0.334 (±0.135) mg/L; At about day 15 after treatment initiation of HDq12 or HDq16, an adjusted bound aflibercept concentration in plasma of about 0.37 (±1.50) or 0.393 (±0.130) mg/L; At about day 22 after treatment initiation of HDq12 or HDq16, an adjusted bound aflibercept concentration in plasma of about 0.25 (±3.00) or 0.335 (±0.155) mg/L; At about day 29 after treatment initiation of HDq12 or HDq16, an adjusted bound aflibercept concentration in plasma of about 0.32 (±1.39) or 0.331 (±0.0953) mg/L; Non-inferior BVCA compared to that of aflibercept which is intravitreally dosed at 2 mg approximately every 4 weeks for the first 5 injections followed by 2 mg approximately once every 8 weeks or once every 2 months; Ocular and non-ocular safety or death rate, in a subject suffering from DME, similar to that of aflibercept which is intravitreally dosed at 2 mg approximately every 4 weeks for the first 3 or 4 or 5 injections followed by 2 mg approximately once every 8 weeks or once every 2 months; An improvement in best corrected visual acuity by 4, 8, 12, 16, 20, 24, 28, 32, 36, 40, 44 or 48 weeks from start of treatment; An increase in best corrected visual acuity, as measured by Early Treatment Diabetic Retinopathy Study (ETDRS) visual acuity chart or Snellen equivalent of ≥ 2 letters, ≥ 3 letters, ≥ 4 letters, ≥ 5 letters, ≥ 6 letters or ≥ 7 letters by 4, 8, 12, 16, 20, 24, 28, 32, 36, 40, 44, 48, 52, 56 or 60 weeks from start of treatment; A retina without fluid (total fluid, intraretinal fluid [IRF] and/or subretinal fluid [SRF]) in center subfield by 4, 8, 12, 16, 20, 24, 28, 32, 36, 40, 44, 48, 52, 56 or 60 weeks from start of treatment; and/or a decrease in central retinal thickness (CRT) of at least 100, 125, 130, 135, 140, 141, 142, 143, 144, 145, 146, 147, 148, 149 or 150 micrometers by 4, 8, 12, 16, 20, 24, 28, 32, 36, 40, 44, 48, 52, 56 or 60 weeks from start of treatment. In an embodiment of the invention, a dry retina lacks intraretinal fluid and/or subretinal fluid; or dry retina is characterized by no intraretinal fluid (IRF) and no subretinal fluid (SRF) in the eye of the subject. In an embodiment of the invention, dry retina is characterized by no intraretinal fluid (IRF) and no subretinal fluid (SRF) in the eye of the subject, after the subject has received three monthly doses of VEGF receptor fusion protein.

In an embodiment of the invention, 1 initial dose, 2 secondary doses and 3 tertiary doses are administered to the subject in the first year; 1 initial dose, 2 secondary doses and 2 tertiary doses are administered to the subject in the first year; or 1 initial dose, 2 secondary doses and 3 tertiary doses are administered to the subject in the first year followed by 2 -4 tertiary doses in the second year.

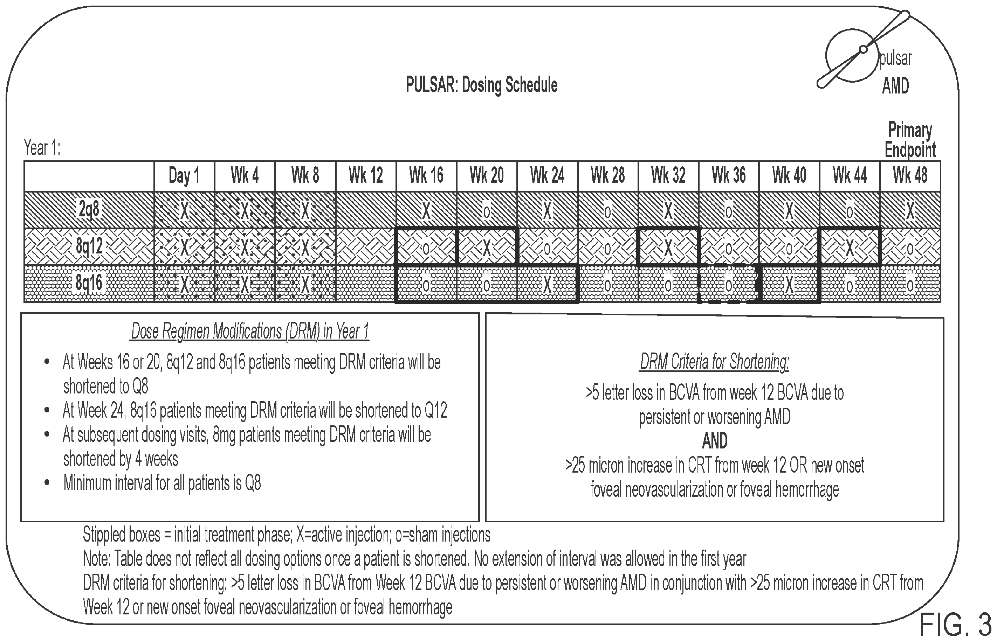

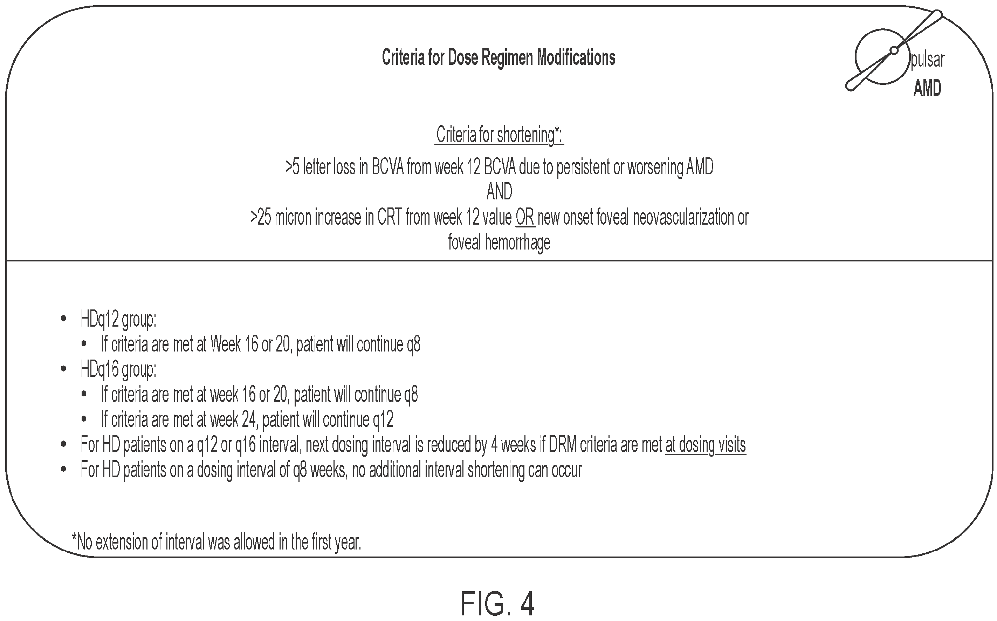

In an embodiment of the invention, the interval between doses are adjusted (increased/maintained/reduced) based on visual and/or anatomic outcomes, *e.g.*, according to the criterial set forth in Figure 3 and/or Figure 4.

In an embodiment of the invention, 12-20 weeks is 12, 13, 14, 15, 16, 17, 18, 19 or 20 weeks; and 2-4 weeks is 2, 3, 4 or 5 weeks.

Preferably, a VEGF receptor fusion protein herein is aflibercept.

The present invention provides a kit comprising a container comprising VEGF receptor fusion protein, preferably aflibercept; and Instruction for use of VEGF receptor fusion protein, wherein the container is a vial or a pre-filled syringe, wherein the container comprises > 100 mg/mL VEGF receptor fusion protein, wherein the container comprises > 114.3 mg/mL VEGF receptor fusion protein, wherein the instruction comprises instruction for the administration of VEGF receptor fusion protein to DME/AMD patients, wherein the instruction comprises instruction that VEGF receptor fusion protein 8 mg treatment is initiated with 1 injection per month (every 4 weeks) for 3 consecutive doses, wherein the instruction comprises instruction that after the initial 3 consecutive doses the injection interval may be extended up to every 16 weeks or every 20 weeks, and wherein the instruction comprises instruction that the treatment interval may be adjusted based on the physician's judgement of visual and/or anatomic outcomes.

The present invention provides aflibercept for use in the treatment or prevention of neovascular age related macular degeneration (nAMD) in a subject in need thereof comprising administering one or more doses of aflibercept at an interval and quantity whereby the clearance of free aflibercept from the ocular compartment is about 0.37-0.46 mL/day after an intravitreal injection of aflibercept, the time for the amount for free aflibercept to reach the lower limit of quantitation (LLOQ) in the ocular compartment of a subject after said intravitreal injection of aflibercept is about 15 weeks; and the time for free aflibercept to reach the lower limit of quantitation (LLOQ) in the plasma of the subject after said intravitreal injection of aflibercept is about 3.5 weeks.

The present invention provides aflibercept for use in a method for slowing the clearance of free aflibercept from the ocular compartment after an intravitreal injection relative to the rate of clearance of aflibercept from the ocular compartment after an intravitreal injection of < 4 mg aflibercept wherein the method comprises intravitreally injecting into an eye of a subject in need thereof,a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose.

The present invention provides aflibercept for use a method for increasing the time for the amount of free aflibercept to reach the lower limit of quantitation (LLOQ) in the ocular compartment of a subject after an intravitreal injection of aflibercept relative to the time to reach LLOQ of the amount of free aflibercept in the ocular compartment of a subject after an intravitreal injection of about 2 mg aflibercept, wherein the method comprises intravitreally injecting into an eye of a subject in need thereof,a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks after the immediately preceding dose.

The present invention provides aflibercept for use in a method for increasing the time for free aflibercept to reach the lower limit of quantitation (LLOQ) in the plasma of a subject after an intravitreal injection of aflibercept relative to the time to reach LLOQ of free aflibercept in the plasma of a subject after an intravitreal injection of about 2 mg aflibercept,
wherein the method comprises intravitreally injecting into an eye of a subject in need thereof,
a single initial dose of about 8 mg or more of aflibercept, followed by
one or more secondary doses of about 8 mg or more of the aflibercept, followed by one or more tertiary doses of about 8 mg or more of the aflibercept;
wherein each secondary dose is administered about 2 to 4 weeks after the immediately preceding dose; and wherein each tertiary dose is administered about 12-20 weeks after the immediately preceding dose.

The present invention provides a VEGF receptor fusion protein for use in a method
- for treating or preventing neovascular age related macular degeneration (nAMD), in a subject in need thereof,
- for improving best corrected visual acuity in a subject in need thereof with nAMD; or
- for promoting retinal drying in a subject with nAMD in need thereof;
wherein the method comprises administering to an eye of the subject, one or more doses of about 8 mg or more of VEGF receptor fusion protein once every 12, 13, 14, 15, 16, 17, 18, 19 or 20 or 12-20 or 12-16 or 16-20 weeks.

The present invention provides aflibercept for use in the treatment or prevention of neovascular age related macular degeneration (nAMD) in a subject in need thereof, wherein the treatment or prevention comprises initiating the treatment with 1 injection of 8 mg aflibercept per month (every 4 weeks) for three consecutive doses followed by one or more injection once every 8 - 16 weeks or 8 - 20 weeks,wherein the concentration of aflibercept of each said dose is 114.3 mg/mL or wherein the application volume of each said dose is 70 µL. In an embodiment of the invention the treatment interval between two subsequent administrations of >8 mg aflibercept is adjusted (increased/maintained/reduced) based on visual and/or anatomic outcomes such as but not limited to letter gain or letter loss in BCVA; increase or reduction in CRT; presence or absence of subretinal fluid; or presence or absence of hemorraghe. In an embodiment of the invention the treatment interval is reduced by 2-4 weeks, 2 weeks, 3 weeks or by 4 weeks compared to the previous treatment interval in case said subject has been identified as one with meeting at least one of the following criteria for reduction of the treatment interval: BCVA loss >5 letters; >25 micrometers increase in central retinal thickness (CRT); new foveal hemorrhage; or new foveal neovascularization. In an embodiment of the invention the treatment interval is extended by 2-4 weeks, 2 weeks, 3 weeks or by 4 weeks compared to the previous treatment interval in case said subject has been identified as one with meeting at least one of the following criteria for extending the treatment interval: BCVA loss <5 letters, no fluid at the central subfield; no new onset foveal hemorrhage; or no foveal neovascularization.

The present invention provides a VEGF receptor fusion protein for use in the treatment or prevention neovascular age-related macular degeneration (nAMD), in a subject in need thereof, wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 8 - 16 weeks (2 - 4 months, +/- 7 days) or every 8 - 20 weeks (2 - 5 months, +/- 7 days).

The present invention provides a VEGF receptor fusion protein for use in the treatment or prevention of neovascular age-related macular degeneration (nAMD), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).

The present invention provides a VEGF receptor fusion protein for use in the treatment or prevention of neovascular age-related macular degeneration (nAMD), in a subject in need thereof wherein the method comprises administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).

The present invention provides a VEGF receptor fusion protein for use in the treatment or prevention of, in a subject in need thereof wherein the method comprises administering 8 mg VEGF neovascular age-related macular degeneration (nAMD) receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (2 - 4 months, +/- 7 days).

The present invention provides a VEGF receptor fusion protein for use in the treatment or prevention of neovascular age-related macular degeneration (nAMD), in a subject in need thereof wherein:
(1) the subject has received an initial 2 mg dose of VEGF receptor fusion protein then the method comprises, after 1 month, administering to the subject the initial ≥8 mg dose of VEGF receptor fusion protein and, 1 month thereafter, the 1^{st} ≥8 mg secondary dose of VEGF receptor fusion protein; and, 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(2) the subject has received an initial 2 mg dose of VEGF receptor fusion protein, then the method comprises, after 1 month, administering to the subject, the first ≥8 mg secondary dose of VEGF receptor fusion protein and, 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(3) the subject has received an initial 2 mg dose of VEGF receptor fusion protein, then the method comprises, after 1 month, administering to the subject the 2^{nd} ≥8 mg secondary dose of VEGF receptor fusion protein and then, every 12 or 16 or 20 weeks thereafter, one or more ≥8 mg maintenance doses of VEGF receptor fusion protein according to the HDq12 or HDq16 or HDq20 dosing regimen;
(4) the subject has received an initial 2 mg dose of VEGF receptor fusion protein, then the method comprises, after 1 month, administering to the subject the 1^{st} ≥8 mg maintenance dose of VEGF receptor fusion protein and all further ≥8 mg maintenance doses of VEGF receptor fusion protein every 12 or 16 or 20 weeks according to the HDq12 or HDq16 or HDq20 dosing regimen;
(5) the subject has received an initial 2 mg dose of VEGF receptor fusion protein and a 1^{st} 2 mg secondary dose of VEGF receptor fusion protein after 1 month, then the method comprises, after another 1 month, administering to the subject the initial ≥8 mg dose of VEGF receptor fusion protein and, 1 month thereafter, the 1^{st} ≥8 mg secondary dose of VEGF receptor fusion protein; and 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(6) the subject has received an initial 2 mg dose of VEGF receptor fusion protein and a 1^{st} 2 mg secondary dose of VEGF receptor fusion protein after 1 month, then the method comprises, after another 1 month, administering to the subject a first ≥8 mg secondary dose of VEGF receptor fusion protein and, 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(7) the subject has received an initial 2 mg dose of VEGF receptor fusion protein and a 1^{st} 2 mg secondary dose of VEGF receptor fusion protein after 1 month, then the method comprises, after another 1 month, administering to the subject the 2^{nd} ≥8 mg secondary dose of VEGF receptor fusion protein and then, every 12 or 16 or 20 weeks thereafter, one or more ≥8 mg maintenance doses of VEGF receptor fusion protein according to the HDq12 or HDq16 or HDq20 dosing regimen;
(8) the subject has received an initial 2 mg dose of VEGF receptor fusion protein and a 1^{st} 2 mg secondary dose of VEGF receptor fusion protein after 1 month, then the method comprises, after another 1 month, administering to the subject the 1^{st} ≥8 mg maintenance dose of VEGF receptor fusion protein and all further ≥8 mg maintenance doses of VEGF receptor fusion protein every 12 or 16 or 20 weeks according to the HDq12 or HDq16 or HDq20 dosing regimen;
(9) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the initial ≥8 mg dose of VEGF receptor fusion protein and, 1 month thereafter, the 1^{st} ≥8 mg secondary dose of VEGF receptor fusion protein; and 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(10) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the first ≥8 mg secondary dose of VEGF receptor fusion protein and, 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(11) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the 2^{nd} ≥8 mg secondary dose of VEGF receptor fusion protein and then, every 12 or 16 or 20 weeks thereafter, one or more ≥8 mg maintenance doses of VEGF receptor fusion protein according to the HDq12 or HDq16 or HDq20 dosing regimen;
(12) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after 2 months, administering to the subject the 1^{st} ≥8 mg maintenance dose of VEGF receptor fusion protein and, all further ≥8 mg maintenance doses of VEGF receptor fusion protein every 12 or 16 or 20 weeks according to the HDq12 or HDq16 or HDq20 dosing regimen;
(13) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 1^{st} 2 mg maintenance dose of VEGF receptor fusion protein after 8 weeks, then the method comprises, up to 2 months after the last dose of VEGF receptor fusion protein, administering to the subject the initial ≥8 mg dose of VEGF receptor fusion protein and 1 month thereafter, the 1^{st} ≥8 mg secondary dose of VEGF receptor fusion protein; and 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(14) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the first ≥8 mg secondary dose of VEGF receptor fusion protein and 1 month thereafter, the 2^{nd} ≥8 mg secondary dose of VEGF receptor fusion protein; and then, every 12 or 16 or 20 weeks thereafter, one or more ≥8 mg maintenance doses of VEGF receptor fusion protein according to the HDq12 or HDq16 or HDq20 dosing regimen;
(15) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 2^{nd} ≥8 mg secondary dose of VEGF receptor fusion protein and then, every 12 or 16 or 20 weeks thereafter, one or more ≥8 mg maintenance doses of VEGF receptor fusion protein according to the HDq12 or HDq16 or HDq20 dosing regimen;
(16) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 1^{st} ≥8 mg maintenance dose of VEGF receptor fusion protein and all further >8 mg maintenance doses of VEGF receptor fusion protein every 12 or 16 or 20 weeks according to the HDq12 or HDq16 or HDq20 dosing regimen;
   wherein,
   (i) said HDq12 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein; wherein each secondary dose is administered about 2 to 4 weeks after the immediately preceding dose; and
      wherein each tertiary dose is administered about 12 weeks after the immediately preceding dose;
   (ii) said HDq16 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein; wherein each secondary dose is administered about 2 to 4 weeks after the immediately preceding dose; and
      wherein each tertiary dose is administered about 16 weeks after the immediately preceding dose;
      and
   (iii) said HDq20 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein; wherein each secondary dose is administered about 2 to 4 weeks after the immediately preceding dose; and
      wherein each tertiary dose is administered about 20 weeks after the immediately preceding dose.

The present invention provides aflibercept for use in the treatment or prevention of neovascular age related macular degeneration (nAMD), in a subject in need thereof, comprising administering to an eye of the subject, a single initial dose >8 mg aflibercept, followed by one or more tertiary doses of about ≥8 mg of aflibercept; wherein each tertiary dose is administered about 8, 12, 16, or 20 weeks after the immediately preceding dose. In an embodiment of the invention, the subject is not a treatment naïve subject, or the subject was pre-treated with a VEGF antagonist or preferably the subject was pre-treated with 8 mg aflibercept or with 2 mg aflibercept.

The present invention provides aflibercept for use in the treatment or prevention of neovascular age related macular degeneration (nAMD), in a subject which was pre-treated with 2 mg aflibercept, comprising administering to an eye of the subject a single initial dose of about ≥8 mg aflibercept, followed by one or more secondary doses of about ≥8 mg of aflibercept, followed by one or more tertiary doses of about 8 mg aflibercept, wherein each secondary dose is administered about 4 weeks after the immediately preceding dose and wherein each tertiary dose is administered about 8, 10, 12, 14, 16, 18 or 20 weeks after the immediately preceding dose. In an embodiment of the invention, the administration of one or more doses of 8 mg aflibercept to an eye of the subject is according to HDq12, HDq16, HDq20, or treat and extent dosing regimen.

The present invention provides a VEGF receptor fusion protein for use in the treatment or prevetion of neovascular age related macular degeneration (nAMD), in a subject in need thereof who has been on a dosing regimen for treating or preventing said disorder wherein:
(a) the subject has received an initial ≥8 mg dose of VEGF receptor fusion protein then the method comprises, after 1 month, administering to the subject the first ≥8 mg secondary dose of VEGF receptor fusion protein and 1 month thereafter, administering the 2nd ≥8 mg secondary dose of VEGF receptor fusion protein; and then, every 12 or 16 or 20 weeks thereafter, administering one or more ≥8 mg maintenance doses of VEGF receptor fusion protein according to the HDq12 or HDq16 or HDq20 dosing regimen;
   or
(b) the subject has received an initial ≥8 mg dose of VEGF receptor fusion protein & 1^{st} ≥8 mg secondary dose of VEGF receptor fusion protein after 1 month, then the method comprises, after another 1 month, administering to the subject the 2^{nd} ≥8 mg secondary dose of VEGF receptor fusion protein; and then, every 12 or 16 or 20 weeks thereafter, one or more >8 mg maintenance doses of VEGF receptor fusion protein according to the HDq12 or HDq16 or HDq20 dosing regimen;
   or
(c) the subject has received an initial ≥8 mg dose of VEGF receptor fusion protein & 1^{st} ≥8 mg secondary dose of VEGF receptor fusion protein after 1 month & the 2^{nd} ≥8 mg secondary dose of VEGF receptor fusion protein after another month, then the method comprises, after 12 or 16 or 20 weeks administering to the subject the 1^{st} ≥8 mg maintenance dose of VEGF receptor fusion protein and all further ≥8 mg maintenance doses of VEGF receptor fusion protein every 12 or 16 or 20 weeks according to the HDq12 or HDq16 or HDq20 dosing regimen;
   or
(d) the subject has received an initial ≥8 mg dose of VEGF receptor fusion protein & a 1^{st} ≥8 mg secondary dose of VEGF receptor fusion protein after 1 month & the 2^{nd} ≥8 mg secondary dose of VEGF receptor fusion protein after another month, then every 12 or 16 or 20 weeks thereafter, the subject has received one or more ≥8 mg maintenance doses of VEGF receptor fusion protein; and, then the method comprises, after 12 or 16 or 20 weeks from the last maintenance dose of VEGF receptor fusion protein, administering to the subject one or more ≥8 mg maintenance doses of VEGF receptor fusion protein and all further ≥8 mg maintenance doses of VEGF receptor fusion protein every 12 or 16 or 20 weeks according to the HDq12 or HDq16 or HDq20 dosing regimen;
wherein,
(i) said HDq12 dosing regimen comprises:
   a single initial dose of about ≥8 mg or more of VEGF receptor fusion protein, followed by one or more secondary doses of about ≥8 mg or more of the VEGF receptor fusion protein, followed by
   one or more tertiary doses of about ≥8 mg or more of the VEGF receptor fusion protein;
   wherein each secondary dose is administered about 2 to 4 weeks after the immediately preceding dose; and
   wherein each tertiary dose is administered about 12 weeks after the immediately preceding dose;
(ii) said HDq16 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 16 weeks after the immediately preceding dose;
   and
(iii) said HDq20 dosing regimen comprises:
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein; wherein each secondary dose is administered about 2 to 4 weeks after the immediately preceding dose; and
   wherein each tertiary dose is administered about 20 weeks after the immediately preceding dose.

The present invention provides a VEGF receptor fusion protein for use in the treatment or prevention of neovascular age related macular degeneration (nAMD), in a subject in need thereof who has been on a dosing regimen for treating or preventing the nAMD calling for a single initial dose of about 2 mg of VEGF receptor fusion protein, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen, comprising administering to an eye of the subject, an ≥8 mg dose of VEGF receptor fusion protein, evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with ≥8 mg VEGF receptor fusion protein; or evaluating the subject in about 8 or 10 or 12 weeks after said administering and, if, in the judgment of the treating physician dosing every 12 weeks is appropriate, then administering another ≥8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.

The present invention provides a VEGF receptor fusion protein for use in the treatment and prevention of neovascular age related macular degeneration (nAMD), in a subject in need thereof, wherein the treatment or prevention comprises administering to an eye of the subject, a single initial dose of about 8 mg or more of a VEGF receptor fusion protein, followed by one or more secondary doses, preferably 2 doses, of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 weeks after the immediately preceding dose;
further comprising, after receiving one or more of said tertiary doses about 12 or 16 after the immediately preceding dose, lengthening the tertiary dose interval from
   - 12 weeks to 16 weeks;
   - 12 weeks to 20 weeks; or
   - 16 weeks to 20 weeks,
after the immediately preceding dose.

The present invention provides a VEGF receptor fusion protein for use in the treatment and prevention of neovascular age related macular degeneration (nAMD), in a subject in need thereof, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 or 16 or 20 weeks after the immediately preceding dose; further comprising, after receiving one or more of said tertiary doses about 12 or 16 or 20 weeks after the immediately preceding dose, shortening the tertiary dose interval from
- 12 weeks to 8 weeks;
- 16 weeks to 12 weeks;
- 16 weeks to 8 weeks,
- 20 weeks to 8 weeks,
- 20 weeks to 12 weeks, or
- 20 weeks to 16 weeks.

The present invention provides a VEGF receptor fusion protein for use in the treatment and prevention of neovascular age related macular degeneration (nAMD), in a subject in need thereof, comprising administering to an eye of the subject 3 doses of about >8 mg VEGF receptor fusion protein in a formulation that comprises about 114.3 mg/ml VEGF receptor fusion protein at an interval of once every 4 weeks; wherein after said 3 doses, administering one or more doses of the VEGF receptor fusion protein at an interval which is lengthened up to 12, 16 or 20 weeks.

The present invention provides a VEGF receptor fusion protein for use in the treatment and prevention of neovascular age related macular degeneration (nAMD), in a subject in need thereof, comprising administering to an eye of the subject, a single initial dose of about 8 mg or more of VEGF receptor fusion protein, followed by 2 secondary doses of about 8 mg or more of the VEGF receptor fusion protein,
wherein each secondary dose is administered about 2 to 4 weeks after the immediately preceding dose; and, after said doses,
   a) determining if the subject meets at least one criterion for reducing or lengthening one or more intervals by 2 weeks, 3 weeks, 4 weeks or 2-4 weeks between doses of the VEGF receptor fusion protein; and
   b) if said determination is made, administering further doses of the VEGF receptor fusion protein at said reduced or lengthened intervals between doses
wherein criteria for lengthening the interval include:
   1. BCVA loss >5 letters;
   2. >25 micrometers increase in central retinal thickness (CRT);
   3. new foveal hemorrhage; and/or
   4. new foveal neovascularization
and, wherein criteria for reducing the interval include:
   1. BCVA loss <5 letters,
   2. No fluid at the central subfield;
   3. No new onset foveal hemorrhage; and/or
   4. No foveal neovascularization.

The present invention provides a VEGF receptor fusion protein for use in the treatment and prevention of of neovascular age related macular degeneration (nAMD), in a subject in need thereof that has been pre-treated with one or more 2 mg doses of VEGF receptor fusion protein, comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks after the immediately preceding dose.

The present invention provides a VEGF receptor fusion protein for use in the treatment and prevention of an angiogenic eye disorder, in a subject in need thereof, comprising administering to an eye of the subject,
(1) a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 8 weeks after the immediately preceding dose; or
(2) one or more doses of 8 mg or more of VEGF receptor fusion protein about every 4 weeks.

A VEGF receptor fusion protein for use in the treatment and prevention of an angiogenic eye disorder wherein the treatment or prevention comprises, prior to each administration, providing
- one single-dose glass vial having a protective plastic cap and a stopper containing an aqueous formulation comprising 8 mg VEGF receptor fusion protein in about 70 microliters;
- one 18-gauge x 1½-inch, 5-micron, filter needle that includes a tip and a bevel;

packaged together; then
   (1) visually inspecting the aqueous formulation in the vial and, if particulates, cloudiness, or discoloration are visible, then using another vial of aqueous formulation containing the VEGF receptor fusion protein;
   (2) removing the protective plastic cap from the vial; and
   (3) cleaning the top of the vial with an alcohol wipe; then
using aseptic technique:
   (4) removing the 18-gauge x 1½-inch, 5-micron, filter needle and the 1 mL syringe from their packaging;
   (5) attaching the filter needle to the syringe by twisting it onto the Luer lock syringe tip;
   (6) pushing the filter needle into the center of the vial stopper until the needle is completely inserted into the vial and the tip touches the bottom or a bottom edge of the vial;
   (7) withdrawing all of the VEGF receptor fusion protein vial contents into the syringe, keeping the vial in an upright position, slightly inclined, while ensuring the bevel of the filter needle is submerged into the liquid;
   (8) continuing to tilt the vial during withdrawal keeping the bevel of the filter needle submerged in the formulation;
   (9) drawing the plunger rod sufficiently back when emptying the vial in order to completely empty the filter needle;
   (10) removing the filter needle from the syringe and disposing of the filter needle;
   (11) removing the 30-gauge x ½-inch injection needle from its packaging and attaching the injection needle to the syringe by firmly twisting the injection needle onto the Luer lock syringe tip;
   (12) holding the syringe with the needle pointing up, and checking the syringe for bubbles, wherein if there are bubbles, gently tapping the syringe with a finger until the bubbles rise to the top; and
   (13) slowly depressing the plunger so that the plunger tip aligns with the graduation line that marks 70 microliters on the syringe.

The present invention provides a VEGF receptor fusion protein for use in the treatment and prevention of of neovascular age related macular degeneration (nAMD) in a subject in need thereof, wherein ≥8 mg of a VEGF receptor fusion protein is in an aqueous pharmaceutical formulation comprising about 103-126 mg/ml VEGF receptor fusion protein, histidine-based buffer and arginine.

The present invention provides a VEGF receptor fusion protein for use in the treatment and prevention of of neovascular age related macular degeneration (nAMD) in a subject in need thereof wherein ≥8 mg of a VEGF receptor fusion protein is an aqueous pharmaceutical formulation comprising about 114.3 mg/ml VEGF receptor fusion protein, histidine-based buffer and arginine.

The present invention provides aflibercept for use in the treatment and prevention of neovascular age-related macular degeneration (nAMD) in a subject in need thereof wherein the ≥8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8°C.

The present invention provides a VEGF receptor fusion protein for use in the treatment and prevention of neovascular age-related macular degeneration (nAMD) in a subject in need thereof wherein the ≥8 mg VEGF receptor fusion protein is in an aqueous pharmaceutical formulation comprising:
at least about 100 mg/ml of a VEGF receptor fusion protein;
about 10-100 mM L-arginine;
sucrose;
a histidine-based buffer; and
a surfactant;
wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

The present invention provides a VEGF receptor fusion protein for use in the treatment and prevention of neovascular age-related macular degeneration (nAMD) in a subject in need thereof wherein ≥8 mg of VEGF receptor fusion protein is in an aqueous pharmaceutical formulation comprising
- >100 mg/ml VEGF receptor fusion protein, histidine-based buffer and L-arginine;
- 140 mg/ml aflibercept; 20 mM histidine-based buffer; 5 % sucrose; 0.03 % polysorbate 20; 10 mM L-arginine; pH 5.8;
- 150 + 15 mg/ml aflibercept, 10 mM phosphate-based buffer, 8 + 0.8% (w/v) sucrose, 0.02-0.04% (w/v) polysorbate 20 and 50 mM L-arginine, pH 5.9-6.5;
- 103-126 mg/ml aflibercept, 10 + 1 mM histidine-based buffer, 5 + 0.5% (w/v) sucrose, 0.02-0.04% (w/v) polysorbate 20, and 50 + 5 mM L-arginine, pH 5.5-6.1;
- 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
- 114.3 mg/ml aflibercept, 10 mM histidine-based buffer, 5% (w/v) sucrose, 0.03% (w/v) polysorbate 20 and 50 mM L-arginine, pH 5.8;
- >100 mg/ml aflibercept, histidine-based buffer and L-arginine;
- >100 mg/ml aflibercept at about pH 5.8, wherein the formulation forms <3% HMW aggregates after incubation at 5°C for 2 months;
- About 114.3 mg/mL aflibercept; 10 mM - 50 mM histidine-based buffer, sugar, non-ionic surfactant, L-Arginine, pH 5.8;
- About 114.3 mg/mL aflibercept; 10 mM His/His-HCl-based buffer, 5% sucrose, 0.03% polysorbate-20, 50 mM L-Arginine, pH 5.8; or
- about 114.3 mg/mL aflibercept; arginine monohydrochloride; histidine; histidine hydrochloride, monohydrate; polysorbate 20; sucrose and water for injection.
- Increase in best corrected visual acuity (BCVA)
- Increase in best corrected visual acuity (BCVA) by ≥5, ≥10, ≥15, or ≥20 letters;
- No decrease in best corrected visual acuity (BCVA);
- Elimination of retinal fluid;
- No retinal fluid leakage according to fluorescein angiography
- Reduction in choroidal neovascularization size
- No intraretinal fluid (IRF) and/or subretinal fluid in the center subfield;
- Decrease in total lesion choroidal neovascularization (CNV) area;
- Loss of or decrease in intraretinal fluid;
- Loss of or decrease in subretinal fluid;
- Decrease in central subfield retinal thickness (CST);
- Increase in vision-related quality of life (*e.g.*, as measured by NEI-VFQ-25 total score);
- Lack of treatment-emergent adverse events (AEs) and/or serious AEs (SAEs);
- ETDRS letter score of at least 69 (approximate 20/40 Snellen equivalent);
- Increase in BCVA as measured by the Early Treatment Diabetic Retinopathy Study (ETDRS) visual acuity chart by >5, >10, >15, or >20 letters, or lack of loss thereof during the course of treatment;
- Increase in BCVA as averaged over a period of 12 weeks;
- No intraretinal fluid (IRF) and no subretinal fluid;
- Decrease in choroidal neovascularization (CNV) size;
- Decrease in total lesion CNV area from baseline;
- Loss of IRF and/or SRF;
- Decrease in central subfield retinal thickness (CST);
- Increase in vision-related quality of life as measured by the National Eye Institute Visual Functioning Questionnaire-25 (NEI-VFQ-25);
- Lack of treatment-emergent adverse events (AEs) and/or serious AEs (SAEs);
- Efficacy and/or safety, in a subject suffering from nAMD, similar to that of aflibercept which is intravitreally dosed at 2 mg approximately every 4 weeks for the first 3 months, followed by 2 mg approximately once every 8 weeks or once every 2 months wherein efficacy is measured as increase in BCVA and/or reduction in central retinal thickness, and wherein safety is as measured as the incidence of adverse events such as blood pressure increase, intraocular pressure increase, visual impairment, vitreous floaters, vitreous detachment, iris neovascularization and/or vitreous hemorrhage;
- No detectable anti-drug antibody during receipt of treatment;
- Improvement in best corrected visual acuity (BVCA) by week 4, week 8, week 12, week 16, week 20, week 24, week 28, week 32, week 36, week 40, week 44, week 48, week 52, week 56 or week 60 from start of treatment (baseline);
- Increase in BCVA as measured by the Early Treatment Diabetic Retinopathy Study (ETDRS) visual acuity chart or Snellen equivalent by ≥2 letters, ≥3 letters, ≥4 letters, ≥5 letters, ≥6 letters or >7 letters;
- Improvement in BCVA, by 4 weeks after initiation of treatment, of about 2 or 3 letters (ETDRS or Snellen equivalent) when on HDq12 regimen; or of about 3 letters (ETDRS or Snellen equivalent) when on HDq16 regimen;
- Improvement in BCVA, by 8 weeks after initiation of treatment, of about 5 letters (ETDRS or Snellen equivalent) when on HDq12 regimen; or of about 4 or 5 letters (ETDRS or Snellen equivalent) when on HDq16 regimen;
- Improvement in BCVA, by 12 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
- Improvement in BCVA, by 16 weeks after initiation of treatment, of about 6 letters (ETDRS or Snellen equivalent) when on HDq12 regimen; or of about 6 or 7 letters (ETDRS or Snellen equivalent) when on HDq16 regimen;
- Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
- Improvement in BCVA, by 24 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
- Improvement in BCVA, by 28 weeks after initiation of treatment, of about 7 letters (ETDRS or Snellen equivalent) when on HDq12 regimen; or of about 6 letters (ETDRS or Snellen equivalent) when on HDq16 regimen;
- Improvement in BCVA, by 32 weeks after initiation of treatment, of about 6 or 7 letters (ETDRS or Snellen equivalent) when on HDq12 regimen; or of about 7 letters (ETDRS or Snellen equivalent) when on HDq16 regimen;
- Improvement in BCVA, by 36 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 40 weeks after initiation of treatment, of about 6 or 7 letters (ETDRS or Snellen equivalent) when on HDq12 regimen; or of about 5 or 6 letters (ETDRS or Snellen equivalent) when on HDq16 regimen;
- Improvement in BCVA, by 44 weeks after initiation of treatment, of about 7 letters (ETDRS or Snellen equivalent) when on HDq12 regimen; or of about 5 or 6 letters (ETDRS or Snellen equivalent) when on HDq16 regimen;
- Improvement in BCVA, by 48 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 52 weeks after initiation of treatment, of about 7 or 8 letters (ETDRS or Snellen equivalent) when on HDq12 regimen; or of about 6 or 7 letters (ETDRS or Snellen equivalent) when on HDq16 regimen;
- Improvement in BCVA, by 56 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
- Improvement in BCVA, by 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
- Improvement in BCVA, from about 48 to about 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
- Improvement in BCVA, by 60 weeks after initiation of treatment, of about 5, 10 or 15 letters (ETDRS or Snellen equivalent) when on the HDq12 regimen; or the HDq16 regimen;
- An improvement in BCVA by about week 8, 9, 10, 11 or 12 after initiation of treatment which is maintained (within about +1 or +2 ETDRS letters or Snellen equivalent) thereafter during the treatment regimen;
- A BCVA by 4 weeks after initiation of treatment of about 63 letters (ETDRS or Snellen equivalent) when on the HDq12 regimen; or a BCVA of about 63 letters (ETDRS or Snellen equivalent) when on the HDq16 regimen;
- A BCVA by 8 weeks after initiation of treatment of about 65 letters (ETDRS or Snellen equivalent) when on the HDq12 regimen; or a BCVA of about 65 letters (ETDRS or Snellen equivalent) when on the HDq16 regimen;
- A BCVA by12 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 16 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 20 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 24 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 28 weeks after initiation of treatment of about 67 letters (ETDRS or Snellen equivalent) when on the HDq12 regimen; or a BCVA of about 66letters (ETDRS or Snellen equivalent) when on the HDq16 regimen;
- A BCVA by 32 weeks after initiation of treatment of about 67 letters (ETDRS or Snellen equivalent) when on the HDq12 regimen; or a BCVA of about 67 letters (ETDRS or Snellen equivalent) when on the HDq16 regimen;
- A BCVA by 36 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA by 40 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA by 44 weeks after initiation of treatment of about 68 letters (ETDRS or Snellen equivalent) when on the HDq12 regimen; or a BCVA of about 66 letters (ETDRS or Snellen equivalent) when on the HDq16 regimen;
- A BCVA by 48 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA by 52 weeks after initiation of treatment of about 67 or 68 letters (ETDRS or Snellen equivalent) when on the HDq12 regimen; or a BCVA of about 66 or 67 letters (ETDRS or Snellen equivalent) when on the HDq16 regimen;
- A BCVA by 56 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
- A BCVA by 60 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
- A BCVA between about week 48 and about 60 week after initiation of treatment of about 66 to about 72 letters (ETDRS or Snellen equivalent) when on the HDq12 regimen; or a BCVA of about 66 to about 70 letters (ETDRS or Snellen equivalent) when on the HDq16 regimen;
- Retina without fluid (total fluid, intraretinal fluid [IRF] and/or subretinal fluid [SRF]) in center subfield;
- No subretinal pigment epithelium fluid;
- Lack of fluid leakage on fluorescein angiography (FA);
- Decrease in central retinal thickness (CRT) by at least about 100, 125, 130, 135, 140, 141, 142, 143, 144, 145, 146, 147, 148, 149 or 150 micrometers;
- A change in central retinal thickness, by 4 weeks after initiation of treatment of about - 120 or -121 or -122 or -122.4 or -120.2 micrometers (± about 10, 11 or 12 micrometers) when on the HDq12 regimen; or of about -126 or -127 or-126.6 or -126.3 micrometers (± about 10, 11 or 12 micrometers) when on the HDq16 regimen;
- A change in central retinal thickness, by 8 weeks after initiation of treatment of about - 132, -133, -134, -135 or -136 or -136.2 or -132.8 micrometers (± about 10, 11 or 12 micrometers) when on the HDq12 regimen; or of about -139 or -140 or -139.5 or -139.6 micrometers (± about 10, 11 or 12 micrometers) when on the HDq16 regimen;
- A change in central retinal thickness, by 12 weeks after initiation of treatment of about - 136, -137, -138, -139, -140 or -141 or -140.9 or -136.6 micrometers (± about 10, 11 or 12 micrometers) when on the HDq12 regimen; or of about -144 or -143 or -143.5 micrometers (± about 10, 11 or 12 micrometers) when on the HDq16 regimen
- A change in central retinal thickness, by 16 weeks after initiation of treatment of about - 120 or -121 or -122 or -123 or -124 or -123.4 or -120.1 micrometers (± about 10, 11 or 12 micrometers) when on the HDq12 regimen; or of about -132 or -133 or -132.1 or -133.1 micrometers (± about 10, 11 or 12 micrometers) when on the HDq16 regimen;
- A change in central retinal thickness, by 20 weeks after initiation of treatment of about - 110 or -111 or -112 or -113 or -114 or -113.6 or -110.9 micrometers (± about 10, 11 or 12 micrometers) when on the HDq12 regimen; or of about -115 or -116 or -117 or -118 or -115.8 or -117.7 micrometers (± about 10, 11 or 12 micrometers) when on the HDq16 regimen;
- A change in central retinal thickness, by 24 weeks after initiation of treatment of about - 134, -135, -136 or -137 or -138 or -137.6 or -134.9 micrometers (± about 10, 11 or 12 micrometers) when on the HDq12 regimen; or of about -105 or -106 or -107 or -108 or -105.3 or -107.8 micrometers (± about 10, 11 or 12 micrometers) when on the HDq16 regimen;
- A change in central retinal thickness, by 28 weeks after initiation of treatment of about - 130, -131 or -132 or -133 or -134 or -133.7 or -130.7 micrometers (± about 10, 11 or 12 micrometers) when on the HDq12 regimen; or of about -144 or -145 or -146 or -147 or -148 or - 144.7 or -147.2 micrometers (± about 10, 11 or 12 micrometers) when on the HDq16 regimen;
- A change in central retinal thickness, by 32 weeks after initiation of treatment of about - 118 or -19 or -120 or -121 or -120.4 or -118.1 micrometers (± about 10, 11 or 12 micrometers) when on the HDq12 regimen; or of about -141 or -142 or -143 or -144 or -141.5 or -144 micrometers (± about 10, 11 or 12 micrometers) when on the HDq16 regimen;
- A change in central retinal thickness, by 36 weeks after initiation of treatment of about - 142 or -143 or -144 or -144.2 or -142.2 micrometers (± about 10, 11 or 12 micrometers) when on the HDq12 regimen; or of about -126 or -127, -128 or -129 or -130 or -131 or -126.4 or - 130.5 micrometers (± about 10, 11 or 12 micrometers) when on the HDq16 regimen;
- A change in central retinal thickness, by 40 weeks after initiation of treatment of about - 131, -132, -133 or -134 or -133.8 or -131.2 micrometers (± about 10, 11 or 12 micrometers) when on the HDq12 regimen; or of about -127, -128 or -127.5 micrometers (± about 10, 11 or 12 micrometers) when on the HDq16 regimen;
- A change in central retinal thickness, by 44 weeks after initiation of treatment of about - 120 or -121 or -122 or -123 or -124 or -125 or -124.7 or -120.3 micrometers (± about 10, 11 or 12 micrometers) when on the HDq12 regimen; or of about -143, -144 or -145 or -144.8 micrometers (± about 10, 11 or 12 micrometers) when on the HDq16 regimen;
- A change in central retinal thickness, by 48 weeks after initiation of treatment of about - 142 or -143 or -144 or -144.4 or -142.3 micrometers (± about 10, 11 or 12 micrometers) when on the HDq12 regimen; or of about -143 or -144 or -145 or -146 or -147 or -148 or -143.8 or - 147.1 micrometers (± about 10, 11 or 12 micrometers) when on the HDq16 regimen;
- A change in central retinal thickness, by 52 weeks after initiation of treatment of about - 143.2 micrometers (± about 10, 11 or 12 micrometers) when on the HDq12 regimen; or of about -139.6 micrometers (± about 10, 11 or 12 micrometers) when on the HDq16 regimen;
- A change in central retinal thickness, by 56 weeks after initiation of treatment of about - 136.3 micrometers (± about 10, 11 or 12 micrometers) when on the HDq12 regimen; or of about -137.5 micrometers (± about 10, 11 or 12 micrometers) when on the HDq16 regimen;
- A change in central retinal thickness, by 60 weeks after initiation of treatment of about - 151.8 micrometers (± about 10, 11 or 12 micrometers) when on the HDq12 regimen; or of about -148.8 micrometers (± about 10, 11 or 12 micrometers) when on the HDq16 regimen;
- A central retinal thickness by week 4 after initiation of treatment of about 248.2 micrometers when on the HDq12 regimen; or of about 244.1 micrometers when on the HDq16 regimen;
- A central retinal thickness by week 8 after initiation of treatment of about 234.4 micrometers when on the HDq12 regimen; or of about 231.2 micrometers when on the HDq16 regimen;
- A central retinal thickness by week 12 after initiation of treatment of about 229.7 micrometers when on the HDq12 regimen; or of about 226.7 micrometers when on the HDq16 regimen;
- A central retinal thickness by week 16 after initiation of treatment of about 247.2 micrometers when on the HDq12 regimen; or of about 238.6 micrometers when on the HDq16 regimen;
- A central retinal thickness by week 20 after initiation of treatment of about 257 micrometers when on the HDq12 regimen; or of about 254.9 micrometers when on the HDq16 regimen;
- A central retinal thickness by week 24 after initiation of treatment of about 233 micrometers when on the HDq12 regimen; or of about 265.4 micrometers when on the HDq16 regimen;
- A central retinal thickness by week 28 after initiation of treatment of about 236.9 micrometers when on the HDq12 regimen; or of about 226 micrometers when on the HDq16 regimen;
- A central retinal thickness by week 32 after initiation of treatment of about 250.2 micrometers when on the HDq12 regimen; or of about 229.2 micrometers when on the HDq16 regimen;
- A central retinal thickness by week 36 after initiation of treatment of about 226.4 micrometers when on the HDq12 regimen; or of about 244.3 micrometers when on the HDq16 regimen;
- A central retinal thickness by week 40 after initiation of treatment of about 236.8 micrometers when on the HDq12 regimen; or of about 243.7 micrometers when on the HDq16 regimen;
- A central retinal thickness by week 44 after initiation of treatment of about 245.9 micrometers when on the HDq12 regimen; or of about 227.7 micrometers when on the HDq16 regimen;
- A central retinal thickness by week 48 after initiation of treatment of about 226.2 micrometers when on the HDq12 regimen; or of about 226.9 micrometers when on the HDq16 regimen;
- A central retinal thickness by week 52 after initiation of treatment of about 227.4 micrometers when on the HDq12 regimen; or of about 231.1 micrometers when on the HDq16 regimen;
- A central retinal thickness by week 56 after initiation of treatment of about 234.3 micrometers when on the HDq12 regimen; or of about 233.2 micrometers when on the HDq16 regimen;
- A central retinal thickness by week 60 after initiation of treatment of about 218.8 micrometers when on the HDq12 regimen; or of about 221.9 micrometers when on the HDq16 regimen;
- A CRT by about week 4, 5, 6, 7 or 8 after initiation of treatment or a reduction in CRT by week 4, 5, 6, 7 or 8 after initiation of treatment which is maintained (within about ±10, ±11 or ±12 micrometers) thereafter during the treatment regimen;
- At about 4 hours after treatment initiation of HDq12 or HDq16, a free aflibercept concentration in plasma of about 0.0409 (±0.0605) or 0 mg/L (or <0.0156 mg/L);
- At about 8 hours after treatment initiation of HDq12 or HDq16, a free aflibercept concentration in plasma of about 0.05 (±3.78), 0.0973 (±0.102) or 0.0672 mg/L;
- At about day 2 after treatment initiation of HDq12 or HDq16, a free aflibercept concentration in plasma of about 0.11 (±2.21), 0.146 (±0.110) or 0.0903 mg/L;
- At about day 3 after treatment initiation of HDq12 or HDq16, a free aflibercept concentration in plasma of about 0.11 (±2.06), 0.137 (±0.0947) or 0.112 mg/L;
- At about day 5 after treatment initiation of HDq12 or HDq16, a free aflibercept concentration in plasma of about 0.08 (±1.86), 0.0933 (±0.0481) or 0.0854 mg/L;
- At about day 8 after treatment initiation of HDq12 or HDq16, a free aflibercept concentration in plasma of about 0.07 (±1.75), 0.0794 (±0.0413) or 0.0682 mg/L ;
- At about day 15 after treatment initiation of HDq12 or HDq16, a free aflibercept concentration in plasma of about 0.04 (±1.76), 0.0435 (±0.0199) or 0.0385 mg/L ;
- At about day 22 after treatment initiation of HDq12 or HDq16, a free aflibercept concentration in plasma of about 0.02 (±1.76), 0.0213 (±0.0148) or 0.0232 mg/L;
- At about day 29 after treatment initiation of HDq12 or HDq16, a free aflibercept concentration in plasma of about 0.00766 (±0.00958) or 0 mg/L (or <0.0156 mg/L);
- At about 4 hours post-dose after treatment initiation of HDq12 or HDq16, an adjusted bound aflibercept concentration in plasma of about 0.00 mg/L;
- At about 8 hours post-dose after treatment initiation of HDq12 or HDq16, an adjusted bound aflibercept concentration in plasma of about 0.00 mg/L;
- At about day 2 after treatment initiation of HDq12 or HDq16, an adjusted bound aflibercept concentration in plasma of about 0.06 (±3.50) or 0.124 (±0.186) mg/L;
- At about day 3 after treatment initiation of HDq12 or HDq16, an adjusted bound aflibercept concentration in plasma of about 0.13 (±2.07) or 0.173 (±0.155) mg/L;
- At about day 5 after treatment initiation of HDq12 or HDq16, an adjusted bound aflibercept concentration in plasma of about 0.18 (±1.88) or 0.223 (±0.157) mg/L;
- At about day 8 after treatment initiation of HDq12 or HDq16, an adjusted bound aflibercept concentration in plasma of about 0.31 (±1.56) or 0.334 (±0.135) mg/L;
- At about day 15 after treatment initiation of HDq12 or HDq16, an adjusted bound aflibercept concentration in plasma of about 0.37 (±1.50) or 0.393 (±0.130) mg/L;
- At about day 22 after treatment initiation of HDq12 or HDq16, an adjusted bound aflibercept concentration in plasma of about 0.25 (±3.00) or 0.335 (±0.155) mg/L;
- At about day 29 after treatment initiation of HDq12 or HDq16, an adjusted bound aflibercept concentration in plasma of about 0.32 (±1.39) or 0.331 (±0.0953) mg/L;
- Non-inferior BVCA compared to that of aflibercept which is intravitreally dosed at 2 mg approximately every 4 weeks for the first 5 injections followed by 2 mg approximately once every 8 weeks or once every 2 months;
- Ocular and non-ocular safety or death rate, in a subject suffering from DME, similar to that of aflibercept which is intravitreally dosed at 2 mg approximately every 4 weeks for the first 3 or 4 or 5 injections followed by 2 mg approximately once every 8 weeks or once every 2 months;
- An improvement in best corrected visual acuity by 4, 8, 12, 16, 20, 24, 28, 32, 36, 40, 44 or 48 weeks from start of treatment;
- An increase in best corrected visual acuity, as measured by Early Treatment Diabetic Retinopathy Study (ETDRS) visual acuity chart or Snellen equivalent of ≥ 2 letters, ≥ 3 letters, ≥ 4 letters, ≥ 5 letters, ≥ 6 letters or > 7 letters by 4, 8, 12, 16, 20, 24, 28, 32, 36, 40, 44, 48, 52, 56 or 60 weeks from start of treatment;
- A retina without fluid (total fluid, intraretinal fluid [IRF] and/or subretinal fluid [SRF]) in center subfield by 4, 8, 12, 16, 20, 24, 28, 32, 36, 40, 44, 48, 52, 56 or 60 weeks from start of treatment; and/or
- a decrease in central retinal thickness (CRT) of at least 100, 125, 130, 135, 140, 141, 142, 143, 144, 145, 146, 147, 148, 149 or 150 micrometers by 4, 8, 12, 16, 20, 24, 28, 32, 36, 40, 44, 48, 52, 56 or 60 weeks from start of treatment.

The present invention provides a VEGF receptor fusion protein for use in the treatment and prevention of neovascular age-related macular degeneration (nAMD) in a subject in need thereof wherein
- 1 initial dose, 2 secondary doses and 3 tertiary doses of said ≥8 mg VEGF receptor fusion protein are administered to the subject in the first year;
- wherein 1 initial dose, 2 secondary doses and 2 tertiary doses of said ≥8 mg VEGF receptor fusion protein are administered to the subject in the first year; or
- wherein 1 initial dose, 2 secondary doses and 3 tertiary doses of said ≥8 mg VEGF receptor fusion protein are administered to the subject in the first year followed by 2 -4 tertiary doses in the second year.

The present invention provides a VEGF receptor fusion protein for use in the treatment and prevention of neovascular age-related macular degeneration (nAMD) in a subject in need thereof wherein the interval between doses of ≥8 mg VEGF receptor fusion protein is adjusted (increased/maintained/reduced) based on visual and/or anatomic outcomes.

The present invention provides a VEGF receptor fusion protein for use in the treatment and prevention of neovascular age-related macular degeneration (nAMD) in a subject in need thereof wherein the doses of ≥8 mg VEGF receptor fusion protein are administered according to pro re nata (PRN), capped PRN or treat and extend (T&E) dosing regimen.

The present invention also provides a kit comprising i) a container comprising a VEGF receptor fusion protein, preferably aflibercept and ii) instruction for use of the VEGF fusion protein. In an embodiment of the invention, the container is a vial or a pre-filled syringe. The vial a type I glass vial containing a nominal fill volume of abut 0.26 mL solution for intravitreal injection. In an embodiment of the invention the container comprises the VEGF receptor fusion protein at a concentration of more or equal to 100 mg/mL or the container comprises aflibercept at a concentration of about 114.3 mg/mL. In an embodiment of the invention, the instruction for use comprising instruction for use of the VEGF fusion protein or aflibercept for the treatment of DME and /or AMD. In an embodiment of the invention, the instruction for use comprises the information that i) the container comprises ≥8 mg (114.3 mg/mL) aflibercept solution for intravitreal injection, ii) each single-dose vial provides a usable amount to deliver a single dose of 70 microliters containing 8 mg aflibercept to adult patients, iii) the recommended dose is ≥8 mg aflibercept (equivalent to 70 microliters solution for injection), iv) ≥8 mg aflibercept treatment is initiated with 1 injection per month (every 4 weeks) for 3 consecutive doses, v) injection intervals may then be extended up to every 16 weeks or 20 weeks vi) the treatment interval may be adjusted based on the physician's judgement of visual and/or anatomic outcomes and /or vii) that ≥8 mg aflibercept / 0.07 mL is provided as a sterile, aqueous solution containing arginine monohydrochloride; histidine; histidine hydrochloride, monohydrate; polysorbate 20; sucrose and water for injection.

### BRIEF DESCRIPTION OF THE FIGURES

**Figure 1**: Summary of PULSAR clinical trial.
**Figure 2**: Key eligibility criteria (inclusion criteria and exclusion criteria) of PULSAR clinical trial.
**Figure 3**: Dosing schedule and dose regimen modification (DRM) criteria for PULSAR clinical trial.
**Figure 4**: Criteria for dose regimen modifications (DRMs) of PULSAR clinical trial.
**Figure 5**: Patient disposition at week 48 in PULSAR clinical trial.
**Figure 6**: Baseline demographics of subjects in PULSAR clinical trial.
**Figure 7**: Baseline characteristics of the study eye of subjects in PULSAR clinical trial.
**Figure 8**: Mean number of injections through week 48 in PULSAR clinical trial.
**Figure 9*******: Mean change in Best Corrected Visual Acuity (BCVA) through week 48 in PULSAR clinical trial. Least squares mean change from baseline at week 48 shown (see data below).

| **Week** | **2q8** | **HDq12** | **HDq16** |
|---|---|---|---|
| 0 | 0.0 | 0.0 | 0.0 |
| 4 | 4.6 | 2.7 | 3.2 |
| 8 | 6.3 | 5 | 4.8 |
| 12 | 6.6 | 5.5 | 5.8 |
| 16 | 6.7 | 6 | 6.5 |
| 20 | 7.7 | 6 | 6.3 |
| 24 | 7.4 | 5.9 | 5.8 |
| 28 | 8.1 | 7.2 | 6.3 |
| 32 | 7.6 | 6.8 | 7.3 |
| 36 | 8.2 | 6.7 | 6.4 |
| 40 | 7.8 | 6.9 | 5.7 |
| 44 | 8 | 7.2 | 5.9 |
| 48 | 7.6 | 6.7 | 6.2 |

| | | | |
|---|---|---|---|
| *the present invention includes methods for achieving approximately the indicated improvement in BVCA by the indicated timepoint when receiving the indicated treatment regimen for treating wetAMD. | | | |

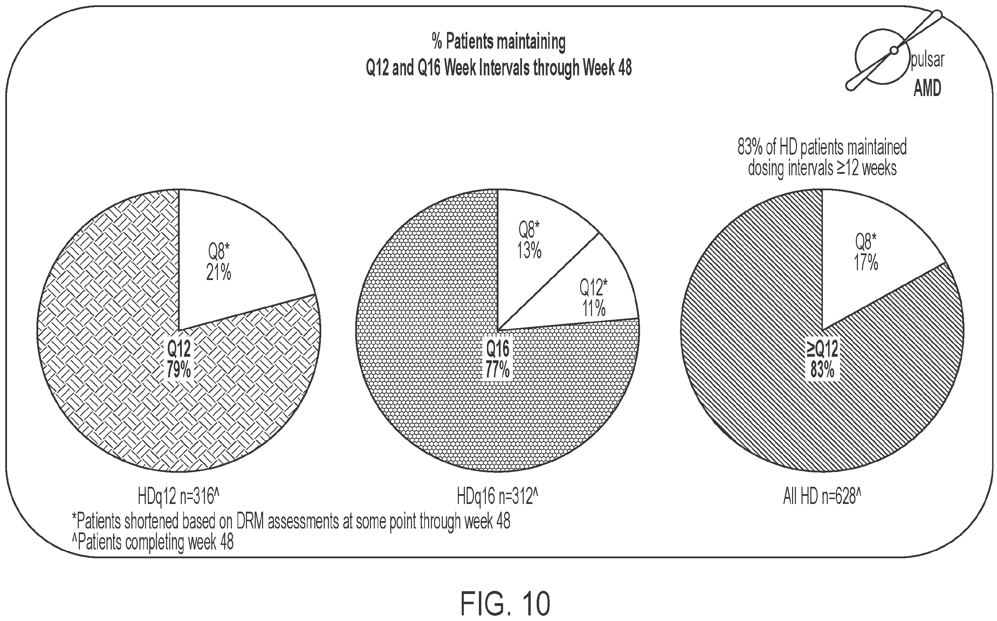

**Figure 10**: Percentage of subjects maintaining Q12 and Q16 week intervals through week 48 in PULSAR clinical trial.

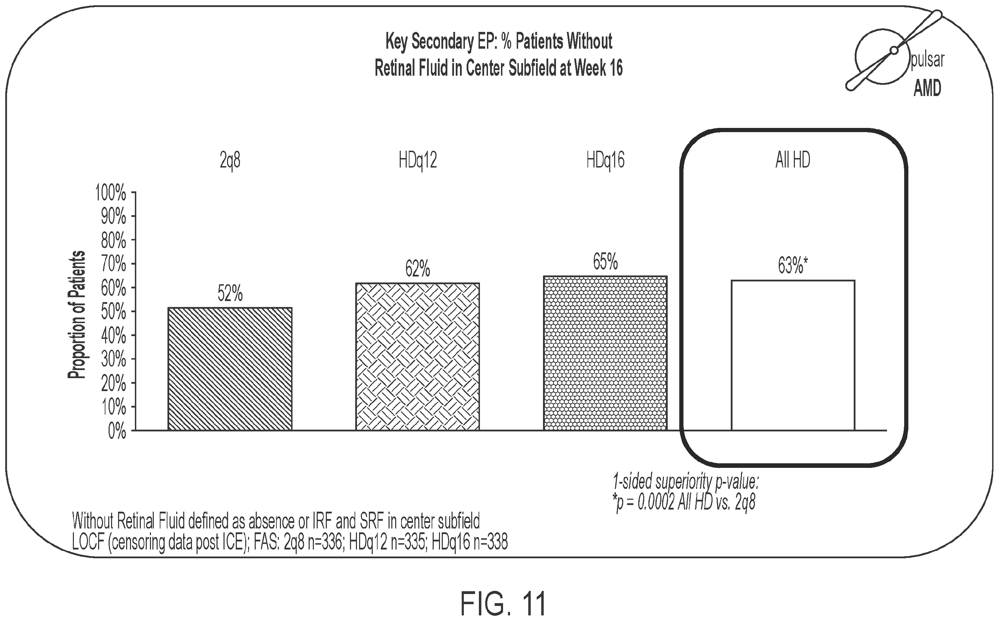

**Figure 11**: Key secondary endpoint of percentage of subjects without retinal fluid in center subfield at week 16 in PULSAR clinical trial.

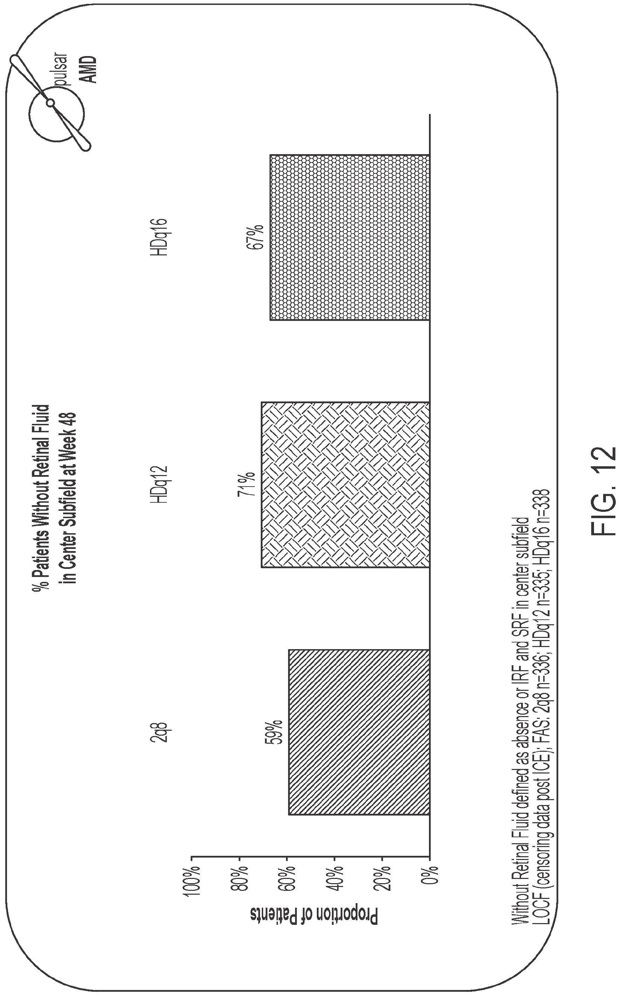

**Figure 12**: Percentage of subjects without retinal fluid in center subfield at week 48 in PULSAR clinical trial.

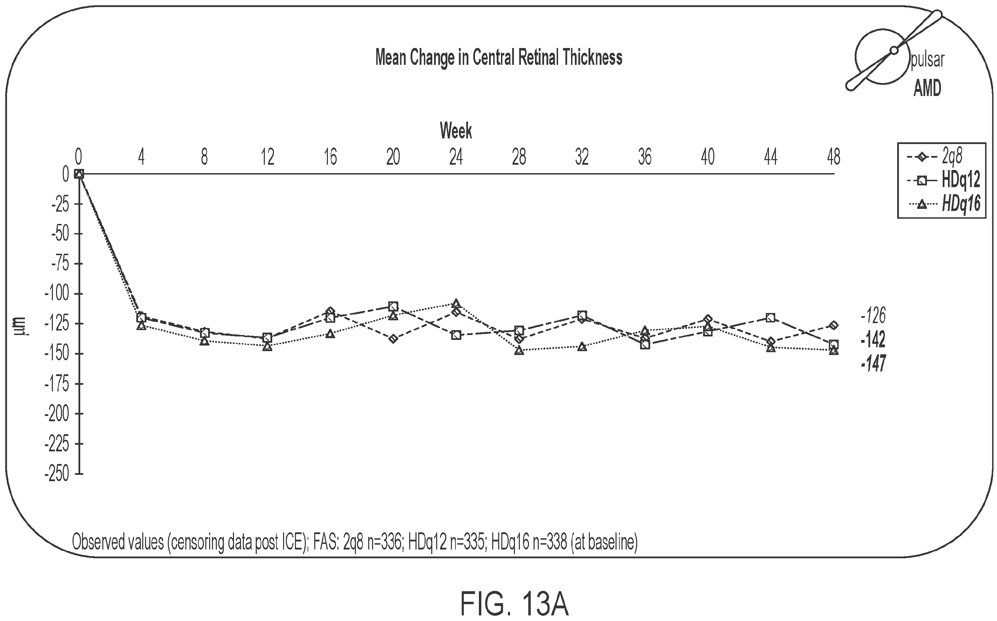

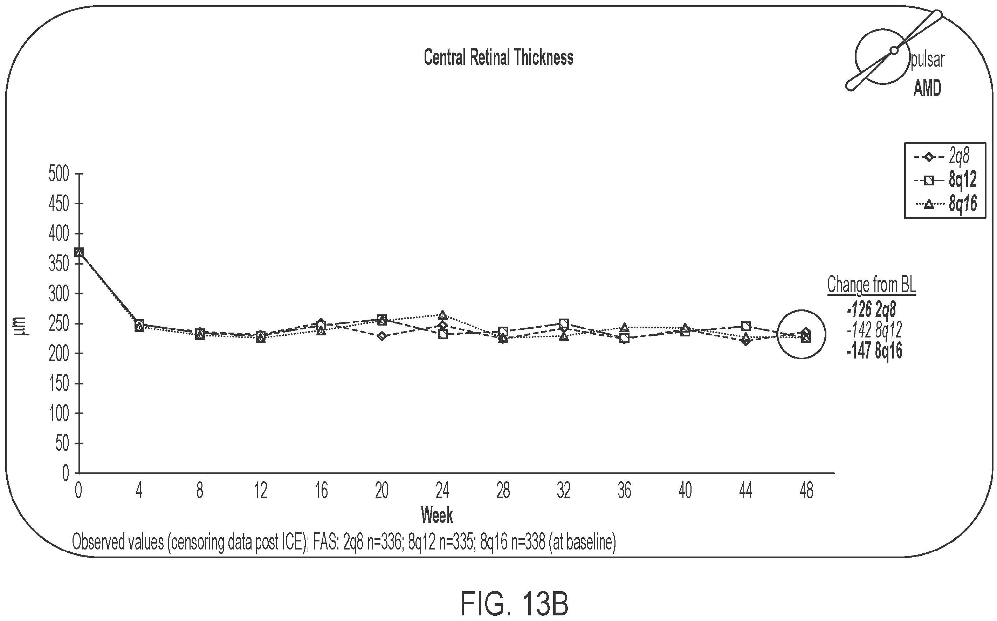

**Figure 13A** **and** **13B**: Mean change from baseline in central retinal thickness through week 48 (Fig 13A)

| **Week** | **2q8** | **HDq12** | **HDq16** |
|---|---|---|---|
| 0 | 0 | 0 | 0.0 |
| 4 | -119.1 | -120.2 | -126.3 |
| 8 | -131.8 | -132.8 | -139.6 |
| 12 | -137.2 | -136.6 | -143.5 |
| 16 | -114.7 | -120.1 | -133.1 |
| 20 | -137.8 | -110.9 | -117.7 |
| 24 | -115.5 | -134.9 | -107.8 |
| 28 | -138.2 | -130.7 | -147.2 |
| 32 | -121.1 | -118.1 | -144 |
| 36 | -137.5 | -142.2 | -130.5 |
| 40 | -121.4 | -131.2 | -127.5 |
| 44 | -140 | -120.3 | -144.8 |
| 48 | -126.3 | -142.3 | -147.1 |

| | | | |
|---|---|---|---|
| *the present invention includes methods for achieving approximately the indicated reduction in central retinal thickness by the indicated timepoint when receiving the indicated treatment regimen for treating wetAMD. ; and Central retinal thickness through week 48 (Fig. 13B), in PULSAR clinical trial (see data below). | | | |

| **Week** | **2q8** | **HDq12** | **HDq16** |
|---|---|---|---|
| 0 | 367.1 | 370.6 | 370.7 |
| 4 | 246.6 | 248.2 | 244.1 |
| 8 | 235.7 | 234.4 | 231.2 |
| 12 | 231 | 229.7 | 226.7 |
| 16 | 252 | 247.2 | 238.6 |
| 20 | 228.7 | 257 | 254.9 |
| 24 | 247.3 | 233 | 265.4 |
| 28 | 224.6 | 236.9 | 226 |
| 32 | 242.5 | 250.2 | 229.2 |
| 36 | 224.1 | 226.4 | 244.3 |
| 40 | 240.3 | 236.8 | 243.7 |
| 44 | 220.9 | 245.9 | 227.7 |
| 48 | 236.3 | 226.2 | 226.9 |

| | | | |
|---|---|---|---|
| *the present invention includes methods for achieving approximately the indicated central retinal thickness by the indicated timepoint when receiving the indicated treatment regimen for treating wetAMD. | | | |

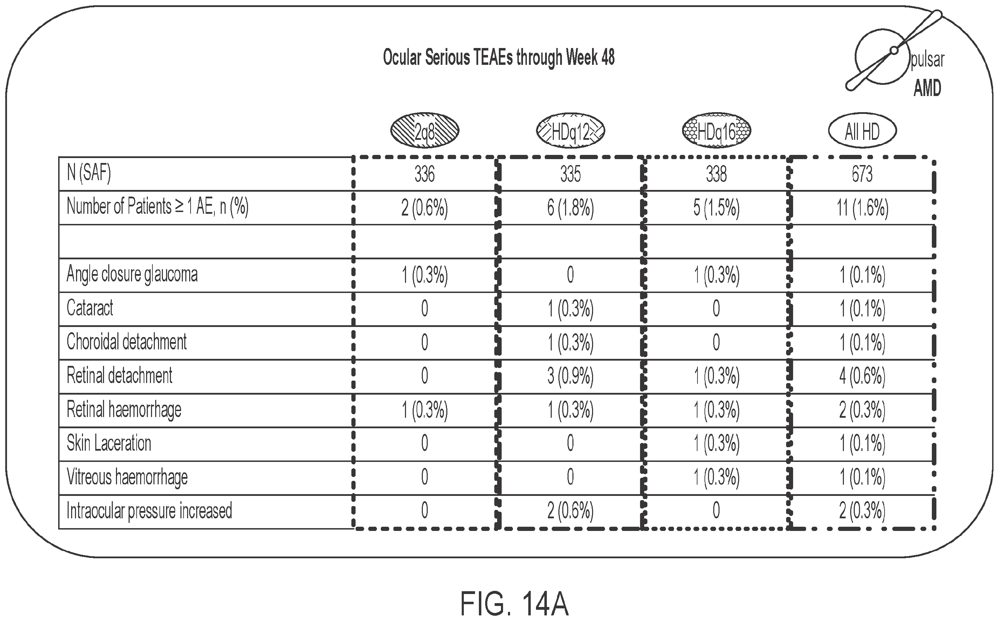

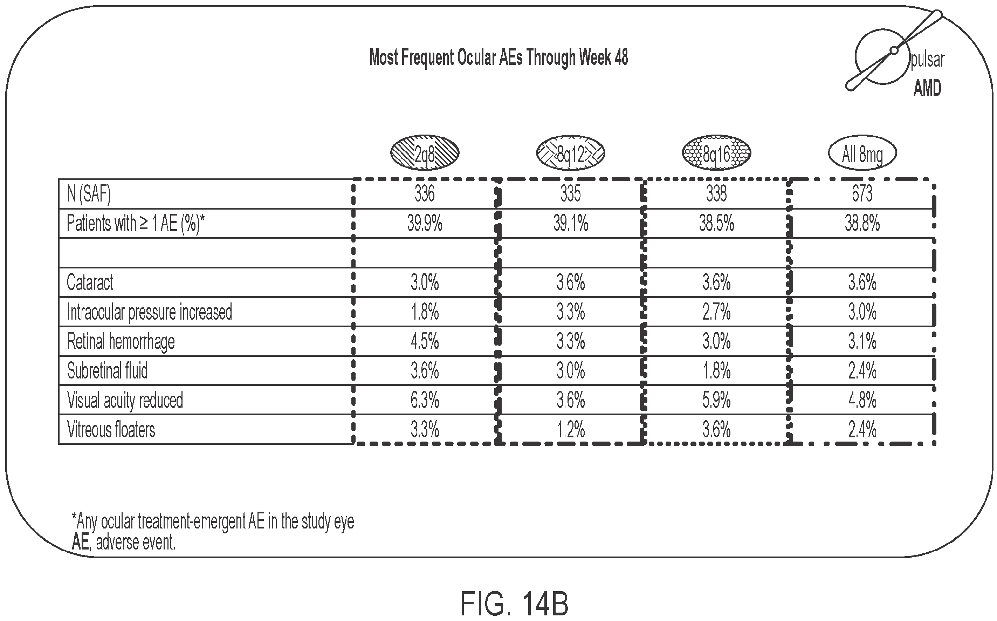

**Figures 14A** **and** **14B**: Ocular serious Treatment Emergent Adverse Events (TEAEs) through week 48 (Fig. 14A); Most Frequent Ocular Adverse Events (AEs) through week 48 (Fig. 14B) in PULSAR clinical trial.

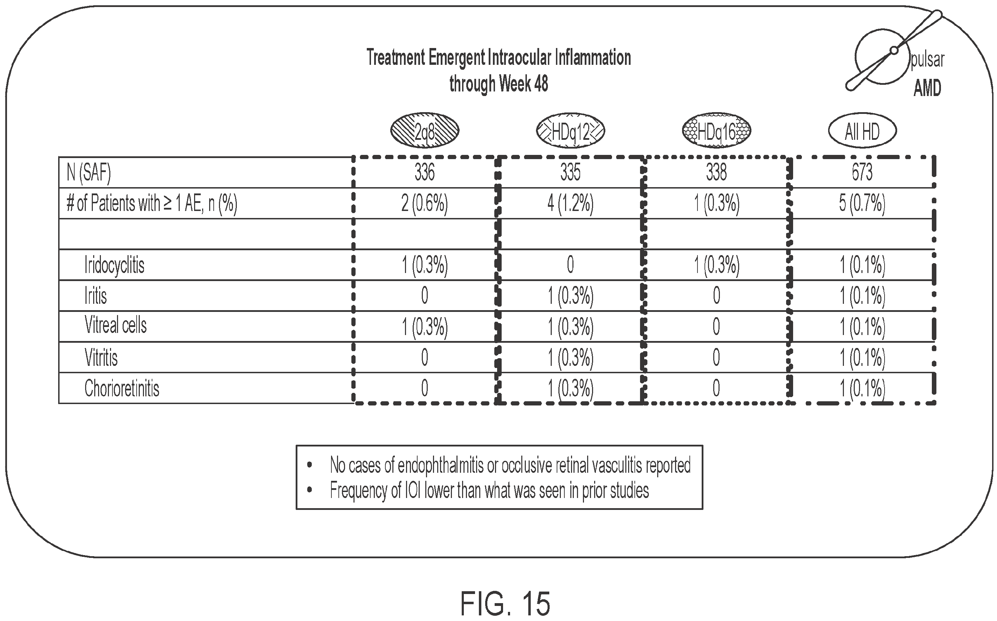

**Figure 15**: Treatment emergent intraocular inflammation through week 48 in PULSAR clinical trial.

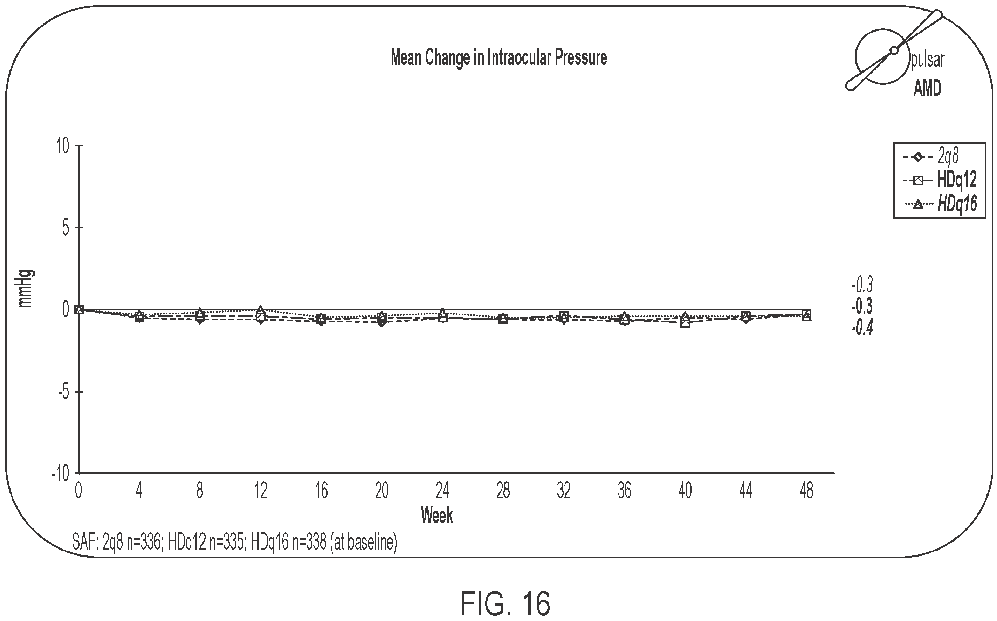

**Figure 16**: Mean change from baseline in intraocular pressure through week 48 in PULSAR clinical trial.

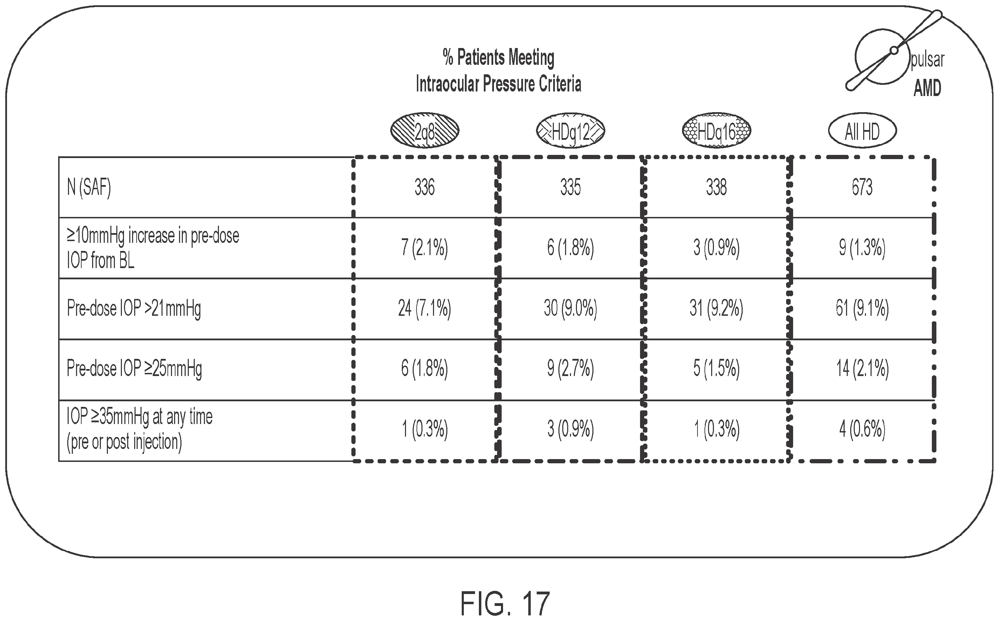

**Figure 17**: Percentage of subjects meeting intraocular pressure criteria in PULSAR clinical trial.

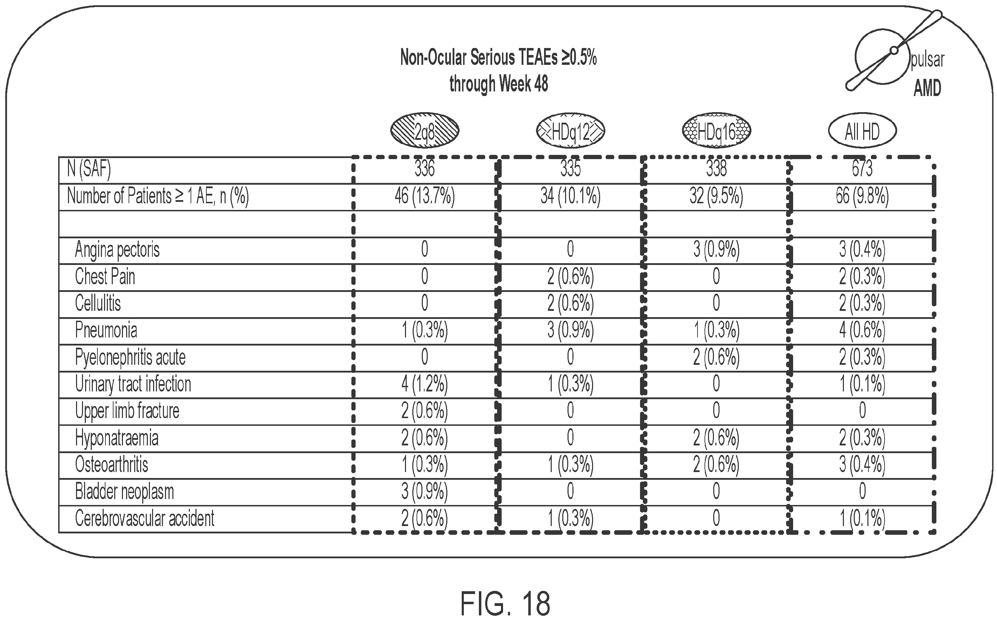

**Figure 18**: Non-Ocular Serious TEAEs ≥0.5% through week 48 in PULSAR clinical trial.

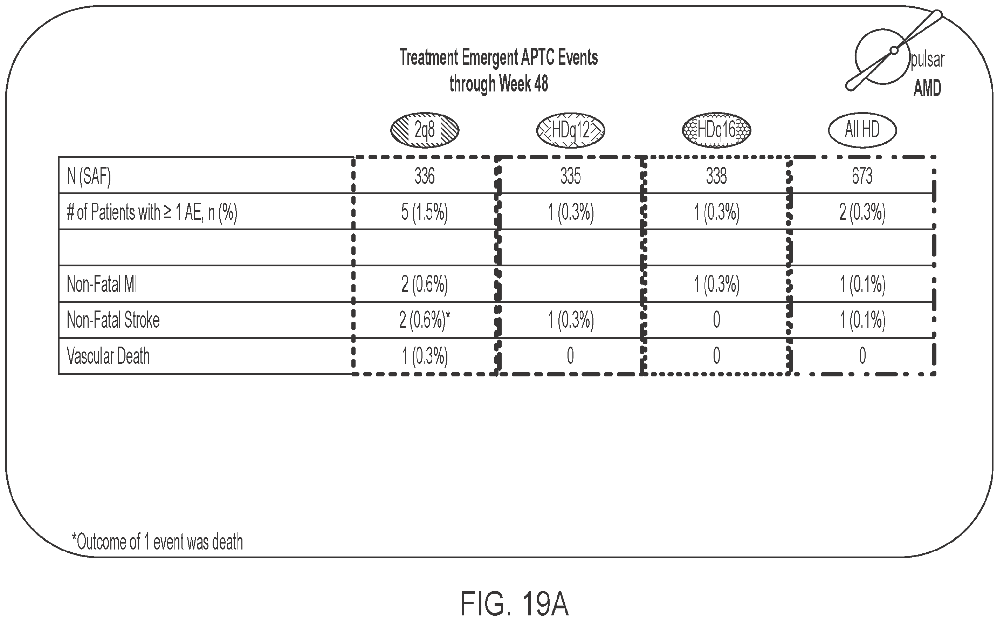

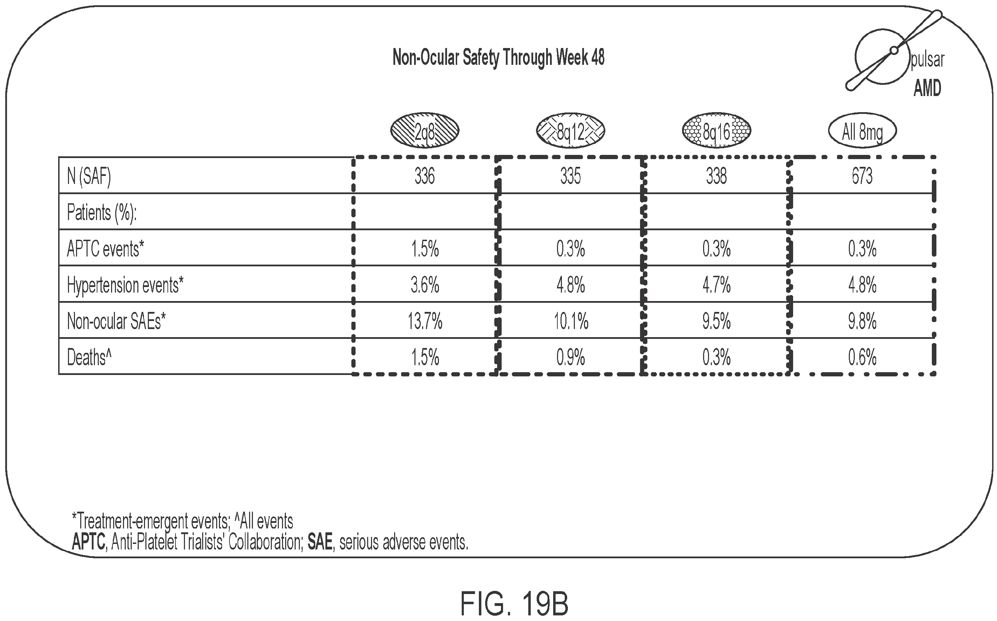

**Figures 19A** **and** **19B**: Treatment emergent Anti-Platelet Trialists' Collaboration (APTC) events through week 48 (Fig. 19A); Non-Ocular Safety through week 48 (Fig. 19B) in PULSAR clinical trial.

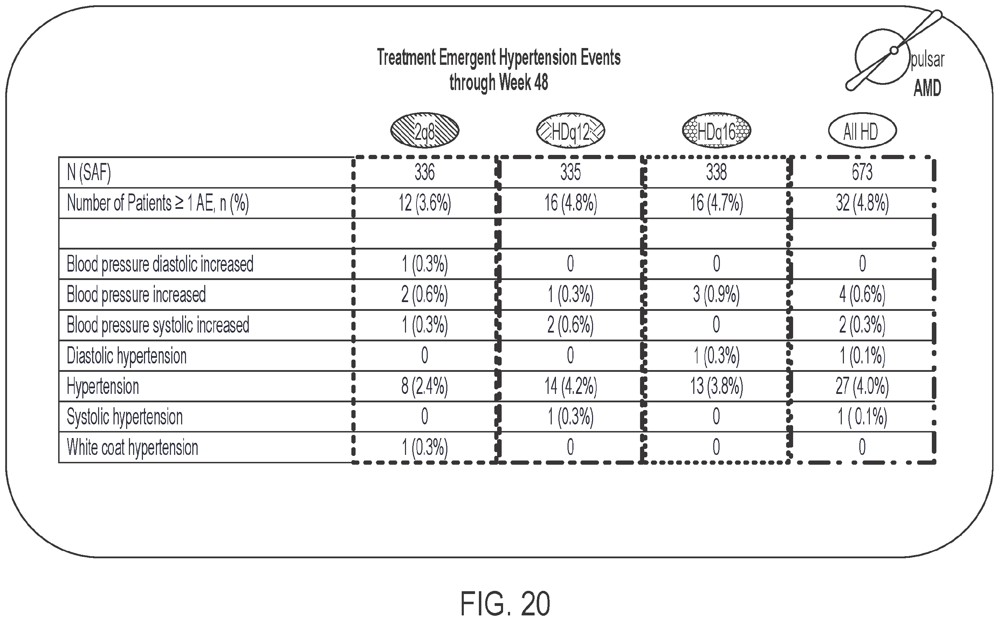

**Figure 20**: Treatment emergent hypertension events though week 48 in PULSAR clinical trial.

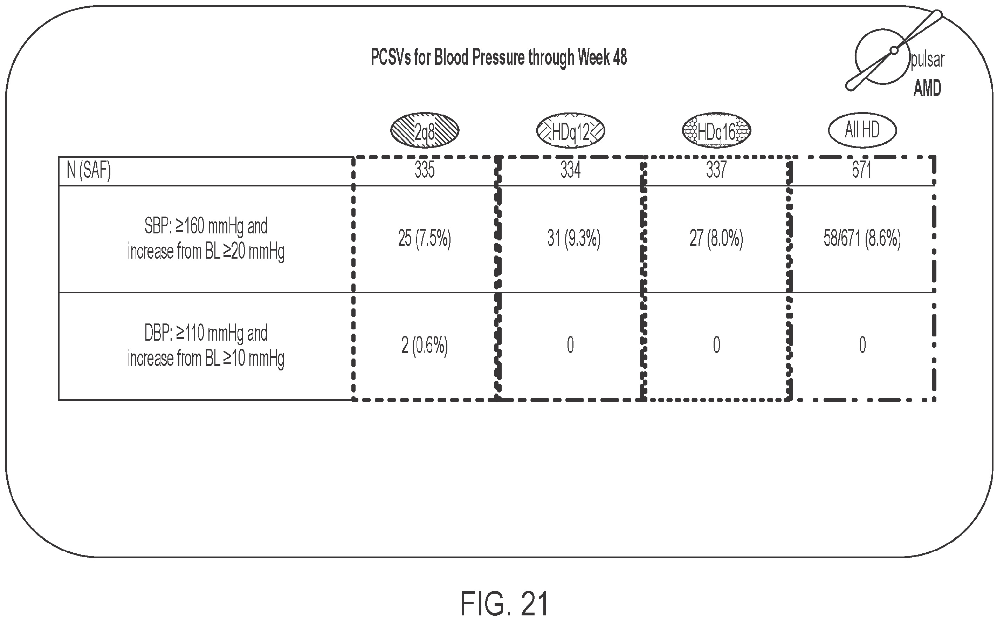

**Figure 21**: Potentially Clinically Significant Values (PCSVs) for blood pressure through week 48 in PULSAR clinical trial.

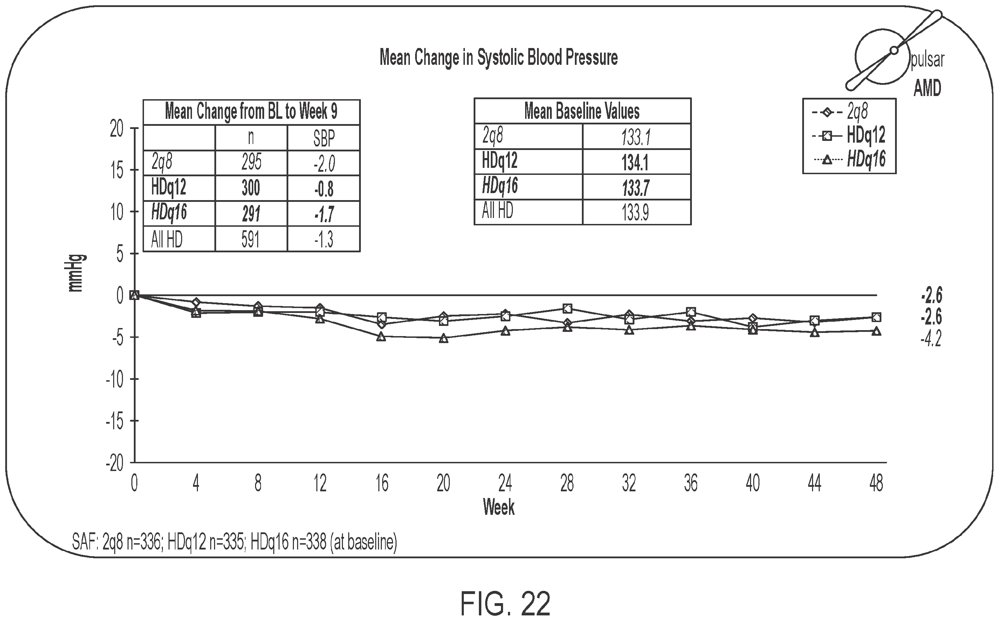

**Figure 22**: Mean change from baseline in systolic blood pressure through week 48 in PULSAR clinical trial. Mean change from baseline to week 9 and mean baseline pressure shown in insets.

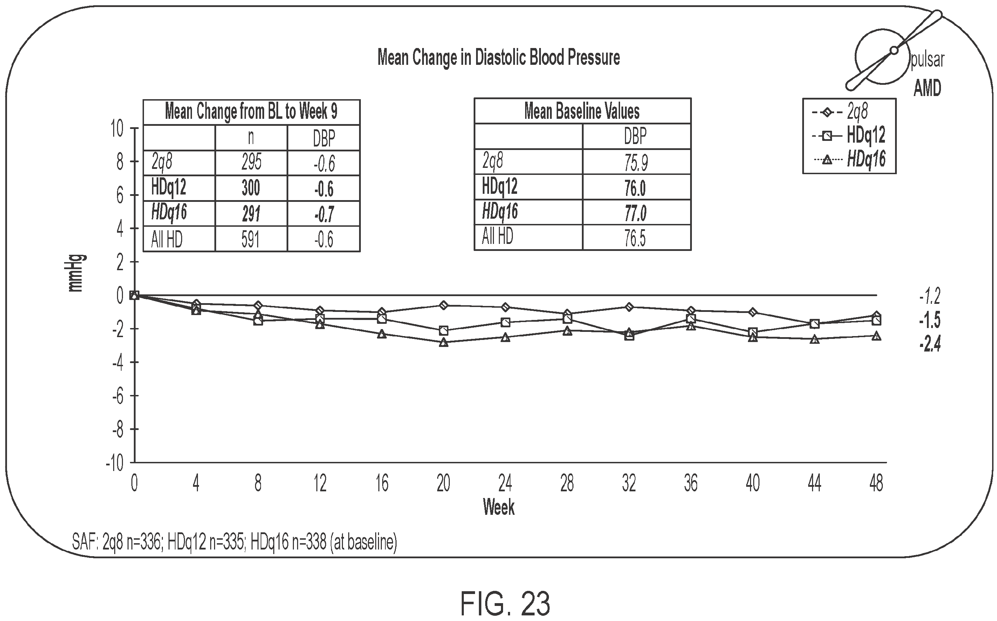

**Figure 23**: Mean change from baseline in diastolic blood pressure through week 48 in PULSAR clinical trial. Mean change from baseline to week 9 and mean baseline pressure shown in insets.

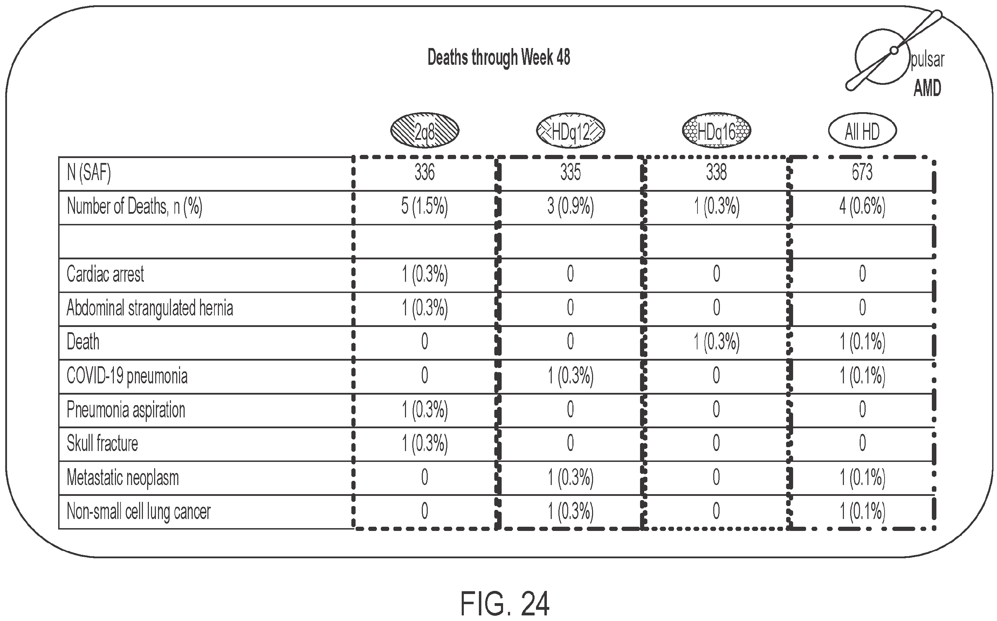

**Figure 24**: Deaths through week 48 in PULSAR clinical trial.

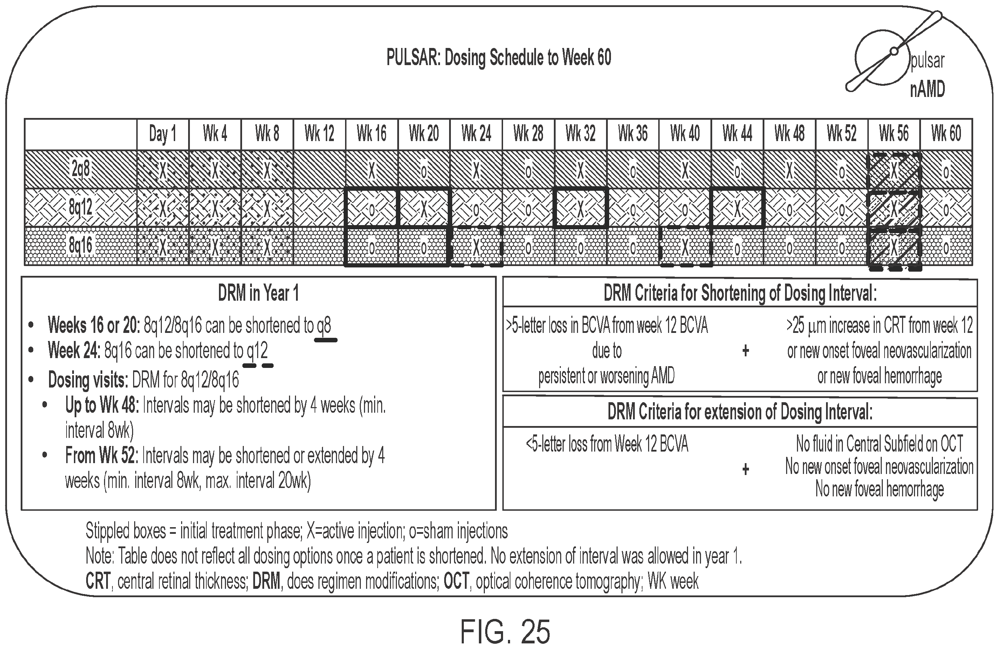

**Figure 25**: PULSAR dosing schedule out to week 60. Dose regimen modification criteria are set forth in the inset.

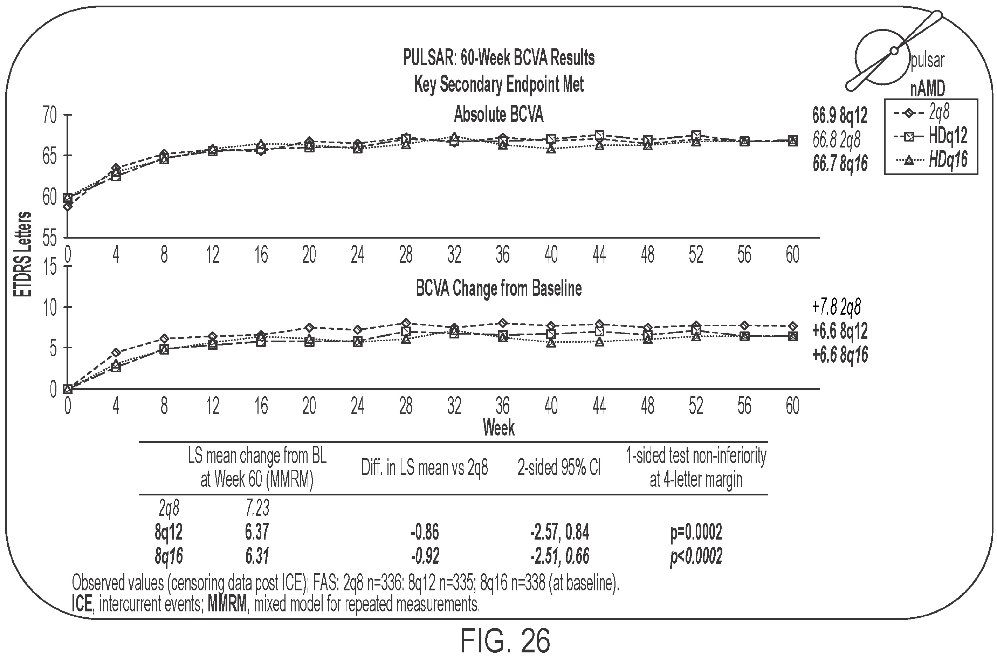

**Figure 26**: Absolute BCVA and change in BCVA from baseline (ETDRS letters) out to week 60. Least squares mean change from baseline at week 60 shown.

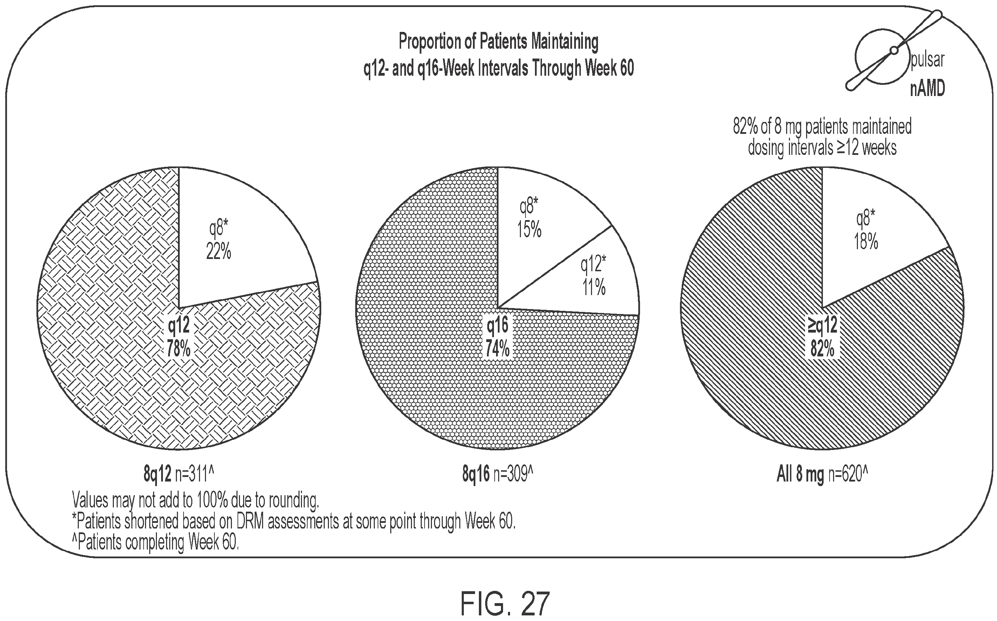

**Figure 27**: Proportion of Patients Maintaining HDq12 (8q12)- and HDq16 (8q16) Intervals Through Week 60.

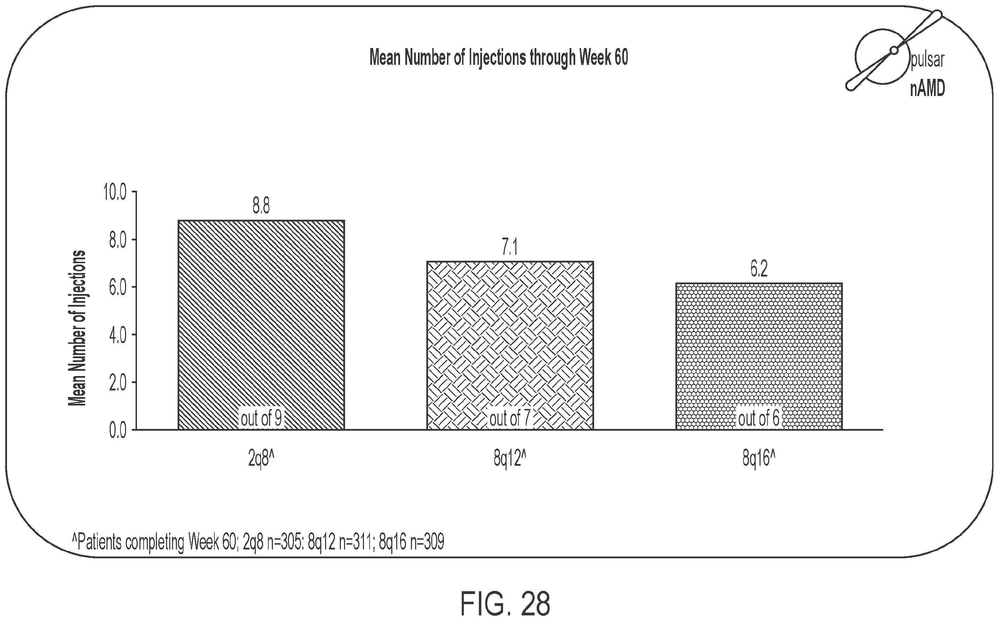

**Figure 28**: Mean Number of Injections through Week 60 in each group.

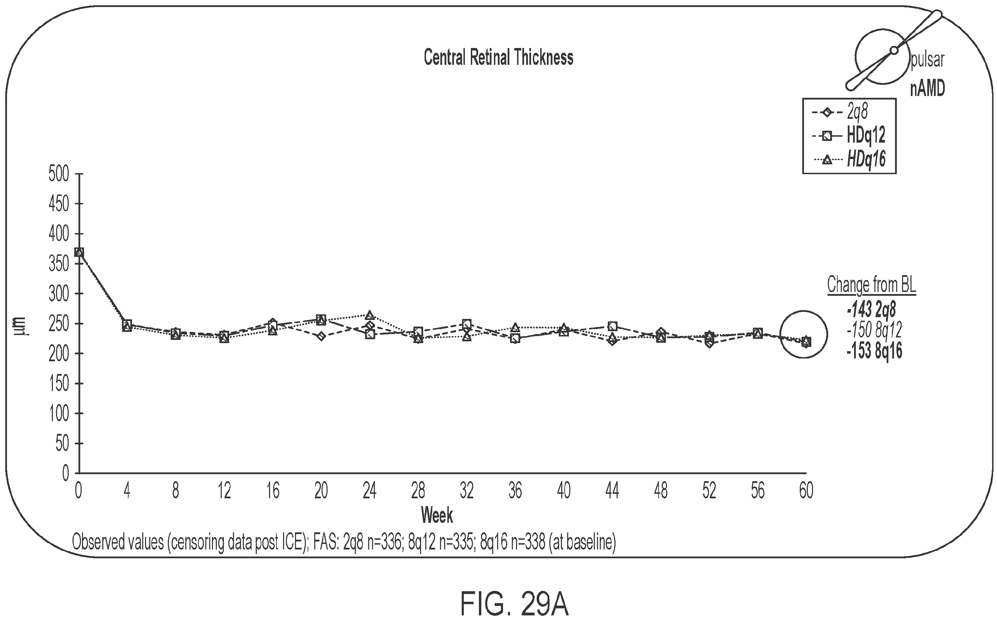

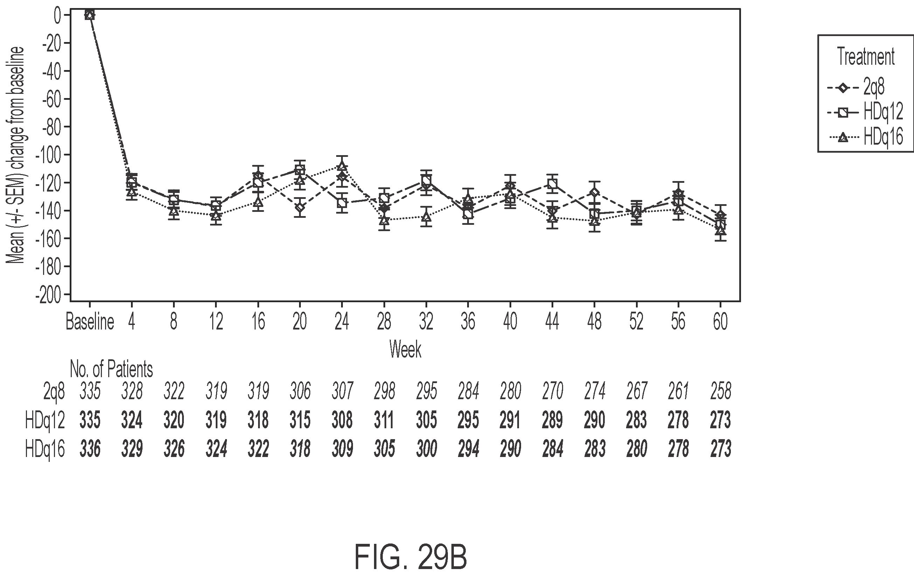

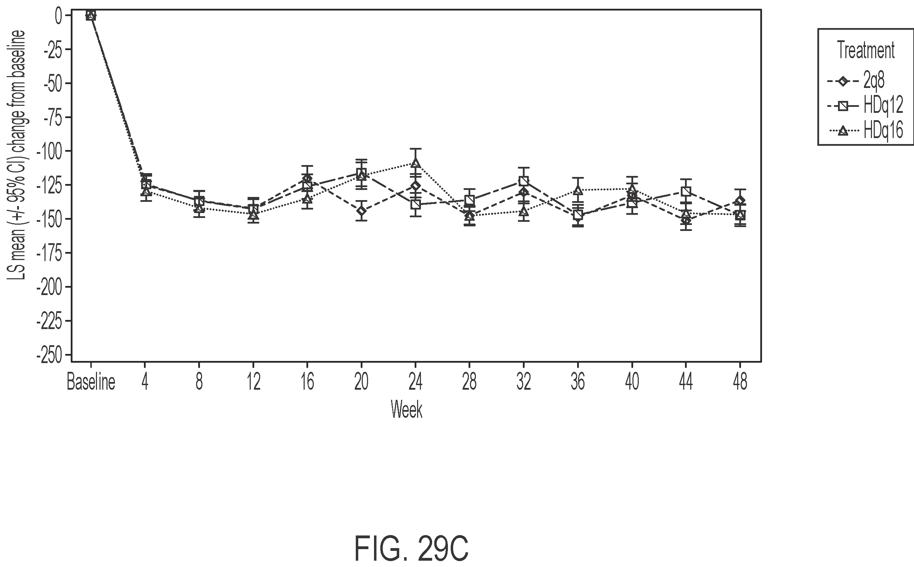

**Figure 29A****,** **29B** **and** **29C**: Central Retinal Thickness (CRT) and Change from Baseline to through Week 60. (A) central retinal thickness (micrometers) over time (observed values-censoring data post ICE); (B) Mean change from baseline in CST (central subfield retinal thickness (interchangeable with CRT); micrometers) by visit through week 60, OC prior to ICE in the full analysis set; (C) LSₘₑₐₙ (95% CIs) changes from baseline in CST (micrometers) by visit, MMRM (mixed model for repeated measurements) in the full analysis set (to week 48).

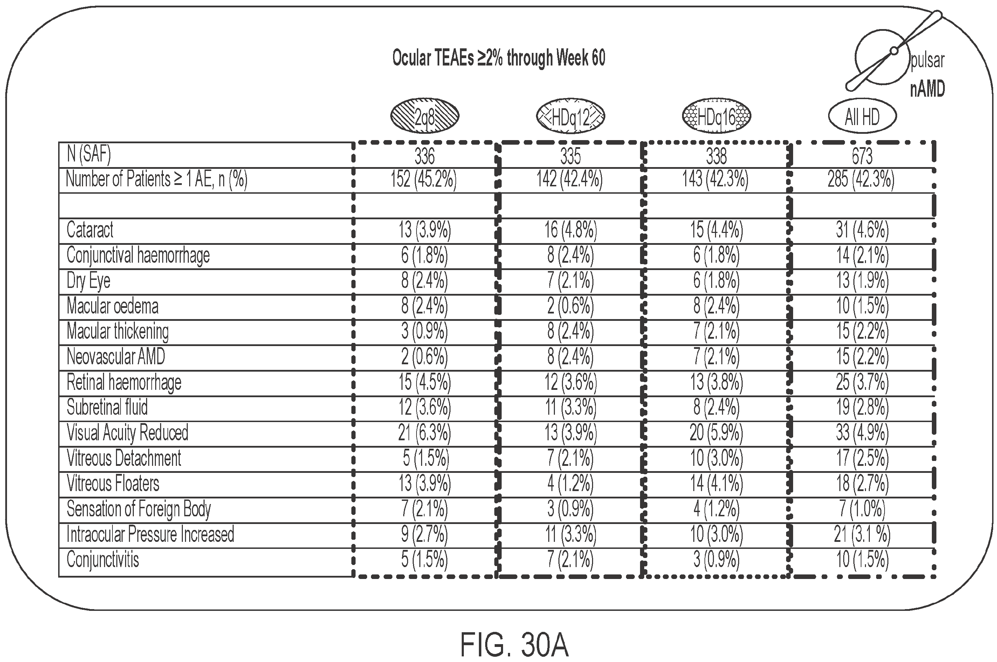

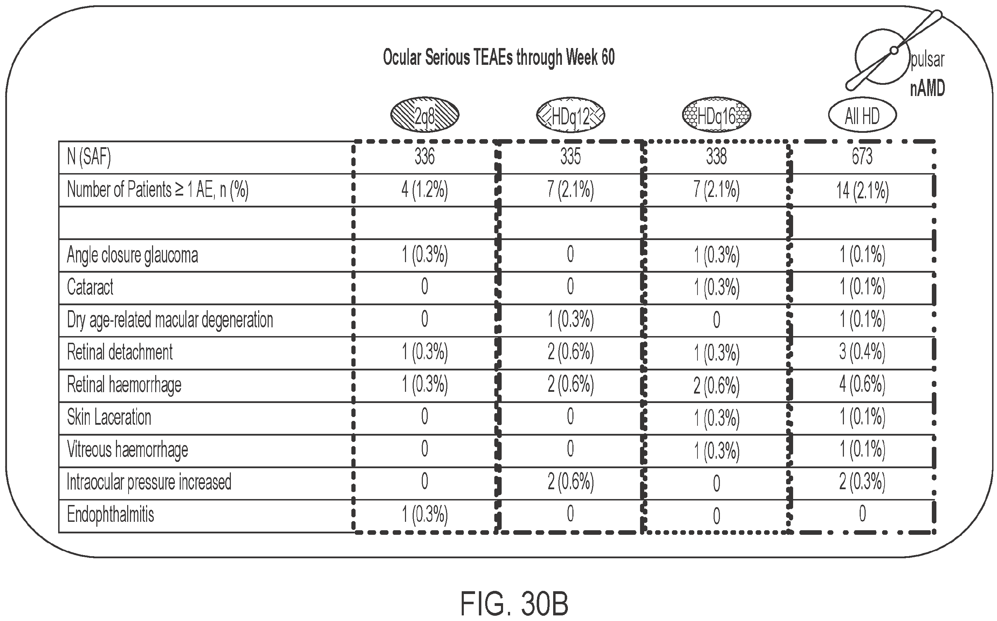

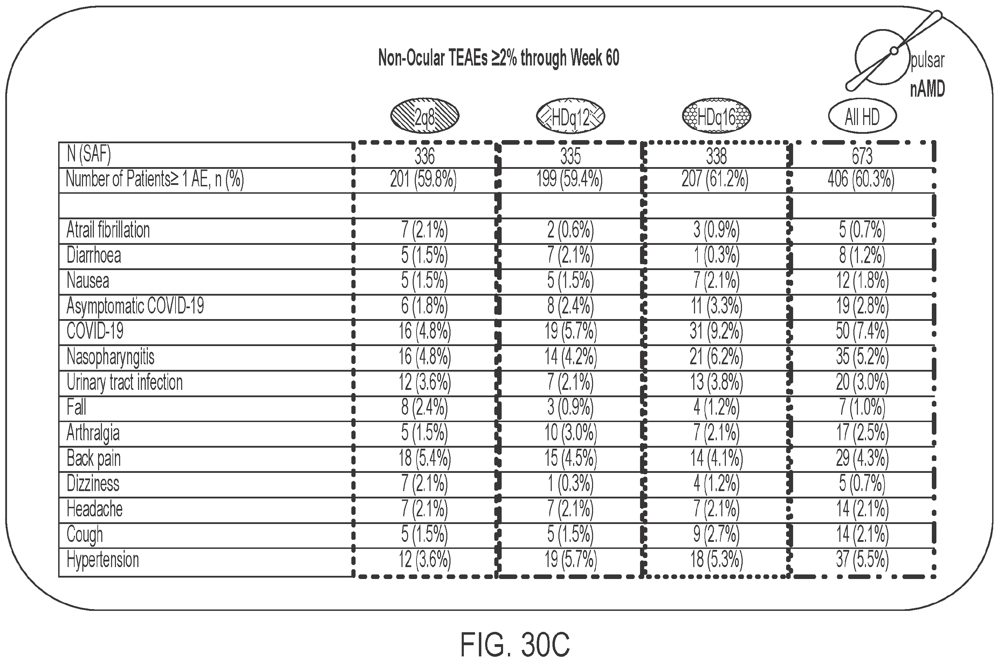

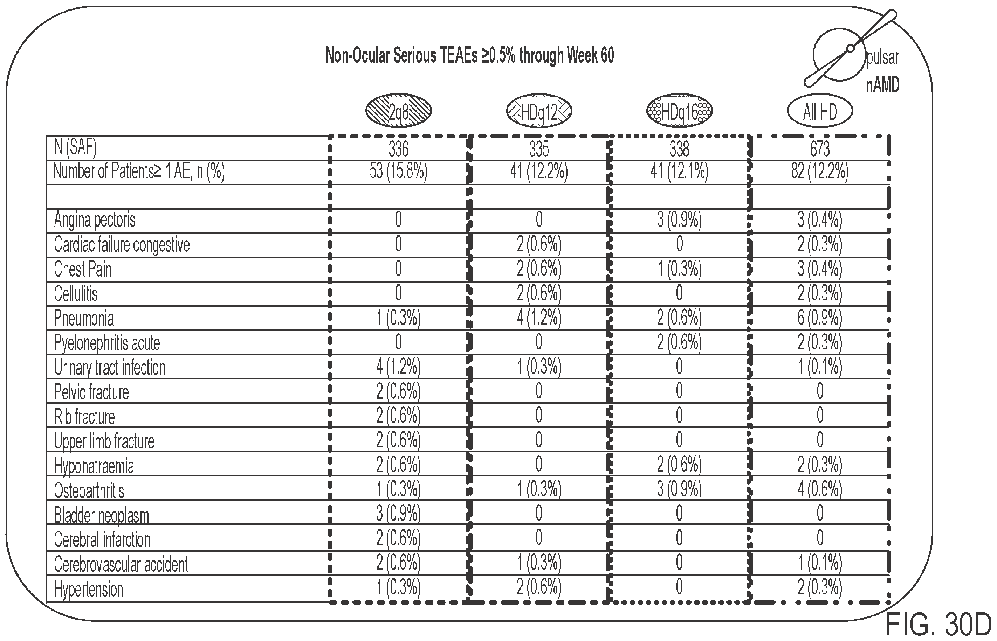

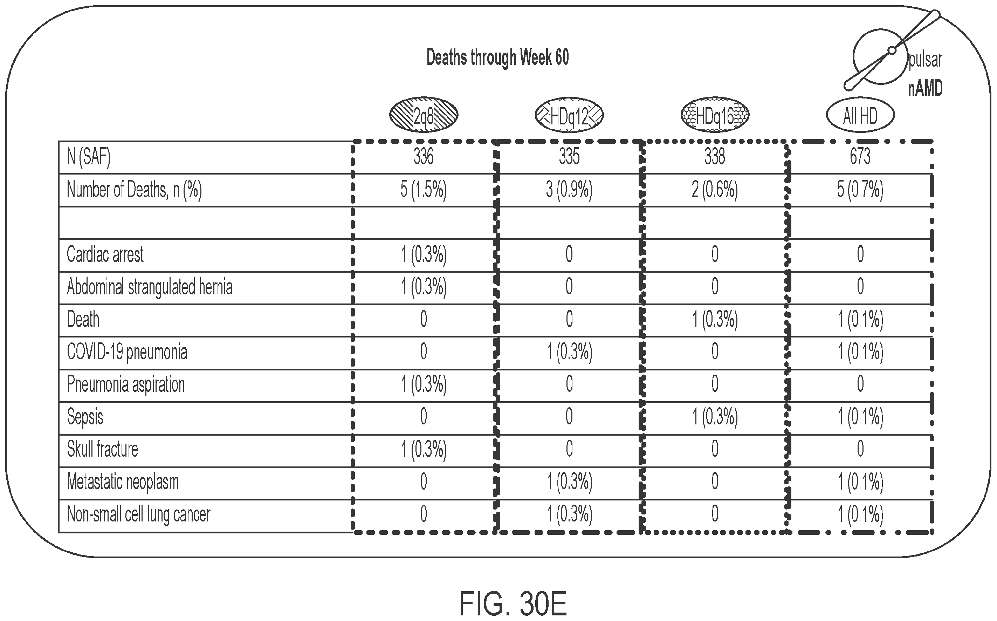

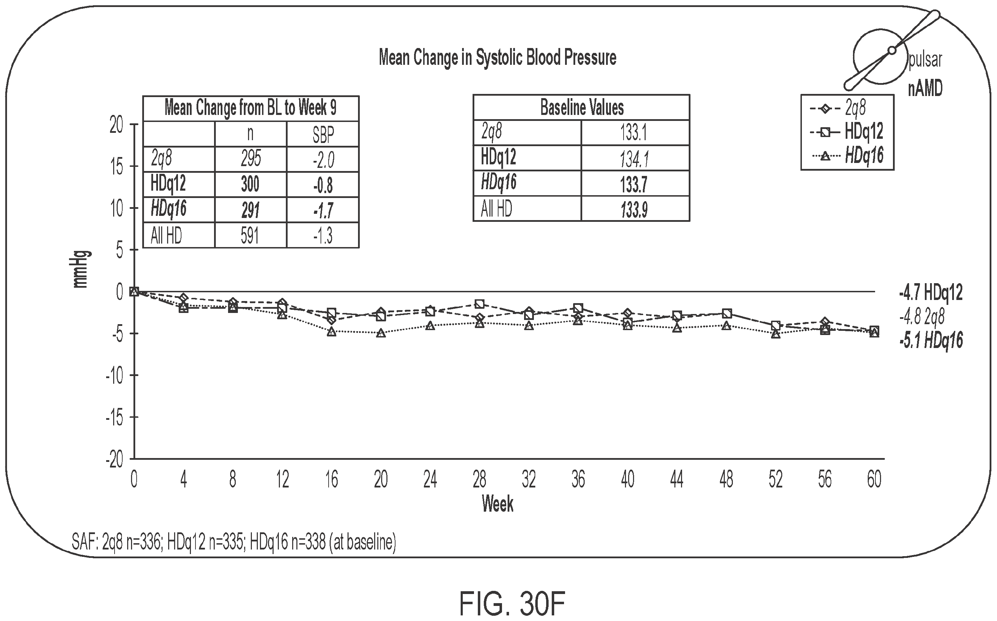

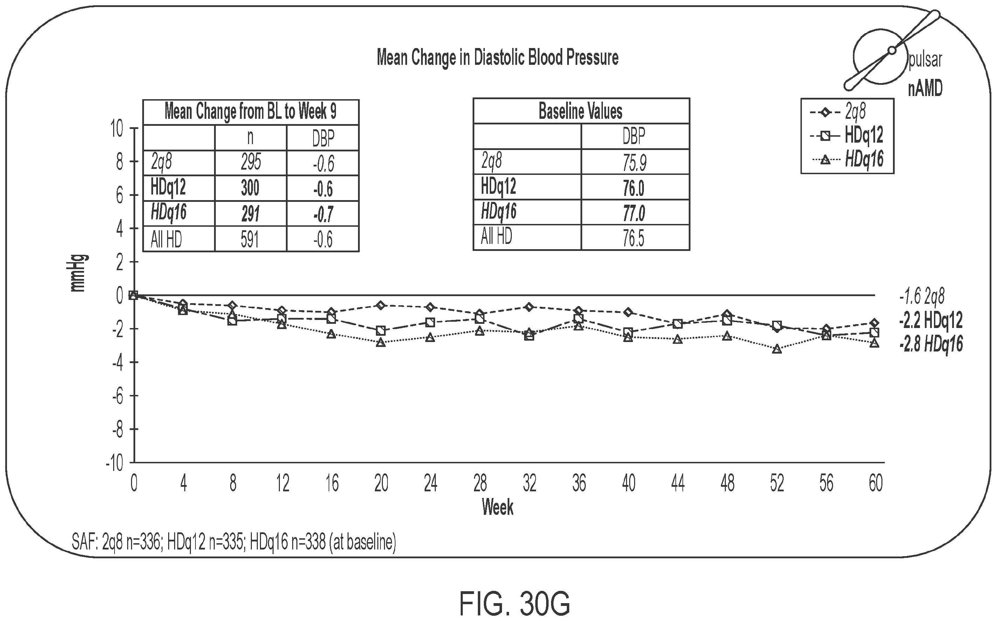

**Figures 30A****,** **30B****,** **30C****,** **30D****,** **30E****,** **30F** **and** **30G**: Safety data summary, (Fig. 30A) Ocular TEAEs ≥2% through Week 60, (Fig. 30B) Ocular Serious TEAEs through Week 60, (Fig. 30C) Non-Ocular TEAEs ≥2% through Week 60, (Fig. 30D) Non-Ocular Serious TEAEs ≥0.5% through Week 60, (Fig. 30E) Deaths through Week 60, (Fig. 30F) Mean Change in Systolic Blood Pressure at week 60, and (Fig. 30G) Mean Change in Diastolic Blood Pressure at week 60, for PULSAR.

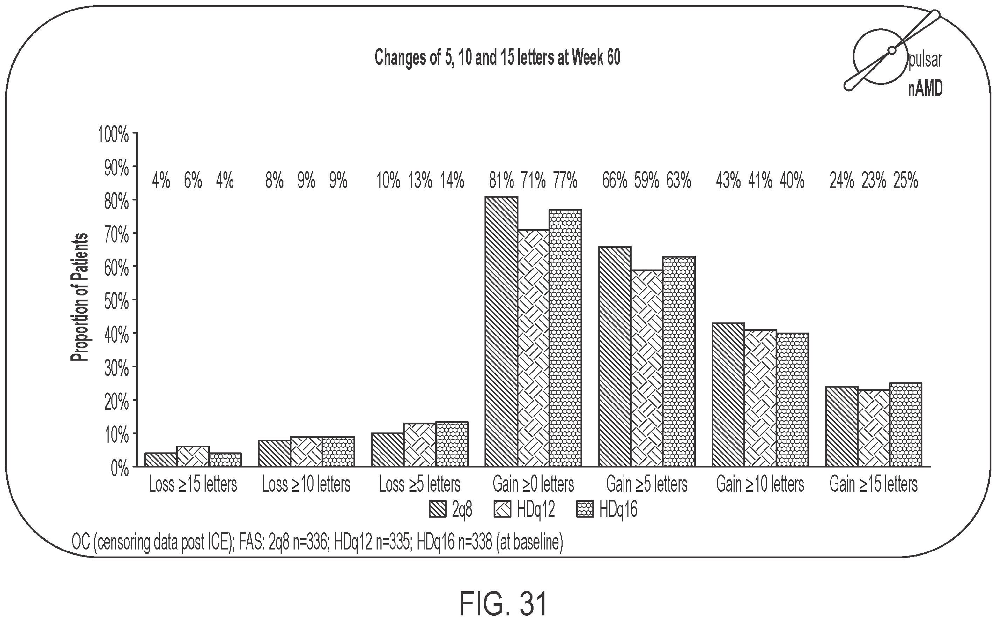

**Figure 31**: Changes of 5, 10 and 15 letters at Week 60. Observed (OC) (censoring data post ICE)

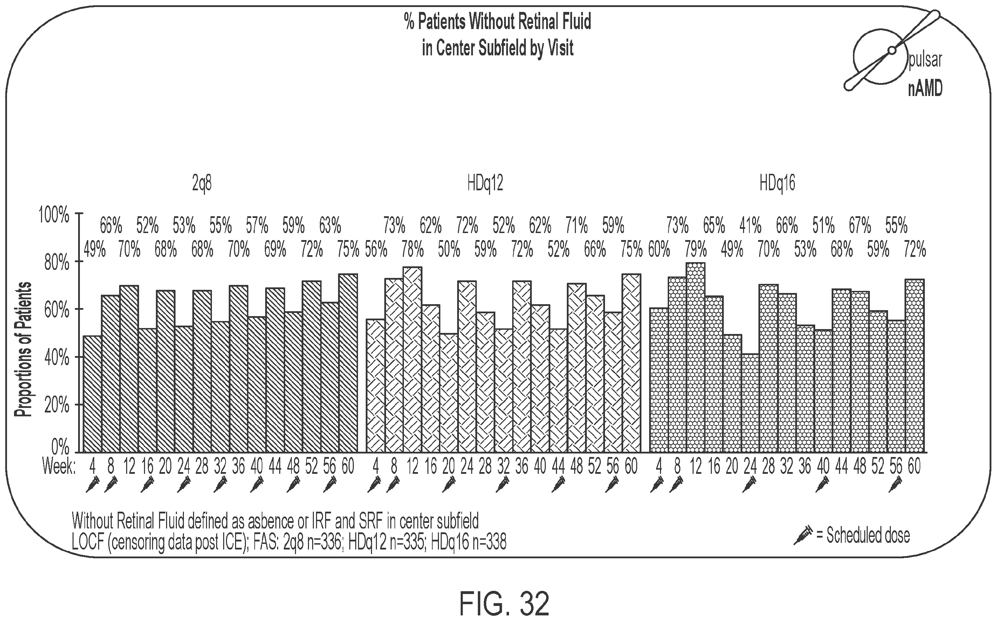

**Figure 32**: % Subjects Without Retinal Fluid in Center Subfield by Visit (weeks and schedules doses shown) to week 60. LOCF (censoring data post ICE)

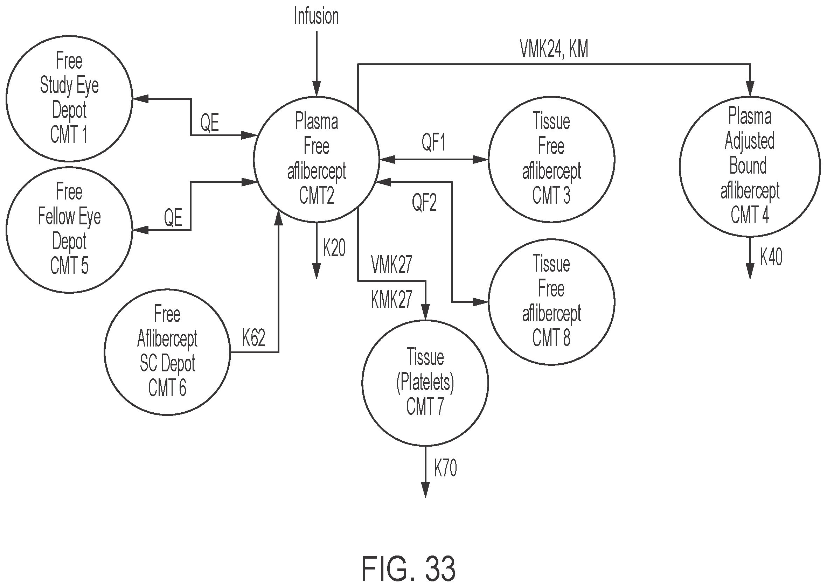

**Figure 33****:** Structural Representation of a Population Pharmacokinetic Model Following IV, SC, and IVT Administration of Aflibercept. CMT = compartment, IV = intravenous, IVT = intravitreal, K20 = elimination rate constant for free aflibercept, K40 = elimination rate constant for adjusted bound aflibercept, K62 = rate of absorption from subcutaneous injection depot compartment, K70 = elimination rate constant from tissue (platelet) compartment; QE = inter-compartmental clearance between ocular compartment and central compartment of free aflibercept, QF1 and QF2 = inter-compartmental clearances of free aflibercept, VMK24, KM = saturable Michaelis-Menten type binding of free aflibercept with VEGF; VMK27, KMK27 = saturable elimination from plasma compartment to tissue compartment (platelets) CMT 2 and CMT 4 are both representative of the plasma compartment and volumes are assumed to be equal.

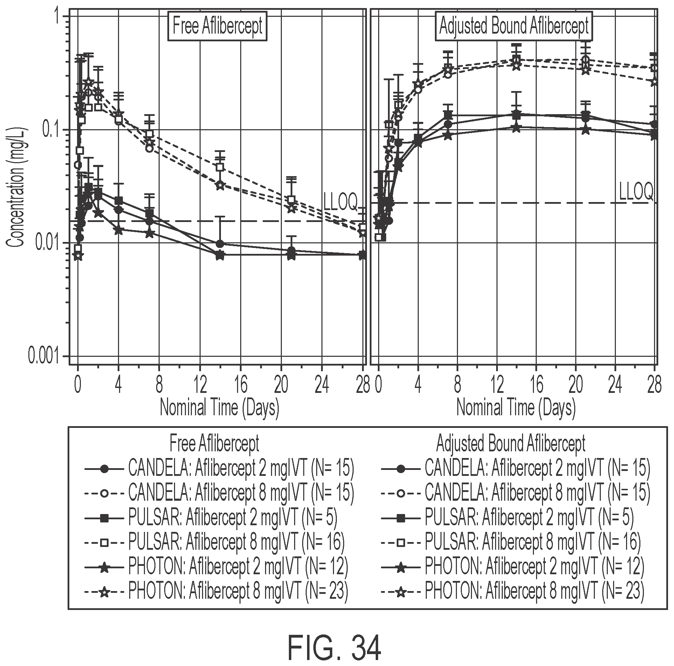

**Figure 34****:** Mean (±SD) Concentrations (mg/l) of Free and Adjusted Bound Aflibercept Over 28 Days for Single 2 mg and 8 mg IVT Doses of Aflibercept in nAMD or DME in the Dense PK Sub-studies (DPKS, Log-Scaled). LQ = below limit of quantification, DME = Diabetic Macular Edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, SD = standard deviation Adjusted Bound Aflibercept = 0.717*Bound Aflibercept Note: Concentrations below the LLOQ (0.0156 mg/L for Free and 0.0224 mg/L for Adjusted Bound Aflibercept) were set to LLOQ/2. Note: 8 mg HD aflibercept data for the first 28 days (obtained from PULSAR or PHOTON) is a combination of data from participants who received HDq12 or HDq16. One participant in PULSAR with an outlier free aflibercept concentration at day 28 that is greater than 10-fold of the mean concentration is excluded. Records after fellow-eye treatment are excluded. Data Source: drug concentration data from the week 48 database lock for PULSAR and PHOTON and final lock for CANDELA.

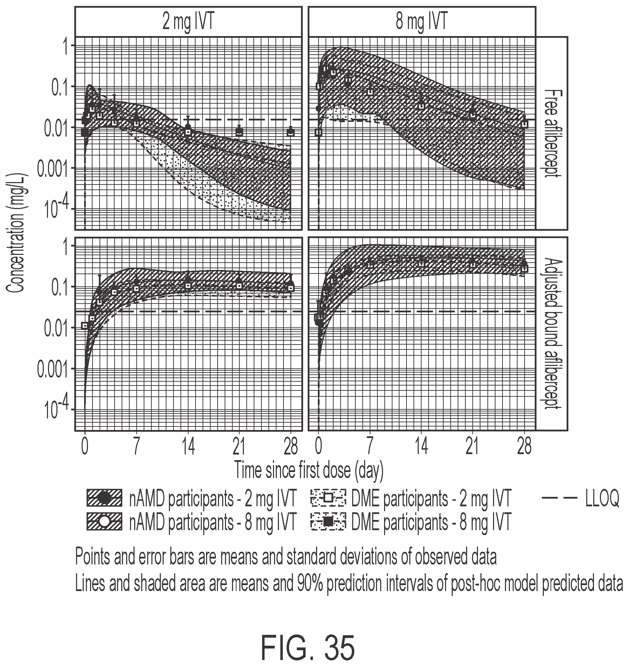

**Figure 35**: Observed and Model-Predicted Concentrations (mg/l) of Free and Adjusted Bound Aflibercept in Plasma Over 28 days After a Single IVT Injection for Participants with nAMD or DME in the Dense PK Sub-studies (DPKS), Stratified by Dose and Population. DME = diabetic macular edema, IVT = intravitreally, LLOQ = lower limit of quantitation, nAMD = neovascular age-related macular degeneration, PK = pharmacokinetic Observed concentrations below the lower limit of quantitation (LLOQ; 0.0156 mg/L for free and 0.0224 mg/L for adjusted bound aflibercept) were set to LLOQ/2. Data source: Drug concentration data from dense PK sub-study in PHOTON, PULSAR, and CANDELA.

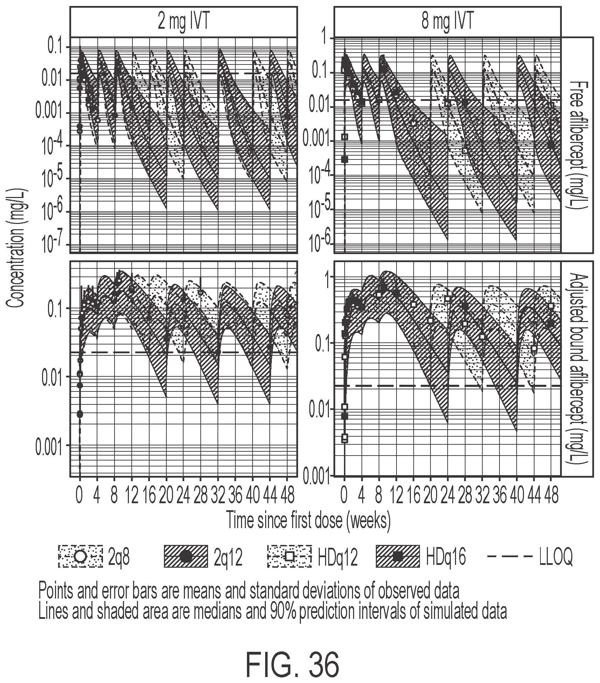

**Figure 36**: Overlay of Observed and Model-Predicted Concentrations (mg/l) of Free and Adjusted Bound Aflibercept in Plasma for Combined nAMD and DME Populations. 2q8 = aflibercept 2 mg administered every 8 weeks, after 3 initial injections at 4-week intervals, 2q12 = aflibercept 2 mg administered every 12 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, nAMD = neovascular age-related macular degeneration Observed concentrations below the lower limit of quantitation (LLOQ; 0.0156 mg/L for free and 0.0224 mg/L for adjusted bound aflibercept) were set to LLOQ/2. Data source: Drug concentration data from CANDELA, PHOTON, and PULSAR.

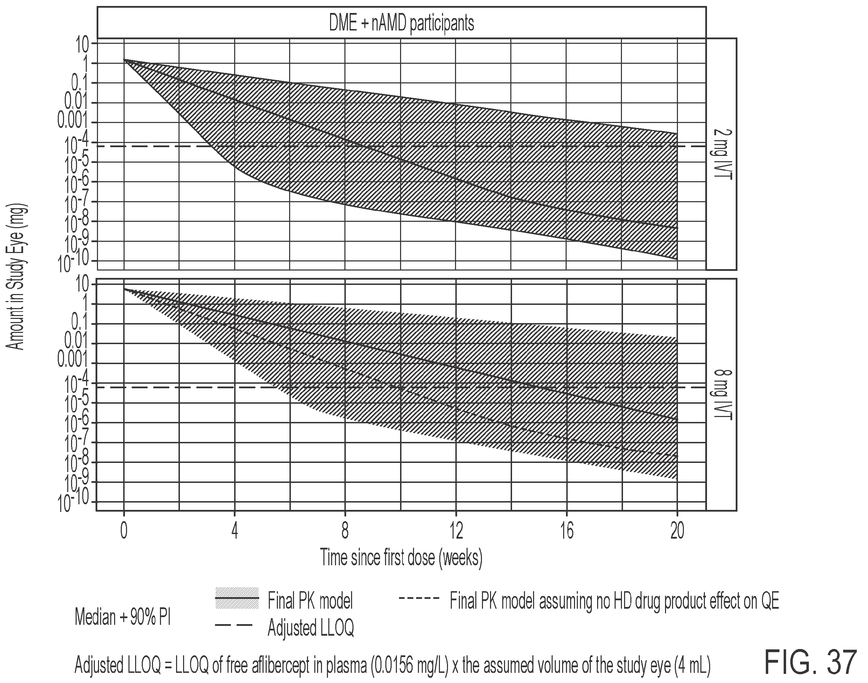

**Figure 37**: Model-Predicted Amounts (mg) of Aflibercept Exposures After a Single IVT Injection, Stratified by Dosing Regimen in Combined Participants with nAMD and DME. DME = diabetic macular edema, HD = aflibercept 8 mg, IVT = intravitreal, nAMD = neurovascular age-related macular degeneration, PI = prediction interval, PK = pharmacokinetics, QE = inter-compartmental clearance between ocular compartment and central compartment of free aflibercept Adjusted LLOQ (0.0624 µg), set as the LLOQ of free aflibercept concentration in plasma (that is, 0.0156 mg/L) times the assumed volume of the study eye compartment in the PK model (that is, 4 mL). Since the concentrations of (free or bound) aflibercept were not measured in the study eye in the clinical studies included in the Population PK analysis dataset, this target was selected arbitrarily on the basis of the LLOQ in plasma and was used as reference for comparison across dosing regimens and to assess the effect of the effect of HD aflibercept on QE.

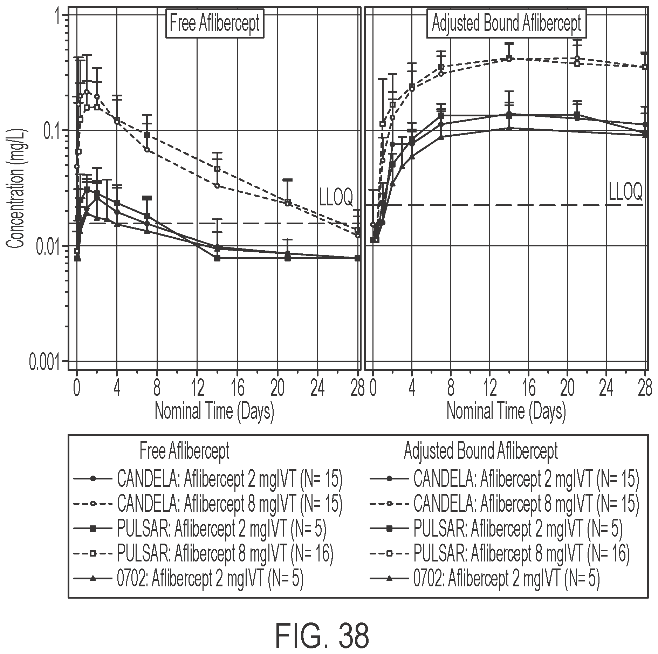

**Figure 38**: Mean (±SD) Concentrations (mg/l) of Free and Adjusted Bound Aflibercept Over 28 Days for Single 2 mg and 8 mg IVT Doses of Aflibercept in Participants with nAMD in the Dense PK Sub-studies (DPKS, Log-Scaled) - No Outlier. DME = diabetic macular edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, PK = pharmacokinetic, SD = standard deviation Adjusted Bound Aflibercept = 0.717*Bound Aflibercept. Note: Concentrations below the LLOQ (0.0156 mg/L for Free and 0.0224 mg/L for Adjusted Bound Aflibercept) were set to LLOQ/2. Note: 8 mg data for the first 28 days (obtained from PULSAR or PHOTON) is a combination of data from participants who received HDq12 or HDq16 Data source: drug concentration from the week 48 lock for PULSAR and PHOTON and final lock for CANDELA. Records after fellow-eye treatment are excluded.

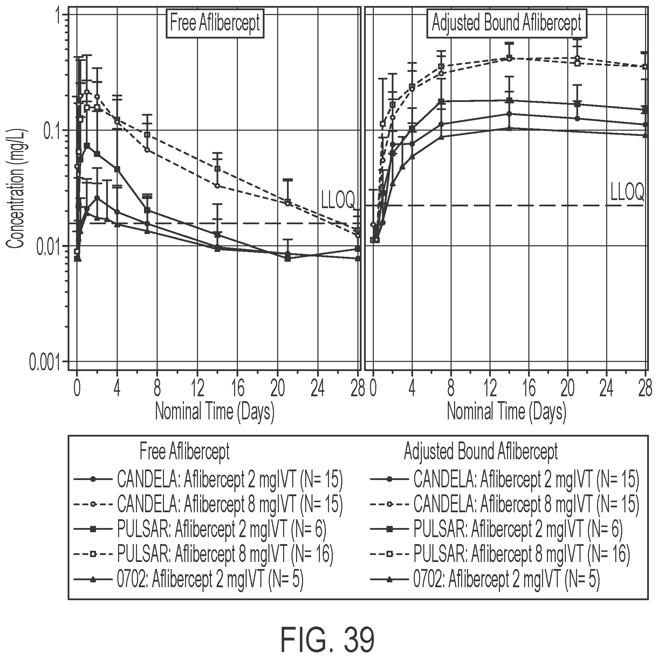

**Figure 39**: Mean (±SD) Concentrations (mg/l) of Free and Adjusted Bound Aflibercept Over 28 Days for Single 2 mg and 8 mg IVT Doses of Aflibercept in Participants with nAMD in the Dense PK Sub-study (DPKS, Log-Scaled) -Outlier Included. DME = diabetic macular edema, DPKS = dense pharmacokinetic sub-studies, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, PK = pharmacokinetic, SD = standard deviation Adjusted Bound Aflibercept = 0.717*Bound Aflibercept. Note: Concentrations below the lower limit of quantification (LLOQ, 0.0156 mg/L for Free and 0.0224 mg/L for Adjusted Bound Aflibercept) were set to LLOQ/2. Data source: drug concentration from the week 48 lock for PULSAR and final lock for CANDELA. Data from VGFTOD-0702 are included as a reference (the concentration in PK sub-study is subtracted by pre-dose concentration when it is >LLOQ). Records after fellow-eye treatment are excluded.

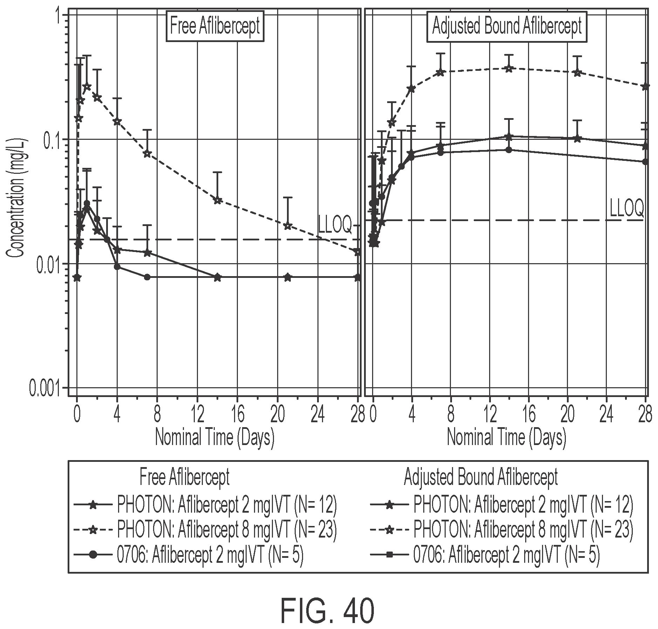

**Figure 40**: Mean (±SD) Concentrations (mg/l) of Free and Adjusted Bound Aflibercept Over 28 Days for Single 2 mg and 8 mg IVT Doses of Aflibercept in Participants with DME in the Dense PK Sub-studies (DPKS, Log-Scaled). BLQ = below limit of quantification, DME = diabetic macular edema, DPKS = dense pharmacokinetic analysis set, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, N = number of participants, nAMD = neovascular age-related macular degeneration, SD = standard deviation Note: Concentrations below the LLOQ (0.0156 mg/L for Free and 0.0224 mg/L for Adjusted Bound Aflibercept) were set to LLOQ/2. Adjusted Bound Aflibercept = 0.717*Bound Aflibercept. Note: 8 mg data for the first 28 days (obtained from PULSAR or PHOTON) is a combination of data from participants who received HDq12 or HDq16. Note: The Concentration is subtracted by baseline concentration if participants took the Aflibercept prior to study drug started within 12 weeks and the baseline concentration is > BLQ. Data source: drug concentration data from the week 48 lock for PHOTON. Drug concentration data from VGFT-OD-0706 (historical data) are included as a reference. Records after fellow-eye treatment are excluded.

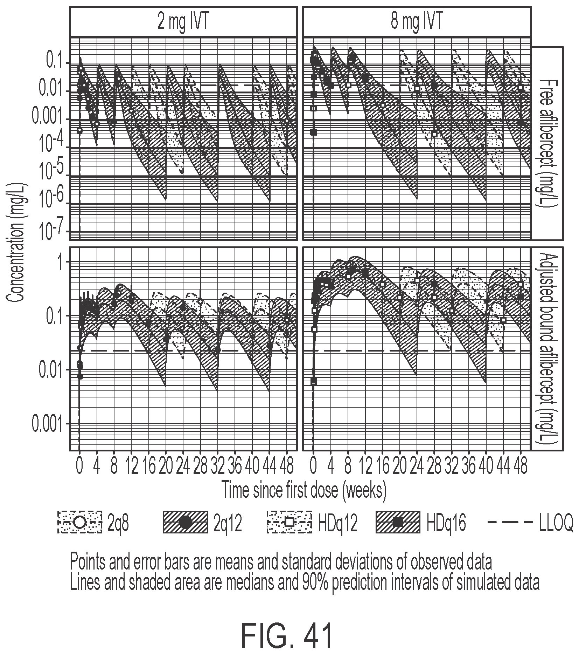

**Figure 41**: Overlay of Observed and Model-Predicted Concentrations (mg/l) of Free and Adjusted Bound Aflibercept in Plasma for Participants with nAMD. 2q8 = aflibercept 2 mg administered every 8 weeks, after 3 initial injections at 4-week intervals, 2q12 = aflibercept 2 mg administered every 12 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantitation, nAMD = neovascular age-related macular degeneration, PK = pharmacokinetics Observed concentrations below the lower limit of quantitation (LLOQ; 0.0156 mg/L for free and 0.0224 mg/L for adjusted bound aflibercept) were set to LLOQ/2. Data from PULSAR and CANDELA.

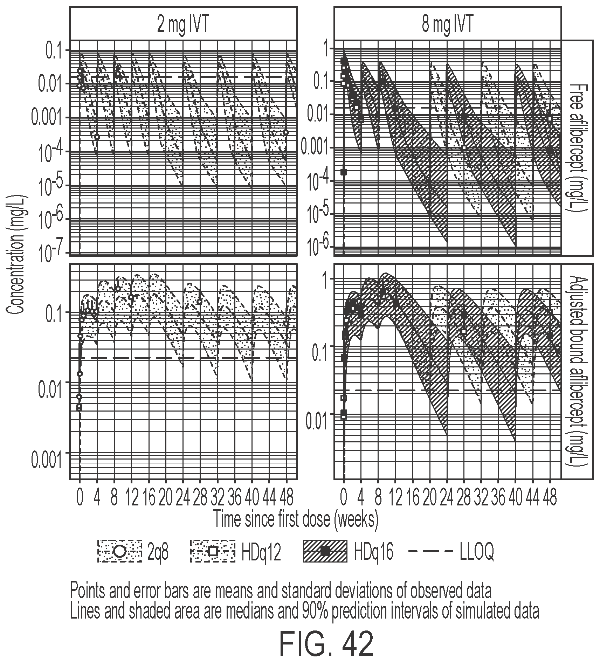

**Figure 42**: Overlay of Observed and Model-Predicted Concentrations (mg/l) of Free and Adjusted Bound Aflibercept in Plasma for Participants with Diabetic Macular Edema in study PHOTON. 2q8 = aflibercept 2 mg administered every 8 weeks, after 3 initial injections at 4-week intervals, HDq12 = aflibercept 8 mg administered every 12 weeks, after 3 initial injections at 4-week intervals, HDq16 = aflibercept 8 mg administered every 16 weeks, after 3 initial injections at 4-week intervals, IVT = intravitreally, LLOQ = lower limit of quantitation Data from PHOTON.

### DETAILED DESCRIPTION OF THE INVENTION

The present invention provides, in part, a safe and effective high-dose aflibercept IVT injection which extends the maintenance dosing interval past 8 weeks, with at least similar functional and potentially improved anatomic outcomes. The regimen exhibited an unexpectedly high level of durability in subjects which exceeded that which would have been expected simply based on administration of more aflibercept.

EYLEA has become the standard-of-care nAMD. Eylea is prescribed for nAMD at a dose of 2 mg once a month for 3 doses followed by maintenance dosing every 8 weeks. The dosing regimen of the present invention has demonstrated that a remarkably high percentage of subjects can be maintained on 12- and 16-week dosing intervals. In trials testing these dosing regimens, nearly 90% of subjects with diabetic macular edema were able to maintain a 16-week dosing regimen. These durability data coupled with a safety profile consistent with that of EYLEA support high-dose aflibercept as a potential new standard-of-care in angiogenic eye disorders such as nAMD. The data presented herein demonstrated that aflibercept 8 mg 12- and 16-week dosing regimens have achieved a high bar, sustaining improvements in visual acuity and anatomic measures of retinal fluid across 48 weeks in subjects with nAMD. These results were all achieved in subjects who were rapidly initiated on extended dosing intervals with the vast majority not requiring regimen modification. Altogether, the pivotal data support aflibercept 8 mg as providing a longer duration of action while maintaining a safety profile similar to EYLEA.

Prior to initiating the HD aflibercept clinical development program, pharmacokinetic simulations of free aflibercept concentration-time profiles in human vitreous using a 1-compartment ocular model predicted that an 8 mg IVT dose of aflibercept could extend the dosing interval by approximately 20 days (two half-lives) relative to a 2 mg IVT dose. The aflibercept HDq12 and HDq16 regimens exhibited a duration of efficacy in the HD clinical studies that was longer than predicted. A subsequent population PK analysis that integrated data from the CANDELA PHOTON and PULSAR phase 3 studies indicated that ocular clearance of free aflibercept was 34% slower for the HD aflibercept drug product compared to 2 mg IVT aflibercept administered as the Eylea drug product, and the slower ocular clearance for HD aflibercept was predicted to result in both a longer persistence of free aflibercept in the eye and an approximate 6-week longer duration of efficacy compared to 2 mg. The magnitude of reduction in ocular clearance for the HD aflibercept drug product compared to the 2 mg Eylea drug product was greater than expected and attributed to an "HD aflibercept drug product effect", a highly statistically significant effect in the population PK model that cannot be explained by just an increase in the dose from 2 mg to 8 mg.

The Population PK predicted median time for free aflibercept concentrations in plasma to reach the lower limit of quantification (LLOQ) following 2 mg IVT aflibercept was estimated to be 1.5 weeks compared to 3.5 weeks for 8 mg HD aflibercept. The longer duration of systemic exposure to free aflibercept, representative of the movement of free aflibercept from the eye, for the HD aflibercept regimen was attributed to not only a higher administered dose and nonlinear systemic target-mediated elimination, but also to a 34% slower ocular clearance of free aflibercept. The slower ocular clearance of the HD aflibercept drug product was predicted to provide a 6-week longer duration of efficacy compared to that of the 2 mg aflibercept drug product, as the population PK estimated time to achieve the free aflibercept amount in the ocular compartment for the 2q8 regimen at the end of an 8-week dosing interval occurs 6 weeks later for the HD aflibercept drug product. Exposure-response analyses estimated that the slower ocular clearance for 8 mg aflibercept, attributable to the HD drug product effect, resulted in a 20.6% lower rate of dosing regimen modification (DRM) than would have been expected if the HD drug product had the same ocular clearance as 2 mg aflibercept.

General methods for protein purification including immunoprecipitation, chromatography, electrophoresis, centrifugation, and crystallization are described (Coligan et al. (2000) Current Protocols in Protein Science, Vol. 1, John Wiley and Sons, Inc., New York). Chemical analysis, chemical modification, post-translational modification, production of fusion proteins, glycosylation of proteins are described (see *e.g.*, Coligan et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, N.Y., pp. 16.0.5-16.22.17; Sigma-Aldrich, Co. (2001) Products for Life Science Research, St. Louis, Mo.; pp. 45-89; Amersham Pharmacia Biotech (2001) BioDirectory, Piscataway, N.J., pp. 384-391). Production, purification, and fragmentation of polyclonal and monoclonal antibodies are described (Coligan et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, N.Y.; Harlow and Lane, *supra*). Standard techniques for characterizing ligand/receptor interactions are available (see, *e.g.*, Coligan et al. (2001) Current Protocols in Immunology, Vol. 4, John Wiley, Inc., New York).

Methods for flow cytometry, including fluorescence activated cell sorting (FACS), are available (see, *e.g.*, Owens et al. (1994) Flow Cytometry Principles for Clinical Laboratory Practice, John Wiley and Sons, Hoboken, N.J.; Givan (2001) Flow Cytometry, 2nd ed.; Wiley-Liss, Hoboken, N.J.; Shapiro (2003) Practical Flow Cytometry, John Wiley and Sons, Hoboken, N.J.). Fluorescent reagents suitable for modifying nucleic acids, including nucleic acid primers and probes, polypeptides, and antibodies, for use, *e.g.*, as diagnostic reagents, are available (Molecular Probes (2003) Catalogue, Molecular Probes, Inc., Eugene, Oreg.; Sigma-Aldrich (2003) Catalogue, St. Louis, Mo.).

Standard methods of histology of the immune system are described (see *e.g.*, Muller-Harmelink (ed.) (1986) Human Thymus: Histopathology and Pathology, Springer Verlag, New York, N.Y.; Hiatt et al. (2000) Color Atlas of Histology, Lippincott, Williams, and Wilkins, Phila, Pa.; Louis et al. (2002) Basic Histology: Text and Atlas, McGraw-Hill, New York, N.Y.).

"Isolated" VEGF antagonists and VEGF receptor fusion proteins (*e.g.*, aflibercept), polypeptides, polynucleotides and vectors, are at least partially free of other biological molecules from the cells or cell culture from which they are produced. Such biological molecules include nucleic acids, proteins, other VEGF antagonists and VEGF receptor fusion proteins, lipids, carbohydrates, or other material such as cellular debris and growth medium. An isolated VEGF antagonist or VEGF receptor fusion protein may further be at least partially free of expression system components such as biological molecules from a host cell or of the growth medium thereof. Generally, the term "isolated" is not intended to refer to a complete absence of such biological molecules (*e.g.*, minor or insignificant amounts of impurity may remain) or to an absence of water, buffers, or salts or to components of a pharmaceutical formulation that includes the VEGF antagonists or VEGF receptor fusion proteins.

"Isolated" VEGF antagonists and VEGF receptor fusion proteins (*e.g.*, aflibercept), polypeptides, polynucleotides and vectors, are at least partially free of other biological molecules from the cells or cell culture from which they are produced. Such biological molecules include nucleic acids, proteins, other VEGF antagonists and VEGF receptor fusion proteins, lipids, carbohydrates, or other material such as cellular debris and growth medium. An isolated VEGF antagonist or VEGF receptor fusion protein may further be at least partially free of expression system components such as biological molecules from a host cell or of the growth medium thereof. Generally, the term "isolated" is not intended to refer to a complete absence of such biological molecules (*e.g.*, minor or insignificant amounts of impurity may remain) or to an absence of water, buffers, or salts or to components of a pharmaceutical formulation that includes the VEGF antagonists or VEGF receptor fusion proteins.

Doses mentioned herein, for example, 8 mg, encompass embodiments including the specified amount ±10%, *e.g.*, 8 mg (±0.8 mg). Concentrations or amounts mentioned herein, for example, of formulation excipients also encompass embodiments including the specified amount ± 10%.

Subject and patient are used interchangeably herein. A subject or patient is a mammal, for example a human, mouse, rabbit, monkey or non-human primate, preferably a human. A subject or patient may be said to be "suffering from" an angiogenic eye disorder such as nAMD. Such a subject or patient has the disorder in one or both eyes. In an embodiment of the invention, a subject or patient (preferably a human) has one or more of the following characteristics (presently or in the past):
1. ≥50 years of age, *e.g.*, 61, 62, 63, 74 or 75;
2. Has active subfoveal CNV secondary to nAMD, *e.g.*, with a total area of >50% of the total lesion area, *e.g.*, including juxtafoveal lesions that affect the fovea in an eye;
3. Has Best Corrected Visual Acuity (BCVA) Early Treatment Diabetic Retinopathy Study (ETDRS) letter score of about 78 to 24, 73-78, <73, 58, 59, 60, 61 or 62 (Snellen equivalent of 20/40, 20/63, 20/50, 20/32 or 20/320), *e.g.*, due to DME or nAMD;
4. Central retinal thickness of ≥300 micrometers or ≥320 micrometers, or about 367, 368, 369, 370, 450, 451, 452, 453, 454 or 455 micrometers; or and/or DME with central involvement in an eye with CRT ≥300 micrometers (or ≥320 micrometers on Spectralis);
5. Total lesion area of about 6 or 7 mm², *e.g.*, wherein the lesion type is occult, predominantly classic or minimally classic;
6. DRSS score of better or equal to Level 43, Level 47 or worse;
7. Type 1 or type 2 diabetes mellitus; and/or
8. Presence of intraretinal and/or subretinal fluid in the central subfield on OCT in an eye;
9. Body mass index of about 27 or 28 or greater; and/or
10. Hypertension; and/or
11. A history of any one or more of: cataract, dry age-related macular degeneration, vitreous detachment, dry eye, cataract nuclear, glaucoma, astigmatism, presbyopia, choroidal neovascularisation, open angle glaucoma, hypermetropia, macular degeneration, myopia, posterior capsule opacification, epiretinal membrane, retinal degeneration, dermatochalasis, retinopathy hypertensive, retinal drusen, exfoliation syndrome, vitreous floaters, cataract cortical, eyelid ptosis, retinal tear, vitreous disorder, pterygium, vitreous degeneration, asthenopia, cataract operation, intraocular lens implant, lens capsulotomy, blepharoplasty, keratomileusis, laser therapy, neoplasms (benign, malignant and unspecified (including cysts and polyps)), or eye naevus,
and/or,
has or lacks any one or more of the following characteristics:
1. Subretinal hemorrhage in an eye that is ≥50% of the total lesion area;
2. Intraocular pressure ≥25 mmHg in an eye;
3. Evidence of infectious blepharitis, keratitis, scleritis, or conjunctivitis in an eye;
4. Any intraocular inflammation and/or ocular infection in an eye;
5. Any history of macular hole of stage 2 and above in an eye;
6. Current iris neovascularization, vitreous hemorrhage, or tractional retinal detachment visible in an eye;
7. Uncontrolled BP (defined as systolic >140 mm Hg or diastolic >90 mm Hg);
8. Variation by more than 10% in the 3 BP measurements;
9. History of cerebrovascular accident/ transient ischemic attack or myocardial infarction/ acute coronary syndrome;
10. Renal failure, dialysis, or history of renal transplant;
11. Known sensitivity to aflibercept;
12. Pregnant or breastfeeding woman;
13. Woman of childbearing potential who are unwilling to practice highly effective contraception;
14. Any intraocular surgery within 12 weeks (84 days); and/or
15. History of corneal transplant or corneal dystrophy in an eye.

Thus, the present invention includes a method for treating or preventing neovascular age related macular degeneration (nAMD), in a subject in need thereof
1. who is ≥50 years of age;
2. who has active subfoveal CNV secondary to nAMD;
3. who has Best Corrected Visual Acuity (BCVA) Early Treatment Diabetic Retinopathy Study (ETDRS) letter score of about 78 to 24;
4. has a central retinal thickness of ≥300 micrometers or ≥320 micrometers;
5. who has a lesion area of about 6 or 7 mm²;
6. who has a DRSS score of better or equal to Level 43, Level 47 or worse; and/or
7. who has Type 1 or type 2 diabetes mellitus;
and/or
1. who does not have subretinal hemorrhage in an eye that is ≥50% of the total lesion area;
2. who does not have an Intraocular pressure ≥25 mmHg in an eye;
3. for whom there is no evidence of infectious blepharitis, keratitis, scleritis, or conjunctivitis in an eye;
4. who does not have any intraocular inflammation and/or ocular infection in an eye;
5. who does not have any history of macular hole of stage 2 and above in an eye;
6. who does not have current iris neovascularization, vitreous hemorrhage, or tractional retinal detachment visible in an eye;
7. who does not have uncontrolled BP;
8. who does not have a variation, by more than 10%, in 3 BP measurements;
9. who does not have a history of cerebrovascular accident/transient ischemic attack or myocardial infarction/acute coronary syndrome;
10. who does not have renal failure, dialysis, and/or history of renal transplant;
11. who does not have a known sensitivity to aflibercept;
12. who is not pregnant or a breastfeeding woman;
13. who is not a woman of childbearing potential who is unwilling to practice highly effective contraception;
14. who has not had any intraocular surgery within 12 weeks (84 days); and/or
15. does not have a history of corneal transplant or corneal dystrophy in an eye;
comprising administering to an eye of the subject, a single initial dose of about 8 mg or more of a VEGF receptor fusion protein, preferably aflibercept, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks after the immediately preceding dose.

### VEGF Antagonists

The present invention includes methods for using a VEGF antagonist for treating or preventing angiogenic eye disorders. VEGF antagonists include molecules which interfere with the interaction between VEGF and a natural VEGF receptor, *e.g.*, molecules which bind to VEGF or a VEGF receptor and prevent or otherwise hinder the interaction between VEGF and a VEGF receptor. Specific, exemplary VEGF antagonists include anti-VEGF antibodies, anti-VEGF receptor antibodies, and VEGF receptor fusion proteins. Though VEGF receptor fusion proteins, such as aflibercept, are preferred for use in connection with the methods set forth herein, the scope of the present invention includes such methods wherein any of the VEGF antagonists described herein (*e.g.*, scFvs, DARPins, anti-VEGF antibodies) are used in place of such fusion proteins.

For purposes herein, a "VEGF receptor fusion protein" refers to a molecule that comprises one or more VEGF receptors or domains thereof, fused to another polypeptide, which interferes with the interaction between VEGF and a natural VEGF receptor, *e.g.*, wherein two of such fusion polypeptides are associated thereby forming a homodimer or other multimer. Such VEGF receptor fusion proteins may be referred to as a "VEGF-Trap" or "VEGF Trap". VEGF receptor fusion proteins within the context of the present disclosure that fall within this definition include chimeric polypeptides which comprise two or more immunoglobulin (Ig)-like domains of a VEGF receptor such as VEGFR1 (also known as Flt1) and/or VEGFR2 (also known as Flk1 or KDR), and may also contain a multimerizing domain (for example, an Fc domain).

An exemplary VEGF receptor fusion protein is a molecule referred to as VEGF1R2-FcΔC1(a) which is encoded by the nucleic acid sequence of SEQ ID NO:1 or nucleotides 79-1374 or 79-1371 thereof.

VEGF1R2-FcΔC1(a) comprises three components:
(1) a VEGFR1 component comprising amino acids 27 to 129 of SEQ ID NO:2;
(2) a VEGFR2 component comprising amino acids 130 to 231 of SEQ ID NO:2; and
(3) a multimerization component ("FcΔC1(a)") comprising amino acids 232 to 457 of SEQ ID NO:2 (the C-terminal amino acids of SEQ ID NO:2, *i*.*e.*, K458, may or may not be included in the VEGF receptor fusion proteins, see U.S. Patent No. 7,396,664 or 7,354,579, incorporated herein for all purposes). Note that amino acids 1 to 26 of SEQ ID NO:2 are the signal sequence.

If the multimerizing component (MC) of a VEGF receptor fusion protein is derived from an IgG (*e.g.*, IgG1) Fc domain, then the MC has no fewer amino acids than are in amino acids 232 to 457 of SEQ ID NO:2. Thus, the IgG of the MC cannot be truncated to be shorter than 226 amino acids.

In an embodiment of the invention, the VEGF receptor fusion protein comprises amino acids 27-458 or 27-457 of SEQ ID NO: 2 (*e.g.*, in the form of a homodimer). 
atggtcagctactgggacaccggggtcctgctgtgcgcgctgctcagctgtctgcttctcacaggatctagttccgg

aagtgataccggtagacctttcgtagagatgtacagtgaaatccccgaaattatacacatgactgaaggaagggagc

tcgtcattccctgccgggttacgtcacctaacatcactgttactttaaaaaagtttccacttgacactttgatccct

gatggaaaacgcataatctgggacagtagaaagggcttcatcatatcaaatgcaacgtacaaagaaatagggcttct

gacctgtgaagcaacagtcaatgggcatttgtataagacaaactatctcacacatcgacaaaccaatacaatcatag

atgtggttctgagtccgtctcatggaattgaactatctgttggagaaaagcttgtcttaaattgtacagcaagaact

gaactaaatgtggggattgacttcaactgggaatacccttcttcgaagcatcagcataagaaacttgtaaaccgaga

cctaaaaacccagtctgggagtgagatgaagaaatttttgagcaccttaactatagatggtgtaacccggagtgacc

aaggattgtacacctgtgcagcatccagtgggctgatgaccaagaagaacagcacatttgtcagggtccatgaaaag

gacaaaactcacacatgcccaccgtgcccagcacctgaactcctggggggaccgtcagtcttcctcttccccccaaa

acccaaggacaccctcatgatctcccggacccctgaggtcacatgcgtggtggtggacgtgagccacgaagaccctg

aggtcaagttcaactggtacgtggacggcgtggaggtgcataatgccaagacaaagccgcgggaggagcagtacaac

agcacgtaccgtgtggtcagcgtcctcaccgtcctgcaccaggactggctgaatggcaaggagtacaagtgcaaggt

ctccaacaaagccctcccagcccccatcgagaaaaccatctccaaagccaaagggcagccccgagaaccacaggtgt

acaccctgcccccatcccgggatgagctgaccaagaaccaggtcagcctgacctgcctggtcaaaggcttctatccc

agcgacatcgccgtggagtgggagagcaatgggcagccggagaacaactacaagaccacgcctcccgtgctggactc

cgacggctccttcttcctctacagcaagctcaccgtggacaagagcaggtggcagcaggggaacgtcttctcatgct

ccgtgatgcatgaggctctgcacaaccactacacgcagaagagcctctccctgtctccgggtaaatga
(SEQ ID NO: 1) 
MVSYWDTGVLLCALLSCLLLTGSSSGSDTGRPFVEMYSEIPEIIHMTEGRELVIPCRVTS
PNITVTLKKFPLDTLIPDGKRIIWDSRKGFIISNATYKEIGLLTCEATVNGHLYKTNYLT
HRQTNTIIDVVLSPSHGIELSVGEKLVLNCTARTELNVGIDFNWEYPSSKHQHKKLVNRD
LKTQSGSEMKKFLSTLTIDGVTRSDQGLYTCAASSGLMTKKNSTFVRVHEKDKTHTCPPC
PAPELLGGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
(SEQ ID NO: 2)

In an embodiment of the invention, the VEGF receptor fusion protein comprises
(1) an immunoglobin-like (Ig) domain 2 of a first VEGF receptor (*e*.*g*., VEGFR1), and
(2) an Ig domain 3 of a second VEGF receptor (*e*.*g*., VEGFR2),
(3) and, optionally, further including an Ig domain 4 of the second VEGF receptor (*e*.*g*., VEGFR2) and
(4) a multimerizing component (*e*.*g*., Fc domain of IgG including the hinge, CH2 and CH3 domains).

For example, in an embodiment of the invention, the VEGF receptor fusion protein has the following arrangement of said domains:
- [VEGFR1 Ig domain 2]-[VEGFR2 Ig domain 3]-[MC] (*e*.*g*., a homodimer thereof) or
- [VEGFR1 Ig domain 2]-[VEGFR2 Ig domain 3]-[VEGFR2 Ig domain 4]-[MC] (*e*.*g*., a homodimer thereof).

Note that the present disclosure also includes, within its scope, high concentration formulations including, instead of a VEGF receptor fusion protein, a VEGF binding molecule or anti-VEGF antibody or antigen-binding fragments thereof or biopolymer conjugate thereof (*e*.*g*., KSI-301), *e*.*g*.,
- bevacizumab (*e*.*g*., at a concentration of about 80-90 or 88 mg/ml),
- ranibizumab (*e*.*g*., at a concentration of about 20-40 mg/ml, *e*.*g*., 21-35, 21 or 35 mg/ml),
- an anti-VEGF aptamer such as pegaptanib (*e*.*g*., pegaptanib sodium),
- a single chain (*e*.*g*., V_{L}-V_{H}) anti-VEGF antibody such as brolucizumab (*e*.*g*., at a concentration of about 200-400 or 200, 210, 400 or 420 mg/ml),
- an anti-VEGF DARPin such as the Abicipar Pegol DARPin (*e*.*g*., at a concentration of about 70-140, 70 or 140 mg/ml), or
- a bispecific anti-VEGF antibody, *e*.*g*., which also binds to ANG2, such as RG7716 (faricimab) (*e*.*g*., at a concentration of about 100-400, 100, 105, 400 or 420 mg/ml).

In order to minimize the repetitiveness of the embodiments discussed herein, it is contemplated that the scope of the present invention includes embodiments wherein any of the formulations discussed herein include, in place of a VEGF receptor fusion protein, an anti-VEGF antibody or antibody fragment or other VEGF binding molecule as discussed herein (*e*.*g*., substituted with an anti-VEGF DARPin) at any of the concentrations discussed herein. For example, the present invention includes a formulation having 35 or 80 mg/ml ranibizumab, a buffer, a thermal stabilizer, a viscosity reducing agent and a surfactant.

DARPins are Designed Ankyrin Repeat Proteins. DARPins generally contain three to four tightly packed repeats of approximately 33 amino acid residues, with each repeat containing a β-turn and two anti-parallel α-helices. This rigid framework provides protein stability whilst enabling the presentation of variable regions, normally comprising six amino acid residues per repeat, for target recognition.

An "anti-VEGF" antibody or antigen-binding fragment of an antibody refers to an antibody or fragment that specifically binds to VEGF.

Illustrative VEGF receptor fusion proteins include aflibercept (EYLEA^{®}, Regeneron Pharmaceuticals, Inc.) or conbercept (sold commercially by Chengdu Kanghong Biotechnology Co., Ltd.). See International patent application publication no. WO2005/121176 or WO2007/112675. The terms "aflibercept" and "conbercept" include biosimilar versions thereof. A biosimilar version of a reference product (*e*.*g*., aflibercept) generally refers to a product comprising the identical amino acid sequence, but includes products which are biosimilar under the U.S. Biologics Price Competition and Innovation Act.

The present invention also includes embodiments including administering one or more further therapeutic agents in addition to VEGF antagonist, for example, administering (one or more doses of) a second VEGF antagonist, an antibiotic, anesthetic (*e*.*g*., local anesthetic) to the eye receiving an injection, a non-steroidal anti-inflammatory drug (NSAID), a steroid (*e*.*g*., a corticosteroid, dexamethasone), triamcinolone acetonide (TA), methotrexate, rapamycin, an anti-tumor necrosis factor alpha drug (*e*.*g*., infliximab), daclizumab, and/or a complement component (*e*.*g*., C3 or C5) inhibitor.

### Pharmaceutical Formulations

The present invention includes methods in which the VEGF antagonist that is administered to the subject's eye is contained within a pharmaceutical formulation. The pharmaceutical formulation includes a VEGF antagonist along with a pharmaceutically acceptable carrier. Other agents may be incorporated into the pharmaceutical formulation to provide improved transfer, delivery, tolerance, and the like. The term "pharmaceutically acceptable" means approved by a regulatory agency of the Federal or a state government or listed in the U.S. Pharmacopeia or other generally recognized pharmacopeia for use in animals, and more particularly, in humans. The term "carrier" refers to a diluent, adjuvant, excipient, or vehicle with which the VEGF antagonist is administered. A multitude of appropriate formulations can be found in the formulary known to all pharmaceutical chemists: Remington's Pharmaceutical Sciences (15th ed, Mack Publishing Company, Easton, Pa., 1975), *e.g.*, Chapter 87 by Blaug, Seymour, therein.

Pharmaceutical formulations for use in a method of the present invention can be "high concentration". High concentration pharmaceutical formulations of the present invention include VEGF antagonist, *e.g.*, VEGF receptor fusion protein, at a concentration of at least 41 mg/ml, of at least 80 mg/ml, of at least 100 mg/ml, of at least 125 mg/ml, of at least 140 mg/ml, of at least 150 mg/ml, of at least 175 mg/ml, of at least 200 mg/ml, of at least 225 mg/ml, of at least 250 mg/ml, or of at least 275 mg/ml. "High concentration" can refer to formulations that include a concentration of VEGF antagonist of from about 140 mg/ml to about 160 mg/ml, at least about 140 mg/ml but less than 160 mg/ml, from about 41 mg/ml to about 275 mg/ml, from about 70 mg/ml to about 75 mg/ml or from about 80 mg/ml to about 250 mg/ml. In some aspects, the VEGF antagonist concentration in the formulation is about any of the following concentrations: 41 mg/ml; 42 mg/ml; 43 mg/ml; 44 mg/ml; 45 mg/ml; 46 mg/ml; 47 mg/ml; 48 mg/ml; 49 mg/ml; 50mg/ml; 51 mg/ml; 52 mg/ml; 53 mg/ml; 54 mg/ml; 55 mg/ml; 56 mg/ml; 57 mg/ml; 58 mg/ml; 59 mg/ml; 60 mg/ml; 61 mg/ml; 62 mg/ml; 63 mg/ml; 64 mg/ml; 65 mg/ml; 66 mg/ml; 67 mg/ml; 68 mg/ml; 69 mg/ml; 70 mg/ml; 71 mg/ml; 72 mg/ml; 73 mg/ml; 74 mg/ml; 75 mg/ml; 76 mg/ml; 77 mg/ml; 78 mg/ml; 79 mg/ml; 80 mg/ml; 81 mg/ml; 82mg/ml; 83 mg/ml; 84 mg/ml; 85 mg/ml; 86 mg/ml; 87mg/ml; 88 mg/ml; 89 mg/ml; 90 mg/ml; 91 mg/ml; 92 mg/ml; 93 mg/ml; 94 mg/ml; 95mg/ml; 96 mg/ml; 97 mg/ml; 98 mg/ml; 99 mg/ml; 100 mg/ml; 101 mg/ml; 102 mg/ml; 103 mg/ml; 104 mg/ml; 105 mg/ml; 106mg/ml; 107 mg/ml; 108 mg/ml; 109 mg/ml; 110 mg/ml; 111 mg/ml; 112 mg/ml; 113 mg/ml; 113.3 mg/ml; 114 mg/ml; 114.1 mg/ml; 114.2 mg/ml; 114.3 mg/ml; 114.4 mg/ml; 114.5 mg/ml; 114.6 mg/ml, 114.7 mg/ml, 114.8 mg/ml; 114.9 mg/ml; 115 mg/ml; 116 mg/ml; 117 mg/ml; 118 mg/ml; 119 mg/ml; 120 mg/ml; 121 mg/ml; 122 mg/ml; 123 mg/ml; 124 mg/ml; 125 mg/ml; 126 mg/ml; 127mg/ml; 128 mg/ml; 129 mg/ml; 130 mg/ml; 131 mg/ml; 132 mg/ml; 133 mg/ml; 133.3 mg/ml; 133.4 mg/ml, 134 mg/ml; 135 mg/ml; 136 mg/ml; 137 mg/ml; 138 mg/ml; 139 mg/ml; 140 mg/ml; 141 mg/ml; 142 mg/ml; 143 mg/ml; 144 mg/ml; 145 mg/ml; 146 mg/ml; 147 mg/ml; 148 mg/ml; 149 mg/ml; 150 mg/ml; 151 mg/ml; 152 mg/ml; 153 mg/ml; 154mg/ml; 155 mg/ml; 156 mg/ml; 157mg/ml; 158 mg/ml; 159 mg/ml; 160 mg/ml; 161 mg/ml; 162 mg/ml; 163 mg/ml; 164 mg/ml; 165 mg/ml; 166 mg/ml; 167 mg/ml; 168 mg/ml; 169 mg/ml; 170 mg/ml; 171 mg/ml; 172 mg/ml; 173 mg/ml; 174 mg/ml; 175 mg/ml; 176 mg/ml; 177 mg/ml; 178 mg/ml; 179 mg/ml; 180 mg/ml; 181 mg/ml; 182 mg/ml; 183 mg/ml; 184 mg/ml; 185 mg/ml; 186 mg/ml; 187 mg/ml; 188 mg/ml; 189 mg/ml; 190 mg/ml; 191 mg/ml; 192 mg/ml; 193 mg/ml; 194 mg/ml; 195 mg/ml; 196 mg/ml; 197 mg/ml; 198 mg/ml; 199 mg/ml; 200 mg/ml; 201 mg/ml; 202 mg/ml; 203 mg/ml; 204 mg/ml; 205 mg/ml; 206 mg/ml; 207 mg/ml; 208 mg/ml; 209 mg/ml; 210 mg/ml; 211 mg/ml; 212 mg/ml; 213 mg/ml; 214 mg/ml; 215 mg/ml; 216 mg/ml; 217 mg/ml; 218 mg/ml; 219 mg/ml; 220 mg/ml; 221 mg/ml; 222 mg/ml; 223 mg/ml; 224 mg/ml; 225 mg/ml; 226 mg/ml; 227 mg/ml; 228 mg/ml; 229 mg/ml; 230 mg/ml; 231 mg/ml; 232 mg/ml; 233 mg/ml; 234 mg/ml; 235 mg/ml; 236 mg/ml; 237mg/ml; 238 mg/ml; 239 mg/ml; 240 mg/ml; 241 mg/ml; 242 mg/ml; 243 mg/ml; 244 mg/ml; 245 mg/ml; 246 mg/ml; 247 mg/ml; 248 mg/ml; 249 mg/ml; 250 mg/ml; 251 mg/ml; 252 mg/ml; 253 mg/ml; 254 mg/ml; 255 mg/ml; 256 mg/ml; 257 mg/ml; 258 mg/ml; 259 mg/ml; 260 mg/ml; 261 mg/ml; 262 mg/ml; 263 mg/ml; 264 mg/ml; 265 mg/ml; 266 mg/ml; 267 mg/ml; 268 mg/ml; 269 mg/ml; 270 mg/ml; 271 mg/ml; 272 mg/ml; 273 mg/ml; 274 mg/ml; or 275 mg/ml. Other VEGF antagonist concentrations are contemplated herein, as long as the concentration functions in accordance with embodiments herein.

In an embodiment of the invention, a pharmaceutical formulation for use in a method of the present invention is of such a concentration as to contain about 4, 6, 8, 10, 12, 14, 16, 18 or 20 mg VEGF receptor fusion protein (*e*.*g*., aflibercept), or the amount of such protein in any of the acceptable doses thereof which are discussed herein, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl.

The present invention includes methods of using (as discussed herein) any of the formulations set forth under "*Illustrative Formulations*" herein, but wherein the concentration of the VEGF receptor fusion protein (*e*.*g*., aflibercept) is substituted with a concentration which is set forth in this section ("VEGF Receptor Fusion Proteins and Other VEGF inhibitors").

Buffers for use in pharmaceutical formulations herein that may be used in a method of the present invention refer to solutions that resist pH change by use of acid-base conjugates. Buffers are capable of maintaining pH in the range of from about 5.0 to about 6.8, and more typically, from about 5.8 to about 6.5, and most typically, from about 6.0 to about 6.5. In some cases, the pH of the formulation of the present invention is about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, or about 6.8. Example buffers for inclusion in formulations herein include histidine-based buffers, for example, histidine with histidine hydrochloride or histidine acetate. Buffers for inclusion in formulations herein can alternatively be phosphate-based buffers, for example, comprising sodium phosphate, acetate-based buffers, for example, comprising sodium acetate or acetic acid, or can be citrate-based, for example, comprising sodium citrate or citric acid. It is also recognized that buffers can be a mix of the above, as long as the buffer functions to buffer the formulations in the above-described pH ranges. In some cases, the buffer is from about 5 mM to about 25 mM, or more typically, about 5 mM to about 15 mM. Buffers can be about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, or about 25 mM.

In an embodiment of the invention, a histidine-based buffer is prepared using histidine and histidine monohydrochloride.

Surfactant for use herein refers to ingredients that protect the higher concentration of VEGF antagonist, *e.g.*, VEGF receptor fusion protein, from various surface and interfacial induced stresses. As such, surfactants can be used to limit or minimize VEGF receptor fusion protein aggregation, and promote protein solubility. Suitable surfactants herein have been shown to be non-ionic, and can include surfactants that have a polyoxyethylene moiety. Illustrative surfactants in this category include: polysorbate 20, polysorbate 80, poloxamer 188, polyethylene glycol 3350, and mixtures thereof. Surfactants in the formulations can be present at from about 0.02% to about 0.1% weight per volume (w/v), and more typically, about 0.02% to about 0.04% (w/v). In some cases, the surfactant is about 0.02% (w/v), about 0.03% (w/v), about 0.04% (w/v), about 0.05% (w/v), about 0.06% (w/v), about 0.07% (w/v), about 0.08% (w/v), about 0.09% (w/v), or about 0.1% (w/v).

Thermal stabilizers for use in pharmaceutical formulations that may be used in methods set forth herein refers to ingredients that provide thermal stability against thermal denaturation of the VEGF antagonist, *e.g.*, VEGF receptor fusion protein, as well as protect against loss of VEGF receptor fusion protein potency or activity. Suitable thermal stabilizers include sugars, and can be sucrose, trehalose, sorbitol or mannitol, or can be amino acids, for example L-proline, L-arginine (*e.g.*, L-arginine monohydrochloride), or taurine. Additionally, thermal stabilizers may also include substituted acrylamides or propane sulfonic acid, or may be compounds like glycerol.

In some cases, the pharmaceutical formulations for use in a method herein include both a sugar and taurine, a sugar and an amino acid, a sugar and propane sulfonic acid, a sugar and taurine, glycerol and taurine, glycerol and propane sulfonic acid, an amino acid and taurine, or an amino acid and propane sulfonic acid. In addition, formulations can include a sugar, taurine and propane sulfonic acid, glycerol, taurine and propane sulfonic acid, as well as L-proline, taurine and propane sulfonic acid.

Embodiments herein may have thermal stabilizers present alone, each independently present at a concentration of, or present in combination at a total concentration of, from about 2% (w/v) to about 10% (w/v) or 4% (w/v) to about 10% (w/v), or about 4% (w/v) to about 9% (w/v), or about 5% (w/v) to about 8% (w/v). Thermal stabilizers in the formulation can be at a concentration of about 2% (w/v), about 2.5% (w/v), about 3% (w/v), about 4% (w/v), about 5% (w/v), about 6% (w/v), about 7% (w/v), about 8% (w/v), about 9% (w/v), about 10% (w/v) or about 20% (w/v).

With respect to taurine and propane sulfonic acid, in an embodiment of the invention, these thermal stabilizers can be present in the formulations at about from 25 mM to about 100 mM, and more typically from about 50 mM to about 75 mM (as compared to the other thermal stabilizers).

Viscosity reducing agents typically are used to reduce or prevent protein aggregation. Viscosity reducing agents for inclusion herein include: sodium chloride, magnesium chloride, D- or L-arginine (*e.g.*, L-arginine monohydrochloride), lysine, or mixtures thereof. When present herein, viscosity reducing agents can be present at from about 10 mM to about 100 mM, and more typically from about 30 mM to about 75 mM, and even more typically from about 40 mM to about 70 mM. In some cases, the viscosity reducing agent is present at about 10 mM, about 15 mM, about 20 mM, about 25 mM, about 30 mM, about 35 mM, about 40 mM, about 45 mM, about 50 mM, about 55 mM, about 60 mM, about 65 mM, about 70 mM, about 75 mM, about 80 mM, about 85 mM, about 90 mM, about 95 mM or about 100 mM.

Pharmaceutical *f*ormulations for use in a method as set forth herein can also have a pharmaceutically acceptable viscosity for ocular administration, for example, intravitreal injection. Viscosity generally refers to the measure of resistance of a fluid which is being deformed by either shear stress or tensile stress (typically measured by techniques known in the art, viscometer or rheometer, for example). Typical viscosities of formulations for use in a method set forth herein are from about 5.0 cP (centipoise) to about 15 cP, from about 11 cP to about 14 cP, from about 12 cP to about 15 cP or from about 11 cP to about 12 cP. As such, formulation viscosity herein can be about 5.0 cP, about 6.0, about 7.1 cP, about 7.2 cP, about 7.3 cP, about 7.4 cP, about 7.5 cP, about 7.6 cP, about 10 cP, about 10.5 cP, about 11.0 cP, about 11.5 cP, about 12.0 cP, about 12.5 cP, about 13.0 cP, about 13.5 cP, about 14.0 cP, about 14.5 cP, or about 15.0 cP (*e.g.*, when measured at 20°C).

Various embodiments herein do not require inclusion of an inorganic salt, or other viscosity reducing agent, to maintain these highly useful viscosities. Typically, high concentration protein solutions require viscosity reducing agents to avoid protein aggregation and higher viscosity, making the formulations difficult for intravitreal injection and reducing the potency of the VEGF receptor fusion protein. As such, embodiments herein include methods of using formulations that have had substantially no, or no added, sodium chloride (NaCl), magnesium chloride (MgCl₂), D- or L-arginine (*e.g.*, D- or L- arginine hydrochloride), lysine or other viscosity reducing agent.

Osmolality is a critical attribute for injectable pharmaceutical formulations for use in a method of the present invention. It is desirable to have products match physiological osmotic conditions. Furthermore, osmolality provides confirmation of soluble content in solution. In an embodiment of the invention, the osmolality of a formulation for use in a method of the present invention is less than or equal to about 506 mmol/Kg or from about 250 to about 506 mmol/Kg., *e.g.*, about 250, 260, 270, 280, 290, 299, 300, 310, 314, 315, 316, 324, 343, 346, 349, 369, 384, 403, 426, 430 or 506 mmol/Kg. In an embodiment of the invention, the osmolality is lower than about 250 mmol/Kg.

Illustrative pharmaceutical formulations for use in the methods of the present invention include the following:
Formulation A: 80 mg/ml aflibercept, 10 mM histidine-based buffer, 5 % (w/v) sucrose, 0.03% (w/v) polysorbate 20, and 40 mM sodium chloride, with a pH of 5.8 to 6.2.
Formulation B: 80 mg/ml aflibercept, 10 mM phosphate-based buffer, 5 % (w/v) sucrose, 0.03% (w/v) polysorbate 20, and 40 mM sodium chloride, with a pH of 5.8 to 6.2.
Formulation C: 80 mg/ml aflibercept, 10 mM citrate-based buffer, 5 % (w/v) sucrose, 0.03% (w/v) polysorbate 20, and 40 mM sodium chloride, with a pH of 5.8 to 6.2.
Formulation D: 80 mg/ml aflibercept, 10 mM histidine-based buffer, 5 % (w/v) sucrose, 0.03 % (w/v) polysorbate 80, and 40 mM sodium chloride, with a pH of 6.2.
Formulation E: 80 mg/ml aflibercept, 10 mM phosphate-based buffer, 5 % (w/v) sucrose, 0.03 % (w/v) polysorbate 80, and 40 mM sodium chloride, with a pH of 5.8 to 6.2.
Formulation F: 80 mg/ml aflibercept, 10 mM citrate-based buffer, 5 % (w/v) sucrose, 0.03 % (w/v) polysorbate 80, and 40 mM sodium chloride, with a pH of 5.8 to 6.2.
Formulation G: 80 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation H: 80 mg/ml aflibercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation I: 80 mg/ml aflibercept, 10 mM citrate-based buffer, 8 % (w/v) sucrose, and 0.0 3% (w/v) polysorbate 20, with a pH of 5.8 to 6.2, and, optionally, specifically excluding a viscosity reducing agent.
Formulation J: 80 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation K: 80 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation L: 80 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation M: 150 mg/ml aflibercept, 10 mM histidine-based buffer, 5 % (w/v) sucrose, 0.03 % (w/v) polysorbate 20, and 40 mM sodium chloride, with a pH of 5.8 to 6.2.
Formulation N: 150 mg/ml aflibercept, 10 mM phosphate-based buffer, 5 % (w/v) sucrose, 0.03 % (w/v) polysorbate 20, and 40 mM sodium chloride, with a pH of 5.8 to 6.2.
Formulation O: 150 mg/ml aflibercept, 10 mM citrate-based buffer, 5 % (w/v) sucrose, 0.03 % (w/v) polysorbate 20, and 40 mM sodium chloride, with a pH of 5.8 to 6.2.
Formulation P: 150 mg/ml aflibercept, 10 mM histidine-based buffer, 5 % (w/v) sucrose, 0.03 % (w/v) polysorbate 80, and 40 mM sodium chloride, with a pH of 6.2.
Formulation Q: 150 mg/ml aflibercept, 10 mM phosphate-based buffer, 5 % (w/v) sucrose, 0.03 % (w/v) polysorbate 80, and 40 mM sodium chloride, with a pH of 5.8 to 6.2.
Formulation R: 150 mg/ml aflibercept, 10 mM citrate-based buffer, 5 % (w/v) sucrose, 0.03 % (w/v) polysorbate 80, and 40 mM sodium chloride, with a pH of 5.8 to 6.2.
Formulation S: 150 mg/ml aflibercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation T: 150 mg/ml aflibercept, 10 mM phosphate-based buffer, 8 % (w/v) sucrose, and 0.03 % (w/v) polysorbate 20, with a pH of 5.8 to 6.2 (*e.g.*, 6.2), and, optionally, specifically excluding a viscosity reducing agent.
Formulation U: 150 mg/ml aflibercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation V: 150 mg/ml aflibercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation W: 150 mg/ml aflibercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation X: 150 mg/ml aflibercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation Y: 80 mg/ml conbercept, 10 mM histidine-based buffer, 5 % (w/v) sucrose, 0.03 % (w/v) polysorbate 20, and 40 mM sodium chloride, with a pH of 5.8 to 6.2.
Formulation Z: 80 mg/ml conbercept, 10 mM phosphate-based buffer, 5 % (w/v) sucrose, 0.03 % (w/v) polysorbate 20, and 40 mM sodium chloride, with a pH of 5.8 to 6.2.
Formulation AA: 80 mg/ml conbercept, 10 mM citrate-based buffer, 5 % (w/v) sucrose, 0.03 % (w/v) polysorbate 20, and 40 mM sodium chloride, with a pH of 5.8 to 6.2.
Formulation BB: 80 mg/ml conbercept, 10 mM histidine-based buffer, 5 % (w/v) sucrose, 0.03 % (w/v) polysorbate 80, and 40 mM sodium chloride, with a pH of 6.2.
Formulation CC: 80 mg/ml conbercept, 10 mM phosphate-based buffer, 5 % (w/v) sucrose, 0.03 % (w/v) polysorbate 80, and 40 mM sodium chloride, with a pH of 5.8 to 6.2.
Formulation DD: 80 mg/ml conbercept, 10 mM citrate-based buffer, 5 % (w/v) sucrose, 0.03 % (w/v) polysorbate 80, and 40 mM sodium chloride, with a pH of 5.8 to 6.2.
Formulation EE: 80 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation FF: 80 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation GG: 80 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation HH: 80 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation II: 80 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation JJ: 80 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation KK: 150 mg/ml conbercept, 10 mM histidine-based buffer, 5 % (w/v) sucrose, 0.03 % (w/v) polysorbate 20, and 40 mM sodium chloride, with a pH of 5.8 to 6.2.
Formulation LL: 150 mg/ml conbercept, 10 mM phosphate-based buffer, 5 % (w/v) sucrose, 0.03 % (w/v) polysorbate 20, and 40 mM sodium chloride, with a pH of 5.8 to 6.2.
Formulation MM: 150 mg/ml conbercept, 10 mM citrate-based buffer, 5 % (w/v) sucrose, 0.03 % (w/v) polysorbate 20, and 40 mM sodium chloride, with a pH of 5.8 to 6.2.
Formulation NN: 150 mg/ml conbercept, 10 mM histidine-based buffer, 5 % (w/v) sucrose, 0.03 % (w/v) polysorbate 80, and 40 mM sodium chloride, with a pH of 6.2.
Formulation OO: 150 mg/ml conbercept, 10 mM phosphate-based buffer, 5 % (w/v) sucrose, 0.03 % (w/v) polysorbate 80, and 40 mM sodium chloride, with a pH of 5.8 to 6.2.
Formulation PP: 150 mg/ml conbercept, 10 mM citrate-based buffer, 5 % (w/v) sucrose, 0.03 % (w/v) polysorbate 80, and 40 mM sodium chloride, with a pH of 5.8 to 6.2.
Formulation QQ: 150 mg/ml conbercept, 10 mM histidine-based buffer, 8 % (w/v) sucrose, and 0.03 % (w/v) polysorbate 20, with a pH of 5.8 to 6.2, and, optionally, specifically excluding a viscosity reducing agent.
Formulation RR: 150 mg/ml conbercept, 10 mM phosphate-based buffer, 8 % (w/v) sucrose, and 0.03 % (w/v) polysorbate 20, with a pH of 5.8 to 6.2, and, optionally, specifically excluding a viscosity reducing agent.
Formulation SS: 150 mg/ml conbercept, 10 mM citrate-based buffer, 8 % (w/v) sucrose, and 0.03 % (w/v) polysorbate 20, with a pH of 5.8 to 6.2, and, optionally, specifically excluding a viscosity reducing agent.
Formulation TT: 150 mg/ml conbercept, 10 mM histidine-based buffer, 8 % (w/v) sucrose, and 0.03 % (w/v) polysorbate 80, with a pH of 5.8 to 6.2, and, optionally, specifically excluding a viscosity reducing agent.
Formulation UU: 150 mg/ml conbercept, 10 mM phosphate-based buffer, 8 % (w/v) sucrose, and 0.03 % (w/v) polysorbate 80, with a pH of 5.8 to 6.2, and, optionally, specifically excluding a viscosity reducing agent.
Formulation VV: 150 mg/ml conbercept, 10 mM citrate-based buffer, 8 % (w/v) sucrose, and 0.03 % (w/v) polysorbate 80, with a pH of 5.8 to 6.2, and, optionally, specifically excluding a viscosity reducing agent.
Formulation WW: 140 mg/ml VEGF receptor fusion protein (*e.g.*, aflibercept), 10 mM histidine-based buffer, 5 % (w/v) sucrose, 0.03 % (w/v) polysorbate 20, and 50 mM taurine, with a pH of 5.8.
Formulation XX: 140 mg/ml VEGF receptor fusion protein (*e*.*g*., aflibercept), 20 mM histidine-based buffer, 4 % (w/v) proline, 0.03 % (w/v) polysorbate 20, and 50 mM arginine (*e*.*g*., arginine hydrochloride), with a pH of 5.8.
Formulation YY: 140 mg/ml VEGF receptor fusion protein (*e*.*g*., aflibercept), 20 mM histidine-based buffer, 2.5 % (w/v) sucrose, 2.0 % (w/v) proline, 0.03 % (w/v) polysorbate 20, and 50 mM taurine, with a pH of 5.8.
Formulation ZZ: 140 mg/ml VEGF receptor fusion protein (*e*.*g*., aflibercept), 10 mM histidine-based buffer, 2.5 % (w/v) sucrose, 2.0 % (w/v) proline, 0.03 % (w/v) polysorbate 20, and 50 mM arginine (*e*.*g*., arginine hydrochloride), with a pH of 5.8.
Formulation AAA: 140 mg/ml VEGF receptor fusion protein (*e*.*g*., aflibercept), 20 mM histidine-based buffer, 5 % (w/v) sucrose, 0.03% (w/v) polysorbate 20, and 50 mM PSA, with a pH of 5.8.
Formulation BBB: 140 mg/ml VEGF receptor fusion protein (*e*.*g*., aflibercept), 20 mM histidine-based buffer, 2.5 % (w/v) sucrose, 2.0 % (w/v) proline, 0.03 % (w/v) polysorbate 20, and 50 mM PSA, with a pH of 5.8.
Formulation CCC: 80, 100, 120 or 140 mg/ml VEGF receptor fusion protein (*e*.*g*., aflibercept), 20 mM histidine-based buffer, 5 % (w/v) sucrose, 0.03 % (w/v) polysorbate 20, and 50 mM arginine (*e*.*g*., arginine hydrochloride), with a pH of 5.8.
Formulation DDD: 140 mg/ml VEGF receptor fusion protein (*e*.*g*., aflibercept), 10 mM histidine-based buffer, 4 % (w/v) proline, 0.03 % (w/v) polysorbate 20, and 50 mM PSA, with a pH of 5.8.
Formulation EEE: 140 mg/ml VEGF receptor fusion protein (*e*.*g*., aflibercept), 20 mM histidine-based buffer, 5 % (w/v) sucrose, and 0.03 % (w/v) polysorbate 20 and, optionally, no thermal stabilizer, with a pH of 5.8.
Formulation FFF: 140 mg/ml VEGF receptor fusion protein (*e*.*g*., aflibercept), 10mM sodium phosphate, 5 % (w/v) sucrose and 0.03 % polysorbate 20 with a pH of 6.2.
Formulation GGG: 140 mg/ml VEGF receptor fusion protein (*e*.*g*., aflibercept); 20 mM histidine, pH 5.8; 5% sucrose; 0.03 % polysorbate 20; 50 mM sodium sulfate
Formulation HHH: 140 mg/ml VEGF receptor fusion protein (*e*.*g*., aflibercept); 20 mM histidine, pH 5.8; 5% sucrose; 0.03 % polysorbate 20; 50 mM sodium thiocyanate
Formulation III: 140 mg/ml VEGF receptor fusion protein (*e*.*g*., aflibercept); 20 mM histidine, pH 5.8; 5 % sucrose, 0.03 % polysorbate 20; 40 mM sodium citrate
Formulation JJJ: 140 mg/ml VEGF receptor fusion protein (*e*.*g*., aflibercept); 20 mM histidine, pH 5.8; 5% Sucrose, 0.03 % polysorbate 20; 50 mM glycine
Formulation KKK: 140 mg/ml VEGF receptor fusion protein (*e*.*g*., aflibercept); 20 mM histidine, pH 5.8; 5 % sucrose, 0.03 % polysorbate 20; 50 mM sodium chloride
Formulation LLL: 140 mg/ml VEGF receptor fusion protein (*e*.*g*., aflibercept); 20 mM histidine, pH 5.8; 5 % sucrose; 0.03 % polysorbate 20; 50 mM lysine
Formulation MMM: 140 mg/ml VEGF receptor fusion protein (*e*.*g*., aflibercept); 20 mM histidine, pH 5.8; 5 % sucrose; 0.03% polysorbate 20; 50 mM sodium aspartate
Formulation NNN: 140 mg/ml VEGF receptor fusion protein (*e*.*g*., aflibercept); 20 mM histidine, pH 5.8; 5 % sucrose; 0.03 % polysorbate 20; 50 mM sodium glutamate
Formulation OOO: 140 mg/ml VEGF receptor fusion protein (*e*.*g*., aflibercept); 20 mM histidine, pH 5.8; 5 % sucrose; 0.03% polysorbate 20; 50 mM sodium citrate; 50 mM arginine (*e*.*g*., arginine hydrochloride)
Formulation PPP: 140 mg/ml VEGF receptor fusion protein (*e*.*g*., aflibercept); 20 mM histidine, pH 5.8; 5 % sucrose; 0.03% polysorbate 20; 50 mM glycine; 50 mM arginine (*e*.*g*., arginine hydrochloride)
Formulation QQQ: 140 mg/ml VEGF receptor fusion protein (*e*.*g*., aflibercept); 20 mM histidine, pH 5.8; 5 % sucrose; 0.03% polysorbate 20; 50 mM sodium aspartate; 50 mM arginine (*e*.*g*., arginine hydrochloride)
Formulation RRR: 140 mg/ml VEGF receptor fusion protein (*e*.*g*., aflibercept); 20 mM histidine, pH 5.8; 5 % sucrose; 0.03% polysorbate 20; 50 mM sodium glutamate; 50 mM arginine (*e*.*g*., arginine hydrochloride)
Formulation SSS: 140 mg/ml VEGF receptor fusion protein (*e*.*g*., aflibercept); 20 mM His, pH 5.8; 5 % sucrose; 0.03 % polysorbate 20; 10 mM L-arginine (L-arginine hydrochloride)
Formulation TTT: 140 mg/ml VEGF receptor fusion protein (*e*.*g*., aflibercept); 20 mM His, pH 5.8; 5 % sucrose; 0.03 % polysorbate 20; 100 L-arginine (L-arginine hydrochloride)
Formulation UUU: 30 mg/ml VEGF receptor fusion protein (*e*.*g*., aflibercept), 10 % sucrose, 10 mM phosphate, 0.03 % polysorbate 20, pH 6.2
Formulation VVV: 30 mg/ml VEGF receptor fusion protein (*e*.*g*., aflibercept), 20 % sucrose, 10 mM phosphate, 0.03 % polysorbate 20, pH 6.2
Formulation WWW: 60 mg/ml VEGF receptor fusion protein (*e*.*g*., aflibercept), 10 % sucrose, 10 mM phosphate, 0.03 % polysorbate 20, pH 6.2
Formulation XXX: 60 mg/ml VEGF receptor fusion protein (*e*.*g*., aflibercept), 20 % sucrose, 10 mM phosphate, 0.03 % polysorbate 20, pH 6.2
Formulation YYY: 120 mg/ml VEGF receptor fusion protein (*e*.*g*., aflibercept), 10 % sucrose, 10 mM phosphate, 0.03 % polysorbate 20, pH 6.2
Formulation ZZZ: 120 mg/ml VEGF receptor fusion protein (*e*.*g*., aflibercept), 20 % sucrose, 10 mM phosphate, 0.03 % polysorbate 20, pH 6.2
Formulation AAAA: 120 mg/ml VEGF receptor fusion protein (*e*.*g*., aflibercept), 10 % sucrose, 10 mM phosphate, 0.03 % polysorbate 20, 50 mM NaCl, pH 6.2
Formulation BBBB: 120 mg/ml VEGF receptor fusion protein (*e*.*g*., aflibercept), 20 % sucrose, 10 mM phosphate, 0.03 % polysorbate 20, 50 mM NaCl, pH 6.2
Formulation CCCC: 140 mg/ml VEGF receptor fusion protein (*e*.*g*., aflibercept), 10 mM sodium phosphate, 5 % sucrose, 40 mM sodium chloride, 0.03 % PS20, pH 6.2
Formulation DDDD: 80 mg/ml VEGF receptor fusion protein (*e*.*g*., aflibercept), 20 mM histidine-based buffer, 5 % (w/v) sucrose, 0.03 % (w/v) polysorbate 20, and 50 mM L-arginine (L-arginine monohydrochloride), with a pH of 5.8.
Formulation EEEE: 120.0 mg/ml VEGF receptor fusion protein (*e*.*g*., aflibercept) (*e*.*g*., ± 12 mg/ml), 20 mM histidine-based buffer (*e*.*g*., ± 2 mM), 5 % (w/v) sucrose (*e.g*., ± 0.5%), 0.03 % (w/v) polysorbate 20 (*e*.*g*., 0.02-0.04%), and 50 mM L-arginine (L-arginine monohydrochloride) (*e*.*g*., ± 5 mM), with a pH of 5.8 (*e*.*g*., 5.6-6.0 or 5.5-6.1).
Formulation FFFF: 113.3 mg/ml VEGF receptor fusion protein (*e*.*g*., aflibercept) (*e*.*g*., 102-125 mg/ml), 20 mM histidine-based buffer (*e*.*g*., ± 2 mM), 5 % (w/v) sucrose (*e.g.*, ± 0.5%), 0.03 % (w/v) polysorbate 20 (*e*.*g*., 0.02-0.04%), and 50 mM L-arginine (L-arginine monohydrochloride) (*e*.*g*., ± 5 mM), with a pH of 5.8 (*e*.*g*., 5.6-6.0 or 5.5-6.1).
Formulation GGGG: 114.3 mg/ml VEGF receptor fusion protein (*e.g.*, aflibercept) (*e.g.*, 103-126 mg/ml), 10 mM histidine-based buffer, for example, including Histidine and Histine-HCl (*e.g.*, + 1 mM), 5 % (w/v) sucrose (*e.g.*, + 0.5%), 0.03 % (w/v) polysorbate 20 (*e.g.*, 0.02-0.04%), and 50 mM L-arginine (*e.g.*, L-arginine monohydrochloride) (*e.g.*, + 5 mM), with a pH of 5.8 (*e.g.*, 5.6-6.0 or 5.5-6.1).
Formulation HHHH: 100.0 mg/ml VEGF receptor fusion protein (*e.g.*, aflibercept) (*e.g.*, + 10 mg/ml), 20 mM histidine-based buffer (*e.g.*, + 2 mM), 5 % (w/v) sucrose (*e.g.*, + 0.5%), 0.03 % (w/v) polysorbate 20 (*e.g.*, 0.02-0.04%), and 50 mM L-arginine (L-arginine monohydrochloride) (*e.g.*, + 5 mM), with a pH of 5.8 (*e.g.*, 5.6-6.0 or 5.5-6.1).
Formulation IIII: 133.3 mg/ml VEGF receptor fusion protein (*e.g.*, aflibercept) (*e.g.*, + 13 mg/ml), 20 mM histidine-based buffer (*e.g.*, ± 2 mM), 5 % (w/v) sucrose (*e.g.*, ± 0.5%), 0.03 % (w/v) polysorbate 20 (*e.g.*, 0.02-0.04%), and 50 mM L-arginine (L-arginine monohydrochloride) (*e.g.*, ± 5 mM), with a pH of 5.8 (*e.g.*, 5.6-6.0 or 5.5-6.1).
Formulation JJJJ: 150 mg/ml aflibercept (*e.g.*, aflibercept) (*e.g.*, ± 15 mg/ml), 10 mM sodium phosphate, 8% (w/v) sucrose (*e.g.*, ± 0.8%), 0.03% (w/v) polysorbate 20 (*e.g.*, 0.02-0.04%) and 50 mM L-arginine (L-arginine hydrochloride), pH 6.2 (*e.g.*, 6.0-6.4 or 5.9-6.5).
Formulation KKKK: 114.3 mg/ml VEGF receptor fusion protein (*e.g.*, aflibercept) (*e.g.*, ± 14 mg/ml), 20 mM histidine-based buffer (*e.g.*, ± 2 mM), 5% (w/v) sucrose (*e.g.*, ± 0.5%), 0.03% (w/v) polysorbate 20 (*e.g.*, 0.02-0.04%), and 50 mM L-arginine (L-arginine monohydrochloride) (*e.g.*, ± 5 mM), with a pH of 5.8 (*e.g.*, 5.6-6.0 or 5.5-6.1);

See International Patent Application Publication No. WO2019/217927.

In an embodiment of the invention, the ≥8 mg VEGF receptor fusion protein, preferably aflibercept, when administered, is in an aqueous pharmaceutical formulation comprising: a VEGF receptor fusion protein comprising two polypeptides that each comprises an immunoglobin-like (Ig) domain 2 of VEGFR1, an Ig domain 3 of VEGFR2, and a multimerizing component (*e.g.*, which comprises amino acids 27-457 of SEQ ID NO: 2) at a concentration of at least about 100 mg/ml; about 5% sucrose; L-arginine (*e.g.*, L-arginine monohydrochloride); a histidine-based buffer (*e.g.*, containing histidine HCl); and about 0.03% surfactant; wherein the formulation has a pH of about 5.0 to about 6.8 (*e.g.*, 5.8 to 6.5, for example 5.8). Preferably the formulation is suitable for intravitreal administration. Other components that may be included are sodium sulfate, sodium thiocyanate, glycine, NaCl, sodium aspartate and/or sodium glutamate. In an embodiment of the invention, the VEGF receptor fusion protein is at a concentration of: about 100 mg/ml; about 111.5 mg/ml; about 112.0 mg/ml; about 113.3 mg/ml; about 114.3 mg/ml; about 115.6 mg/ml; about 116.3 mg/ml; about 120 mg/ml; about 133 mg/ml; about 140 mg/ml; about 150 mg/ml; about 200 mg/ml; or about 250 mg/ml. The formulation may be characterized by (i) an osmolality of about 299 to about 506 mmol/Kg; and/or (ii) a viscosity of from about 6-15 cP at 20°C. The surfactant may be a non-ionic surfactant such as polysorbate 20, polysorbate 80, poloxamer 188, polyethylene glycol 3350 or mixtures thereof. The histidine-based buffer may be at a concentration of about 10 mM to 20 mM. In an embodiment of the invention, the VEGF receptor fusion protein has less than about 3.5% high molecular weight species immediately after manufacture and purification and/or less than or equal to about 6% high molecular weight species after storage for about 24 months at about 2-8°C.

In an embodiment of the invention, the ≥8 mg VEGF receptor fusion protein is, when administered in an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.

In an embodiment of the invention, the aqueous formulation includes:
- ≥ about 100 mg/ml VEGF receptor fusion protein (*e*.*g*., aflibercept), histidine-based buffer and L-arginine;
- about 140 mg/ml aflibercept; 20 mM histidine-based buffer; 5% sucrose; 0.03% polysorbate 20; 10 mM L-arginine; pH 5.8;
- about 150 ± 15 mg/ml aflibercept, 10 mM phosphate-based buffer, 8 ± 0.8% (w/v) sucrose, 0.02-0.04% (w/v) polysorbate 20 and 50 mM L-arginine, pH 5.9-6.5;
- about 103-126 mg/ml aflibercept, 10 ± 1 mM histidine-based buffer, 5 ± 0.5% (w/v) sucrose, 0.02-0.04% (w/v) polysorbate 20, and 50 ± 5 mM L-arginine, pH 5.5-6.1;
- about 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
- about 114.3 mg/ml aflibercept, 10 mM histidine-based buffer, 5% (w/v) sucrose, 0.03% (w/v) polysorbate 20 and 50 mM L-arginine, pH 5.8;
- ≥about 100 mg/ml aflibercept, histidine-based buffer and L-arginine;
- ≥about 100 mg/ml aflibercept at about pH 5.8, wherein the formulation forms <3% HMW aggregates after incubation at 5°C for 2 months;
- about 114.3 mg/mL aflibercept; 10 mM - 50 mM histidine-based buffer, sugar, non-ionic surfactant, L-Arginine, pH 5.8;
   or
- about 114.3 mg/mL aflibercept; 10 mM His/His-HCl-based buffer, 5% sucrose, 0.03% polysorbate-20, 50 mM L-Arginine, pH 5.8.

In an embodiment of the invention, the ≥8 mg VEGF receptor fusion protein is, when administered in an aqueous pharmaceutical formulation comprising
aflibercept at a concentration of at least about 100 mg/ml (*e*.*g*., about 111.5 mg/ml; 112.0 mg/ml; 113.3 mg/ml; about 114.3 mg/ml; about 115.6 mg/ml; or about 116.3 mg/ml);
a thermal stabilizer which is a sugar, an amino acid, sucrose, mannitol, sorbitol, trehalose, L-proline, glycine, glycerol, taurine or propane sulfonic acid (*e*.*g*., at about 2% (w/v) to about 10% (w/v), for example, 5% (w/v));
a buffer which is a histidine-based buffer, a phosphate-based buffer, an acetate-based buffer (*e.g.*, at a concentration of about 5-25 mM, *e*.*g*., 10 mM or 20 mM); or a citrate-based buffer;
a non-ionic surfactant, such as for example, polyoxyethylene-based, polysorbate 20, polysorbate 80, poloxamer 188 or polyethylene glycol 3350 (*e*.*g*., at a concentration of about 0.02% to about 0.1 % (w/v), *e*.*g*., 0.03% (w/v)); and
a viscosity reducing agent which is NaCl, MgCl₂, D-arginine, L-arginine or lysine (*e*.*g*., at a concentration of about 10-100 mM, *e*.*g*., 50 mM),
wherein the formulation has a pH of about 5.0 to about 6.8 (*e*.*g*., 5.0-6.0 or 5.8).

In an embodiment of the invention, the aflibercept is at a concentration in the aqueous pharmaceutical formulation of about 100 mg/ml; 101 mg/ml; 102 mg/ml; 103 mg/ml; 104 mg/ml; 105 mg/ml; 106 mg/ml; 107 mg/ml; 108 mg/ml; 109 mg/ml; 110 mg/ml; 111 mg/ml; 112 mg/ml; 113 mg/ml; 113.3 mg/ml; 114 mg/ml; 114.1 mg/ml; 114.2 mg/ml; 114.3 mg/ml; 114.4 mg/ml; 114.5 mg/ml; 114.6 mg/ml, 114.7 mg/ml, 114.8 mg/ml; 114.9 mg/ml; 115 mg/ml; 116 mg/ml; 117 mg/ml; 118 mg/ml; 119 mg/ml; 120 mg/ml; 121 mg/ml; 122 mg/ml; 123 mg/ml; 124 mg/ml; 125 mg/ml; 126 mg/ml; 127mg/ml; 128 mg/ml; 129 mg/ml; 130 mg/ml; 131 mg/ml; 132 mg/ml; 133 mg/ml; 133.3 mg/ml; 133.4 mg/ml, 134 mg/ml; 135 mg/ml; 136 mg/ml; 137 mg/ml; 138 mg/ml; 139 mg/ml; 140 mg/ml; 141 mg/ml; 142 mg/ml; 143 mg/ml; 144 mg/ml; 145 mg/ml; 146 mg/ml; 147 mg/ml; 148 mg/ml; 149 mg/ml; 150 mg/ml; 151 mg/ml; 152 mg/ml; 153 mg/ml; 154mg/ml; 155 mg/ml; 156 mg/ml; 157mg/ml; 158 mg/ml; 159 mg/ml; 160 mg/ml; 161 mg/ml; 162 mg/ml; 163 mg/ml; 164 mg/ml; 165 mg/ml; 166 mg/ml; 167 mg/ml; 168 mg/ml; 169 mg/ml; 170 mg/ml; 171 mg/ml; 172 mg/ml; 173 mg/ml; 174 mg/ml; 175 mg/ml; 176 mg/ml; 177 mg/ml; 178 mg/ml; 179 mg/ml; 180 mg/ml; 181 mg/ml; 182 mg/ml; 183 mg/ml; 184 mg/ml; 185 mg/ml; 186 mg/ml; 187 mg/ml; 188 mg/ml; 189 mg/ml; 190 mg/ml; 191 mg/ml; 192 mg/ml; 193 mg/ml; 194 mg/ml; 195 mg/ml; 196 mg/ml; 197 mg/ml; 198 mg/ml; 199 mg/ml; 200 mg/ml; 201 mg/ml; 202 mg/ml; 203 mg/ml; 204 mg/ml; 205 mg/ml; 206 mg/ml; 207 mg/ml; 208 mg/ml; 209 mg/ml; 210 mg/ml; 211 mg/ml; 212 mg/ml; 213 mg/ml; 214 mg/ml; 215 mg/ml; 216 mg/ml; 217 mg/ml; 218 mg/ml; 219 mg/ml; 220 mg/ml; 221 mg/ml; 222 mg/ml; 223 mg/ml; 224 mg/ml; 225 mg/ml; 226 mg/ml; 227 mg/ml; 228 mg/ml; 229 mg/ml; 230 mg/ml; 231 mg/ml; 232 mg/ml; 233 mg/ml; 234 mg/ml; 235 mg/ml; 236 mg/ml; 237mg/ml; 238 mg/ml; 239 mg/ml; 240 mg/ml; 241 mg/ml; 242 mg/ml; 243 mg/ml; 244 mg/ml; 245 mg/ml; 246 mg/ml; 247 mg/ml; 248 mg/ml; 249 mg/ml; 250 mg/ml; 251 mg/ml; 252 mg/ml; 253 mg/ml; 254 mg/ml; 255 mg/ml; 256 mg/ml; 257 mg/ml; 258 mg/ml; 259 mg/ml; 260 mg/ml; 261 mg/ml; 262 mg/ml; 263 mg/ml; 264 mg/ml; 265 mg/ml; 266 mg/ml; 267 mg/ml; 268 mg/ml; 269 mg/ml; 270 mg/ml; 271 mg/ml; 272 mg/ml; 273 mg/ml; 274 mg/ml; or 275 mg/ml.

In an embodiment of the invention, the aqueous pharmaceutical formulation includes aflibercept at a concentration of at least about 100 mg/ml; sucrose, mannitol, sorbitol, trehalose; a histidine-based buffer; polysorbate 20 or polysorbate 80; and L-arginine, at a pH of about 5.0 to about 6.8; wherein the aflibercept has less than about 3.5% high molecular weight species immediately after manufacture and purification and/or less than or equal to about 6% high molecular weight species after storage for about 24 months at about 2-8°C.

In an embodiment of the invention, the sucrose, mannitol, sorbitol or trehalose is at a concentration of about 2-10% (w/v); the L-arginine is at a concentration of about 10-100 mM; the polysorbate 20 or polysorbate 80 is at a concentration of about 0.02-0.1% (w/v); and the histidine-based buffer is at a concentration of about 5-25 mM; at a pH of about 5.0 to about 6.8.

### Treatment and Administration

The present invention provides methods for treating angiogenic eye disorders (*e.g.,* neovascular age related macular degeneration (nAMD)) by sequentially administering initial loading doses (*e.g.*, 2 mg or more, 4 mg or more or, preferably, 8 mg or more of VEGF antagonist or inhibitor, for example, a VEGF receptor fusion protein such as aflibercept) (*e.g.*, about every 2-4 or 3-5 weeks) followed by additional doses every 12-20 weeks, preferably 12-16 weeks, 12 weeks, 16 weeks or 20 weeks. For example, the present invention provides methods for treating or preventing angiogenic eye disorders, such as neovascular age related macular degeneration (nAMD), by administering, sequentially, one or more (*e.g.*, 3 or 4 or 5) doses of about 8 mg or more of VEGF antagonist (*e.g.*, a VEGF receptor fusion protein such as aflibercept) about every 2-4 or 3-5 weeks, *e.g.*, every month (or about every 28 days, 28 ± 5 days or about every 4 weeks), followed by one or more doses of about 8 mg or more VEGF antagonist (*e.g.*, a VEGF receptor fusion protein such as aflibercept) every 12 weeks (or about every 3 months or about every quarter year or about every 84 days) or every 16 weeks (or about every 4 months or about every 1/3 years or about every 112 days) or every 20 weeks. The dosing regimen including the 12 week tertiary dosing interval may be referred to herein as a 12 week dosing regimen or 8q12 or HDq12; the dosing regimen including the 16 week tertiary dosing interval may be referred to herein as a 16 week dosing regimen or 8q16 or HDq16; and the dosing regimen including the 20 week tertiary dosing interval may be referred to herein as a 20 week dosing regimen or 8q20 or HDq20. A dosing regimen including tertiary dosing intervals of between 12 and 20 weeks may be referred to herein as a 12-20 week dosing regimen or 8q12-20 or HDq12-20.

In addition, the present invention includes methods for treating angiogenic eye disorders (*e.g.*, neovascular age related macular degeneration (nAMD)) by administering, one or more times, ≥8 mg VEGF receptor fusion protein, preferably aflibercept, every 4 weeks, 8 weeks, 12-20 weeks, 12-16 weeks, 12 weeks or 16 weeks; as well as every 4 weeks for the first 3, 4 or 5 doses followed by dosing about every 8 weeks.

In an embodiment of the invention, a subject begins receiving the ≥8 mg maintenance doses of every 12 or 16 or 20 weeks after the ≥8 mg monthly loading doses with no intervening doses. The subject enters the maintenance dose phase rapidly/immediately after the loading dose phase. In an embodiment of the invention, the subject continues receiving the ≥8 mg 12-20, 12 or 16 or 20 week doses without any intervening doses.

In an embodiment of the invention, the subject does not receive a dosing regimen modification (DRM) or does not terminate treatment for at least 1, 2, 3, 4 or 5 years.

The terms "initial dose," "secondary doses," and "tertiary doses," refer to the temporal sequence of administration of the VEGF antagonist (*e.g.*, a VEGF receptor fusion protein such as aflibercept). Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of VEGF antagonist (*e.g.*, a VEGF receptor fusion protein such as aflibercept), but will generally differ from one another in terms of frequency of administration. In certain embodiments, however, the amount of VEGF antagonist (*e.g.*, a VEGF receptor fusion protein such as aflibercept) contained in the initial, secondary and/or tertiary doses will vary from one another (*e*.*g*., adjusted up or down as appropriate) during the course of treatment.

Thus, a dosing regimen of the present invention may be expressed as follows:
a method for treating an angiogenic eye disorder (*e*.*g*., nAMD) in a subject in need thereof including administering (*e.g*., intravitreally) to a subject in need thereof:
a single initial dose of about ≥8 mg (in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of a VEGF antagonist (*e*.*g*., a VEGF receptor fusion protein such as aflibercept), followed by
one or more (*e*.*g*., 2, or 3 or 4, preferably 2) secondary doses of the VEGF antagonist (*e*.*g*., a VEGF receptor fusion protein such as aflibercept), followed by
one or more tertiary doses of the VEGF antagonist (*e*.*g*., a VEGF receptor fusion protein such as aflibercept);
wherein each secondary dose is administered 2 to 4 weeks (preferably, 4 weeks) after the immediately preceding dose; and
wherein each tertiary dose is administered at least 12 or 16 or 20 weeks (preferably, 12-20, 12-16, 12 or 16 weeks) after the immediately preceding dose.

The present invention includes methods wherein one or more additional, non-scheduled doses, in addition to any of the scheduled initial, secondary and/or tertiary doses of VEGF antagonist (*e*.*g*., a VEGF receptor fusion protein such as aflibercept) are administered to a subject. Such doses are typically administered at the discretion of the treating physician depending on the particular needs of the subject. The present invention also provides methods for treating angiogenic eye disorders by administering to a subject in need thereof about ≥8 mg (for example, in about 100 µl or less, about 75 µl or less or about 70 µl or less, *e*.*g*., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of VEGF antagonist (*e*.*g*., a VEGF receptor fusion protein such as aflibercept) on a PRN basis.

A *pro re nata* (PRN) treatment protocol calls for intervals between doctor visits to remain fixed (*e*.*g*., once every 2, 3, 4, 8, 12, 16 or 20 weeks) and decisions to carry out an injection of VEGF receptor fusion protein to be based on the anatomic findings at each respective visit. A capped PRN dosing regimen is PRN wherein subjects must be treated at a certain minimal frequency, *e.g.*, at least once every 2 or 3 or 4 months.

Treat & Extend (T&E) regimens call for the time interval between doctor visits to be adjusted based on the patient's clinical course-*e.g.*, if a subject shows no sign of an active disease (*e.g.*, the macula remains dry, without any leakage), the next one or more intervals can be extended; if there is fluid accumulation, the next interval will be shortened. At each visit following T&E, an injection of VEGF receptor fusion protein will be performed; the current clinical status only has an impact on the duration of the next injection interval.

The present invention includes embodiments wherein, at any point during a HDq12-20, HDq12, HDq16 or HDq20 treatment regimen, the patient can be switched to a PRN, capped PRN or T&E regimen. The PRN, capped PRN and/or T&E may be continued indefinitely or can be stopped at any point and then the HDq12, HDq16 or HDq20 regimen is re-initiated at any phase thereof. Any HDq12-20, HDq12, HDq16 or HDq20 regimen can be preceded or followed by a period of PRN, capped PRN and/or T&E.

The present invention includes methods comprising administering the required doses of the HDq12-20 or HDq12 or HDq16 or HDq20 regimen, wherein each of the tertiary doses is administered 12-20 or 12 or 16 or 20 weeks after the immediately preceding dose, wherein the treatment interval between two tertiary doses is extended (*e.g.*, from 12 weeks to 13, 14, 15, 16 or 20 weeks or from 16 weeks to 17, 18, 19, or 20 weeks) until signs of disease activity or visual impairment deteriorate or recur and then either continuing dosing at the last tertiary interval used or the penultimate tertiary interval used. In an embodiment of the invention, the subject receives the initial, secondary and, then, 12 or 16 week tertiary intervals and, then, after about 1 year, extending the tertiary intervals to about 20 weeks.

The present invention includes methods comprising administering the required doses of the HDq12-20 or HDq12 or HDq16 or HDq20 regimen, wherein the treatment interval between any two tertiary doses is reduced (*e.g.*, from 20 weeks to 8, 12 or 16 weeks, from 16 weeks to 15, 14, 13, 12, 11, 10, 9, 8, 7, 6, 5, 4, 3, or 2 weeks or from 12 weeks to 11, 10, 9, 8, 7, 6, 5, 4, 3 or 2 weeks), *e.g.*, until signs of disease activity or visual impairment improve (*e.g.*, BCVA stabilizes or improves and/or CRT stabilizes or reduces) whereupon, optionally, the interval between doses can be extended, *e.g.*, back to a greater interval length.

For example, in an embodiment of the invention, the interval between doses, *e.g.*, during the 12-20, 12 week or 16 week or 20 week dosing phase, can be shortened (*e.g.*, to 8 weeks) if:
- greater than 5 letters are lost in BCVA (ETDRS or Snellen equivalent) (*e*.*g*., relative to the BCVA observed at about 12 weeks after treatment initiation), for example, due to persistent or worsening AMD; and/or
- a greater than 25 micrometers increase in CRT is observed (*e*.*g*., relative to the CRT observed at about 12 weeks after treatment initiation) and/or if a new onset foveal neovascularization or foveal hemorrhage is observed.

In an embodiment of the invention, if the criteria for reducing the interval between doses is met in a subject receiving the HDq12 regimen at week 16 or 20, the interval between doses is decreased to 8 weeks.

In an embodiment of the invention, if the criteria for reducing the interval between doses is met in a subject receiving the HDq16 regimen at week 16 or 20, the interval between doses is decreased to 8 weeks; and if the criteria for reducing the interval between doses is met in a subject receiving the HDq16 regimen at week 24, the interval between doses is decreased to 12 weeks.

In an embodiment of the invention, the interval is not decreased to anything shorter than 8 weeks.

For example, in an embodiment of the invention, the interval between doses, *e*.*g*., during the 12-20, 12 week or 16 week or 20 week dosing phase, can be lengthened (*e*.*g*., by 4 week increments, *e*.*g*., from 12 to 16 weeks, or 16 to 20 weeks) if:
- BCVA loss <5 letters (*e*.*g*., from Week 12),
- No fluid at the central subfield, e.g., on OCT, and/or
- No new onset foveal hemorrhage or foveal neovascularization
(*e*.*g*., if all three criteria are met)

See Figure 3 or Figure 4.

The present invention also provides methods for treating angiogenic eye disorders (*e*.*g*., nAMD) by administering
doses of about ≥8 mg (for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) about once every 4, 5, 6, 7, 8, 12, 16 or 20 weeks;
   or
a single initial dose (*e*.*g*., about ≥8 mg, for example, in about 100 µl or less, about 75 µl or less or about 70 µl or less, e.g., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl)) of a VEGF antagonist (*e*.*g*., a VEGF receptor fusion protein such as aflibercept), followed by
one or more (*e*.*g*., 2, or 3 or 4, preferably 2 or 4) secondary doses of the VEGF antagonist (*e.g*., a VEGF receptor fusion protein such as aflibercept), followed by
one or more tertiary doses of the VEGF antagonist (*e*.*g*., a VEGF receptor fusion protein such as aflibercept);
wherein each secondary dose is administered 2 to 4 (preferably, 4) weeks after the immediately preceding dose; and
wherein each tertiary dose is administered at about 4, 5, 6, 7 or 8 (*e*.*g*., 8) weeks after the immediately preceding dose
   or
about ≥8 mg (for example, in about 100 µl or less, about 75 µl or less or about 70 µl or less, *e*.*g*., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of VEGF antagonist (*e*.*g*., a VEGF receptor fusion protein such as aflibercept) once every about 4 weeks (q4w);
   or
less than about 8 doses (*e*.*g*., about 5 doses or 6 doses) of about ≥8 mg (for example, in about 100 µl or less, about 75 µl or less or about 70 µl or less, *e*.*g*., about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl) of a VEGF antagonist (*e*.*g*., a VEGF receptor fusion protein such as aflibercept) over the course of about 48 weeks.

Dosing every "month" or after a "month" refers to dosing after about 28 days, about 4 weeks, or about 28 (± 5 days) and may encompass up to 5 weeks (± 5 days).

Dosing every "4 weeks" or after "4 weeks" refers to dosing after about 28 days (± 5 days), about a month or about 28 (± 5 days), and may encompass up to every 5 weeks (± 5 days).

Dosing every "2-4 weeks" or after "2-4 weeks" refers to dosing after about 2 weeks (± 5 days), 3 weeks (± 5 days) or 4 weeks (± 5 days).

Dosing every "8 weeks" or after "8 weeks" refers to dosing after about 2 months (± 5 days) or about 56 (± 5 days).

Dosing every "12 weeks" or after "12 weeks" refers to dosing after about 3 months, about 84 days (± 5 days), about 90 days (± 5 days) or about 84 (± 5 days).

Dosing every "16 weeks" or after "16 weeks" refers to dosing after about 4 months or about 112 days (± 5 days).

Dosing every "12-20 weeks" or after "12-20 weeks" refers to dosing after about 12, 13, 14, 15, 16, 17, 18, 19 or 20 weeks (± 5 days), preferably about 12-16 weeks (± 5 days), about 12 weeks (± 5 days), about 16 weeks (± 5 days) or about 20 weeks (± 5 days).

Dosing every "12-20 weeks" refers to dosing after about 12, 13, 14, 15, 16, 17, 18, 19 or 20 weeks (± 5 days), preferably about 12-16 weeks (± 5 days), about 12 weeks (± 5 days), about 16 weeks (± 5 days) or about 20 weeks (± 5 days).

A dose of ≥ 8 mg encompasses a dose of about 8 mg or doses exceeding 8 mg, for example, about 9, 10, 11, 12, 13, 14, 15, 16, 17, 18, 19 or 20 mg.

Any dosing frequency specified herein may, in an embodiment of the invention, be expressed as the specific frequency "± 5 days" (*e.g.*, where "4 weeks" is stated, the present invention also includes embodiments such as 4 weeks ± 5 days). The term ± 5 days includes ±1, ±2, ±3, ±4 and/or ±5 days.

"Sequentially administering" means that each dose of VEGF antagonist (*e.g.*, a VEGF receptor fusion protein such as aflibercept) is administered to the eye of a subject at a different point in time, *e.g.*, on different days separated by a predetermined interval (*e.g.*, hours, days, weeks or months). The present invention includes methods which comprise sequentially administering to the eye of a subject a single initial dose of a VEGF antagonist (*e.g.*, a VEGF receptor fusion protein such as aflibercept), followed by one or more secondary doses of the VEGF antagonist (*e.g.*, a VEGF receptor fusion protein such as aflibercept), followed by one or more tertiary doses of the VEGF antagonist (*e.g.*, a VEGF receptor fusion protein such as aflibercept).

An effective or therapeutically effective dose of VEGF antagonist (*e.g.*, a VEGF receptor fusion protein such as aflibercept) for treating or preventing an angiogenic eye disorder refers to the amount of VEGF antagonist (*e*.*g*., a VEGF receptor fusion protein such as aflibercept) sufficient to alleviate one or more signs and/or symptoms of the disease or condition in the treated subject, whether by inducing the regression or elimination of such signs and/or symptoms or by inhibiting the progression of such signs and/or symptoms. In an embodiment of the invention, an effective or therapeutically effective dose of VEGF antagonist (*e*.*g*., a VEGF receptor fusion protein such as aflibercept) is about ≥8 mg every month, for 3 doses, followed by once every 12-20 weeks. In an embodiment of the invention, the alleviation of signs and/or symptoms is achievement, e.g., by 1 year, of a gain of ≥5, 10 or 15 letters BCVA (relative to baseline) (*e.g*., ≥5 letters improvement in a nAMD subject and/or 8-14 letters improvement in a DME patient/subject); achieving a BCVA ≥ 69 letters; achieving no fluid at foveal center; reduction in central retinal thickness (CRT) by about 150 micrometers or more (*e*.*g*., below 300 micrometers in an nAMD subject/patient; and/or reduction by at least about 200 micrometers in a DR or RVO patient/subject) or achievement of normal CRT (*e*.*g*., about 300 micrometers or less); and/or achievement of no leakage on fluorescein angiography.

In an embodiment of the invention, the subject receiving HDq12-20, HDq16 or HDq16 receives fewer inejctions per month or quarter, *e*.*g*., by week 60 from treatment initiation, than that of a subject receiving a 2q8 regimen. For example, wherein the subject receiving HDq12 receives about 7 injections over 60 weeks and/or the subject receiving the HDq16 regimen freceives about 6 injections over 60 weeks.

An "angiogenic eye disorder" means any disease of the eye which is caused by or associated with the growth or proliferation of blood vessels or by blood vessel leakage. Nonlimiting examples of angiogenic eye disorders that are treatable or preventable using the methods of the present invention include:
- age-related macular degeneration (neovascular (nAMD)),
- macular edema (ME),
- macular edema following retinal vein occlusion (ME-RVO),
- retinal vein occlusion (RVO),
- central retinal vein occlusion (CRVO),
- branch retinal vein occlusion (BRVO),
- diabetic macular edema (DME),
- choroidal neovascularization (CNV),
- iris neovascularization,
- neovascular glaucoma,
- post-surgical fibrosis in glaucoma,
- proliferative vitreoretinopathy (PVR),
- optic disc neovascularization,
- corneal neovascularization,
- retinal neovascularization,
- vitreal neovascularization,
- pannus,
- polypoidal choroidal vasculopathy (PCV), optionally, wherein the subject also has nAMD,
- pterygium,
- vascular retinopathy,
- diabetic retinopathies (DR) (*e*.*g*., non-proliferative diabetic retinopathy (*e*.*g*., characterized by a Diabetic Retinopathy Severity Scale (DRSS) level of about 47 or 53) or proliferative diabetic retinopathy; *e*.*g*., in a subject that does not suffer from DME), and
- Diabetic retinopathy in a subject who has diabetic macular edema (DME).

The present invention provides methods for treating angiogenic eye disorders (*e*.*g*., nAMD) in a subject in need thereof, by sequentially administering initial loading doses (*e*.*g*., 2 mg or more, 4 mg or more or, preferably, about 8 mg or more of VEGF antagonist or inhibitor, for example, a VEGF receptor fusion protein such as aflibercept) (*e*.*g*., about every 2-4 or 3-5 weeks, preferably every 4 weeks; preferably, three initial loading doses) followed by additional doses every 12-20 weeks, preferably 12-16 weeks, 12 weeks, 16 weeks or 20 weeks wherein the subject achieves and/or maintains, *e.g*., by week 4, 8, 12, 16, 20, 24, 28, 32, 36, 40, 44, 48, 52, 56, 60, 64, 68, 72, 76, 80, 84, 88, 92 or 96 weeks after treatment initiation:
- Increase in BCVA, *e*.*g*., as measured by the Early Treatment Diabetic Retinopathy Study (ETDRS) visual acuity chart (*e.g*., by week 4, 8, 12, 16, 20, 24, 28, 32, 36, 40, 44, 48, 52, 56, 60, 64, 68, 72, 76, 80, 84, 88, 92 or 96), for example, by 1, 2, 3, 4, 5, 6, 7, 8, 9 or 10 letters, or ≥5, ≥10, ≥15, or ≥20 letters, or lack of loss thereof during the course of treatment;
- Does not receive a dose regimen modification, *e*.*g*., wherein the interval between doses (*e*.*g*., tertiary doses) is reduced from the HDq12-20 or HDq12 or HDq16 or HDq20 treatment regimen once started, *e*.*g*., for at least 48, 60 or 96 weeks;
- Receives 100% of all scheduled doses, *e*.*g*., for at least 48, 60 or 96 weeks;
- Increase in BCVA as averaged over a period of 12 weeks, *e*.*g*., by week 36 or 48;
- No intraretinal fluid (IRF) and/or no subretinal fluid and/or subretinal epithelium fluid (*e*.*g*., by week 4, 8, 12, 16, 20, 24, 28, 32, 36, 40, 44, 48, 52, 56, 60, 64, 68, 72, 76, 80, 84, 88, 92 or 96) *e*.*g*., wherein the subject has nAMD and PCV;
- Retina without fluid (total fluid, intraretinal fluid [IRF] and/or subretinal fluid [SRF] and/or subretinal epithelium fluid) in center subfield (*e*.*g*., by about 4, 8, 12, 16, 20, 24, 28, 32, 36, 40, 44, 48, 52, 56, 60, 64, 68, 72, 76, 80, 84, 88, 92 or 96 weeks from start of treatment); *e*.*g*., wherein the subject has nAMD and PCV;
- No subretinal pigment epithelium fluid (*e*.*g*., by about 4, 8, 12, 16, 20, 24, 28, 32, 36, 40, 44, 48, 52, 56, 60, 64, 68, 72, 76, 80, 84, 88, 92 or 96 weeks from start of treatment);
- Lack of fluid leakage on fluorescein angiography (FA) (*e*.*g*., by about 4, 8, 12, 16, 20, 24, 28, 32, 36, 40, 44, 48, 52, 56, 60, 64, 68, 72, 76, 80, 84, 88, 92 or 96 weeks from start of treatment);
- Decrease in choroidal neovascularization (CNV) size, e.g., by about 2.9, 3, 3.5, 3.65 or 4 mm² (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment), for example wherein the baseline size is about 6 or 6.5 mm²;
- Decrease in total lesion CNV area from baseline (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment);
- Loss of IRF and/or SRF, *e*.*g*., in the center subfield (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment);
- Decrease in central subfield retinal thickness (CST) (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment), *e*.*g*., by about 125, 150, 175 or 200 micrometers;
- Increase, *e*.*g*, by about 3 or 4 points, in vision-related quality of life, *e*.*g*., as measured by the National Eye Institute Visual Functioning Questionnaire-25 (NEI-VFQ-25) (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment), *e*.*g*., wherein the baseline score is about 76 or 77;
- Lack of treatment-emergent adverse events (AEs) and/or serious AEs (SAEs), for example, treatment-emergent adverse events occurring anytime within 30 days of any injection) such as blood pressure increase, intraocular pressure increase, visual impairment, vitreous floaters, vitreous detachment, iris neovascularization and/or vitreous hemorrhage (*e*.*g*., through week 4, 8, 12, 16, 20, 24, 28, 32, 36, 40, 44, 48, 52, 56, 60, 64, 68, 72, 76, 80, 84, 88, 92 or 96 from start of treatment);
- ETDRS letter score of at least 69 (approximate 20/40 Snellen equivalent) (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment); and/or
- Efficacy and/or safety, in a subject suffering from nAMD, similar to that of aflibercept which is intravitreally dosed at 2 mg approximately every 4 weeks for the first 3 months, followed by 2 mg approximately once every 8 weeks or once every 2 months, *e*.*g*., wherein efficacy is measured as increase in BCVA and/or reduction in central retinal thickness, *e*.*g*., wherein safety is as measured as the incidence of adverse events (treatment-emergent adverse events occurring anytime within 30 days of any injection) such as blood pressure increase, intraocular pressure increase, visual impairment, vitreous floaters, vitreous detachment, iris neovascularization and/or vitreous hemorrhage.

The present invention provides methods for treating angiogenic eye disorders (*e*.*g*., nAMD) in a subject in need thereof, by sequentially administering initial loading doses (*e*.*g*., 2 mg or more, 4 mg or more or, preferably, about 8 mg or more of VEGF antagonist or inhibitor, for example, a VEGF receptor fusion protein such as aflibercept) (*e*.*g*., about every 2-4 or 3-5 weeks, preferably every 4 weeks; preferably, three initial loading doses) followed by additional doses every 12-20 weeks, preferably 12-16 weeks, 12 weeks, 16 weeks or 20 weeks wherein the subject achieves and/or maintains, e.g., by week 4, 8, 12, 16, 20, 24, 28, 32, 36, 40, 44, 48, 52, 56, 60, 64, 68, 72, 76, 80, 84, 88, 92 or 96 weeks after treatment initiation:
- No detectable anti-drug antibody (*e*.*g*., anti-aflibercept) during receipt of treatment;
- Improvement in best corrected visual acuity (BVCA) by week 4, 8, 12, 16, 20, 24, 28, 32, 36, 40, 44, 48, 52, 56, 60, 64, 68, 72, 76, 80, 84, 88, 92 or 96 from start of treatment;
- Increase in BCVA, *e*.*g*., as measured by the Early Treatment Diabetic Retinopathy Study (ETDRS) visual acuity chart or Snellen equivalent (*e*.*g*., by week 4, 8, 12, 16, 20, 24, 28, 32, 36, 40, 44, 48, 52, 56, 60, 64, 68, 72, 76, 80, 84, 88, 92 or 96 from start of treatment) by ≥2 letters, ≥3 letters, ≥4 letters, ≥5 letters, ≥6 letters or ≥7 letters;
- Improvement in BCVA, by 4 weeks after initiation of treatment, of about 2 or 3 letters (ETDRS or Snellen equivalent) when on HDq12 regimen; or of about 3 letters (ETDRS or Snellen equivalent) when on HDq16 regimen;
- Improvement in BCVA, by 8 weeks after initiation of treatment, of about 5 letters (ETDRS or Snellen equivalent) when on HDq12 regimen; or of about 4 or 5 letters (ETDRS or Snellen equivalent) when on HDq16 regimen;
- Improvement in BCVA, by 12 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
- Improvement in BCVA, by 16 weeks after initiation of treatment, of about 6 letters (ETDRS or Snellen equivalent) when on HDq12 regimen; or of about 6 or 7 letters (ETDRS or Snellen equivalent) when on HDq16 regimen;
- Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
- Improvement in BCVA, by 24 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
- Improvement in BCVA, by 28 weeks after initiation of treatment, of about 7 letters (ETDRS or Snellen equivalent) when on HDq12 regimen; or of about 6 letters (ETDRS or Snellen equivalent) when on HDq16 regimen;
- Improvement in BCVA, by 32 weeks after initiation of treatment, of about 6 or 7 letters (ETDRS or Snellen equivalent) when on HDq12 regimen; or of about 7 letters (ETDRS or Snellen equivalent) when on HDq16 regimen;
- Improvement in BCVA, by 36 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 40 weeks after initiation of treatment, of about 6 or 7 letters (ETDRS or Snellen equivalent) when on HDq12 regimen; or of about 5 or 6 letters (ETDRS or Snellen equivalent) when on HDq16 regimen;
- Improvement in BCVA, by 44 weeks after initiation of treatment, of about 7 letters (ETDRS or Snellen equivalent) when on HDq12 regimen; or of about 5 or 6 letters (ETDRS or Snellen equivalent) when on HDq16 regimen;
- Improvement in BCVA, by 48 weeks after initiation of treatment, of about 6 or 7 letters (ETDRS or Snellen equivalent) when on HDq12 regimen; or of about 6 letters (ETDRS or Snellen equivalent) when on HDq16 regimen;
- Improvement in BCVA, by 52 weeks after initiation of treatment, of about 7 or 8 letters (ETDRS or Snellen equivalent) when on HDq12 regimen; or of about 6 or 7 letters (ETDRS or Snellen equivalent) when on HDq16 regimen;
- Improvement in BCVA, by 56 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
- Improvement in BCVA, by 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
- Improvement in BCVA, from about 36 to about 48 weeks after initiation of treatment, of about 6 or 7 letters, or up to 46 letters (ETDRS or Snellen equivalent) when on HDq12 regimen; or of about 6 or 7 letters, or up to 40 letters (ETDRS or Snellen equivalent) when on HDq16 regimen;
- Improvement in BCVA, from about 48 to about 60 weeks after initiation of treatment, of about 6 or 7 letters, or up to 46 letters (ETDRS or Snellen equivalent) when on HDq12 regimen; or of about 6 or 7 letters, or up to 40 letters (ETDRS or Snellen equivalent) when on HDq16 regimen;
- Improvement in BCVA, by 48 or 60 weeks after initiation of treatment, of at least about 5, 10 or 15 letters (ETDRS or Snellen equivalent) when on the HDq12 regimen; or the HDq16 regimen;
- Does not lose 5, 10 or 15 letters (ETDRS letters or Snellen equivalent) in BCVA, by 48 or 60 or 96 weeks after initiation of treatment, when on the HDq12 regimen; or the HDq16 regimen;
- An improvement in BCVA by about week 8, 9, 10, 11 or 12 after initiation of treatment which is maintained (*e*.*g*., within about ±1 or ±2 ETDRS letters or Snellen equivalent) thereafter during the treatment regiment, *e*.*g*., to at least week 48.
- A BCVA of at least 69 letters (ETDRS letters or Snellen equivalent) by about week 48 or 60 when receiving the HDq12 or HDq16 regimen.
- A BCVA by 4 weeks after initiation of treatment of about 63 letters (ETDRS or Snellen equivalent) when on the HDq12 regimen; or a BCVA of about 63 letters (ETDRS or Snellen equivalent) when on the HDq16 regimen;
- A BCVA by 8 weeks after initiation of treatment of about 65 letters (ETDRS or Snellen equivalent) when on the HDq12 regimen; or a BCVA of about 65 letters (ETDRS or Snellen equivalent) when on the HDq16 regimen;
- A BCVA by12 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 16 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 20 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 24 weeks after initiation of treatment of about 66 letters (ETDRS or Snellen equivalent) when on the HDq12 regimen; or a BCVA of about 66 letters (ETDRS or Snellen equivalent) when on the HDq16 regimen;
- A BCVA by 28 weeks after initiation of treatment of about 67 letters (ETDRS or Snellen equivalent) when on the HDq12 regimen; or a BCVA of about 66letters (ETDRS or Snellen equivalent) when on the HDq16 regimen;
- A BCVA by 32 weeks after initiation of treatment of about 67 letters (ETDRS or Snellen equivalent) when on the HDq12 regimen; or a BCVA of about 67 letters (ETDRS or Snellen equivalent) when on the HDq16 regimen;
- A BCVA by 36 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA by 40 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA by 44 weeks after initiation of treatment of about 68 letters (ETDRS or Snellen equivalent) when on the HDq12 regimen; or a BCVA of about 66 letters (ETDRS or Snellen equivalent) when on the HDq16 regimen;
- A BCVA by 48 weeks after initiation of treatment of about 67 letters (ETDRS or Snellen equivalent) when on the HDq12 regimen; or a BCVA of about 66 letters (ETDRS or Snellen equivalent) when on the HDq16 regimen;
- A BCVA between weeks 36 and 48 weeks after initiation of treatment of about 66, 67, 68, 69, 70, 71 or 72 letters or up to 91 letters (ETDRS or Snellen equivalent) when on the HDq12 regimen;
- A BCVA between weeks 36 and 48 weeks after initiation of treatment of about 66, 67, 68, 69 or 70 letters or up to 94 letters (ETDRS or Snellen equivalent) when on the HDq16 regimen;
- A BCVA between weeks 48 and 60 weeks after initiation of treatment of about 66, 67, 68, 69, 70, 71 or 72 letters or up to 91 letters (ETDRS or Snellen equivalent) when on the HDq12 regimen;
- A BCVA between weeks 48 and 60weeks after initiation of treatment of about 66, 67, 68, 69 or 70 letters or up to 94 letters (ETDRS or Snellen equivalent) when on the HDq16 regimen;
- An improvement in BCVA of about 7, 8, 9, 10 or 11 letters (ETDRS or Snellen equivalent), *e*.*g*., wherein the baseline BCVA is ≤54 letters, by about week 48;
- An improvement in BCVA of about 5, 6 or 7 letters (ETDRS or Snellen equivalent), *e*.*g*., wherein the baseline BCVA is about 55-73 letters, by about week 48;
- An improvement in BCVA of about 1, 2 or 3 letters (ETDRS or Snellen equivalent), *e*.*g*., wherein the baseline BCVA is about ≥74 letters, by about week 48;
- An improvement in BCVA of about 4, 5 or 6 letters (ETDRS or Snellen equivalent), *e*.*g*., wherein the baseline BCVA is about 63 letters and the baseline CRT is <400 micrometers, by about week 48;
- An improvement in BCVA of about 5, 6, 7 8 or 9 letters (ETDRS or Snellen equivalent), *e*.*g*., wherein the baseline BCVA is about 52 o 53 letters and the baseline CRT is ≥400 micrometers, by about week 48;
- An improvement in BCVA of about 2, 3, 4, 5 or 6 letters (ETDRS or Snellen equivalent), *e*.*g*., wherein the baseline BCVA is about 56, 57 or 58 letters and the subject has a minimally classic CNV, by about week 48;
- An improvement in BCVA of about 5 letters (ETDRS or Snellen equivalent), *e*.*g*., wherein the baseline BCVA is about 62, 63 or 64 letters and the subject has an occult only CNV, by about week 48;
- An improvement in BCVA of about 6, 7, 8, 9 or 10 letters (ETDRS or Snellen equivalent), *e*.*g*., wherein the baseline BCVA is about 53 or 54 letters and the subject has a predominantly classic CNV, by about week 48;
- A BCVA by 52 weeks after initiation of treatment of about 67 or 68 letters (ETDRS or Snellen equivalent) when on the HDq12 regimen; or a BCVA of about 66 or 67 letters (ETDRS or Snellen equivalent) when on the HDq16 regimen;
- A BCVA by 56 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
- A BCVA by 60 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
- A BCVA between about week 48 and about 60 week after initiation of treatment of about 66 to about 72 letters (ETDRS or Snellen equivalent) when on the HDq12 regimen; or a BCVA of about 66 to about 70 letters (ETDRS or Snellen equivalent) when on the HDq16 regimen;
   - A BCVA of about 59 letters (ETDRS) when receiving the HDq12 regimen (*e*.*g*., wherein the baseline BCVA is about 57 letters); and/or a BCVA of about 64 letters (ETDRS) when receiving the HDq16 regimen (*e*.*g*., wherein the baseline BCVA is about 60 letters); by about week 4 after treatment initiation; in a subject having polypoidal choroidal vasculopathy (PCV) and neovascular age related macular degeneration (nAMD);
   - A BCVA of about 63 letters (ETDRS) when receiving the HDq12 regimen (*e*.*g*., wherein the baseline BCVA is about 57 letters); and/or a BCVA of about 65 letters (ETDRS) when receiving the HDq16 regimen (*e*.*g*., wherein the baseline BCVA is about 60 letters); by about week 8 after treatment initiation; in a subject having polypoidal choroidal vasculopathy (PCV) and neovascular age related macular degeneration (nAMD);
   - A BCVA of about 63letters (ETDRS) when receiving the HDq12 regimen (*e*.*g*., wherein the baseline BCVA is about 57 letters); and/or a BCVA of about 66 letters (ETDRS) when receiving the HDq16 regimen (*e*.*g*., wherein the baseline BCVA is about 60 letters); by about week 12 after treatment initiation; in a subject having polypoidal choroidal vasculopathy (PCV) and neovascular age related macular degeneration (nAMD);
   - A BCVA of about 64 letters (ETDRS) when receiving the HDq12 regimen (*e*.*g*., wherein the baseline BCVA is about 57 letters); and/or a BCVA of about 67 letters (ETDRS) when receiving the HDq16 regimen (*e*.*g*., wherein the baseline BCVA is about 60 letters); by about week 16 after treatment initiation; in a subject having polypoidal choroidal vasculopathy (PCV) and neovascular age related macular degeneration (nAMD);
   - A BCVA of about 64 letters (ETDRS) when receiving the HDq12 regimen (*e*.*g*., wherein the baseline BCVA is about 57 letters); and/or a BCVA of about 68 letters (ETDRS) when receiving the HDq16 regimen (*e*.*g*., wherein the baseline BCVA is about 60 letters); by about week 20 after treatment initiation; in a subject having polypoidal choroidal vasculopathy (PCV) and neovascular age related macular degeneration (nAMD);
   - A BCVA of about 64 letters (ETDRS) when receiving the HDq12 regimen (*e*.*g*., wherein the baseline BCVA is about 57 letters); and/or a BCVA of about 67 letters (ETDRS) when receiving the HDq16 regimen (*e*.*g*., wherein the baseline BCVA is about 60 letters); by about week 24 after treatment initiation; in a subject having polypoidal choroidal vasculopathy (PCV) and neovascular age related macular degeneration (nAMD);
   - A BCVA of about 65 letters (ETDRS) when receiving the HDq12 regimen (*e*.*g*., wherein the baseline BCVA is about 57 letters); and/or a BCVA of about 68 letters (ETDRS) when receiving the HDq16 regimen (*e*.*g*., wherein the baseline BCVA is about 60 letters); by about week 28 after treatment initiation; in a subject having polypoidal choroidal vasculopathy (PCV) and neovascular age related macular degeneration (nAMD);
   - A BCVA of about 64 letters (ETDRS) when receiving the HDq12 regimen (*e*.*g*., wherein the baseline BCVA is about 57 letters); and/or a BCVA of about 71 letters (ETDRS) when receiving the HDq16 regimen (*e*.*g*., wherein the baseline BCVA is about 60 letters); by about week 32 after treatment initiation; in a subject having polypoidal choroidal vasculopathy (PCV) and neovascular age related macular degeneration (nAMD);
   - A BCVA of about 64 letters (ETDRS) when receiving the HDq12 regimen (*e*.*g*., wherein the baseline BCVA is about 57 letters); and/or a BCVA of about 71l letters (ETDRS) when receiving the HDq16 regimen (*e*.*g*., wherein the baseline BCVA is about 60 letters); by about week 36 after treatment initiation; in a subject having polypoidal choroidal vasculopathy (PCV) and neovascular age related macular degeneration (nAMD);
   - A BCVA of about 65 letters (ETDRS) when receiving the HDq12 regimen (*e*.*g*., wherein the baseline BCVA is about 57 letters); and/or a BCVA of about 69 letters (ETDRS) when receiving the HDq16 regimen (*e*.*g*., wherein the baseline BCVA is about 60 letters); by about week 40 after treatment initiation; in a subject having polypoidal choroidal vasculopathy (PCV) and neovascular age related macular degeneration (nAMD);
   - A BCVA of about 65 letters (ETDRS) when receiving the HDq12 regimen (*e*.*g*., wherein the baseline BCVA is about 57 letters); and/or a BCVA of about 68 letters (ETDRS) when receiving the HDq16 regimen (*e*.*g*., wherein the baseline BCVA is about 60 letters); by about week 44 after treatment initiation; in a subject having polypoidal choroidal vasculopathy (PCV) and neovascular age related macular degeneration (nAMD);
   - an increase in BCVA of about 3.0 letters (ETDRS) when receiving the HDq12 regimen; and/or an increase of about 3.0 letters when receiving the HDq16 regimen by about week 4 following treatment initiation in a subject having PCV and nAMD;
   - an increase in BCVA of about 6.0 letters (ETDRS) when receiving the HDq12 regimen; and/or an increase of about 5.0 letters when receiving the HDq16 regimen by about week 8 following treatment initiation in a subject having PCV and nAMD;
   - an increase in BCVA of about 7.0 letters (ETDRS) when receiving the HDq12 regimen; and/or an increase of about 6.0 letters when receiving the HDq16 regimen by about week 12 following treatment initiation in a subject having PCV and nAMD;
   - an increase in BCVA of about 7.0 letters (ETDRS) when receiving the HDq12 regimen; and/or an increase of about 7.0 letters when receiving the HDq16 regimen by about week 16 following treatment initiation in a subject having PCV and nAMD;
   - an increase in BCVA of about 7.0 letters (ETDRS) when receiving the HDq12 regimen; and/or an increase of about 7.0 letters when receiving the HDq16 regimen by about week 20 following treatment initiation in a subject having PCV and nAMD;
   - an increase in BCVA of about 7.0 letters (ETDRS) when receiving the HDq12 regimen; and/or an increase of about 5.0 letters when receiving the HDq16 regimen by about week 24 following treatment initiation in a subject having PCV and nAMD;
   - an increase in BCVA of about 9.0 letters (ETDRS) when receiving the HDq12 regimen; and/or an increase of about 7.0 letters when receiving the HDq16 regimen by about week 28 following treatment initiation in a subject having PCV and nAMD;
   - an increase in BCVA of about 8.0 letters (ETDRS) when receiving the HDq12 regimen; and/or an increase of about 10.0 letters when receiving the HDq16 regimen by about week 32 following treatment initiation in a subject having PCV and nAMD;
   - an increase in BCVA of about 8.0 letters (ETDRS) when receiving the HDq12 regimen; and/or an increase of about 10.0 letters when receiving the HDq16 regimen by about week 36 following treatment initiation in a subject having PCV and nAMD;
   - an increase in BCVA of about 9.0 letters (ETDRS) when receiving the HDq12 regimen; and/or an increase of about 8.0 letters when receiving the HDq16 regimen by about week 40 following treatment initiation in a subject having PCV and nAMD;
   - an increase in BCVA of about 9.0 letters (ETDRS) when receiving the HDq12 regimen; and/or an increase of about 8.0 letters when receiving the HDq16 regimen by about week 44 following treatment initiation in a subject having PCV and nAMD;
   - an increase in BCVA of about 9.0 letters (ETDRS) when receiving the HDq12 regimen; and/or an increase of about 9.0 letters when receiving the HDq16 regimen by about week 48 following treatment initiation in a subject having PCV and nAMD;
   - A BCVA of about 65 letters (ETDRS) when receiving the HDq12 regimen (*e*.*g*., wherein the baseline BCVA is about 57 letters); and/or a BCVA of about 69 letters (ETDRS) when receiving the HDq16 regimen (*e*.*g*., wherein the baseline BCVA is about 60 letters); by about week 48 after treatment initiation; in a subject having polypoidal choroidal vasculopathy (PCV) and neovascular age related macular degeneration (nAMD);
- A reduction in total lesion size of about 0.45 (±2.3) or 0.5 (±2.3) or 0.23 mm², or up to 11 mm², when receiving the HDq12 regimen or about 0.40 (±2.6) or 0.13 mm², or up to 17 mm², when receiving the HDq16 regimen, by about week 12 following treatment initiation; for example, wherein baseline total lesion area is about 5 or 6 or 7 mm²;
- A reduction in total lesion size of about 0.36 or 0.4 or 0.46 or 0.26 (±2.9 or ±0.19) mm² when receiving the HDq12 regimen or about 0.2, 0.2856 0.3, or 0.04 (±3.2 or ±0.2) mm², or up to 16 mm², when receiving the HDq16 regimen, by about week 48 following treatment initiation; for example, wherein baseline total lesion area is about 5 or 6 or 7 mm²;
- A reduction in total lesion size of about 0.52 or 0.6, or 0.32 (±2.8) mm² , or up to 14 mm², when receiving the HDq12 regimen or about 0.35 or 0.4 or 0.14 (±3.2) mm² or up to 17mm², when receiving the HDq16 regimen, by about week 60 following treatment initiation; for example, wherein baseline total lesion area is about 5 or 6 or 7 mm²;
- Decrease in central retinal thickness (CRT), for example, by at least about 100, 125, 130, 135, 140, 141, 142, 143, 144, 145, 146, 147, 148, 149 or 150 micrometers (*e*.*g*., by week 4, 8, 12, 16, 20, 24, 28, 32, 36, 40, 44 or 48 from start of treatment);
- A change in central retinal thickness, by 4 weeks after initiation of treatment of about - 120 or -121 or -122 or -122.4 or -120.2 micrometers when on the HDq12 regimen; or of about -126 or -127 or-126.6 or -126.3 micrometers when on the HDq16 regimen;
- A change in central retinal thickness, by 8 weeks after initiation of treatment of about - 132, -133, -134, -135 or -136 or -136.2 or -132.8 micrometers when on the HDq12 regimen; or of about -139 or -140 or -139.5 or -139.6 micrometers when on the HDq16 regimen;
- A change in central retinal thickness, by 12 weeks after initiation of treatment of about - 136, -137, -138, -139, -140 or -141 or -140.9 or -136.6 micrometers when on the HDq12 regimen; or of about -144 or -143 or -143.5 micrometers when on the HDq16 regimen
- A change in central retinal thickness, by 16 weeks after initiation of treatment of about - 120 or -121 or -122 or -123 or -124 or -123.4 or -120.1 micrometers when on the HDq12 regimen; or of about -132 or -133 or -132.1 or -133.1 micrometers when on the HDq16 regimen;
- A change in central retinal thickness, by 20 weeks after initiation of treatment of about - 110 or -111 or -112 or -113 or -114 or -113.6 or -110.9 micrometers when on the HDq12 regimen; or of about -115 or -116 or -117 or -118 or -115.8 or -117.7 micrometers when on the HDq16 regimen;
- A change in central retinal thickness, by 24 weeks after initiation of treatment of about - 134, -135, -136 or -137 or -138 or -137.6 or -134.9 micrometers when on the HDq12 regimen; or of about -105 or -106 or -107 or -108 or -105.3 or -107.8 micrometers when on the HDq16 regimen;
- A change in central retinal thickness, by 28 weeks after initiation of treatment of about - 130, -131 or -132 or -133 or -134 or -133.7 or -130.7 micrometers when on the HDq12 regimen; or of about -144 or -145 or -146 or -147 or -148 or -144.7 or -147.2 micrometers when on the HDq16 regimen;
- A change in central retinal thickness, by 32 weeks after initiation of treatment of about - 118 or -19 or -120 or -121 or -120.4 or -118.1 micrometers when on the HDq12 regimen; or of about -141 or -142 or -143 or -144 or -141.5 or -144 micrometers when on the HDq16 regimen;
- A change in central retinal thickness, by 36 weeks after initiation of treatment of about - 142 or -143 or -144 or -144.2 or -142.2 micrometers when on the HDq12 regimen; or of about -126 or -127, -128 or -129 or -130 or -131 or -126.4 or -130.5 micrometers when on the HDq16 regimen;
- A change in central retinal thickness, by 40 weeks after initiation of treatment of about - 131, -132, -133 or -134 or -133.8 or -131.2 micrometers when on the HDq12 regimen; or of about -127, -128 or -127.5 micrometers when on the HDq16 regimen;
- A change in central retinal thickness, by 44 weeks after initiation of treatment of about - 120 or -121 or -122 or -123 or -124 or -125 or -124.7 or -120.3 micrometers when on the HDq12 regimen; or of about -143, -144 or -145 or -144.8 micrometers when on the HDq16 regimen;
- A change in central retinal thickness, by 48 weeks after initiation of treatment of about-141, -141.9, -142, -142.3, -143, -144, -144.4, -145, -146, -147, -147.37 or -148 micrometers when on the HDq12 regimen, *e.g*., wherein the baseline CRT is about 370 micrometers; or of about -143, -144, -145, -146, -146.76, -147, -147.1, -148, -143.8 or - 147.1 micrometers when on the HDq16 regimen, *e.g*., wherein the baseline CRT is about 370 micrometers;
- A change in central retinal thickness, by 60 weeks after initiation of treatment, of about - 153 micrometers when on the HDq16 regimen;
- A central retinal thickness by week 4 after initiation of treatment of about 248.2 micrometers when on the HDq12 regimen; or of about 244.1 micrometers when on the HDq16 regimen (*e.g*., wherein the initial CRT is about 370 micrometers);
- A central retinal thickness by week 8 after initiation of treatment of about 234.4 micrometers when on the HDq12 regimen; or of about 231.2 micrometers when on the HDq16 regimen (*e.g*., wherein the initial CRT is about 370 micrometers);
- A central retinal thickness by week 12 after initiation of treatment of about 229.7 micrometers when on the HDq12 regimen; or of about 226.7 micrometers when on the HDq16 regimen (*e.g*., wherein the initial CRT is about 370 micrometers);
- A central retinal thickness by week 16 after initiation of treatment of about 247.2 micrometers when on the HDq12 regimen; or of about 238.6 micrometers when on the HDq16 regimen (*e.g*., wherein the initial CRT is about 370 micrometers);
- A central retinal thickness by week 20 after initiation of treatment of about 257 micrometers when on the HDq12 regimen; or of about 254.9 micrometers when on the HDq16 regimen (*e.g*., wherein the initial CRT is about 370 micrometers);
- A central retinal thickness by week 24 after initiation of treatment of about 233 micrometers when on the HDq12 regimen; or of about 265.4 micrometers when on the HDq16 regimen (*e.g*., wherein the initial CRT is about 370 micrometers);
- A central retinal thickness by week 28 after initiation of treatment of about 236.9 micrometers when on the HDq12 regimen; or of about 226 micrometers when on the HDq16 regimen (*e.g*., wherein the initial CRT is about 370 micrometers);
- A central retinal thickness by week 32 after initiation of treatment of about 250.2 micrometers when on the HDq12 regimen; or of about 229.2 micrometers when on the HDq16 regimen (*e.g*., wherein the initial CRT is about 370 micrometers);
- A central retinal thickness by week 36 after initiation of treatment of about 226.4 micrometers when on the HDq12 regimen; or of about 244.3 micrometers when on the HDq16 regimen (*e.g*., wherein the initial CRT is about 370 micrometers);
- A central retinal thickness by week 40 after initiation of treatment of about 236.8 micrometers when on the HDq12 regimen; or of about 243.7 micrometers when on the HDq16 regimen (*e.g*., wherein the initial CRT is about 370 micrometers);
- A central retinal thickness by week 44 after initiation of treatment of about 245.9 micrometers when on the HDq12 regimen; or of about 227.7 micrometers when on the HDq16 regimen (*e.g*., wherein the initial CRT is about 370 micrometers);
- A central retinal thickness by week 48 after initiation of treatment of about 226 micrometers when on the HDq12 regimen; or of about 226.9 micrometers when on the HDq16 regimen (*e.g*., wherein the initial CRT is about 370 micrometers);
- A central retinal thickness by week 52 after initiation of treatment of about 227.4 micrometers when on the HDq12 regimen; or of about 231.1 micrometers when on the HDq16 regimen (*e.g*., wherein the initial CRT is about 370 micrometers);
- A central retinal thickness by week 56 after initiation of treatment of about 234.3 micrometers when on the HDq12 regimen; or of about 233.2 micrometers when on the HDq16 regimen (*e.g*., wherein the initial CRT is about 370 micrometers);
- A central retinal thickness by week 60 after initiation of treatment of about 218.8 micrometers when on the HDq12 regimen; or of about 221.9 micrometers when on the HDq16 regimen (*e.g*., wherein the initial CRT is about 370 micrometers);
- A change in central retinal thickness of about -112 or -113 or -112.4 micrometers, between weeks 0 (treatment initiation) and 4 when on the HDq12 regimen;
- A change in central retinal thickness of about -13 or -14 or -13.8 micrometers, between weeks 4 and 8 when on the HDq12 regimen;
- A change in central retinal thickness of about -4 or -5 or -4.7 micrometers, between weeks 8 and 12 when on the HDq12 regimen;
- A change in central retinal thickness of about -24 or -25 micrometers, between weeks 20 and 24 when on the HDq12 regimen;
- A change in central retinal thickness of about -23 or -24 or -23.8 micrometers, between weeks 32 and 36 when on the HDq12 regimen;
- A change in central retinal thickness of about -19 or -20 or -19.7 micrometers, between weeks 44 and 48 when on the HDq12 regimen;
- A change in central retinal thickness of about -126, -127 or -126.6 micrometers, between weeks 0 and 4 when on the HDq16 regimen;
- A change in central retinal thickness of about -12, -13 or -12.9 micrometers, between weeks 4 and 8 when on the HDq16 regimen;
- A change in central retinal thickness of about -4 or -5 or -4.5 micrometers, between weeks 8 and 12 when on the HDq16 regimen;
- A change in central retinal thickness of about -39, -40 or -39.4 micrometers, between weeks 24 and 28 when on the HDq16 regimen;
- A change in central retinal thickness of about 0 or -1 or -0.6 micrometers, between weeks 36 and 40 when on the HDq16 regimen;
- A change in central retinal thickness of about -15 or -16 micrometers, between weeks 40 and 44 when on the HDq16 regimen;
- A change in central retinal thickness of about 0 or -1 or -0.8 micrometers, between weeks 44 and 48 when on the HDq16 regimen;
- A change in central retinal thickness of about -11, -12 or -11.3 micrometers, between weeks 56 and 60 when on the HDq16 regimen;
- A CRT by about week 4, 5, 6, 7 or 8 after initiation of treatment or a reduction in CRT by week 4, 5, 6, 7 or 8 after initiation of treatment which is maintained (*e.g*., within about ±10, ±11 or ±12 micrometers) thereafter during the treatment regimen, *e.g*., to at least week 48;
   - a change in central retinal thickness (CRT) by about week 4 following treatment initiation of about -130.2 micrometers when receiving the HDq12 regimen; and/or a change in CRT of about -111.6micrometers when receiving the HDq16 regimen, in a subject having PCV and nAMD;
   - a change in central retinal thickness (CRT) by about week 8 following treatment initiation of about -152.5 micrometers when receiving the HDq12 regimen; and/or a change in CRT of about -134.5micrometers when receiving the HDq16 regimen, in a subject having PCV and nAMD;
   - a change in central retinal thickness (CRT) by about week 12 following treatment initiation of about -160.9 micrometers when receiving the HDq12 regimen; and/or a change in CRT of about -147.7micrometers when receiving the HDq16 regimen, in a subject having PCV and nAMD;
   - a change in central retinal thickness (CRT) by about week 16 following treatment initiation of about -150 micrometers when receiving the HDq12 regimen; and/or a change in CRT of about -141.1 micrometers when receiving the HDq16 regimen, in a subject having PCV and nAMD;
   - a change in central retinal thickness (CRT) by about week 20 following treatment initiation of about -135.9 micrometers when receiving the HDq12 regimen; and/or a change in CRT of about -128 micrometers when receiving the HDq16 regimen, in a subject having PCV and nAMD;
   - a change in central retinal thickness (CRT) by about week 24 following treatment initiation of about -162.7 micrometers when receiving the HDq12 regimen; and/or a change in CRT of about -115.7 micrometers when receiving the HDq16 regimen, in a subject having PCV and nAMD;
   - a change in central retinal thickness (CRT) by about week 28 following treatment initiation of about -163.4 micrometers when receiving the HDq12 regimen; and/or a change in CRT of about -147.9 micrometers when receiving the HDq16 regimen, in a subject having PCV and nAMD;
   - a change in central retinal thickness (CRT) by about week 32 following treatment initiation of about -148.2 micrometers when receiving the HDq12 regimen; and/or a change in CRT of about -142.3 micrometers when receiving the HDq16 regimen, in a subject having PCV and nAMD;
   - a change in central retinal thickness (CRT) by about week 36 following treatment initiation of about -167.4 micrometers when receiving the HDq12 regimen; and/or a change in CRT of about -132.6 micrometers when receiving the HDq16 regimen, in a subject having PCV and nAMD;
   - a change in central retinal thickness (CRT) by about week 40 following treatment initiation of about -159.2 micrometers when receiving the HDq12 regimen; and/or a change in CRT of about -121.1 micrometers when receiving the HDq16 regimen, in a subject having PCV and nAMD;
   - a change in central retinal thickness (CRT) by about week 44 following treatment initiation of about -146.7 micrometers when receiving the HDq12 regimen; and/or a change in CRT of about -143.9 micrometers when receiving the HDq16 regimen, in a subject having PCV and nAMD;
   - a change in central retinal thickness (CRT) by about week 48 following treatment initiation of about -170.6 micrometers when receiving the HDq12 regimen; and/or a change in CRT of about -146.1 micrometers when receiving the HDq16 regimen, in a subject having PCV and nAMD;
   - a central retinal thickness (CRT) of about 260.2 micrometers when receiving the HDq12 regimen (*e.g*., wherein the baseline CRT is about 390.4 micrometers; and/or a CRT of about) 263.6micrometers when receiving the HDq16 regimen (*e.g*., when the baseline CRT is about 375.2 micrometers) by about week 4 following treatment initiation in a subject having PCV and nAMD;
   - a central retinal thickness (CRT) of about 237.9 micrometers when receiving the HDq12 regimen (*e.g*., wherein the baseline CRT is about 390.4 micrometers; and/or a CRT of about) 240.7 micrometers when receiving the HDq16 regimen (*e.g*., when the baseline CRT is about 375.2 micrometers) by about week 8 following treatment initiation in a subject having PCV and nAMD;
   - a central retinal thickness (CRT) of about 229.5 micrometers when receiving the HDq12 regimen (*e.g*., wherein the baseline CRT is about 390.4 micrometers; and/or a CRT of about) 227.5micrometers when receiving the HDq16 regimen (*e.g*., when the baseline CRT is about 375.2 micrometers) by about week 12 following treatment initiation in a subject having PCV and nAMD;
   - a central retinal thickness (CRT) of about 240.4 micrometers when receiving the HDq12 regimen (*e.g*., wherein the baseline CRT is about 390.4 micrometers; and/or a CRT of about) 234.1 micrometers when receiving the HDq16 regimen (*e.g*., when the baseline CRT is about 375.2 micrometers) by about week 16 following treatment initiation in a subject having PCV and nAMD;
   - a central retinal thickness (CRT) of about 254.5 micrometers when receiving the HDq12 regimen (*e.g*., wherein the baseline CRT is about 390.4 micrometers; and/or a CRT of about) 247.2 micrometers when receiving the HDq16 regimen (*e.g*., when the baseline CRT is about 375.2 micrometers) by about week 20 following treatment initiation in a subject having PCV and nAMD;
   - a central retinal thickness (CRT) of about 227.7 micrometers when receiving the HDq12 regimen (*e.g*., wherein the baseline CRT is about 390.4 micrometers; and/or a CRT of about) 259.5 micrometers when receiving the HDq16 regimen (*e.g*., when the baseline CRT is about 375.2 micrometers) by about week 24 following treatment initiation in a subject having PCV and nAMD;
   - a central retinal thickness (CRT) of about 227 micrometers when receiving the HDq12 regimen (*e.g*., wherein the baseline CRT is about 390.4 micrometers; and/or a CRT of about) 227.3 micrometers when receiving the HDq16 regimen (*e.g*., when the baseline CRT is about 375.2 micrometers) by about week 28 following treatment initiation in a subject having PCV and nAMD;
   - a central retinal thickness (CRT) of about 242.2 micrometers when receiving the HDq12 regimen (*e.g*., wherein the baseline CRT is about 390.4 micrometers; and/or a CRT of about) 232.9 micrometers when receiving the HDq16 regimen (*e.g*., when the baseline CRT is about 375.2 micrometers) by about week 32 following treatment initiation in a subject having PCV and nAMD;
   - a central retinal thickness (CRT) of about 223 micrometers when receiving the HDq12 regimen (*e.g*., wherein the baseline CRT is about 390.4 micrometers; and/or a CRT of about) 242.6 micrometers when receiving the HDq16 regimen (*e.g*., when the baseline CRT is about 375.2 micrometers) by about week 36 following treatment initiation in a subject having PCV and nAMD;
   - a central retinal thickness (CRT) of about 231.2 micrometers when receiving the HDq12 regimen (*e.g*., wherein the baseline CRT is about 390.4 micrometers; and/or a CRT of about) 254.1 micrometers when receiving the HDq16 regimen (*e.g*., when the baseline CRT is about 375.2 micrometers) by about week 40 following treatment initiation in a subject having PCV and nAMD;
   - a central retinal thickness (CRT) of about 243.7 micrometers when receiving the HDq12 regimen (*e.g*., wherein the baseline CRT is about 390.4 micrometers; and/or a CRT of about) 231.3 micrometers when receiving the HDq16 regimen (*e.g*., when the baseline CRT is about 375.2 micrometers) by about week 44 following treatment initiation in a subject having PCV and nAMD;
   - a central retinal thickness (CRT) of about 219.8 micrometers when receiving the HDq12 regimen (*e.g*., wherein the baseline CRT is about 390.4 micrometers; and/or a CRT of about) 229.1 micrometers when receiving the HDq16 regimen (*e.g*., when the baseline CRT is about 375.2 micrometers) by about week 48 following treatment initiation in a subject having PCV and nAMD;
- A reduction in choroidal neovascularization size of about 0.6, 1.5, 1 or 2 mm² (±3.7 mm²), or up to about 11 or 12 mm², by week 12 when receiving the HDq12 regimen (*e.g*., when the baseline size at treatment initiation is about 4, 5 or 6 mm²);
- A reduction in choroidal neovascularization size of about 2, 3, 4, 3.5 or 2.5 mm² (±5 mm²), or up to about 15 or 16 mm², by week 48 when receiving the HDq12 regimen (*e.g*., when the baseline size at treatment initiation is about 4, 5 or 6 mm²);
- A reduction in choroidal neovascularization size of about 2, 3, 4, 3.8, 2.8 mm² (±5 mm²), or up to 13 mm², by week 60 when receiving the HDq12 regimen (*e.g*., when the baseline size at treatment initiation is about 4, 5 or 6 mm²);
- A reduction in choroidal neovascularization size of about 1, 2, 0.5 or 1.5 mm² (±4.2 mm²), or up to 17 mm², by week 12 when receiving the HDq16 regimen (*e.g*., when the baseline size at treatment initiation is about 4, 4.5, 5, 6, 6.5 or 7 mm²);
- A reduction in choroidal neovascularization size (mm²) of about 1, 2, 1.4 or 3 mm² (±5 mm²), or up to 16 mm², by week 48 when receiving the HDq16 regimen (*e.g*., when the baseline size at treatment initiation is about 4, 4.5, 5, 6, 6.5 or 7 mm²);
- A reduction in choroidal neovascularization size of about 2, 3, 4 or 3.7 mm² (±5.7 mm²), or up to 17mm², by week 60 when receiving the HDq16 regimen (*e.g*., when the baseline size at treatment initiation is about 4, 4.5, 5, 6, 6.5 or 7 mm²);
- At baseline has a free aflibercept concentration in plasma of about 0 mg/L (or <0.0156 mg/L);
- At about 4 hours after treatment initiation of HDq12 or HDq16, has a free aflibercept concentration in plasma of about 0.04 (±0.06) or 0 mg/L (or <0.0156 mg/L);
- At about 8 hours after treatment initiation of HDq12 or HDq16, has a free aflibercept concentration in plasma of about 0.05 (±3.8), 0.1 (±0.102) or 0.067 mg/L;
- At about day 2 after treatment initiation of HDq12 or HDq16, has a free aflibercept concentration in plasma of about 0.11 (+2.21), 0.146 (±0.110) or 0.0903 mg/L;
- At about day 3 after treatment initiation of HDq12 or HDq16, has a free aflibercept concentration in plasma of about 0.11 (±2.06), 0.137 (±0.0947) or 0.112 mg/L;
- At about day 5 after treatment initiation of HDq12 or HDq16, has a free aflibercept concentration in plasma of about 0.08 (±1.86), 0.09 (±0.048) or 0.085 mg/L;
- At about day 8 after treatment initiation of HDq12 or HDq16, has a free aflibercept concentration in plasma of about 0.07 (±1.75), 0.0794 (±0.041) or 0.068 mg/L ;
- At about day 15 after treatment initiation of HDq12 or HDq16, has a free aflibercept concentration in plasma of about 0.04 (+1.76), 0.044 (±0.0199) or 0.039 mg/L ;
- At about day 22 after treatment initiation of HDq12 or HDq16, has a free aflibercept concentration in plasma of about 0.02 (+1.76), 0.021 (±0.0148) or 0.023 mg/L;
- At about day 29 after treatment initiation of HDq12 or HDq16, has a free aflibercept concentration in plasma of about 0.0077 (±0.0096) or 0 mg/L (or <0.0156 mg/L);
- At about 48 weeks after treatment initiation of the HDq12, has a free aflibercept concentration in plasma of about 0.014 mg/L (±0.018);
- At about 48 weeks after treatment initiation of the HDq16, has a free aflibercept concentration in plasma of about 0.00056 mg/L (±0.0038);
- At about 28 weeks after treatment initiation of the HDq12, has a free aflibercept concentration in plasma of about 0.00088 mg/L (±0.0053);
- At about 28 weeks after treatment initiation of the HDq16, has a free aflibercept concentration in plasma of about 0.015 mg/L (±0.016);
- At about 12 weeks after treatment initiation of the HDq12, has a free aflibercept concentration in plasma of about 0.02 mg/L (±2) or about 0.028 mg/L (±0.023);
- At about 12 weeks after treatment initiation of the HDq16, has a free aflibercept concentration in plasma of about 0.02 mg/L (±2) or about 0.029 mg/L (±0.023);
- At about 4 weeks after treatment initiation of the HDq12, has a free aflibercept concentration in plasma of about 0.017 mg/L (±0.016);
- At about 4 weeks after treatment initiation of the HDq16, has a free aflibercept concentration in plasma of about 0.017 mg/L (±0.016);
- At about 4 hours post-dose after treatment initiation of HDq12 or HDq16, has an adjusted bound aflibercept concentration in plasma of about 0.00 mg/L;
- At about 8 hours post-dose after treatment initiation of HDq12 or HDq16, has an adjusted bound aflibercept concentration in plasma of about 0.00 mg/L;
- At about day 2 after treatment initiation of HDq12 or HDq16, has an adjusted bound aflibercept concentration in plasma of about 0.06 (±3.50) or 0.12 (±0.19) mg/L;
- At about day 3 after treatment initiation of HDq12 or HDq16, has an adjusted bound aflibercept concentration in plasma of about 0.13 (±2.07) or 0.17 (±0.155) mg/L;
- At about day 5 after treatment initiation of HDq12 or HDq16, has an adjusted bound aflibercept concentration in plasma of about 0.18 (±1.9) or 0.22 (±0.157) mg/L;
- At about day 8 after treatment initiation of HDq12 or HDq16, has an adjusted bound aflibercept concentration in plasma of about 0.31 (±1.56) or 0.33 (±0.135) mg/L;
- At about day 15 after treatment initiation of HDq12 or HDq16, has an adjusted bound aflibercept concentration in plasma of about 0.37 (±1.50) or 0.39 (±0.130) mg/L;
- At about day 22 after treatment initiation of HDq12 or HDq16, has an adjusted bound aflibercept concentration in plasma of about 0.25 (±3.00) or 0.34 (±0.155) mg/L;
- At about day 29 after treatment initiation of HDq12 or HDq16, has an adjusted bound aflibercept concentration in plasma of about 0.32 (±1.39) or 0.33 (±0.0953) mg/L;
- At about 48 weeks after treatment initiation of HDq12, has an adjusted bound aflibercept concentration in plasma of about 0.3 or 0.38 mg/l (± 2.4 or 0.18);
- At about 48 weeks after treatment initiation of HDq16, has an adjusted bound aflibercept concentration in plasma of about 0.17 or 0.2 mg/l (± 2 or 0.11);
- At about 28 weeks after treatment initiation of HDq12, has an adjusted bound aflibercept concentration in plasma of about 0.2 or 0.22 mg/l (± 1.8 or 0.12);
- At about 28 weeks after treatment initiation of HDq16, has an adjusted bound aflibercept concentration in plasma of about 0.32 or 0.37 mg/l (± 2 or 0.15);
- At about 12 weeks after treatment initiation of HDq12, has an adjusted bound aflibercept concentration in plasma of about 0.6 mg/l (± 1.5 or 0.22);
- At about 12 weeks after treatment initiation of HDq16, has an adjusted bound aflibercept concentration in plasma of about 0.6 mg/l (± 1.6 or 0.22);
- At about 4 weeks after treatment initiation of HDq12, has an adjusted bound aflibercept concentration in plasma of about 0.34 or 0.38 mg/l (± 1.7 or 0.13);
- At about 4 weeks after treatment initiation of HDq16, has an adjusted bound aflibercept concentration in plasma of about 0.33 or 0.36 mg/l (± 1.7 or 0.14);
- Non-inferior BVCA compared to that of aflibercept which is intravitreally dosed at 2 mg approximately every 4 weeks for the first 5 injections followed by 2 mg approximately once every 8 weeks or once every 2 months;
- Ocular (*e.g*., intraocular pressure) and non-ocular safety (*e.g*., hypertensive events or APTC events) or death rate, in a subject suffering from DME, similar to that of aflibercept which is intravitreally dosed at 2 mg approximately every 4 weeks for the first 3 or 4 or 5 injections followed by 2 mg approximately once every 8 weeks or once every 2 months;
- Efficacy and/or safety, in a subject suffering from nAMD, similar to that of aflibercept which is intravitreally dosed at 2 mg approximately every 4 weeks for the first 3 injections followed by 2 mg approximately once every 8 weeks or once every 2 months, *e.g*., wherein efficacy is measured as increase in BCVA and/or reduction in central retinal thickness, *e.g*., wherein safety is as measured as the incidence of adverse events (treatment-emergent adverse events occurring anytime within 30 days of any injection) such as blood pressure increase, intraocular pressure increase, visual impairment, vitreous floaters, vitreous detachment, iris neovascularization and/or vitreous hemorrhage;
- A peak free aflibercept concentration in plasma at about 1, 1.5, 2 or 3 days after initiation of treatment;
- A maximum free aflibercept concentration in plasma of about 0.1, 0.12, 0.13, 0.14 or 0.2 mg/l;
- A plasma half-life of free aflibercept of about 10, 11, 12 or 13 days;
- Peak adjusted bound aflibercept concentration in plasma at about 14 days after initiation of treatment;
- Maximum free aflibercept concentration in plasma of about 0.4 mg/l; and/or
- A plasma half-life of free aflibercept of about 281 days.

Thus, the present invention provides the following:
- A method for achieving a change in central retinal thickness (CRT) by about week 4 following treatment initiation of about -130.2 micrometers when receiving the HDq12 regimen; and/or a change in CRT of about -111.6 micrometers when receiving the HDq16 regimen; a change in central retinal thickness (CRT) by about week 8 following treatment initiation of about -152.5 micrometers when receiving the HDq12 regimen; and/or a change in CRT of about -134.5 micrometers when receiving the HDq16 regimen; a change in central retinal thickness (CRT) by about week 12 following treatment initiation of about -160.9 micrometers when receiving the HDq12 regimen; and/or a change in CRT of about -147.7 micrometers when receiving the HDq16 regimen; a change in central retinal thickness (CRT) by about week 16 following treatment initiation of about -150 micrometers when receiving the HDq12 regimen; and/or a change in CRT of about -141.1 micrometers when receiving the HDq16 regimen; a change in central retinal thickness (CRT) by about week 20 following treatment initiation of about -135.9 micrometers when receiving the HDq12 regimen; and/or a change in CRT of about -128 micrometers when receiving the HDq16 regimen; a change in central retinal thickness (CRT) by about week 24 following treatment initiation of about -162.7 micrometers when receiving the HDq12 regimen; and/or a change in CRT of about -115.7 micrometers when receiving the HDq16 regimen; a change in central retinal thickness (CRT) by about week 28 following treatment initiation of about -163.4 micrometers when receiving the HDq12 regimen; and/or a change in CRT of about -147.9 micrometers when receiving the HDq16 regimen; a change in central retinal thickness (CRT) by about week 32 following treatment initiation of about - 148.2 micrometers when receiving the HDq12 regimen; and/or a change in CRT of about -142.3 micrometers when receiving the HDq16 regimen; a change in central retinal thickness (CRT) by about week 36 following treatment initiation of about -167.4 micrometers when receiving the HDq12 regimen; and/or a change in CRT of about - 132.6 micrometers when receiving the HDq16 regimen; a change in central retinal thickness (CRT) by about week 40 following treatment initiation of about -159.2 micrometers when receiving the HDq12 regimen; and/or a change in CRT of about - 121.1 micrometers when receiving the HDq16 regimen; a change in central retinal thickness (CRT) by about week 44 following treatment initiation of about -146.7 micrometers when receiving the HDq12 regimen; and/or a change in CRT of about - 143.9 micrometers when receiving the HDq16 regimen; a change in central retinal thickness (CRT) by about week 48 following treatment initiation of about -170.6 micrometers when receiving the HDq12 regimen; and/or a change in CRT of about - 146.1 micrometers when receiving the HDq16 regimen; a central retinal thickness (CRT) of about 260.2 micrometers when receiving the HDq12 regimen (*e.g.*, wherein the baseline CRT is about 390.4 micrometers; and/or a CRT of about) 263.6micrometers when receiving the HDq16 regimen (*e.g.*, when the baseline CRT is about 375.2 micrometers) by about week 4 following treatment initiation; a central retinal thickness (CRT) of about 237.9 micrometers when receiving the HDq12 regimen (*e.g.*, wherein the baseline CRT is about 390.4 micrometers; and/or a CRT of about) 240.7 micrometers when receiving the HDq16 regimen (*e.g.*, when the baseline CRT is about 375.2 micrometers) by about week 8 following treatment initiation; a central retinal thickness (CRT) of about 229.5 micrometers when receiving the HDq12 regimen (*e.g.*, wherein the baseline CRT is about 390.4 micrometers; and/or a CRT of about) 227.5micrometers when receiving the HDq16 regimen (*e.g.*, when the baseline CRT is about 375.2 micrometers) by about week 12 following treatment initiation; a central retinal thickness (CRT) of about 240.4 micrometers when receiving the HDq12 regimen (*e.g.*, wherein the baseline CRT is about 390.4 micrometers; and/or a CRT of about) 234.1 micrometers when receiving the HDq16 regimen (*e.g.*, when the baseline CRT is about 375.2 micrometers) by about week 16 following treatment initiation; a central retinal thickness (CRT) of about 254.5 micrometers when receiving the HDq12 regimen (*e.g.*, wherein the baseline CRT is about 390.4 micrometers; and/or a CRT of about) 247.2 micrometers when receiving the HDq16 regimen (*e.g.*, when the baseline CRT is about 375.2 micrometers) by about week 20 following treatment initiation; a central retinal thickness (CRT) of about 227.7 micrometers when receiving the HDq12 regimen (*e.g.*, wherein the baseline CRT is about 390.4 micrometers; and/or a CRT of about) 259.5 micrometers when receiving the HDq16 regimen (*e.g.*, when the baseline CRT is about 375.2 micrometers) by about week 24 following treatment initiation; a central retinal thickness (CRT) of about 227 micrometers when receiving the HDq12 regimen (*e.g.*, wherein the baseline CRT is about 390.4 micrometers; and/or a CRT of about) 227.3 micrometers when receiving the HDq16 regimen (*e.g.*, when the baseline CRT is about 375.2 micrometers) by about week 28 following treatment initiation; a central retinal thickness (CRT) of about 242.2 micrometers when receiving the HDq12 regimen (*e.g.*, wherein the baseline CRT is about 390.4 micrometers; and/or a CRT of about) 232.9 micrometers when receiving the HDq16 regimen (*e.g.*, when the baseline CRT is about 375.2 micrometers) by about week 32 following treatment initiation; a central retinal thickness (CRT) of about 223 micrometers when receiving the HDq12 regimen (*e.g.*, wherein the baseline CRT is about 390.4 micrometers; and/or a CRT of about) 242.6 micrometers when receiving the HDq16 regimen (*e.g.*, when the baseline CRT is about 375.2 micrometers) by about week 36 following treatment initiation; a central retinal thickness (CRT) of about 231.2 micrometers when receiving the HDq12 regimen (*e.g.*, wherein the baseline CRT is about 390.4 micrometers; and/or a CRT of about) 254.1 micrometers when receiving the HDq16 regimen (*e.g.*, when the baseline CRT is about 375.2 micrometers) by about week 40 following treatment initiation; a central retinal thickness (CRT) of about 243.7 micrometers when receiving the HDq12 regimen (*e.g.*, wherein the baseline CRT is about 390.4 micrometers; and/or a CRT of about) 231.3 micrometers when receiving the HDq16 regimen (*e.g.*, when the baseline CRT is about 375.2 micrometers) by about week 44 following treatment initiation; and/or a central retinal thickness (CRT) of about 219.8 micrometers when receiving the HDq12 regimen (*e.g.*, wherein the baseline CRT is about 390.4 micrometers; and/or a CRT of about) 229.1 micrometers when receiving the HDq16 regimen (*e.g*., when the baseline CRT is about 375.2 micrometers) by about week 48 following treatment initiation in a subject in need thereof having PCV and nAMD; comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an increase in BCVA of about 3.0 letters (ETDRS) when receiving the HDq12 regimen; and/or an increase of about 3.0 letters when receiving the HDq16 regimen by about week 4 following treatment initiation; an increase in BCVA of about 6.0 letters (ETDRS) when receiving the HDq12 regimen; and/or an increase of about 5.0 letters when receiving the HDq16 regimen by about week 8 following treatment initiation; an increase in BCVA of about 7.0 letters (ETDRS) when receiving the HDq12 regimen; and/or an increase of about 6.0 letters when receiving the HDq16 regimen by about week 12 following treatment initiation; an increase in BCVA of about 7.0 letters (ETDRS) when receiving the HDq12 regimen; and/or an increase of about 7.0 letters when receiving the HDq16 regimen by about week 16 following treatment initiation; an increase in BCVA of about 7.0 letters (ETDRS) when receiving the HDq12 regimen; and/or an increase of about 7.0 letters when receiving the HDq16 regimen by about week 20 following treatment initiation; an increase in BCVA of about 7.0 letters (ETDRS) when receiving the HDq12 regimen; and/or an increase of about 5.0 letters when receiving the HDq16 regimen by about week 24 following treatment initiation; an increase in BCVA of about 9.0 letters (ETDRS) when receiving the HDq12 regimen; and/or an increase of about 7.0 letters when receiving the HDq16 regimen by about week 28 following treatment initiation; an increase in BCVA of about 8.0 letters (ETDRS) when receiving the HDq12 regimen; and/or an increase of about 10.0 letters when receiving the HDq16 regimen by about week 32 following treatment initiation; an increase in BCVA of about 8.0 letters (ETDRS) when receiving the HDq12 regimen; and/or an increase of about 10.0 letters when receiving the HDq16 regimen by about week 36 following treatment initiation; an increase in BCVA of about 9.0 letters (ETDRS) when receiving the HDq12 regimen; and/or an increase of about 8.0 letters when receiving the HDq16 regimen by about week 40 following treatment initiation; an increase in BCVA of about 9.0 letters (ETDRS) when receiving the HDq12 regimen; and/or an increase of about 8.0 letters when receiving the HDq16 regimen by about week 44 following treatment initiation; an increase in BCVA of about 9.0 or 10 letters (ETDRS) when receiving the HDq12 regimen; and/or an increase of about 9.0 letters when receiving the HDq16 regimen by about week 48 following treatment initiation; in an subject in need thereof having PCV and nAMD comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) after the immediately preceding dose;
- A method for achieving a BCVA of about 59 letters (ETDRS) when receiving the HDq12 regimen (*e.g.*, wherein the baseline BCVA is about 57 letters); and/or a BCVA of about 64 letters (ETDRS) when receiving the HDq16 regimen (*e.g.*, wherein the baseline BCVA is about 60 letters); by about week 4 after treatment initiation; a BCVA of about 63 letters (ETDRS) when receiving the HDq12 regimen (*e.g.*, wherein the baseline BCVA is about 57 letters); and/or a BCVA of about 65 letters (ETDRS) when receiving the HDq16 regimen (*e.g.*, wherein the baseline BCVA is about 60 letters); by about week 8 after treatment initiation; a BCVA of about 63 letters (ETDRS) when receiving the HDq12 regimen (*e.g.*, wherein the baseline BCVA is about 57 letters); and/or a BCVA of about 66 letters (ETDRS) when receiving the HDq16 regimen (*e.g.*, wherein the baseline BCVA is about 60 letters); by about week 12 after treatment initiation; a BCVA of about 64 letters (ETDRS) when receiving the HDq12 regimen (*e.g.*, wherein the baseline BCVA is about 57 letters); and/or a BCVA of about 67 letters (ETDRS) when receiving the HDq16 regimen (*e.g.*, wherein the baseline BCVA is about 60 letters); by about week 16 after treatment initiation; a BCVA of about 64 letters (ETDRS) when receiving the HDq12 regimen (*e.g.*, wherein the baseline BCVA is about 57 letters); and/or a BCVA of about 68 letters (ETDRS) when receiving the HDq16 regimen (*e.g.*, wherein the baseline BCVA is about 60 letters); by about week 20 after treatment initiation; a BCVA of about 64 letters (ETDRS) when receiving the HDq12 regimen (*e.g.*, wherein the baseline BCVA is about 57 letters); and/or a BCVA of about 67 letters (ETDRS) when receiving the HDq16 regimen (*e.g*., wherein the baseline BCVA is about 60 letters); by about week 24 after treatment initiation; a BCVA of about 65 letters (ETDRS) when receiving the HDq12 regimen (*e.g*., wherein the baseline BCVA is about 57 letters); and/or a BCVA of about 68 letters (ETDRS) when receiving the HDq16 regimen (*e.g*., wherein the baseline BCVA is about 60 letters); by about week 28 after treatment initiation; a BCVA of about 64 letters (ETDRS) when receiving the HDq12 regimen (*e.g*., wherein the baseline BCVA is about 57 letters); and/or a BCVA of about 71 letters (ETDRS) when receiving the HDq16 regimen (*e.g*., wherein the baseline BCVA is about 60 letters); by about week 32 after treatment initiation; a BCVA of about 64 letters (ETDRS) when receiving the HDq12 regimen (*e.g*., wherein the baseline BCVA is about 57 letters); and/or a BCVA of about 71I letters (ETDRS) when receiving the HDq16 regimen (*e.g*., wherein the baseline BCVA is about 60 letters); by about week 36 after treatment initiation; a BCVA of about 65letters (ETDRS) when receiving the HDq12 regimen (*e.g*., wherein the baseline BCVA is about 57 letters); and/or a BCVA of about 69 letters (ETDRS) when receiving the HDq16 regimen (*e.g*., wherein the baseline BCVA is about 60 letters); by about week 40 after treatment initiation; a BCVA of about 65letters (ETDRS) when receiving the HDq12 regimen (*e.g*., wherein the baseline BCVA is about 57 letters); and/or a BCVA of about 68 letters (ETDRS) when receiving the HDq16 regimen (*e.g*., wherein the baseline BCVA is about 60 letters); by about week 44 after treatment initiation; and/or a BCVA of about 65 letters (ETDRS) when receiving the HDq12 regimen (*e.g*., wherein the baseline BCVA is about 57 letters); and/or a BCVA of about 69 letters (ETDRS) when receiving the HDq16 regimen (*e.g*., wherein the baseline BCVA is about 60 letters); by about week 48 after treatment initiation; in a subject in need thereof having polypoidal choroidal vasculopathy (PCV) and nAMD; comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose;
- A method for achieving a reduction in total lesion size of about 0.4475 (±2.3) or 0.5 (±2.3) mm² when receiving the HDq12 regimen or about 0.3923 (±2.6) or 0.4 (±2.6) mm² when receiving the HDq16 regimen, by about week 12 following treatment initiation; a reduction in total lesion size of about 0.3628 (±2.9) or 0.4 (±2.9) mm² when receiving the HDq12 regimen or about 0.2856 (±3.2) or 0.3 (±3.2) mm² when receiving the HDq16 regimen, by about week 48 following treatment initiation; and/or, a reduction in total lesion size of about 0.5199 (±2.8) or 0.6 (+2.8) mm² when receiving the HDq12 regimen or about 0.3530 (±3.2) or 0.4 (±3.2) mm² when receiving the HDq16 regimen, by about week 60 following treatment initiation, in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) or 20 weeks (HDq20 regimen) after the immediately preceding dose.
- A method for achieving an increase in BCVA as measured by the Early Treatment Diabetic Retinopathy Study (ETDRS) visual acuity chart by week 4, 8, 12, 16, 20, 24, 28, 32, 36, 40, 44, 48, 52, 56 or 60 after treatment initiation by ≥5, ≥10, ≥15, or ≥20 letters in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving no intraretinal fluid (IRF) and no subretinal fluid by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a decrease in choroidal neovascularization (CNV) size by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a decrease in total lesion CNV area from baseline by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a loss of IRF and/or SRF in the center subfield by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a decrease in central subfield retinal thickness (CST) by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving an increase in vision-related quality of life as measured by the National Eye Institute Visual Functioning Questionnaire-25 (NEI-VFQ-25) by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving an ETDRS letter score of at least 69 (approximate 20/40 Snellen equivalent) of BCVA by week 4, 8, 12, 16, 20, 24, 28, 32, 36, 40, 44, 48, 52, 56 or 60 after treatment initiation; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving an improvement in best corrected visual acuity (BVCA) by week 4, 8, 12, 16, 20, 24, 28, 32, 36, 40, 44, 48, 52, 56 or 60 from start of treatment; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving an increase in BCVA as measured by the Early Treatment Diabetic Retinopathy Study (ETDRS) visual acuity chart or Snellen equivalent by week 4, 8, 12, 16, 20, 24, 28, 32, 36, 40, 44, 48, 52, 56 or 60 weeks from start of treatment by ≥2 letters, ≥3 letters, ≥4 letters, ≥5 letters, ≥6 letters or ≥7 letters; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving an improvement in BCVA, by 4 weeks after initiation of treatment, of about 2 or 3 letters (ETDRS or Snellen equivalent) when on HDq12 regimen; or of about 3 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 8 weeks after initiation of treatment, of about 5 letters (ETDRS or Snellen equivalent) when on HDq12 regimen; or of about 4 or 5 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 12 weeks after initiation of treatment, of about 5 or 6 letters (ETDRS or Snellen equivalent) when on HDq12 regimen; or of about 5 or 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 16 weeks after initiation of treatment, of about 6 letters (ETDRS or Snellen equivalent) when on HDq12 regimen; or of about 6 or 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 24 weeks after initiation of treatment, of about 5 or 6 letters (ETDRS or Snellen equivalent) when on HDq12 regimen; or of about 5 or 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 28 weeks after initiation of treatment, of about 7 letters (ETDRS or Snellen equivalent) when on HDq12 regimen; or of about 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 32 weeks after initiation of treatment, of about 6 or 7 letters (ETDRS or Snellen equivalent) when on HDq12 regimen; or of about 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 36 weeks after initiation of treatment, of about 6 or 7 letters (ETDRS or Snellen equivalent) when on HDq12 regimen; or of about 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 40 weeks after initiation of treatment, of about 6 or 7 letters (ETDRS or Snellen equivalent) when on HDq12 regimen; or of about 5 or 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 44 weeks after initiation of treatment, of about 7 letters (ETDRS or Snellen equivalent) when on HDq12 regimen; or of about 5 or 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 48 weeks after initiation of treatment, of about 6 or 7 letters (ETDRS or Snellen equivalent) when on HDq12 regimen; or of about 6 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 52 weeks after initiation of treatment, of about 7 or 8 letters (ETDRS or Snellen equivalent) when on HDq12 regimen; or of about 6 or 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 56 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, from about 48 to about 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA, by 60 weeks after initiation of treatment, of about 5, 10 or 15 letters (ETDRS or Snellen equivalent) when on the HDq12 regimen; or the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving an improvement in BCVA by about week 8, 9, 10, 11 or 12 after initiation of treatment which is maintained within about ±1 or ±2 ETDRS letters (or Snellen equivalent) thereafter during the treatment regimen to at least week 48 or 60 in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a BCVA by 4 weeks after initiation of treatment of about 63 letters (ETDRS or Snellen equivalent) when on the HDq12 regimen; or a BCVA of about 63 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 8 weeks after initiation of treatment of about 65 letters (ETDRS or Snellen equivalent) when on the HDq12 regimen; or a BCVA of about 65 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by12 weeks after initiation of treatment of about 66 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 16 weeks after initiation of treatment of about 66 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 20 weeks after initiation of treatment of about 66 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 24 weeks after initiation of treatment of about 66 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 28 weeks after initiation of treatment of about 67 letters (ETDRS or Snellen equivalent) when on the HDq12 regimen; or a BCVA of about 66letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 32 weeks after initiation of treatment of about 67 letters (ETDRS or Snellen equivalent) when on the HDq12 regimen; or a BCVA of about 67 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 36 weeks after initiation of treatment of about 67 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 40 weeks after initiation of treatment of about 67 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 44 weeks after initiation of treatment of about 68 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 48 weeks after initiation of treatment of about 67 letters (ETDRS or Snellen equivalent) when on the HDq12 regimen; or a BCVA of about 66 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 52 weeks after initiation of treatment of about 67 or 68 letters (ETDRS or Snellen equivalent) when on the HDq12 regimen; or a BCVA of about 66 or 67 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 56 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA by 60 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a BCVA between about week 48 and about week 60 after initiation of treatment of about 66 to about 72 letters (ETDRS or Snellen equivalent) when on the HDq12 regimen; or a BCVA of about 66 to about 70 letters (ETDRS or Snellen equivalent) when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a retina without fluid (total fluid, intraretinal fluid [IRF] and/or subretinal fluid [SRF]) in center subfield by about 4, 8, 12, 16, 20, 24, 28, 32, 36, 40, 44, 48, 52, 56 or 60 weeks from start of treatment; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving no subretinal pigment epithelium fluid by about 4, 8, 12, 16, 20, 24, 28, 32, 36, 40, 44, 48, 52, 56 or 60 weeks from start of treatment; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving no fluid leakage on fluorescein angiography (FA) in the retina by about 4, 8, 12, 16, 20, 24, 28, 32, 36, 40, 44, 48, 52, 56 or 60 weeks from start of treatment; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a decrease in central retinal thickness (CRT) of at least about 100, 125, 130, 135, 140, 141, 142, 143, 144, 145, 146, 147, 148, 149 or 150 micrometers by week 4, 8, 12, 16, 20, 24, 28, 32, 36, 40, 44, 48 or 60 from start of treatment; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a change in central retinal thickness, by 4 weeks after initiation of treatment of about -120 or -121 or -122 or -122.4 or -120.2 micrometers when on the HDq12 regimen; or of about -126 or -127 or-126.6 or -126.3 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 8 weeks after initiation of treatment of about -132, -133, -134, -135 or -136 or -136.2 or -132.8 micrometers when on the HDq12 regimen; or of about -139 or -140 or -139.5 or -139.6 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 12 weeks after initiation of treatment of about -136, -137, -138, -139, -140 or -141 or -140.9 or -136.6 micrometers when on the HDq12 regimen; or of about -144 or -143 or -143.5 micrometers when on the HDq16 regimen in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 16 weeks after initiation of treatment of about -120 or -121 or -122 or -123 or -124 or -123.4 or -120.1 micrometers when on the HDq12 regimen; or of about -132 or -133 or -132.1 or -133.1 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 20 weeks after initiation of treatment of about -110 or -111 or -112 or -113 or -114 or -113.6 or -110.9 micrometers when on the HDq12 regimen; or of about -115 or -116 or -117 or -118 or - 115.8 or -117.7 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 24 weeks after initiation of treatment of about -134, -135, -136 or -137 or -138 or -137.6 or -134.9 micrometers when on the HDq12 regimen; or of about -105 or -106 or -107 or -108 or -105.3 or -107.8 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 28 weeks after initiation of treatment of about -130, -131 or -132 or -133 or -134 or -133.7 or -130.7 micrometers when on the HDq12 regimen; or of about -144 or -145 or -146 or -147 or -148 or -144.7 or -147.2 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 32 weeks after initiation of treatment of about -118 or -19 or -120 or -121 or -120.4 or -118.1 micrometers when on the HDq12 regimen; or of about -141 or -142 or -143 or -144 or -141.5 or -144 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 36 weeks after initiation of treatment of about -142 or -143 or -144 or -144.2 or -142.2 micrometers when on the HDq12 regimen; or of about -126 or -127, -128 or -129 or -130 or -131 or -126.4 or - 130.5 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 40 weeks after initiation of treatment of about -131, -132, -133 or -134 or -133.8 or -131.2 micrometers when on the HDq12 regimen; or of about -127, -128 or -127.5 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 44 weeks after initiation of treatment of about -120 or -121 or -122 or -123 or -124 or -125 or -124.7 or -120.3 micrometers when on the HDq12 regimen; or of about -143, -144 or -145 or -144.8 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a change in central retinal thickness, by 48 weeks after initiation of treatment of about -142 or -143 or -144 or -144.4 or -142.3 micrometers when on the HDq12 regimen; or of about -143 or -144 or -145 or -146 or -147 or -148 or -143.8 or - 147.1 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 4 after initiation of treatment of about 248.2 micrometers when on the HDq12 regimen; or of about 244.1 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 8 after initiation of treatment of about 234.4 micrometers when on the HDq12 regimen; or of about 231.2 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 12 after initiation of treatment of about 229.7 micrometers when on the HDq12 regimen; or of about 226.7 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 16 after initiation of treatment of about 247.2 micrometers when on the HDq12 regimen; or of about 238.6 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 20 after initiation of treatment of about 257 micrometers when on the HDq12 regimen; or of about 254.9 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 24 after initiation of treatment of about 233 micrometers when on the HDq12 regimen; or of about 265.4 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 28 after initiation of treatment of about 236.9 micrometers when on the HDq12 regimen; or of about 226 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 32 after initiation of treatment of about 250.2 micrometers when on the HDq12 regimen; or of about 229.2 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 36 after initiation of treatment of about 226.4 micrometers when on the HDq12 regimen; or of about 244.3 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 40 after initiation of treatment of about 236.8 micrometers when on the HDq12 regimen; or of about 243.7 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 44 after initiation of treatment of about 245.9 micrometers when on the HDq12 regimen; or of about 227.7 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 48 after initiation of treatment of about 226 micrometers when on the HDq12 regimen; or of about 226.9 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 52 after initiation of treatment of about 227.4 micrometers when on the HDq12 regimen; or of about 231.1 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 56 after initiation of treatment of about 234.3 micrometers when on the HDq12 regimen; or of about 233.2 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by week 60 after initiation of treatment of about 218.8 micrometers when on the HDq12 regimen; or of about 221.9 micrometers when on the HDq16 regimen; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a central retinal thickness by about week 4, 5, 6, 7 or 8 after initiation of treatment or a reduction in CRT by week 4, 5, 6, 7 or 8 after initiation of treatment which is maintained (within about ±10, ±11 or ±12 micrometers) thereafter during the treatment regimen to at least week 48; in a subject in need thereof having an angiogenic eye disorder (preferably nAMD) comprising administering to an eye of the subject, a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving at about 4 hours after treatment initiation of HDq12 or HDq16, a free aflibercept concentration in plasma of about 0.0409 (±0.0605), below the limit of quantitation (BLQ) or 0 mg/L (or <0.0156 mg/L); in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about 8 hours after treatment initiation of HDq12 or HDq16, a free aflibercept concentration in plasma of about 0.05 (±3.78), 0.0973 (±0.102) or 0.0672 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 2 after treatment initiation of HDq12 or HDq16, a free aflibercept concentration in plasma of about 0.11 (±2.21), 0.146 (±0.110) or 0.0903 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 3 after treatment initiation of HDq12 or HDq16, a free aflibercept concentration in plasma of about 0.11 (±2.06), 0.137 (±0.0947) or 0.112 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 5 after treatment initiation of HDq12 or HDq16, a free aflibercept concentration in plasma of about 0.08 (±1.86), 0.0933 (±0.0481) or 0.0854 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 8 after treatment initiation of HDq12 or HDq16, a free aflibercept concentration in plasma of about 0.07 (±1.75), 0.0794 (±0.0413) or 0.0682 mg/L ; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 15 after treatment initiation of HDq12 or HDq16, a free aflibercept concentration in plasma of about 0.04 (±1.76), 0.0435 (±0.0199) or 0.0385 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 22 after treatment initiation of HDq12 or HDq16, a free aflibercept concentration in plasma of about 0.02 (±1.76), 0.0213 (±0.0148) or 0.0232 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 29 after treatment initiation of HDq12 or HDq16, a free aflibercept concentration in plasma of about 0.00766 (±0.00958) or BLQ or 0 mg/L (or <0.0156 mg/L); in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about 4 hours post-dose after treatment initiation of HDq12 or HDq16, an adjusted bound aflibercept concentration in plasma of about 0.00 mg/L; in a subject in need thereof having an angiogenic eye disorder (preferably nAMD) comprising administering to an eye of the subject, a single initial dose of about 8 mg or more aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about 8 hours post-dose after treatment initiation of HDq12 or HDq16, an adjusted bound aflibercept concentration in plasma of about 0.00 mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 2 after treatment initiation of HDq12 or HDq16, an adjusted bound aflibercept concentration in plasma of about 0.06 (±3.50) or 0.124 (±0.186) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 3 after treatment initiation of HDq12 or HDq16, an adjusted bound aflibercept concentration in plasma of about 0.13 (±2.07) or 0.173 (±0.155) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 5 after treatment initiation of HDq12 or HDq16, an adjusted bound aflibercept concentration in plasma of about 0.18 (±1.88) or 0.223 (±0.157) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 8 after treatment initiation of HDq12 or HDq16, an adjusted bound aflibercept concentration in plasma of about 0.31 (±1.56) or 0.334 (±0.135) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 15 after treatment initiation of HDq12 or HDq16, an adjusted bound aflibercept concentration in plasma of about 0.37 (±1.50) or 0.393 (±0.130) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 22 after treatment initiation of HDq12 or HDq16, an adjusted bound aflibercept concentration in plasma of about 0.25 (±3.00) or 0.335 (±0.155) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving at about day 29 after treatment initiation of HDq12 or HDq16, an adjusted bound aflibercept concentration in plasma of about 0.32 (±1.39) or 0.331 (±0.0953) mg/L; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.
- A method for achieving a non-inferior BVCA compared to that of aflibercept which is intravitreally dosed at 2 mg approximately every 4 weeks for the first 5 injections followed by 2 mg approximately once every 8 weeks or once every 2 months; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a peak free aflibercept concentration in plasma at about 1, 1.5, 2 or 3 days after initiation of treatment; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20 regimen) or 12 weeks (HDq12 regimen) or 16 weeks (HDq16 regimen) or 20 weeks (HDq20 regimen) after the immediately preceding dose.
- A method for achieving a maximum free aflibercept concentration in plasma of about 0.1, 0.12, 0.13, 0.14 or 0.2 mg/l; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving a peak adjusted bound aflibercept concentration in plasma at about 14 days after initiation of treatment; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
   and
- A method for achieving a maximum free aflibercept concentration in plasma of about 0.4 mg/l; in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12-20 weeks (HDq12-20) or 12 weeks (HDq12 regimen) or 16 weeks (HDq16 regimen) or 20 weeks (HDq20 regimen) after the immediately preceding dose.
- A method for achieving
   a change in central retinal thickness of about -112 or -113 or -112.4 micrometers, between weeks 0 (treatment initiation) and 4 when on the HDq12 regimen;
   a change in central retinal thickness of about -13 or -14 or -13.8 micrometers, between weeks 4 and 8 when on the HDq12 regimen;
   a change in central retinal thickness of about -4 or -5 or -4.7 micrometers, between weeks 8 and 12 when on the HDq12 regimen;
   a change in central retinal thickness of about -24 or -25 micrometers, between weeks 20 and 24 when on the HDq12 regimen;
   a change in central retinal thickness of about -23 or -24 or -23.8 micrometers, between weeks 32 and 36 when on the HDq12 regimen;
   a change in central retinal thickness of about -19 or -20 or -19.7 micrometers, between weeks 44 and 48 when on the HDq12 regimen;
   a change in central retinal thickness of about -126, -127 or -126.6 micrometers, between weeks 0 and 4 when on the HDq16 regimen;
   a change in central retinal thickness of about -12, -13 or -12.9 micrometers, between weeks 4 and 8 when on the HDq16 regimen;
   a change in central retinal thickness of about -4 or -5 or -4.5 micrometers, between weeks 8 and 12 when on the HDq16 regimen;
   a change in central retinal thickness of about -39, -40 or -39.4 micrometers, between weeks 24 and 28 when on the HDq16 regimen;
   a change in central retinal thickness of about 0 or -1 or -0.6 micrometers, between weeks 36 and 40 when on the HDq16 regimen;
   a change in central retinal thickness of about -15 or -16 micrometers, between weeks 40 and 44 when on the HDq16 regimen;
   a change in central retinal thickness of about 0 or -1 or -0.8 micrometers, between weeks 44 and 48 when on the HDq16 regimen;
   a change in central retinal thickness of about -11, -12 or -11.3 micrometers, between weeks 56 and 60 when on the HDq16 regimen;
   in a subject in need thereof having an angiogenic eye disorder (preferably nAMD) comprising administering to an eye of the subject, a single initial dose of about 8 mg or more of aflibercept, followed by one or more secondary doses of about 8 mg or more of the aflibercept, followed by one or more tertiary doses of about 8 mg or more of the aflibercept; wherein each secondary dose is administered about 2 to 4 weeks after the immediately preceding dose; and wherein each tertiary dose is administered about 12 weeks (HDq12 regimen) or 16 weeks (HDq16 regimen) after the immediately preceding dose.

In an embodiment of the invention, CRT and/or retinal fluid is as measured on spectral domain optical coherence tomography (SD-OCT). In an embodiment of the invention, any of such achievements are maintained as long as the subject is receiving the treatment regimen.

The molecular weight adjusted concentration of bound aflibercept (adjusted bound aflibercept) is calculated by multiplying the observed concentrations by 0.717 to account for the target VEGF weight in the complex in plasma in the concentration-time profiles discussed herein.

In an embodiment of the invention, a subject receiving a treatment for an angiogenic eye disorder, *e.g.*, nAMD, does not experience or is no more likely to experience than a subject receiving Eylea according to the prescribed dosage regimen (2q8):
- Ocular serious TEAE (*e.g.*, through week 48 after treatment initiation), for example, cataract subcapsular, retinal detachment, ulcerative keratitis, vitreous haemorrhage or increased intraocular pressure, angle closure glaucoma, cataract, choroidal detachment, retinal detachment, retinal haemorrhage, skin laceration and/or vitreous haemorrhage;
- TEAE intraocular inflammation (*e.g.*, through week 48 after treatment initiation), for example, chorioretinitis, iridocyclitis, iritis, uveitis, vitreal cells and/or vitritis;
- Non-ocular serious TEAEs (*e.g.*, through week 48 after treatment initiation), for example, acute left ventricular failure, acute myocardial infarction, cardiac arrest, coronary artery disease, myocardial infarction, Covid-19 pneumonia, gangrene, pneumonia, hyponatraemia, cerebrovascular accident, acute kidney injury, acute respiratory failure, angina pectoris, chest pain, cellulitis, pneumonia, pyelonephritis acute, urinary tract infection, upper limb fracture, hyponatraemia, osteoarthritis, bladder neoplasm and/or cerebrovascular accident;
- Treatment emergent APTC event (*e.g.*, through week 48 after treatment initiation), for example, non-fatal myocardial infarction, non-fatal stroke and/or vascular death;
- Treatment emergent hypertension events (*e.g.*, through week 48 after treatment initiation), for example, blood pressure increased (diastolic and/or systolic), hypertension, diastolic hypertension, systolic hypertension, hypertensive crisis, hypertensive emergency, hypertensive urgency, labile hypertension and/or white coat hypertension;
- Potentially clinically significant values (*e.g.*, through week 48 after treatment initiation), for example, systolic blood pressure≥160 mmHg and an increase from baseline of ≥20 mmHg; and/or diastolic blood pressure ≥110 mmHg and an increase from baseline of ≥10 mmHg; and/or
- Death (*e.g.*, through week 48 after treatment initiation), for example, due to cardiac arrest, cardio-respiratory arrest, left ventricular failure, myocardial infarction, death, sudden death, covid-19, pneumonia, diabetic metabolic decompensation, endometrial cancer, acute kidney injury, abdominal strangulated hernia, pneumonia aspiration, skull fracture, metastatic neoplasm and/or non-small cell lung cancer

The present invention further includes methods for achieving a pharmacokinetic effect in a subject suffering from nAMD comprising administering to an eye of the subject, at least one dose (*e.g.*, a first dose) of about >8 mg VEGF antagonist (*e.g.*, a VEGF receptor fusion protein such as aflibercept). The pharmacokinetic effect can be one or more set forth below:
- Decreasing ocular clearance of free VEGF antagonist (*e.g.*, a VEGF receptor fusion protein such as aflibercept) *e.g.*, by about 34% relative to that of a dose of 2 mg of the VEGF antagonist (*e.g.*, a VEGF receptor fusion protein such as aflibercept);
- 0.2-0.3 (*e.g.*, 0.310 or 0.245) mg/l free VEGF antagonist (*e.g.*, a VEGF receptor fusion protein such as aflibercept) in the plasma, *e.g.*, within about 1, 2 or 3 days of the first dose;
- About 0.38 or 0.5 mg/l adjusted bound VEGF antagonist (*e.g.*, a VEGF receptor fusion protein such as aflibercept) in the plasma, *e.g.*, within about 14, 15 or 16 days of the first dose;
- Reaches LLOQ of free VEGF antagonist (*e.g.*, a VEGF receptor fusion protein such as aflibercept) (about 15.6 ng/ml) in the plasma by about 3 or 4 weeks of the first dose;
- Reaches LLOQ of free VEGF antagonist (*e.g.*, a VEGF receptor fusion protein such as aflibercept) (about 15.6 ng/ml) in the ocular compartment by about 15 weeks of the first dose;
- Reaches LLOQ of free VEGF antagonist (*e.g.*, a VEGF receptor fusion protein such as aflibercept) (about 15.6 ng/ml) that is about 6 weeks longer than achieved for a 2 mg dose of the antagonist;
- A clearance of the VEGF antagonist (*e.g.*, a VEGF receptor fusion protein such as aflibercept) from the ocular compartment of about 0.41 ml/day;
for example, wherein the subject is administered:
a single initial dose of about 8 mg or more of a VEGF antagonist (*e.g.*, a VEGF receptor fusion protein such as aflibercept), followed by one or more secondary doses of about 8 mg or more of the VEGF antagonist (*e.g.*, a VEGF receptor fusion protein such as aflibercept), followed by one or more tertiary doses of about 8 mg or more of the VEGF antagonist (*e.g.*, a VEGF receptor fusion protein such as aflibercept); wherein each secondary dose is administered about 2 to 4 weeks (preferably, 4 weeks) after the immediately preceding dose; and wherein each tertiary dose is administered about 12-20 weeks (preferably, 12, 16 or 20 weeks) after the immediately preceding dose

In an embodiment of the invention, the method for treating or preventing nAMD, in a subject in need thereof comprises administering >8 mg aflibercept (0.07 mL or 70 microliters) administered by intravitreal injection every 4 weeks (approximately every 28 days +/- 7 days, monthly) for the first three doses, followed by >8 mg aflibercept (0.07 mL) via intravitreal injection once every 8 - 16 weeks (2 - 4 months, +/- 7 days).

Some subject may be excluded from administration based, for example, on the existence of certain exclusion criteria. For example, in an embodiment of the invention, the criteria are one or more of ocular infection, periocular infection; active intraocular inflammation; and/or hypersensitivity, *e.g.*, to aflibercept or any component of a formulation thereof. The method presented herein may include the step of evaluating the subject for such exclusion criteria and excluding the subject from said administration if any one or more if found in the subject; and proceeding with administration if exclusion criteria are not found.

In an embodiment of the invention, a subject receiving VEGF antagonist (*e.g.*, a VEGF receptor fusion protein such as aflibercept) is monitored for adverse events (AEs) such as conjunctival hemorrhage, cataract, vitreous detachment, vitreous floaters, corneal epithelium defect and/or increased intraocular pressure. If an AE is found, the AE can be treated in the subject and treatment can either be discontinued or continued.

The methods of present invention can include preparatory steps that include use of
- one single-dose glass vial having a protective plastic cap and a stopper containing an aqueous formulation comprising >8 mg aflibercept in about 70 microliters;
- one 18-gauge x 1½-inch, 5-micron, filter needle that includes a tip and a bevel;
- one 30-gauge x ½-inch injection needle; and
- one 1-mL Luer lock syringe having a graduation line marking for 70 microliters of volume;
packaged together (kits including such items form part of the present invention). The steps can include, for example: (1) visually inspecting the aqueous formulation in the vial and, if particulates, cloudiness, or discoloration are visible, then using another vial of aqueous formulation containing the aflibercept; (2) removing the protective plastic cap from the vial; and (3) cleaning the top of the vial with an alcohol wipe; then, using aseptic technique the following steps: (4) removing the 18-gauge x 1½-inch, 5-micron, filter needle and the 1 mL syringe from their packaging; (5) attaching the filter needle to the syringe by twisting it onto the Luer lock syringe tip; (6) pushing the filter needle into the center of the vial stopper until the needle is completely inserted into the vial and the tip touches the bottom or a bottom edge of the vial; (7) withdrawing all of the aflibercept vial contents into the syringe, keeping the vial in an upright position, slightly inclined, while ensuring the bevel of the filter needle is submerged into the liquid; (8) continuing to tilt the vial during withdrawal keeping the bevel of the filter needle submerged in the formulation; (9) drawing the plunger rod sufficiently back when emptying the vial in order to completely empty the filter needle; (10) removing the filter needle from the syringe and disposing of the filter needle; (11) removing the 30-gauge x ½-inch injection needle from its packaging and attaching the injection needle to the syringe by firmly twisting the injection needle onto the Luer lock syringe tip; (12) holding the syringe with the needle pointing up, and checking the syringe for bubbles, wherein if there are bubbles, gently tapping the syringe with a finger until the bubbles rise to the top; and (13) slowly depressing the plunger so that the plunger tip aligns with the graduation line that marks 70 microliters on the syringe.

Preferably injection of aflibercept as performed in methods of the present invention is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.

### Switching

The present invention includes embodiments wherein a subject has a history of receiving one or more doses of afliberept or any other VEGF antagonist (*e.g.*, 2 mg aflibercept such as Eylea (*e.g.*, 2q8 regimen) or one or more doses of about 8 mg aflibercept) and is then switched to a dosing regimen of the present invention, *e.g.*, HDq12, HDq16, HDq20, HDq12-20 or HDq16-20, starting at any step in the regimen.

For example, a subject may have been initially administered aflibercept manufactured by a first process (a first aflibercept) and then is switched to aflibercept manufactured by a different process (*e.g.*, a second aflibercept; *e.g.*, a biosimilar aflibercept); for example, wherein each process is carried out by a different manufacturer.

Subjects may initially be receiving aflibercept according to a 2q8 dosing regimen comprising administering 3 initial monthly doses followed by one or more maintenance doses every 8 weeks (*e.g.*, Eylea) and then switch to a HDq12-20 or HDq12 or HDq16 or HDq20 dosing regimen. The aflibercept administered in the HDq12-20 or HDq12 or HDq16 or HDq20 dosing regimen may have been manufactured by a different process, *e.g.*, by a different manufacturer.

In addition, a subject may be receiving a HDq12-20 or HDq12 or HDq16 or HDq20 dosing regimen with aflibercept and then switch to aflibercept manufactured by a different process, *e.g.*, by a different manufacturer, while remaining on the HDq12-20 or HDq12 or HDq16 or HDq20 dosing regimen.

The present invention encompasses, but is not limited to, methods for treating an angiogenic eye disorder, preferably nAMD, wherein a subject is switched, from a first aflibercept (manufactured by one process) for use in a HDq12-20 or HDq12 or HDq16 or HDq20 regimen to a second aflibercept (manufactured by another process) for use in a HDq12-20 or HDq12 or HDq16 or HDq20 regimen. The present invention includes embodiments wherein the subject initiates treatment of the second aflibercept HDq12 or HDq16 regimen at any dosing phaseinitial, secondary or tertiary/maintenance-after having received the initial dose, one or more secondary doses or one or more tertiary/maintenance doses of the first aflibercept HDq12-20 or HDq12 or HDq16 or HDq20 regimen. Thus, the present invention includes embodiments wherein, the subject is switched from any phase of the first HDq12-20 or HDq12 or HDq16 or HDq20 regimen into any phase of the second HDq12-20 or HDq12 or HDq16 or HDq20 regimen. Preferably, the subject will pick up receiving the second aflibercept HDq12-20 or HDq12 or HDq16 or HDq20 regimen at the dosing phase that corresponds to where dosing was stopped with the first HDq12-20 or HDq12 or HDq16 or HDq20 regimen, *e.g.*, if a particular secondary dose was due with the first aflibercept therapy, the subject would timely receive the same secondary dose with the second aflibercept and, for example, continue receiving the second aflibercept according to the HDq12-20 or HDq12 or HDq16 or HDq20 regimen as needed thereafter.

The present invention also encompasses, but is not limited to, methods for treating an angiogenic eye disorder wherein a subject is switched, from a first aflibercept for use in a 2q8 regimen to a second aflibercept for use in a HDq12-20 or HDq12 or HDq16 or HDq20 regimen. The present invention includes embodiments wherein the subject initiates treatment of the second aflibercept HDq12-20 or HDq12 or HDq16 or HDq20 regimen at any dosing phaseinitial, secondary or tertiary/maintenance-after having received the initial dose, one or more secondary doses or one or more tertiary/maintenance doses of the first aflibercept 2q8 regimen. Thus, the present invention includes embodiments wherein, for example, the subject is switched directly to the maintenance phase of the HDq12-20 or HDq12 or HDq16 or HDq20 regimen with second aflibercept after having received the initial and a single secondary dose in the 2q8 regimen with the first aflibercept.

In an embodiment of the invention, a subject who has received an initial, one or more secondary doses and/or one or more tertiary doses of 2 mg aflibercept (*e.g.*, Eylea) therapy (*e.g.*, 2q8) according to the prescribed dosing regimen may receive an >8 mg dose of aflibercept, undergo an evaluation by a treating physician in about 8 or 10 or 12 weeks and, if, in the judgment of a treating physician, dosing every 12 weeks or every 16 weeks or every 20 weeks is appropriate (*e.g.*, there is no undue loss in BCVA and/or increase in CRT), then continuing to dose the subject every 12-20 or 12 weeks or 16 weeks or 20 weeks with >8 mg aflibercept.

The present invention includes methods for treating or preventing an angiogenic eye disorder, preferably, nAMD, in a subject in need thereof, by administering to said subject >8 mg aflibercept, wherein:
- the subject has received an initial >8 mg dose of aflibercept; then the method comprises after 1 month administering to the subject the first >8 mg secondary dose of aflibercept and 1 month thereafter, administering the 2^{nd} >8 mg secondary dose of aflibercept; and then, every 12-20 or 12 or 16 or 20 weeks thereafter, administering one or more >8 mg maintenance doses of aflibercept according to the HDq12-20 or HDq12 or HDq16 or HDq20 dosing regimen;
   or
- the subject has received an initial >8 mg dose of aflibercept & a 1^{st} >8 mg secondary dose of aflibercept after 1 month, then the method comprises, after another 1 month, administering to the subject the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
   or
- the subject has received an initial >8 mg dose of aflibercept & a 1^{st} >8 mg secondary dose of aflibercept after 1 month & a 2^{nd} >8 mg secondary dose of aflibercept after another month; then the method comprises, after 12-20 or 12 or 16 or 20 weeks, administering to the subject the 1^{st} >8 mg maintenance dose of aflibercept and all further >8 mg maintenance doses of aflibercept every 12-20 or 12 or 16 or 20 weeks according to the HDq12-20 or HDq12 or HDq16 or HDq20 dosing regimen;
   or
- the subject has received an initial >8 mg dose of aflibercept & a 1^{st} >8 mg secondary dose of aflibercept after 1 month & a 2^{nd} >8 mg secondary dose of aflibercept after another month, then, every 12-20 or 12 or 16 or 20 weeks thereafter, the subject has received one or more >8 mg maintenance doses of aflibercept; then the method comprises after 12-20 or 12 or 16 or 20 weeks from the last maintenance dose of aflibercept, administering to the subject one or more >8 mg maintenance doses of aflibercept and all further >8 mg maintenance doses of aflibercept every 12-20 or 12 or 16 or 20 weeks according to the HDq12-20 or HDq12 or HDq16 or HDq20 dosing regimen.

Subjects may switch from a reference 2 mg aflibercept dosing regimen to a particular step in the HDq12-20 or HDq12 or HDq16 or HDq20 dosing regimen. For example, a subject may receive only the initial 2mg dose of reference, and then, skipping the initial and secondary doses of the HDq12-20 or HDq12 or HDq16 or HDq20 dosing regimen, begin receiving the HDq12-20 or HDq12 or HDq16 or HDq20 maintenance doses. The present invention includes methods for treating or preventing an angiogenic eye disorder, preferably nAMD, in a subject in need thereof, by administering to said subject about >8 mg aflibercept, wherein:
(1) the subject has received an initial 2 mg dose of aflibercept then the method comprises, after 1 month, administering to the subject the initial >8 mg dose of aflibercept and, 1 month thereafter, the 1^{st} >8 mg secondary dose of aflibercept; and, 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(2) the subject has received an initial 2 mg dose of aflibercept, then the method comprises, after 1 month, administering to the subject, the first >8 mg secondary dose of aflibercept and, 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(3) the subject has received an initial 2 mg dose of aflibercept, then the method comprises, after 1 month, administering to the subject the 2^{nd} >8 mg secondary dose of aflibercept and then, every 12-20 or 12 or 16 or 20 weeks thereafter, one or more >8 mg maintenance doses of aflibercept according to the HDq12-20 or HDq12 or HDq16 or HDq20 dosing regimen;
(4) the subject has received an initial 2 mg dose of aflibercept, then the method comprises, after 1 month, administering to the subject the 1^{st} >8 mg maintenance dose of aflibercept and all further >8 mg maintenance doses of aflibercept every 12-20 or 12 or 16 or 20 weeks according to the HDq12-20 or HDq12 or HDq16 or HDq20 dosing regimen;
(5) the subject has received an initial 2 mg dose of aflibercept and a 1^{st} 2 mg secondary dose of aflibercept after 1 month, then the method comprises, after another 1 month, administering to the subject the initial >8 mg dose of aflibercept and, 1 month thereafter, the 1^{st} >8 mg secondary dose of aflibercept; and 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(6) the subject has received an initial 2 mg dose of aflibercept and a 1^{st} 2 mg secondary dose of aflibercept after 1 month, then the method comprises, after another 1 month, administering to the subject a first >8 mg secondary dose of aflibercept and, 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(7) the subject has received an initial 2 mg dose of aflibercept and a 1^{st} 2 mg secondary dose of aflibercept after 1 month, then the method comprises, after another 1 month, administering to the subject the 2^{nd} >8 mg secondary dose of aflibercept and then, every 12-20 or 12 or 16 or 20 weeks thereafter, one or more >8 mg maintenance doses of aflibercept according to the HDq12-20 or HDq12 or HDq16 or HDq20 dosing regimen;
(8) the subject has received an initial 2 mg dose of aflibercept and a 1^{st} 2 mg secondary dose of aflibercept after 1 month, then the method comprises, after another 1 month, administering to the subject the 1^{st} >8 mg maintenance dose of aflibercept and all further >8 mg maintenance doses of aflibercept every 12-20 or 12 or 16 or 20 weeks according to the HDq12-20 or HDq12 or HDq16 or HDq20 dosing regimen;
(9) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the initial >8 mg dose of aflibercept and, 1 month thereafter, the 1^{st} >8 mg secondary dose of aflibercept; and 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(10) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the first >8 mg secondary dose of aflibercept and, 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(11) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after another 1 month, administering to the subject the 2^{nd} >8 mg secondary dose of aflibercept and then, every 12-20 or 12 or 16 or 20 weeks thereafter, one or more >8 mg maintenance doses of aflibercept according to the HDq12-20 or HDq12 or HDq16 or HDq20 dosing regimen;
(12) the subject has received an initial 2 mg dose of aflibercept and a 1^{st} 2 mg secondary dose of aflibercept after 1 month and a 2^{nd} 2 mg secondary dose of aflibercept after another 1 month, then the method comprises, after 2 months, administering to the subject the 1^{st} >8 mg maintenance dose of aflibercept and, all further >8 mg maintenance doses of aflibercept every 12-20 or 12 or 16 or 20 weeks according to the HDq12-20 or HDq12 or HDq16 or HDq20 dosing regimen;
(13) the subject has received an initial 2 mg dose of aflibercept and a 1^{st} 2 mg secondary dose of aflibercept after 1 month and a 2^{nd} 2 mg secondary dose of aflibercept after another 1 month and a 1^{st} 2 mg maintenance dose of aflibercept after 8 weeks, then the method comprises, up to 2 months after the last dose of aflibercept, administering to the subject the initial >8 mg dose of aflibercept and 1 month thereafter, the 1^{st} >8 mg secondary dose of aflibercept; and 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(14) the subject has received an initial 2 mg dose of aflibercept and a 1^{st} 2 mg secondary dose of aflibercept after 1 month and a 2^{nd} 2 mg secondary dose of aflibercept after another 1 month and 1 or more 2 mg maintenance doses of aflibercept after 8 weeks, then the method comprises up to 2 months after the last dose of aflibercept, administering to the subject the first >8 mg secondary dose of aflibercept and 1 month thereafter, the 2^{nd} >8 mg secondary dose of aflibercept; and then, every 12-20 or 12 or 16 or 20 weeks thereafter, one or more >8 mg maintenance doses of aflibercept according to the HDq12-20 or HDq12 or HDq16 or HDq20 dosing regimen;
(15) the subject has received an initial 2 mg dose of aflibercept and a 1^{st} 2 mg secondary dose of aflibercept after 1 month and a 2^{nd} 2 mg secondary dose of aflibercept after another 1 month and 1 or more 2 mg maintenance doses of aflibercept after 8 weeks, then the method comprises up to 2 months after the last dose of aflibercept, administering to the subject the 2^{nd} >8 mg secondary dose of aflibercept and then, every 12-20 or 12 or 16 or 20 weeks thereafter, one or more >8 mg maintenance doses of aflibercept according to the HDq12-20 or HDq12 or HDq16 or HDq20 dosing regimen;
(16) the subject has received an initial 2 mg dose of aflibercept and a 1^{st} 2 mg secondary dose of aflibercept after 1 month and a 2^{nd} 2 mg secondary dose of aflibercept after another 1 month and 1 or more 2 mg maintenance doses of aflibercept after 8 weeks, then the method comprises up to 2 months after the last dose of aflibercept, administering to the subject the 1^{st} >8 mg maintenance dose of aflibercept and all further >8 mg maintenance doses of aflibercept every 12-20 or 12 or 16 or 20 weeks according to the HDq12-20 or HDq12 or HDq16 or HDq20 dosing regimen.

### Precision Dose Drug Delivery

The present invention provides methods as set forth herein wherein a VEGF antagonist (*e.g.*, aflibercept) is delivered with a high amount of precision, *e.g.*, with a drug delivery device (DDD) (*e.g.*, with a 0.5 mL volume), whether pre-filled or capable of being filled from a vial, and delivering a volume of between 70 and 100 microliter with an average volume of about 81 or 82 or 81-82 microliters, *e.g.*, with a standard deviation of about 4 or 5 or 4-5 microliters (*e.g.*, about 4.5 or 4.46 microliters) or less. In an embodiment of the invention, the DDD is a syringe, *e.g.*, with a 30 gauge, ½ inch needle.

One means for ensuring precision of a dose to be delivered with a device, such as a syringe, is by employing a syringe wherein the dose volume is device-determined. If the dose volume is device-determined, the device is designed only to deliver a single volume (*e.g.*, 87 microliters) or a single volume with a limited amount of acceptable error (+ 4-5 microliters). Thus, if used properly, the user cannot deliver the wrong dose (*e.g.*, cannot deliver more than the intended volume from the device).

The present invention includes embodiments wherein, a precise dosage of about 8 mg or more is a dose of about 9, 9.3, 9.33, 9.7, 9.8, 9.9, 9.7-9.9 mg or more + about 0.5, or + about 0.51 mg is delivered to a subject's eye. The volume in which a dose is delivered can be, for example, about 70, 81, 82, 81.7, 85, 86, 87, 85-87 microliters + about 4, 4.45, 4.5, or 5 microliters. Doses may be delivered with a dose delivery device (DDD) which is a syringe.

Highly precise doses of VEGF antagonist (*e.g.*, aflibercept) may be delivered, for example, in a volume that is device-determined (wherein the device is a syringe), by a method that includes the steps: (a) priming the syringe (*e.g.*, a pre-filled syringe), thereby removing air from the syringe and, thus avoiding injection of air into the eye, by advancing the plunger rod by a predetermined distance into the syringe body until advancement of the plunger rod is resisted by a stop; (b) rotating the plunger rod about a longitudinal axis; and (c) actuating the plunger rod to dispense a predetermined (device-determined) volume (*e.g.*, about 70, 81, 82, 81.7, 85, 86, 87, 85-87 microliters, + about 4, 4.45, 4.5, or 5 microliters) of the formulation.

In an embodiment of the invention, the drug delivery device (DDD), comprises:
- a barrel including a longitudinal axis, a proximal end region, and a distal end region, the proximal end region including an opening, wherein the barrel is configured to receive a drug therein;
- a plunger rod disposed at least partially inside the barrel and protruding from the opening, wherein the plunger rod includes a rack having a plurality of teeth; and
- a pinion having a plurality of teeth configured to engage with the plurality of teeth of the rack,
wherein rotation of the pinion against the rack moves at least a part of the plunger rod along the longitudinal axis of the barrel; for example, which further comprises a shaft affixed to the pinion, wherein rotation of the shaft rotates the pinion against the rack which may include a knob affixed to the shaft. In an embodiment of the invention, the DDD further includes a magnifier disposed on the distal end region of the barrel. In an embodiment of the invention, the DDD further includes a stopper inside the barrel, wherein the stopper is affixed to a distal end of the plunger rod. In an embodiment of the invention, the DDD further includes a circular ratchet disposed coaxially with the pinion, wherein the circular ratchet has a diameter smaller than a diameter of the pinion; a spring-loaded pawl disposed on an internal circumference of the pinion, wherein the pawl is configured to engage the ratchet; and a shaft affixed to the ratchet, wherein rotation of the shaft in one direction causes rotation of the pinion, and rotation of the shaft in a second direction does not cause rotation of the pinion for example wherein the ratchet is disposed inside the pinion. In an embodiment of the invention, the pinion includes a plurality of teeth having a first height, and a stopper tooth having a second height greater than the first height, for example, wherein the second height of the stopper tooth prevents the pinion from engaging the plurality of teeth of the rack, and/or wherein the second height of the stopper tooth is configured to contact one of the plunger rod and the rack to stop rotation of the pinion. In an embodiment of the invention, the plunger rod includes an inner column and an outer lumen, and wherein the rack is disposed on the inner column, *e.g.*, wherein rotation of the pinion against the rack moves the inner column of the plunger rod independently of the outer lumen, and/or further including a shaft removably affixed to the pinion, wherein the shaft prevents movement of the outer lumen of the plunger rod relative to the barrel, and wherein removal of the shaft allows for movement of the outer lumen of the plunger rod relative to the barrel. In an embodiment of the invention, the plunger rod further includes a body and a flange, the flange extending partially along a longitudinal length of the body and having a width greater than a width of the body; wherein the barrel further comprises a plunger lock, the plunger lock including a through hole configured to allow the flange to pass through the second plunger lock in a specific orientation.

In an embodiment of the invention, the drug delivery device (DDD), comprises:
- a barrel including a longitudinal axis, a proximal end region, a distal end region, and an interior, the proximal end region including an opening and the interior including a threaded region; and
- a plunger rod disposed at least partially inside the barrel and protruding from the opening, the plunger rod including a threaded region configured to engage the threaded region of the barrel interior,
wherein rotation of the plunger rod about the longitudinal axis of the drug delivery device moves the plunger rod along the longitudinal axis. In an embodiment of the invention, the plunger rod further includes a tab protruding from the plunger rod in a first direction and located proximally from the threaded region of the plunger rod, and wherein the threaded region in the interior of the barrel further includes a slot sized and configured to allow for the tab to pass through the threaded region in the interior of the barrel, *e.g.*, wherein the slot includes a first segment parallel to the longitudinal axis of the drug delivery device and a second segment perpendicular to the longitudinal axis of the drug delivery device - the slot may include a third segment parallel to the longitudinal axis of the drug delivery device, wherein the second segment is in between the first segment and the third segment. In an embodiment of the invention, the tab is a first tab, and wherein the plunger rod further includes a second tab protruding from the plunger rod in a second direction opposite to the first direction, and wherein the threaded region in the interior of the barrel further includes a second slot sized and configured to allow for the second tab to pass through the threaded region in the interior of the barrel.

In an embodiment of the invention, the drug delivery device, includes:
- a barrel having a proximal end region, a distal end region, an opening in the proximal end region, an interior, and a threaded region in the interior;
- a sleeve disposed partly inside the barrel and protruding from the opening in the proximal end region of the barrel, the sleeve including a threaded region engaged with the threaded region of the barrel interior;
- a plunger rod disposed at least partially inside the sleeve; and
- a stopper inside the barrel and located distally from the sleeve, the stopper connected to a distal end of the plunger rod,
wherein rotation of the sleeve in a first direction around a longitudinal axis of the drug delivery device moves the sleeve towards the distal end region of the barrel. In an embodiment of the invention, rotation of the sleeve in the first direction moves the stopper towards the distal end region of the barrel. In an embodiment of the invention; the sleeve includes an inner passage, and the stopper has a diameter larger than a diameter of the inner passage; and/or the sleeve includes a tab disposed on an exterior of the sleeve, the tab located proximally from the threaded region of the barrel interior, and wherein the tab stops movement of the sleeve towards the distal end region of the barrel, *e.g.*, wherein the tab is configured to stop movement of the sleeve towards the distal end region of the barrel after the drug delivery device has been primed or wherein the tab is a first tab, and wherein the sleeve further includes a second tab disposed on an exterior of the sleeve, the second tab located distally from the threaded region of the barrel interior, wherein the second tab stops movement of the sleeve towards the proximal end region of the barrel.

In an embodiment of the invention, the drug delivery device, comprises:
- a barrel including a proximal end region and a distal end region, the proximal end region including an opening;
- a plunger rod including a body and a flange, the flange extending partially along a longitudinal length of the body and having a width greater than a width of the body, the plunger rod disposed at least partially inside the barrel and protruding from the opening;
- a first plunger lock disposed on the barrel, the first plunger lock configured to block the flange from entering the barrel; and
- a second plunger lock disposed in the barrel, the second plunger lock including a through hole configured to allow the flange to pass through the second plunger lock in a specific orientation.

For example, in an embodiment of the invention, the first plunger lock is removable and/or frangible. In an embodiment of the invention, a distance between the first plunger lock and the second plunger lock is equivalent to the distance that the stopper must travel to prime the drug delivery device; and/or the plunger rod is rotatable around a longitudinal axis of the drug delivery device.

Substances from such a DDD (*e.g.*, a formulation including aflibercept as described herein), having a plunger rod and a barrel, may be dispensed as follows:
- advancing the plunger rod by a predetermined distance into the barrel until advancement of the plunger rod is resisted by a stop;
- deactivating the stop; and
- actuating the plunger rod (*e.g.*, which includes a flange, wherein the stop includes a lock that prevents the flange from entering the barrel; or which includes a flange, wherein the stop comprises a lock that prevents the flange from entering the barrel) to deliver the substance. Advancing the plunger rod may include the step of rotating a pinion against a rack disposed on the plunger rod, *e.g.*, wherein the stop comprises a shaft removably affixed to the pinion, and wherein deactivating the stop comprises removing the shaft from the pinion. Deactivating the stop may include the step of rotating the plunger rod. In an embodiment of the invention, deactivating the stop includes the step of removing the lock and/or breaking the lock.

In an embodiment of the invention, the drug delivery device, includes:
- a barrel including a longitudinal axis, a proximal end region, and a distal end region, the proximal end region including an opening and a rack disposed on the interior of the barrel, the rack having a plurality of teeth, wherein the barrel is configured to receive a drug therein;
- a plunger rod disposed at least partially inside the barrel and protruding from the opening, wherein the plunger rod includes a rack having a plurality of teeth; a pinion having a plurality of teeth configured to engage with the plurality of teeth of the plunger rod rack; and
- an inner plunger coupled to the pinion by a rod, wherein rotation of the pinion against the plunger rod rack results in movement of the inner plunger along the longitudinal axis of the barrel;
for example, wherein the teeth of the pinion are further configured to engage with the plurality of teeth of the rack disposed on the barrel. In an embodiment of the invention, the pinion is a first pinion, and further includes: a second pinion disposed coaxially with the first pinion, the second pinion having a diameter smaller than a diameter of the first pinion and a plurality of teeth configured to engage with the plurality of the teeth of the rack disposed on the barrel, wherein rotation of the first pinion results in rotation of the second pinion against the rack disposed on the barrel and in movement of the inner plunger along the longitudinal axis of the barrel.

See International patent application publication no. WO2019/118588.

In an embodiment of the invention, the drug delivery device (DDD), includes:
- a body;
- a plunger rod disposed partially inside the body;
- a protrusion extending from the plunger rod; and
- a blocking component coupled to a proximal end portion of the body, wherein the blocking component is a flange piece,
wherein, when the protrusion is in a first position relative to the blocking component, the blocking component restricts distal movement of the plunger rod to a first stopping point, and when the protrusion is in a second position relative to the blocking component, the blocking component restricts distal movement of the plunger rod to a second stopping point. In an embodiment of the invention, the DDD further includes: a stopper disposed in the body, wherein distal movement of the plunger rod distally moves the stopper; and a drug substance disposed in the body in between the stopper and a distal end of the body, wherein distal movement of the plunger rod to the first stopping point primes the drug delivery device, and distal movement of the plunger rod to the second stopping point dispenses a predetermined volume of the drug substance from a distal end of the device.

In an embodiment of the invention, moving the protrusion from the first position to the second position includes twisting the plunger rod relative to the blocking component. In an embodiment of the invention, the DDD further includes: a cavity in a proximal side of the blocking component, the cavity sized and configured to receive a portion of the protrusion, wherein when the protrusion is in the second position relative to the blocking component, the protrusion is positioned proximally from the cavity, such that distal movement of the plunger rod moves the protrusion into the cavity; *e.g.*, wherein the cavity is a first cavity, and further includes: a second cavity in a proximal side of the blocking component, the second cavity sized and configured to receive a portion of the protrusion, wherein the first and second cavity are located on opposite sides of a central longitudinal axis of the drug delivery device. In an embodiment of the invention, the plunger rod passes through an opening in the blocking component. In an embodiment of the invention the DDD further includes an actuation portion at a proximal end portion of the plunger rod, wherein the protrusion extends from the actuation portion, *e.g.*, wherein the actuation portion includes a generally cylindrical shape having a diameter greater than a width of the remainder of the plunger rod, wherein the protrusion extends from a side of the generally cylindrical shape, and wherein the actuation portion further comprises: a thumb pad on a proximal end of the actuation portion; and a ring on an exterior surface on the side of the generally cylindrical shape; *e.g.*, further including a proximal collar on the blocking component, wherein the actuation portion partially fits inside the proximal collar; *e.g.*, wherein the plunger rod further includes a pair of extensions protruding distally from the actuation portion and the blocking component (*e.g.*, which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes one or more indents formed along a bottom wall of the blocking component; and wherein a portion of each extension is configured to be received by the one or more indents upon distal movement of the plunger rod relative to the blocking component to allow distal movement of the plunger rod to the second stopping point; or, which includes a pair of internal grooves formed along a sidewall of the blocking component; and wherein a portion of each extension is configured to be received by at least one of the pair of internal grooves upon rotation of the plunger rod relative to the blocking component to expand the extensions radially-outward from a compressed state to a relaxed state) includes a pair of openings; and wherein a portion of each extension is configured to be received by one of the pair of openings in the first stopping point. In an embodiment of the invention, the protrusion is a first protrusion, and further includes a second protrusion extending from the plunger rod in a direction opposite to the first protrusion. In an embodiment of the invention, the blocking component is slidably coupled to the body and includes a third cavity and a pair of ribs that extend into the third cavity, wherein the body includes a top flange and the pair of ribs are configured to engage the top flange received in the third cavity; wherein the pair of internal ribs are configured to apply a distally-directed force onto the top flange. In an embodiment of the invention, the blocking component is slidably coupled to the body and includes a pair of movable tabs that are configured to engage the body; and the pair of movable tabs are laterally deflectable upon receiving the body in the blocking component and are configured to apply a radially-inward directed force onto the body. In an embodiment of the invention, the blocking component further includes a pair of finger flanges, and each of the finger flanges includes a textured surface having a predefined pattern that increases a grip of the blocking component.

In an embodiment of the invention, the drug delivery device (DDD), includes:
- a body;
- a plunger rod having a distal end contacting a stopper inside the body, and a proximal end including an actuation portion with a thumb pad;
- a plurality of protrusions extending from the actuation portion; and
- a blocking component disposed on the body, the blocking component including a proximal collar having a plurality of slots,

wherein, when the protrusions and the slots are in a first configuration relative to one another, the blocking component restricts distal movement of the plunger rod to a first stopping point, and when the protrusions and the slots are in a second configuration, the blocking component restricts distal movement of the plunger rod to a second stopping point, wherein, in the second configuration, the slots are configured to receive the protrusions upon distal movement of the plunger rod. In an embodiment of the invention, the protrusions and the slots are movable from the first configuration to the second configuration by rotation of the actuation portion about a longitudinal axis in relation to the blocking component, and wherein when the protrusions and the slots are in the second configuration, the protrusions and the slots are not movable to the first configuration; and/or
a difference between the first stopping point and the second stopping point is equivalent to a distance that the stopper must travel to expel a predetermined volume of a drug product from a distal end of the body, and wherein the plunger rod is prevented from moving from the second stopping point to the first stopping point; and/or the plurality of protrusions includes two protrusions disposed symmetrically about the actuation portion; and/or the blocking component further comprises a pair of finger flanges; and/or the drug delivery device is a pre-filled syringe; and/or the drug delivery device is changeable: (a) from a pre-use state to a primed state, by longitudinally moving the plunger rod (*e.g.*, wherein the plunger rod includes a neck disposed distally from the actuation portion, wherein the neck interfaces with an opening in the blocking component to prevent proximal movement of the plunger rod, for example, wherein the neck further interfaces with the opening in the blocking component to prevent movement of the drug delivery device from the delivery state to the primed state) until the plunger rod reaches the first stopping point; (b) from the primed state to a delivery state by rotating the plunger rod in relation to the blocking component until the protrusions and the blocking component are in the second configuration; and (c) from a delivery state to a used state by longitudinally moving the plunger rod until the plunger reaches the second stopping point, wherein the drug delivery device is not changeable from the used state to the delivery state, from the delivery state to the primed state, or from the primed state to the pre-use state. In an embodiment of the invention,
when the plunger rod is at the second stopping point, the stopper does not contact a distal end of the body.

In an embodiment of the invention, a drug delivery device, includes:
- a body;
- a plunger rod, including:
- a distal portion contacting a stopper inside the body;
- a proximal end including a generally cylindrical actuation portion disposed outside of the body; and
- two protrusions extending from opposite sides of the actuation portion in a symmetrical configuration; and
- a blocking component coupled to the body, the blocking component including: a collar configured to accept a distal part of the actuation portion; and two cavities in the collar having proximally-facing openings, wherein each cavity is configured to accept a distal portion of one of the two protrusions;

wherein the plunger rod is longitudinally movable and rotatable about a longitudinal axis relative to the blocking component, and
wherein, when the drug delivery device is in a pre-use state, the protrusions and the cavity openings are not longitudinally aligned, and when the drug delivery device is in a delivery state, the protrusions and the cavity openings are longitudinally aligned. In an embodiment of the invention, the blocking component further includes a finger flange, and further includes a ribbed surface on a side of the actuation portion. In an embodiment of the invention, the plunger rod further includes: two extensions protruding distally from the actuation portion; and a plurality of openings in the collar of the blocking component, wherein a portion of each extension is configured to be received by one of the plurality of openings upon distal movement of the plunger rod relative to the blocking component.

In an embodiment of the invention, a drug delivery device includes:
- a body;
- a stopper disposed inside the body;
- a sleeve having a proximal end and a distal end, the distal end being disposed inside the body, proximally from the stopper; and
- a plunger rod disposed at least partially inside the sleeve;
wherein, when the stopper is in a ready position, distal advancement of one of (a) only the sleeve, (b) only the plunger rod, or (c) both the sleeve and the plunger rod together, relative to the body advances the stopper to a primed position, and wherein, when the stopper is in the primed position, distal advancement of another of (a) only the sleeve, (b) only the plunger rod, or (c) both the sleeve and the plunger rod together, relative to the body advances the stopper to a dose completion position. For example, in an embodiment of the invention, a DDD further includes a removable blocking component (*e.g.*, wherein the blocking component is a clip removably secured around at least a portion of the sleeve) disposed between a proximal portion of the sleeve and a proximal end of the body, the blocking component obstructing distal advancement of the sleeve relative to the body, wherein distal advancement of the sleeve relative to the body after removal of the blocking component advances the stopper to the primed position. In an embodiment of the invention, the DDD further includes a removable locking component (*e.g.*, a pin, a tab, or a bar) that couples the plunger rod to the sleeve, wherein distal advancement of both the sleeve and the plunger rod together relative to the body advances the stopper to the primed position, wherein distal advancement of only the plunger rod relative to the body after removal of the locking component advances the stopper to the dose completion position. In an embodiment of the invention, in the dose completion position, a proximal end of the plunger rod abuts against a distal end of the sleeve, such that the plunger rod is prevented from advancing distally any further relative to the body. In an embodiment of the invention, the DDD further includes a protrusion disposed on the plunger rod; and an inner protrusion disposed on an interior wall of the sleeve distally to the protrusion of the plunger rod, wherein distal advancement of only the plunger rod relative to the body advances the stopper to the primed position and causes the protrusion of the plunger rod to contact the inner protrusion of the sleeve, and wherein distal advancement of both the plunger rod and the sleeve relative to the body, after the protrusion of the plunger rod has contacted the inner protrusion of the sleeve, advances the stopper to the dose completion position. In an embodiment of the invention, the sleeve includes a finger flange. In an embodiment of the invention, the DDD further includes a stop disposed at a proximal end of the body, the stop sized to block distal advancement of the sleeve or the plunger rod once the stopper is in the completion position.

In an embodiment of the invention, a drug delivery device, includes:
- a body;
- a plunger rod having a distal portion disposed inside the body and a proximal portion disposed outside a proximal end of the body, the proximal portion having a width greater than a width of the distal portion; and
- an obstruction that, in an obstructing position relative to the plunger rod, prevents distal advancement of the plunger rod from a primed position to a dose completion position, wherein displacement of the obstruction from the obstructing position permits distal advancement of the plunger rod to the dose completion position, for example, further including a collar affixed to a proximal end portion of the body, the collar surrounding the proximal portion of the plunger rod; and a collar projection extending radially inward from the collar, wherein the proximal portion of the plunger rod includes a channel into which the collar projection protrudes, the channel including a circumferential path and an axial dose completion path, wherein the obstruction comprises the collar projection, which, when disposed in the circumferential path of the channel, prevents distal advancement of the plunger rod to the dose completion position, and wherein displacement of the obstruction from the obstructing position comprises twisting the plunger rod about a longitudinal axis to align the collar projection with the axial dose completion path. For example, in an embodiment of the invention, the channel further includes an axial priming path offset from the axial dose completion path, and connected to the axial dose completion path by the circumferential path, and distal movement of the plunger rod such that the collar projection travels on the axial priming path advances the plunger rod to the primed position. In an embodiment of the invention, the DDD further includes a finger flange. In an embodiment of the invention, the proximal portion of the plunger rod includes a projection extending radially outward, and the drug delivery device further includes: a rotatable alignment component disposed in between the proximal portion of the plunger rod and the body, the alignment component including a channel, the channel sized and configured to accommodate the plunger rod projection, wherein the obstruction comprises a wall of the channel that blocks a distal axial path of the plunger rod projection when the plunger rod is in the primed position, and wherein displacement of the obstruction from the obstructing position comprises rotating the alignment component to remove the wall of the channel from the distal axial path of the plunger rod projection, *e.g.*, further including a finger flange coupled to a proximal end portion of the body, wherein the rotatable alignment component is disposed between the finger flange and the proximal portion of the plunger rod. In an embodiment of the invention, the DDD further includes a flange piece disposed at the proximal end of the body, wherein the obstruction includes a removable cap that, when in the obstructing position relative to the plunger rod, is disposed partially in between the proximal portion of the plunger rod and the flange piece. In an embodiment of the invention, removal of the cap allows the proximal portion of the plunger rod to advance to a dose completion position, wherein, in the dose completion position, the proximal portion of the plunger rod contacts the flange piece. In an embodiment of the invention, the removable cap covers the proximal portion of the plunger rod when in the obstructing position. In an embodiment of the invention, the DDD further includes a collar disposed between the proximal end of the body and the proximal portion of the plunger rod, the collar defining an opening sized to accommodate the proximal portion of the plunger rod upon distal advancement of the plunger rod beyond a primed position; wherein the obstruction comprises a tab protruding radially outward from the proximal portion of the plunger rod, the tab preventing the proximal portion of the plunger rod from fitting into the opening of the collar, and wherein a depth of the collar opening coincides with a distance the plunger rod must travel to advance distally to the dose completion position, *e.g.*, wherein displacement of the obstruction from the obstructing position comprises either removing the tab or compressing the tab into a side of the proximal portion of the plunger rod; and/or wherein the tab is a first tab, and wherein the obstruction further comprises a second tab protruding radially outward from the proximal portion of the plunger rod in a direction opposite the protruding direction of the first tab; and/or wherein the obstruction comprises a tab that, when in the obstructing position, is disposed between the body and the proximal portion of the plunger rod, and wherein the plunger rod includes a geometry disposed proximally from the tab, wherein the geometry cannot advance distally past the tab when the tab is in the obstructing position. For example, displacement of the obstruction may include removing the tab from the drug delivery device by pulling the tab. In an embodiment of the invention, the DDD further includes a flange piece, wherein a portion of the tab is disposed inside a cavity of the flange piece. In an embodiment of the invention, displacement of the obstruction comprises removing the tab from the drug delivery device by breaking the tab. In an embodiment of the invention, the obstruction includes a flange piece that, in the obstructing position, is disposed proximally from the proximal end of the body, between the proximal portion of the plunger rod and the body, and is spaced from the proximal end of the body by a removable blocking component, and wherein displacement of the obstruction from the obstructing position comprises: removing the blocking component; and shifting the flange piece distally towards the proximal end of the body. In an embodiment of the invention, the plunger rod includes a projection extending radially outward, wherein the obstruction includes a lever having an end that, in the obstructing position, is located distally from the projection and blocks distal movement of the projection and thereby distal movement of the plunger rod, and wherein displacement of the obstruction from the obstructing position comprises actuating the lever to remove the end of the lever from its location distal from the projection. In an embodiment of the invention, distal advancement of the plunger rod beyond the dose completion position is prevented by contact between the proximal portion of the plunger rod and a portion of a flange piece coupled to the body.

In an embodiment of the invention, the drug delivery device, includes:
- a body;
- a sleeve affixed to the body, the sleeve including a proximal end, a distal end, and an opening disposed in a circumferential wall of the sleeve;
- a plunger rod passing through the sleeve, the plunger rod including a distal end portion disposed inside the body, and a radially-extending protrusion;
wherein the plunger rod may be distally advanced into the body from a ready position to a primed position, wherein, in the primed position, the protrusion of the plunger rod is disposed inside the opening, and further distal advancement of the plunger rod is resisted by contact between the protrusion and a wall of the opening, and wherein pressure may be exerted on the protrusion to overcome the resistance to further distal advancement of the plunger rod. In an embodiment of the invention, the opening in the sleeve is a second opening, and the sleeve further includes a first opening disposed in the circumferential wall of the sleeve proximally from the second opening, and a third opening disposed in the circumferential wall of the sleeve distally from the second opening, wherein, in the ready position, the protrusion of the plunger rod is disposed in the first opening, and further distal advancement of the plunger rod is resisted by contact between the protrusion and a wall of the first opening, and wherein, after further distal advancement of the plunger rod past the primed position, the protrusion of the plunger rod is disposed in the third opening, and further distal advancement of the plunger rod is prevented. In an embodiment of the invention, the radially-extending protrusion is a first protrusion, and wherein the plunger rod further includes a second radially-extending protrusion opposite the first protrusion, and wherein squeezing the first and second protrusions towards one another while applying axial pressure in the distal direction on the plunger rod overcomes the resistance to further distal advancement of the plunger rod. In an embodiment of the invention, the protrusion includes a distally-tapering profile to aid in distal advancement of the plunger rod.

In an embodiment of the invention, a drug delivery device, includes:
- a body;
- a plunger rod including a distal end portion disposed inside the body and a rotatable element; and
- a sleeve affixed to the body, the sleeve including a proximal opening into which the plunger rod may be advanced,
wherein rotating the rotatable element causes distal advancement of the plunger rod to a primed position, and wherein once the plunger rod is in the primed position, further rotation of the rotatable element is resisted. In an embodiment of the invention, the DDD further includes a collar disposed at a proximal end of the body, an interior of the collar including a proximal threaded portion forming a proximal helical path, wherein the rotatable element comprises a proximal portion of the plunger rod including a protrusion, wherein the proximal portion of the plunger rod may be rotated about a longitudinal axis to cause the protrusion to travel distally along the proximal helical path, and wherein once the protrusion reaches the end of the proximal threaded portion of the collar, the plunger rod is in the primed position, *e.g.*, wherein once the plunger rod is in the primed position, the plunger rod may be depressed axially into the body to distally advance the plunger rod to a dose completion position; and/or wherein the interior of the collar further includes a distal threaded portion, wherein threads of the distal threaded portion form a distal helical path offset from, and opposite to, the proximal helical path, wherein alignment of the protrusion with the distal helical path places the plunger rod in the primed position, and wherein rotation of the proximal portion of the plunger rod to cause the protrusion to travel distally along the distal helical path causes distal advancement of the plunger rod to a dose completion position.

A substance may be dispensed using such a DDD having a plunger rod and a body, may be done by a method including:
(a) advancing the plunger rod by a predetermined distance into the body until advancement of the plunger rod is resisted by a stop;
(b) rotating the plunger rod about a longitudinal axis; and
(c) actuating the plunger rod to dispense a predetermined volume of the substance,
wherein none of steps (a), (b), and (c) are reversible. In an embodiment of the invention, the DDD further includes a flange piece having a collar, and advancing the plunger rod and actuating the plunger rod comprise pressing an actuation portion of the plunger rod into the collar of the flange piece; for example, wherein the plunger rod comprises a protrusion, and wherein the collar of the flange piece abuts against the protrusion to resist advancement of the plunger rod. For example, in an embodiment of the invention, wherein rotating the plunger rod comprises twisting an actuation portion of the plunger rod relative to the flange piece, until a protrusion on the plunger rod becomes longitudinally aligned with a cavity in the collar of the flange piece, which may further include advancing the protrusion into the cavity until the protrusion abuts a distal side of the cavity, wherein the predetermined volume of the substance is dispensed when the protrusion abuts the distal side of the cavity.

See International patent application publication no. WO2020/247686.

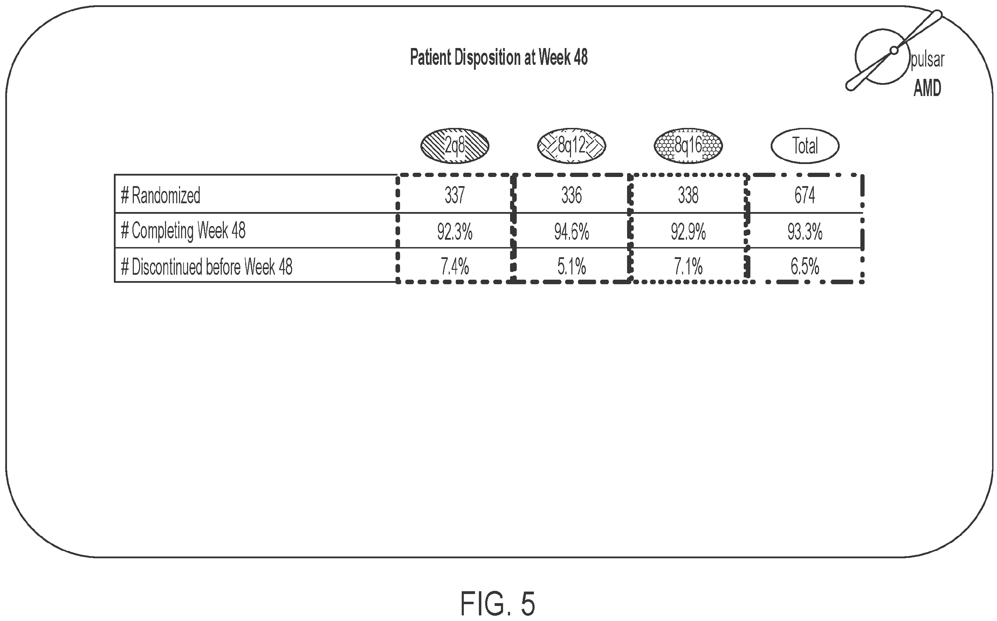

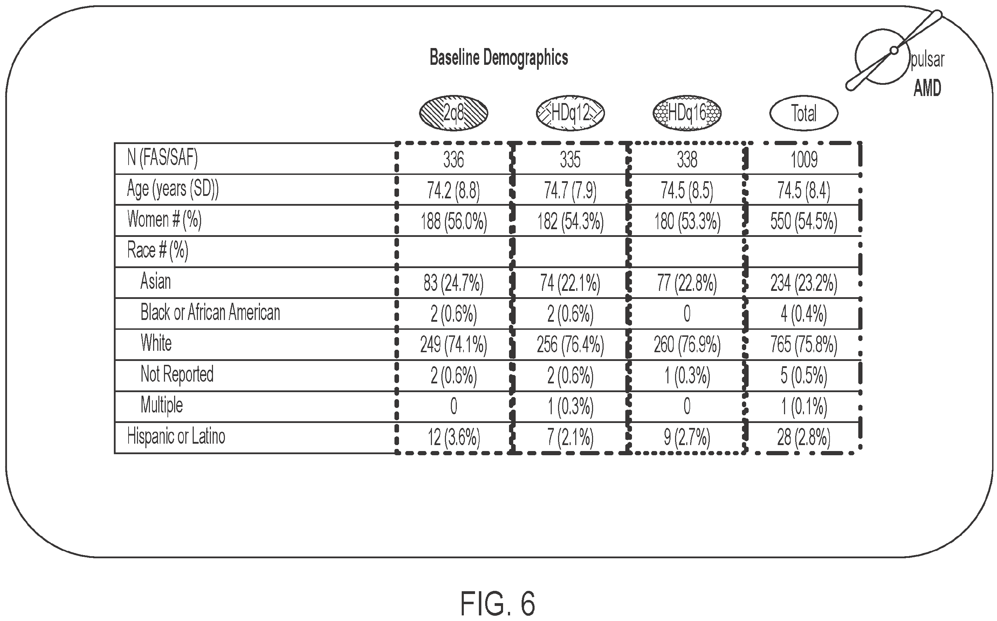

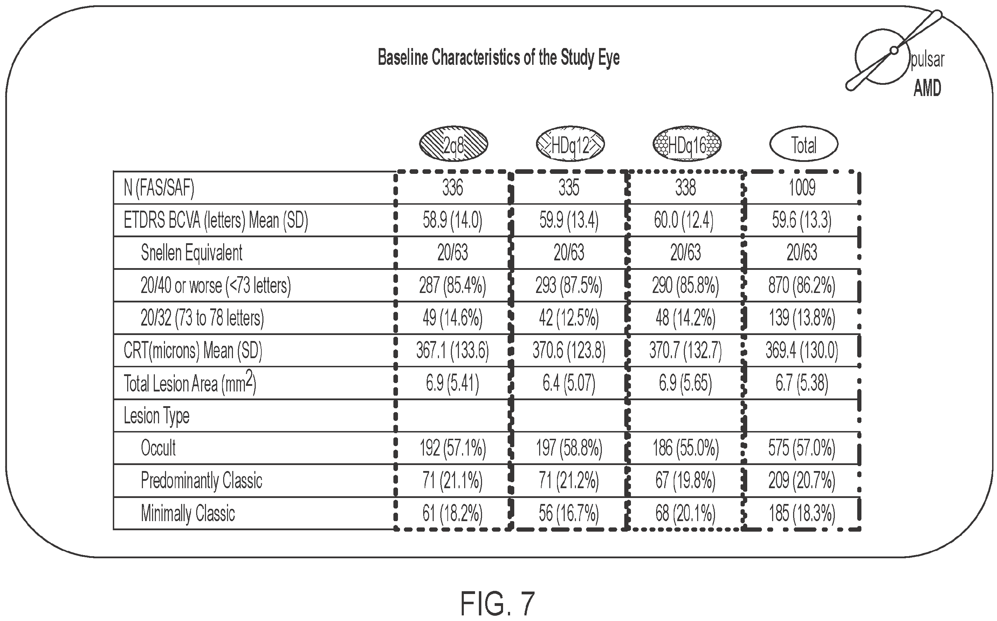

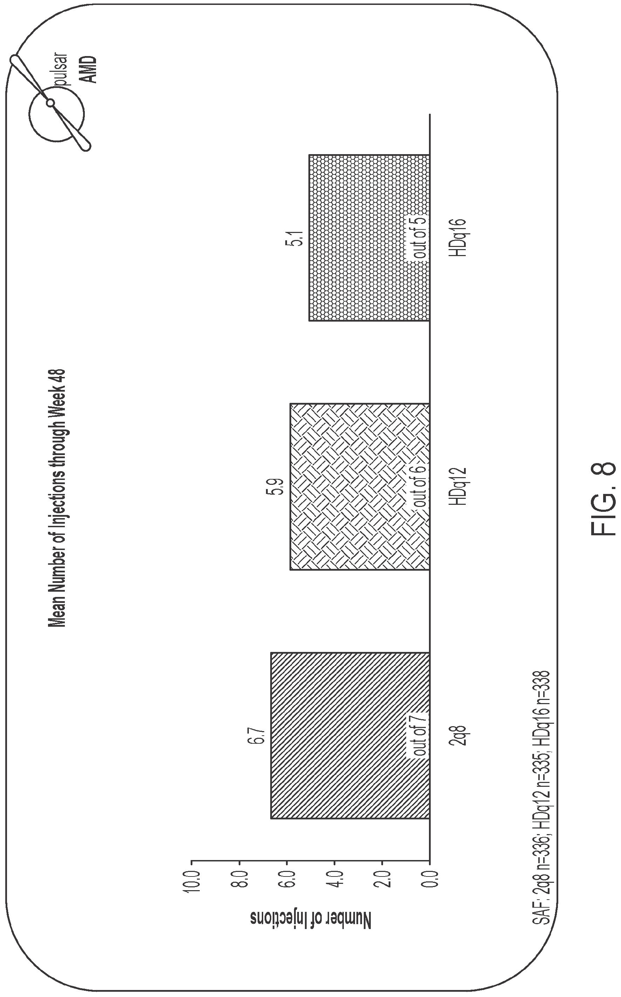

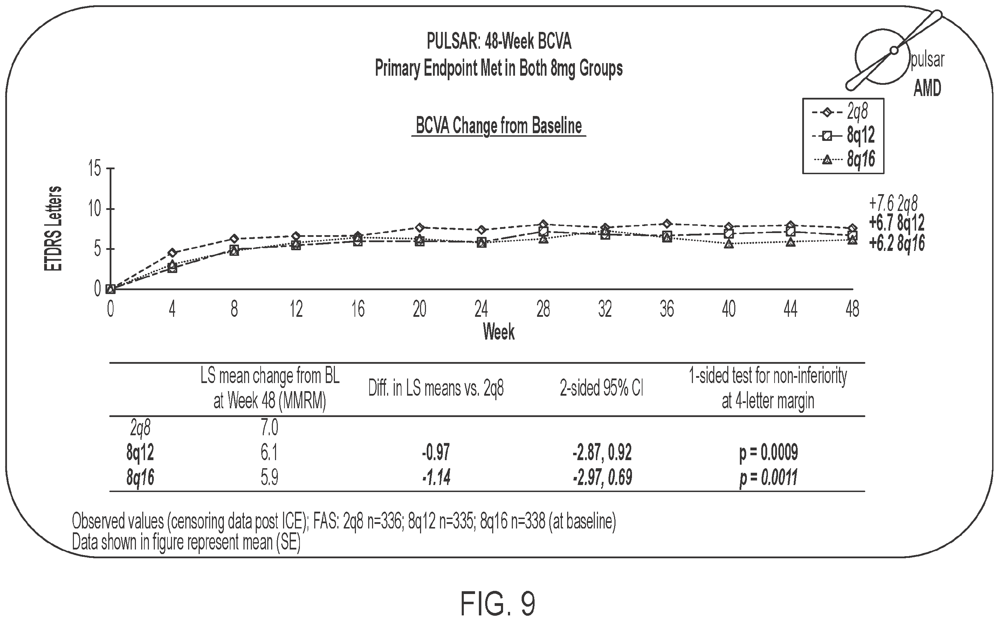

Data from the PULSAR trial through week 48 are provided. Patient disposition data in the PHOTON trial are set forth in Figure 5 and baseline demographics and study eye characteristics data are in Figures 6 and Figure 7. The mean patient exposure to injections per week is summarized in Figure 8. Efficacy data with respect to changes in visual acuity (BCVA; Figure 9); durability (Figure 10); center subfield retinal fluid at weeks 16 and 48 (Figure 11 and Figure 12, respectively); and changes to central retinal thickness (CRT) (Figure 13) are also provided. In addition, safety data are set forth in Figure 14-Figure 24. Week 60 data with respect to absolute BCVA and change in BCVA over time are set forth in Figure 26. Durability is summarized in Figure 27 and the mean number of injections received in each group is summarized in Figure 28. Figure 29 summarizes central retinal thickness over time in the treatment groups.

### EXAMPLES

The present invention includes methods for achieving any of the individual results or PK points, for example, by the period of time after treatment initiation that is indicated (*e.g.*, improvement in BCVA by X ETDRS letters by Y days after treatment initiation) as is set forth in the Examples section in a subject having nAMD by administering an HDq12-20, HDq12, HDq16 or HDq20 treatment regimen to the subject.

### Example 1: Randomized, Double-Masked, Active-Controlled, Phase 3 Study of the Efficacy and Safety of High Dose Aflibercept in Patients with Neovascular Age-Related Macular Degeneration (PULSAR)

This phase 3, multi-center, randomized, double-masked, active-controlled study investigates the efficacy, safety, and tolerability of IVT administration of aflibercept 8 mg (HD) versus aflibercept 2 mg in participants with treatment-naive nAMD.

The study consists of a screening/baseline period, a treatment period with duration of 92 weeks, and an end of study visit at Week 96. No study intervention will be administered at the end of study visit at Week 96.

Approximately 960 eligible participants with nAMD are randomly assigned to receive IVT injections of HD or 2 mg in a 1:1:1 ratio to 3 parallel treatment groups:
- **2q8:** aflibercept 2 mg administered every 8 weeks, after 3 initial injections at 4-week intervals.
- **HDq12:** aflibercept 8 mg administered every 12 weeks, after 3 initial injections at 4-week intervals.
- **HDq16:** aflibercept 8 mg administered every 16 weeks, after 3 initial injections at 4-week intervals.

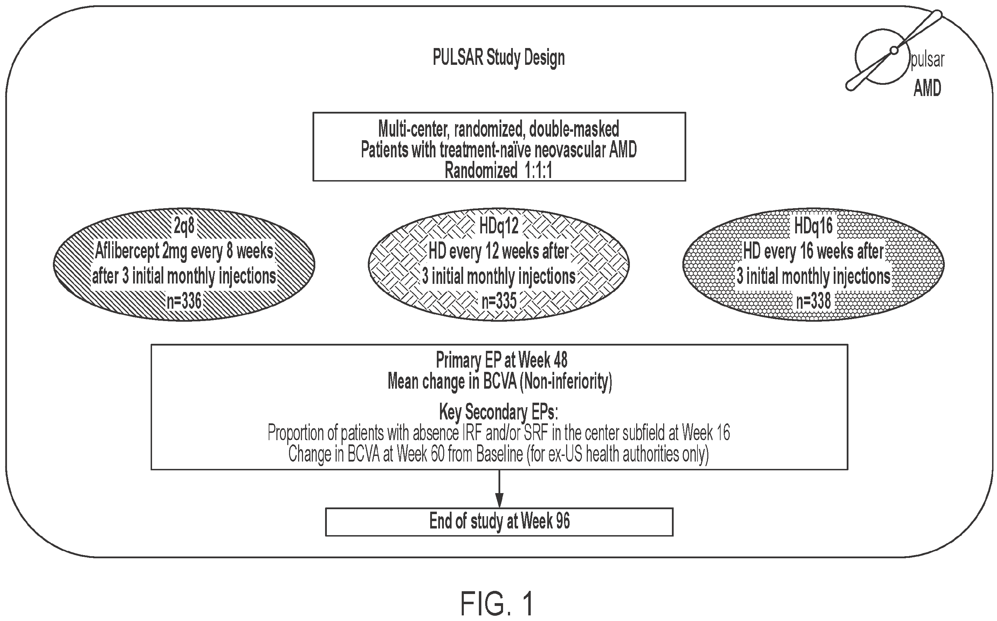

See Figure 1, Figure 3 and Figure 25.

Participants are stratified based on baseline BCVA and geographical region, to ensure balanced distribution of the treatment groups within each stratum. Only one eye can be treated within the study. Sham procedures are done on visits when an active injection is not planned. No sham procedures will be done at the non-treatment visit at Week 12. At all subsequent visits, all participants will receive either active study treatment injection or sham procedure (for masking purposes), depending on their assigned treatment schedule and eligibility for dose regimen modification.

Safety will be assessed by ophthalmic examinations, vital signs (including heart rate, blood pressure and temperature), electrocardiogram (ECG), AEs, and laboratory assessments. All AEs reported in this study will be coded using the currently available version of the Medical Dictionary for Regulatory Activities (MedDRA^{®}).

In all participants, blood samples for measurement of drug concentrations (for PK) will be obtained prior to the first treatment and at pre-specified time points throughout the course of the study. In addition, a deoxyribonucleic acid (DNA) blood sample will be collected from those who sign the informed consent form (ICF) for the optional genomic sub-study.

The study also includes a PK sub-study, with dense PK blood sampling for systemic drug concentrations and PK assessments for approximately 12 Japanese participants from Japan sites and 12 non-Asian participants from Europe or US sites (distributed across all 3 treatment groups). All participants in the PK sub-study will participate in the main study for 96 weeks but will have extra visits. Blood pressure and heart rate measurements will also be taken in these participants at the same timepoints as for the PK sampling.

### Dosing Schedule (Figure 3)

The dosing schedule is set forth below in Table 1-1.

**Table 1-1. Dosing Schedule**

| * | | | | | | | | | | | | | ** |
|---|---|---|---|---|---|---|---|---|---|---|---|---|---|
| | Day1 | Wk4 | Wk8 | Wk12 | Wk16 | Wk20 | Wk24 | Wk28 | Wk32 | Wk36 | Wk40 | Wk44 | Wk48 |
| 2q8 | X | X | X | | X | ○ | X | ○ | X | ○ | X | ○ | X |
| HDq12 | X | X | X | | ○ (a) | X (a) | ○ | ○ | X (c) | ○ | ○ | X (c) | ○ |
| HDq16 | X | X | X | | ○ (b) | o (b) | X (b) | ○ | ○ | ○ | X (c) | ○ | ○ |

| * | | | | | | | | | | | | | |
|---|---|---|---|---|---|---|---|---|---|---|---|---|---|
| | Wk52 | W56 | Wk60 | Wk64 | Wk68 | Wk72 | Wk76 | Wk80 | Wk84 | Wk88 | Wk92 | Wk96 | |
| 2q8 | ○ | X | ○ | X | ○ | X | ○ | X | ○ | X | ○ | | |
| HDq12 | ○ | X (d) | ○ | ○ | X (d) | ○ | ○ | X (d) | ○ | ○ | X (d) | | |
| HDq16 | ○ | X (d) | ○ | ○ | ○ | X (d) | ○ | ○ | ○ | X (d) | ○ | | |

| | | | | | | | | | | | | | |
|---|---|---|---|---|---|---|---|---|---|---|---|---|---|
| * Key 2° Endpoint at week 16 and 60 ** 1° Endpoint at week 48 | | | | | | | | | | | | | |

For masking purposes, DRM assessments will be performed in all participants at all visits (through the IXRS) starting from Week 16.
a HDq12 group: If DRM criteria are met, participants will continue on q8 rescue regimen.
b HDq16 group: If DRM criteria are met at Week 16 or 20, participant will continue on q8 rescue regimen. If DRM criteria are met at Week 24, participant will continue on q12 regimen.
c For participants remaining on a dosing interval of q12 or q16 weeks after Week 24, if DRM criteria are met at an active injection visit, the next dosing interval will be reduced by 4 weeks (to a minimum of q8).
d From Week 52, all participants in HD groups will be eligible for dose interval shortening (to a minimum of q8) or extension (by 4-week increments) according to pre-specified DRM criteria. If DRM criteria are met at an active injection visit, the next dosing interval will be changed by 4 weeks.

This table does not reflect all available dosing options, once a participant's dose regimen is shortened or extended. X=active injection, o=sham procedure 2q8=aflibercept 2 mg administered every 8 weeks, after 3 initial injections at 4-week intervals, HDq12=high dose aflibercept 8 mg administered every 12 weeks, after 3 initial injections at 4-week intervals, HDq16=high dose aflibercept 8 mg administered every 16 weeks, after 3 initial injections at 4-week intervals, 1°=primary, 2°=secondary, DRM=dose regimen modification, HD=high dose, q8=every 8 weeks, q12=every 12 weeks, q16=every 16 weeks, Wk=Week

### Primary Endpoints

The primary endpoint is:
- Change from baseline in BCVA measured by the Early Treatment Diabetic Retinopathy Study (ETDRS) letter score at Week 48

### Secondary Endpoints

The key secondary efficacy endpoints are:
- Change from baseline in BCVA measured by the ETDRS letter score at Week 60
- Proportion of participants with no intraretinal fluid (IRF) and no subretinal fluid (SRF) in central subfield at Week 16

The additional secondary efficacy endpoints are:
- Proportion of participants gaining at least 15 letters in BCVA from baseline at Week 48
- Proportion of participants achieving an ETDRS letter score of at least 69 (approximate 20/40 Snellen equivalent) at Week 48
- Change in choroidal neovascularization (CNV) size from baseline to Week 48
- Change in total lesion area from baseline to Week 48
- Proportion of participants with no IRF and no SRF in the center subfield at Week 48
- Change from baseline in central subfield retinal thickness (CST) at Week 48
- Change from baseline in National Eye Institute Visual Functioning Questionnaire-25 (NEI-VFQ-25) total score at Week 48

### Exploratory Endpoints

The exploratory endpoints are:
- Change from baseline in BCVA measured by the ETDRS letter score at Week 96
- Change from baseline in BCVA averaged over the period from Week 36 to Week 48 and from Week 48 to Week 60
- Proportion of participants gaining at least 15 letters in BCVA from baseline at Week 60 and Week 96
- Proportion of participants achieving an ETDRS letter score of at least 69 (approximate 20/40 Snellen equivalent) at Week 60 and Week 96
- Proportions of participants gaining and losing at least 5 or at least 10 letters in BCVA from baseline at Week 48, Week 60, and Week 96
- Proportion of participants losing at least 15 letters in BCVA from baseline at Week 48, Week 60, and Week 96
- Change in CNV size from baseline to Week 60 and Week 96
- Change in total lesion area from baseline to Week 60 and Week 96
- Change from baseline in CST at Week 60 and Week 96
- Proportion of participants with no IRF and no SRF in the center subfield at Week 96
- Proportion of participants without retinal fluid (total fluid, IRF, and/or SRF) and subretinal pigment epithelium fluid in center subfield at Week 48, Week 60, and Week 96
- Time to fluid-free retina over 48 weeks, 60 weeks, and 96 weeks (total fluid, IRF, and/or SRF in the center subfield)
- Proportion of participants with sustained fluid-free retina over 48 weeks, 60 weeks, and 96 weeks (total fluid, IRF, and/or SRF in the center subfield)
- Change from baseline in BCVA at each visit in relation to fluid outcomes
- Change from baseline in NEI-VFQ-25 total score at Week 60 and Week 96

### Number of Patients Planned

The study will enroll approximately 960 eligible participants with nAMD that will be randomly assigned to receive IVT injections of 8 mg or 2 mg in a 1:1:1 ratio in three parallel treatment groups.

### Study Population

The study population consists of treatment-naïve patients with nAMD.

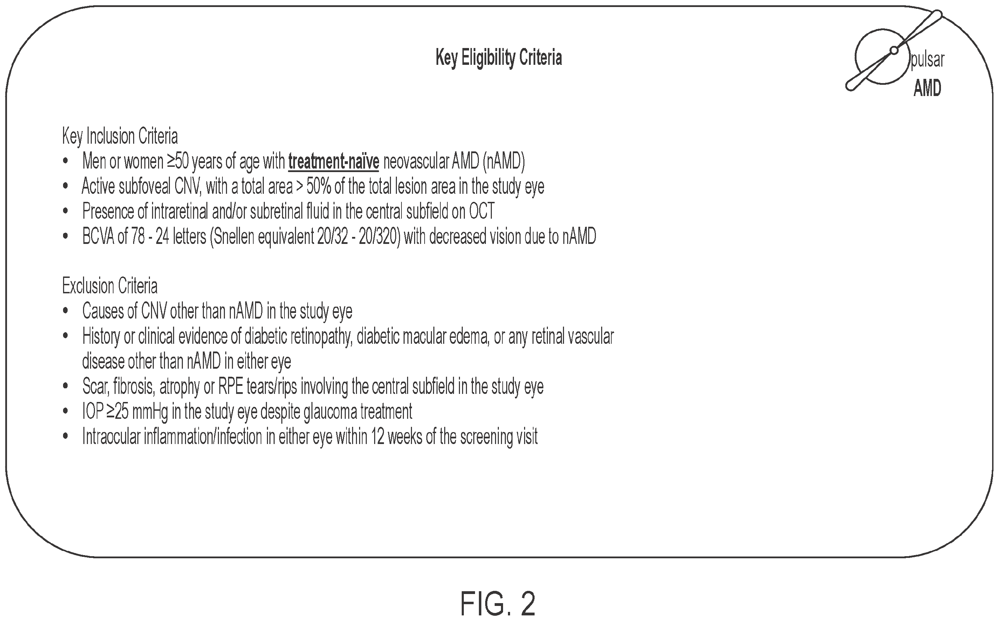

### Inclusion Criteria (Figure 2)

Participants are eligible to be included in the study only if all of the following criteria apply at both screening and baseline:
1. At least 50 years of age at the time of signing the informed consent.
2. Active subfoveal CNV secondary to nAMD, including juxtafoveal lesions that affect the fovea as assessed in the study eye.
3. Total area of CNV (including both classic and occult components) must comprise greater than 50% of the total lesion area in the study eye.
4. BCVA ETDRS letter score of 78 to 24 (corresponding to a Snellen equivalent of approximately 20/32 to 20/320) in the study eye.
5. Decrease in BCVA determined to be primarily the result of nAMD in the study eye.
6. Presence of IRF and/or SRF affecting the central subfield of the study eye on OCT. The central subfield is defined as a circle with diameter 1 mm, centered on the fovea.
7. Male or female.
8. Contraceptive use by men or women should be consistent with local regulations regarding the methods of contraception for those participating in clinical studies.
   a. Male participants: Men who are sexually active with partners of childbearing potential must agree to use highly effective contraception prior to the initial dose/start of the first treatment, during the study, and for at least 3 months after the last administration of study intervention.
   b. Female participants: Women of childbearing potential (WOCBP) must practice highly effective contraception prior to the initial dose/start of the first treatment, during the study, and for at least 3 months after the last administration of study intervention. Pregnancy testing and contraception are not required for women not considered WOCBP.
9. Capable of giving signed informed consent, which includes compliance with the requirements and restrictions listed in the ICF and in this protocol.

### Exclusion Criteria (Figure 2)

Participants are excluded from the study if any of the following criteria apply at either screening or baseline:

### Medical Conditions - Per Eye

1. Causes of CNV other than nAMD in the study eye.
2. Prior or concomitant conditions in the study eye:
   a. Subretinal hemorrhage that is at least 50% of the total lesion area, or if the blood under the fovea is 1 or more disc areas in size in the study eye.
   b. Scar or fibrosis making up more than 50% of the total lesion in the study eye.
   c. Scar, fibrosis, or atrophy involving the central subfield in the study eye.
   d. Presence of retinal pigment epithelial tears or rips involving the central subfield in the study eye.
   e. Total lesion size >12 disc areas (30.5 mm², including blood, scars, and neovascularization) as assessed by FA in the study eye.
   f. Uncontrolled glaucoma (defined as IOP >25 mmHg despite treatment with anti-glaucoma medication) in the study eye.
   g. History of idiopathic or autoimmune uveitis in the study eye.
   h. Vitreomacular traction or epiretinal membrane in the study eye evident on biomicroscopy or OCT that is thought to affect central vision.
   i. Any history of macular hole of stage 2 and above in the study eye.
   j. Structural damage to the center of the macula in the study eye that is likely to preclude improvement in BCVA following the resolution of retinal fluid including but not limited to, atrophy of the retinal pigment epithelium, subretinal fibrosis or scar or significant macular ischemia.
   k. History of, or likely future need of, filtration or tube shunt surgery on the study eye.
   l. Aphakia, or pseudophakia with absence of posterior capsule (unless it occurred as a result of a yttrium-aluminum-garnet [YAG] posterior capsulotomy performed more than 4 weeks (28 days) before screening), in the study eye.
   m. Myopia of a spherical equivalent of at least 8 diopters in the study eye prior to any refractive or cataract surgery.
   n. Significant media opacities, including cataract, that interfere with BCVA assessment, fundus photography or OCT imaging in the study eye.
   o. History of corneal transplant or corneal dystrophy in the study eye.
   p. History of irregular astigmatism or amblyopia with chronic limitation of BCVA in the study eye. Medical Conditions - Per Participant
3. Prior or concomitant conditions:
   a. History or clinical evidence of diabetic retinopathy, diabetic macular edema, or any retinal vascular disease other than nAMD in either eye.
   b. Evidence of extraocular or periocular infection or inflammation (including infectious blepharitis, keratitis, scleritis, or conjunctivitis) in either eye at the time of screening/randomization.
   c. Any intraocular inflammation/infection in either eye within 12 weeks (84 days) of the screening visit.
   d. Only 1 functional eye, even if that eye was otherwise eligible for the study (*e.g.*, BCVA of counting fingers or less in the eye with worse vision).
   e. Ocular conditions with poorer prognosis in the fellow eye.
4. Uncontrolled blood pressure (defined as systolic >160 mmHg or diastolic >95 mmHg). Participants may be treated with up to 3 agents known to have anti-hypertensive effects for arterial hypertension to achieve adequate blood pressure control. This limit applies to drugs that could be used to treat hypertension even if their primary indication in the participant was not for blood pressure control. Any recent changes in medications known to affect blood pressure need to be stable for 12 weeks prior to screening.
5. History of cerebrovascular accident or myocardial infarction within 24 weeks (168 days) before the screening visit.
6. Renal failure requiring dialysis, or renal transplant at screening or potentially during the study.
7. Allergy or hypersensitivity to any of the compounds/excipients in the study interventions formulations.
8. Presence of any contraindications indicated in the locally approved label for aflibercept.
9. History of other disease, metabolic dysfunction, physical examination finding, or clinical laboratory finding giving reasonable suspicion of a disease or condition that contraindicates the use of an investigational drug, might affect interpretation of the results of the study, or renders the participant at high risk for treatment complications.
10. Members of the clinical site study team and/or his/her immediate family, unless prior approval granted by the sponsor.
11. Pregnant or breastfeeding women.

### Prior Therapy

12. Any prior or concomitant ocular (in the study eye) or systemic treatment (with an investigational or approved, anti-VEGF or other agent) or surgery for nAMD, except dietary supplements or vitamins.

13. Prior treatment of the study eye with any of the following drugs (any route of ophthalmic administration) or procedures before baseline visit (Day 1):
a. Anti-angiogenic drugs at any time including investigational therapy (*e.g.*, with anti-angiopoietin/anti-VEGF bispecific monoclonal antibodies).
b. Long-acting steroids, within 16 weeks (112 days) before the screening visit, or any treatment with IVT implant, gene therapy, or cell therapy at any time.
c. Ocriplasmin (Jetrea^{®}) at any time.
d. Vitreoretinal surgery and/or including scleral buckling at any time.
e. Any other intraocular surgery within 90 days before the screening visit.
f. Panretinal laser photocoagulation or macular laser photocoagulation within 90 days before the screening visit.
g. YAG capsulotomy in the study eye within 30 days before the screening visit.

14. Prior treatment of the fellow eye with any of the following:
a. Investigational therapy (*e.g.*, with anti-angiopoietin/anti-VEGF bispecific monoclonal antibodies) within 180 days before the screening visit.
b. IVT implant, gene therapy, or cell therapy at any time.

Prior treatment in the fellow eye with approved anti-VEGF therapy is allowed. Prior treatment in the fellow eye with bevacizumab (although not approved but a component of standard of care in some countries) is also allowed.

15. Participation in other clinical trials requiring administration of investigational treatments (other than vitamins and minerals) at the time of screening, or within 30 days or 5 half-lives of administration of the previous study intervention, whichever is longer.

### Additional Exclusion Criteria for the Dense PK Sub-study

Participants who meet any of the following criteria will be not be eligible for the Dense PK Sub-study:
1. Prior treatment with IVT aflibercept in the fellow eye within 12 weeks (84 days) before the screening visit.
2. Active CNV in the fellow eye requiring anti-VEGF treatment at the time of screening visit.
3. Other IVT anti-VEGF treatment (ranibizumab, bevacizumab, brolucizumab, conbercept, pegaptanib sodium) in the fellow eye within 4 weeks (28 days) before the screening visit.
4. Systolic blood pressure >140 mmHg or diastolic blood pressure >90 mmHg.
5. Known cardiac arrhythmia, based on medical history and/or outcome of ECG at screening.
6. Variation by more than 10% in the 3 pre-randomization blood pressure measures recorded at the screening visits and at randomization
7. Participants who, in the opinion of the investigator, are unlikely to have stable blood pressure over the course of the study (*e.g.*, due to known or suspected non-compliance with medication).

### Investigational and Reference Treatments

The HD will be provided as a liquid formulation in a vial. The target concentration of aflibercept is 114.3 mg/mL. The dose will be delivered in an injection volume of 70 µl. The IAI will be provided as a liquid formulation in a vial. The target concentration of aflibercept is 40 mg/mL. The dose will be delivered in an injection volume of 50 µl.

### Study Assessments and Procedures

Study procedures and their timing are summarized in the following tables.

**Table 1-2. Schedule of Activities - Year 1**

| **Visit** | **1 Screening** | **2 Baseline** | **3** | **4** | **5** | **6** | **7** | **8** | **9** | **10** | **11** | **12** | **13** | **14** | **15** |
|---|---|---|---|---|---|---|---|---|---|---|---|---|---|---|---|
| **Week** | | | 4 | 8 | | 12 | 16 | 20 | 24 | 28 | 32 | 36 | 40 | 44 | 48 |
| Day | -21 to -1 | 1 | 29 | 57 | 60-64 | 85 | 113 | 141 | 169 | 197 | 225 | 253 | 281 | 309 | 337 |
| **Window (day)^{a}** | | | ±5 | ±5 | ^{b} | ±5 | ±5 | ±5 | ±5 | ±5 | ±5 | ±5 | ±5 | ±5 | ±5 |

| **Administrative:** | | | | | | | | | | | | | | | |
|---|---|---|---|---|---|---|---|---|---|---|---|---|---|---|---|
| Informed Consent (ICF) | X | | | | | | | | | | | | | | |
| Dense PK Substudy ICF^{c} | X | | | | | | | | | | | | | | |
| Genomic Substudy ICF^{d} | X | | | | | | | | | | | | | | |
| FBR ICF^{e} | X | | | | | | | | | | | | | | |
| Inclusion/Exclusion Eligibility | X | X^{f} | | | | | | | | | | | | | |
| Medical History | X | | | | | | | | | | | | | | |
| Demographics | X | | | | | | | | | | | | | | |
| Concomitant Medications | X | X | X | X | X | X | X | X | X | X | X | X | X | X | X |
| Randomization | | X | | | | | | | | | | | | | |
| **Study Intervention^{g}:** | | | | | | | | | | | | | | | |
| Study Intervention (active or sham) | | X | X | X | | | X | X | X | X | X | X | X | X | X |
| DRM Assessment^{h} | | | | | | | X | X | X | X | X | X | X | X | X |

| **Ocular Efficacy and Safety (bllatera unless Indicated):** | | | | | | | | | | | | | | | |
|---|---|---|---|---|---|---|---|---|---|---|---|---|---|---|---|
| NEI-VFQ-25' | | X | | | | | | | X | | | | | | X |
| BCVA (ETDRS) and Refraction | X | x | X | X | | X | X | X | X | X | X | X | X | X | X |
| IOP^{j} | X | x | X | X | | X | X | X | X | X | X | X | X | X | X |
| Slit Lamp Examination^{k} | X | x | X | X | | X | X | X | X | X | X | X | X | X | X |
| Indirect Ophthalmoscopy^{l} | X | X | X | X | | X | X | X | X | X | X | X | X | X | X |
| FA, FP^{m} | X | | | | | X | | | X | | | X | | | X |
| SD-OCT^{m} | X | X | X | X | | X | X | X | X | X | X | X | X | X | X |
| ICGAⁿ | X | | | | | | | | | | | | | | X |
| OCT-A^{o} | X | | | | | X | | | X | | | X | | | X |

| **Visit** | **1 Screening** | **2 Baseline** | **3** | **4** | **5** | **6** | **7** | **8** | **9** | **10** | **11** | **12** | **13** | **14** | **15** |
|---|---|---|---|---|---|---|---|---|---|---|---|---|---|---|---|
| **Week** | | | 4 | 8 | | 12 | 16 | 20 | 24 | 28 | 32 | 36 | 40 | 44 | 48 |
| Day | -21 to -1 | 1 | 29 | 57 | 60-64 | 85 | 113 | 141 | 169 | 197 | 225 | 253 | 281 | 309 | 337 |
| **Window (day)^{a}** | | | ±5 | ±5 | ^{b} | ±5 | ±5 | ±5 | ±5 | ±5 | ±5 | ±5 | ±5 | ±5 | ±5 |

| **Nonocular Safety:** | | | | | | | | | | | | | | | |
|---|---|---|---|---|---|---|---|---|---|---|---|---|---|---|---|
| Physical Examination | X | | | | | | | | | | | | | | |
| Vital Signs^{p} | X | X | X | X | X^{o} | X | X | X | X | X | X | X | X | X | X |
| ECG | X | | | | | | | | | | | | | | X |
| Adverse Events | X | X | X | X | X | X | X | X | X | X | X | X | X | X | X |

| **Laboratory Testing^{q}:** | | | | | | | | | | | | | | | |
|---|---|---|---|---|---|---|---|---|---|---|---|---|---|---|---|
| Hematology | X | | | | | | | | | | | | | | X |
| Blood Chemistry | X | | | | | | | | | | | | | | X |
| Pregnancy Test (WOCBP)^{r} | X Serum | X Urine | X Urine | X Urine | | X Urine | X Urine | X Urine | X Urine | X Urine | X Urine | X Urine | X Urine | X Urine | X Urine |
| Urinalysis, UPCR | X | | | | | | | | | | | | | | X |

| **Pharmacokinetics and Other Sampling:** | | | | | | | | | | | | | | | |
|---|---|---|---|---|---|---|---|---|---|---|---|---|---|---|---|
| PK Samples (Sparse)^{s} | | X | X | | X | X | | | | X | | | | | X |
| PK Samples (Dense)^{c} | | See Table 1-3 | | | | | | | | | | | | | |
| Anti-drup Antibody Serum Sample^{q,t} | | X | | | | | | | | | | | | | X |
| Genomic DNA Sample (optional)^{d} | | X | | | | | | | | | | | | | |

| | | | | | | | | | | | | | | | |
|---|---|---|---|---|---|---|---|---|---|---|---|---|---|---|---|
| BCVA=best corrected visual acuity, DNA=deoxyribonucleic acid, DRM = dose regimen modification, ECG=electrocardiogram, ETDRS=Early Treatment Diabetic Retinopathy Study, FA=fluorescein angiography, FBR=future biomedical research, FP=fundus photography, ICF=informed consent form, ICGA=indocyanine green angiography, IOP=Intraocular pressure, NEI-VFQ-25=National Eye Institute Visual Functioning Questionnaire-25, OCT-A=optical coherence tomography angiography, PK=pharmacokinetics, SD-OCT=spectral domain optical coherence tomography, UPCR=urine protein:creatinine ratio, WOCBP=women of childbearing potential | | | | | | | | | | | | | | | |

**Table 1-3. Schedule of Activities - Year 2**

| **Visit** | **16** | **17** | **18** | **19** | **20** | **21** | **22** | **23** | **24** | **25** | **26** | **27 EOS or ED** |
|---|---|---|---|---|---|---|---|---|---|---|---|---|
| **Week** | 52 | 56 | 60 | 64 | 68 | 72 | 76 | 80 | 84 | 88 | 92 | 96 |
| Day | 365 | 393 | 421 | 449 | 477 | 505 | 533 | 561 | 589 | 617 | 645 | 673 |
| **Window (day)**^{a} | ±5 | ±5 | ±5 | ±5 | ±5 | ±5 | ±5 | ±5 | ±5 | ±5 | ±5 | ±5 |

| **Administrative:** | | | | | | | | | | | | |
|---|---|---|---|---|---|---|---|---|---|---|---|---|
| Concomitant medications | X | X | X | X | X | X | X | X | X | X | X | X |

| **Study Intervention^{g}:** | | | | | | | | | | | | |
|---|---|---|---|---|---|---|---|---|---|---|---|---|
| Study Intervention (active or sham) | X | X | X | X | X | X | X | X | X | X | X | |
| DRM Assessment^{h} | X | X | X | X | X | X | X | X | X | X | X | |

| **Ocular Efficacy and Safety (bilateral unless indicated):** | | | | | | | | | | | | |
|---|---|---|---|---|---|---|---|---|---|---|---|---|
| NEI-VFQ-25ⁱ | | | X | | | | | | | | | X |
| BCVA (ETDRS) and refraction | X | X | X | X | X | X | X | X | X | X | X | X |
| IOP^{j} | X | X | X | X | X | X | X | X | X | X | X | X |
| Slit lamp examination^{k} | X | X | X | X | X | X | X | X | X | X | X | X |
| Indirect ophthalmoscopy^{l} | X | X | X | X | X | X | X | X | X | X | X | X |
| FA, FP^{m} | | | X | | | | | | | | | X |
| SD-OCT^{m} | X | X | X | X | X | X | X | X | X | X | X | X |
| ICGAⁿ | | | | | | | | | | | | X |
| OCT-A^{o} | | | X | | | | | | | | | X |

| **Nonocular Safety:** | | | | | | | | | | | | |
|---|---|---|---|---|---|---|---|---|---|---|---|---|
| Physical examination | | | | | | | | | | | | X |
| Vital signs^{p} | X | X | X | X | X | X | X | X | X | X | X | X |
| ECG | | | | | | | | | | | | X |
| Adverse events | X | X | X | X | X | X | X | X | X | X | X | X |

| **Laboratory Testing^{q}:** | | | | | | | | | | | | |
|---|---|---|---|---|---|---|---|---|---|---|---|---|
| Hematology | | | | | | | | | | | | X |
| Blood Chemistry | | | | | | | | | | | | X |
| Pregnancy Test (women of childbearing potential)^{r} | X Urine | X Urine | X Urine | X Urine | X Urine | X Urine | X Urine | X Urine | X Urine | X Urine | X Urine | X Urine |
| Urinalysis, UPCR | | | | | | | | | | | | X |
| Anti-drug Antibody Serum Sample^{q,t} | | | | | | | | | | | | X |

| | | | | | | | | | | | | |
|---|---|---|---|---|---|---|---|---|---|---|---|---|
| BCVA=best corrected visual acuity, DNA=deoxyribonucleic acid, DRM=dose regimen modification, ECG=electrocardiogram, ED=Early Discontinuation, EOS=End of Study, ETDRS=Early Treatment Diabetic Retinopathy Study, FA=fluorescein angiography, FP=fundus photography, ICF=informed consent form, ICGA=indocyanine green angiography, IOP=Intraocular pressure, NEI-VFQ-25=National Eye Institute Visual Functioning Questionnaire-25, OCT-A=optical coherence tomography angiography, PK=pharmacokinetics, SD- OCT=spectral domain optical coherence tomography, UPCR=urine protein:creatinine ratio, WOCBP=women of childbearing potential | | | | | | | | | | | | |

Footnotes for the Schedule of Activities Tables
a Visit schedules may deviate by up to ±5 days. Set schedule visits (except Visit 5) use baseline for the calculation. The procedures required at each visit have to be complete within 3 days, i.e., split visits are allowed. Additionally, all procedures have to be complete within the 5-day window. Slit lamp (anterior segment), IOP measurement, and indirect ophthalmoscopy are recommended to take place on the same day as the IVT injection.
b Visit 5 must be within 3 to 7 days after the Week 8 injection. This visit uses the date of Visit 4 for calculation.
c Dense PK sampling will be performed in a subgroup including participants at Japanese and non-Asian sites. The Dense PK Sub-study ICF should be presented and signed at the screening visit. Refer to Table 1-3. Participants in the Dense PK Sub-study have extra visits but otherwise participate in the main study with a 96 week duration.
d The optional genomic sub-study ICF should be presented to participants at the screening visit and may be signed at any subsequent visit at which the participant chooses to participate after screening. The genomic DNA blood sample should be collected on Day 1/baseline (pre-injection) or at any time during the study, only from participants who consent to participate in the genomic sub-study. Participants from China will not be enrolled in this optional sub-study.
e The optional FBR ICF should be presented to participants and signed at the screening visit. No additional blood sample is required - remaining blood samples (from *e.g.*, PK or anti-drug antibody [ADA] sampling) may be used.
f Inclusion/exclusion criteria will be evaluated at screening and baseline to confirm subject's eligibility. The investigator is responsible for confirming that any changes between screening and baseline do not affect the participant's eligibility.
g Following study intervention injection or sham procedure, participants will be observed for at least 30 minutes.
h For masking purposes, assessments for dose regimen modification (DRM) or potential shortening to the rescue regimen (8 mg q8) will be performed in all participants at all visits starting from Week 16. Actual DRMs will be implemented. (See Figure 3, Figure 4 and Figure 25)
i NEI-VFQ-25 to be administered in a quiet room by a masked study-related person trained to administer this type of questionnaire, preferably before other visit procedures are performed.
j IOP will be measured at all study visits (bilateral). On days when study intervention is administered, IOP should be measured pre-injection (bilaterally) and approximately 30 minutes after administration of study intervention (study eye only). IOP will be measured using Goldman applanation tonometry, rebound tonometry Icare, or Tonopen and the same method of measurement must be used in each participant throughout the study.
k Slit lamp examination will be performed bilaterally.
l Indirect ophthalmoscopy will be performed bilaterally at all visits. On days when study intervention is administered, it should also be performed immediately after administration of study intervention (study eye only).
m The same SD-OCT/FA/FP imaging system used at screening and Day 1 must be used at all follow-up visits in each participant. Images will be taken in both eyes before dosing (active or sham injection).
n Optional at all sites that have the relevant equipment. ICGA will be used to diagnose and characterize the polypoidal choroidal vascularization (PCV) subtype of nAMD. If ICGA cannot be performed at screening visit, it may be done at baseline visit.
o OCT-A is optional at all sites that have the relevant equipment. If OCT-A cannot be performed at screening visit, it may be done at baseline visit.
p Vital signs (temperature, blood pressure, heart rate) should be measured per the procedure outlined in the study manual. At Visit 5, only blood pressure and heart rate are required. Vital signs should be measured prior to injection and any blood sampling. When possible, timing of all blood pressure assessments should be within 2 hours of clock time of dosing on Day 1. Measurements will be taken pre-dose (active or sham injection).
q All samples collected for laboratory assessments should be obtained prior to administration of fluorescein and/or indocyanine green, and prior to administration of study intervention.
r For women of childbearing potential, a negative serum pregnancy test at screening is required for eligibility. A negative urine pregnancy test is required before any treatment (including rescue regimen) is administered at subsequent visits.
s Sparse PK sampling will be performed in all participants (optional for participants in China). Any PK sampling will be done prior to dosing if scheduled at the sampling time point.
t Anti-drug antibody sample collection is optional for participants in China.

**Table 1-4. Schedule of Activities - Dense PK Sub-Study**

| Visit | **Dose** | **Assessment Day** | **Assessment Time (h)** | **PK Sample** | **Heart Rate and Blood Pressure^{a}** |
|---|---|---|---|---|---|
| Screening 2^{b} | | -21 to -1 | ±2h | | X |
| Visit 2 (Baseline) | | 1 | Pre-dose^{c} | X (pre-iniection) | X |
| | X | | 4^{c} | X | |
| | | | 8^{c} | X | |
| | | 2 | ±2h^{c} | X | X |
| | | 3 | ±2h^{c} | X | X |
| | | 5 | ±2h^{c} | X | X |
| | | 8 | ±2h^{c} | X | X |
| | | 15 | ±2h^{c} | X | X |
| | | 22 | ±2h^{c} | X | X |

| | | | | | |
|---|---|---|---|---|---|
| PK=pharmacokinetics | | | | | |

Participants enrolled in the Dense PK Sub-study will also have blood pressure and heart rate assessed at each visit within the sub-study.

Participants enrolled in the Dense PK Sub-study will also have blood pressure and heart rate assessed at each visit within the sub-study.
a Timing of all blood pressure assessments must be within ±2 hours of the clock time of dosing on Day 1. Blood pressure assessments for participants in the Dense PK Sub-study will be taken prior to blood sample collection using automated office blood pressure (AOBP) measurement with the Omron Model HEM 907XL (or comparable). Measures will be recorded in the electronic case report form (eCRF). Detailed instructions can be found in the study manual.
b Additional blood pressure assessment between screening and baseline, to confirm eligibility for participants in the Dense PK Sub-study. Screening 2 may occur on the same day as the screening visit.
c On Day 1, the 4 hour and 8 hour PK sampling is to be within ±30 minutes and ±2 hours, respectively, of the scheduled time. For subsequent days, PK sampling is to be performed within ±2 hours of the clock time of dosing on Day 1.

### Ophthalmic and General Examinations

All ophthalmic examinations are described, irrespective of whether they are used for efficacy or safety assessments. All ophthalmic examinations are to be conducted pre-injection in both eyes and post-injection in the study eye only, unless indicated otherwise. At any visit, ophthalmic examinations not stipulated by this protocol may take place outside of this protocol at the discretion of the investigator.

*Best Corrected Visual Acuity (BCVA)* - Visual function will be assessed using the ETDRS protocol (2) starting at 4 meters. Refraction is to be done at each visit. Visual acuity examiners must be certified to ensure consistent measurement of BCVA. Any certified and trained study personnel may perform this assessment (including but not limited to ophthalmologist, optometrist, or technician) and must remain masked to treatment assignment. For each participant, the same examiner must perform all assessments whenever possible. BCVA should be done before any other ocular procedures are performed.

*Intraocular Pressure (IOP)* - IOP will be measured using Goldman applanation tonometry, rebound tonometry Icare, or Tonopen and the same method of measurement must be used in each participant throughout the study. At all visits, IOP should be measured bilaterally by the masked investigator (or designee). On days when study intervention is administered, IOP should also be measured approximately 30 minutes after administration of study intervention (study eye only) by the unmasked investigator (or designee). If multiple post-injection measurements are performed, the final measurement before the participant leaves should be documented in the eCRF. Any injection-related increase in IOP (and treatment) should be documented in a masked fashion.

*Slit Lamp Examination* - The slit lamp examination will be performed according to local medical practice and applicable medical standards at the site. Participants' anterior eye structure and ocular adnexa will be examined bilaterally (pre-dose on visits with active injection) at each study visit using a slit lamp.

*Indirect Ophthalmoscopy* - Indirect ophthalmoscopy will be performed according to local medical practice and applicable medical standards at the site. Participants' posterior pole and peripheral retina will be examined by indirect ophthalmoscopy at each study visit pre-dose (bilateral) by the masked investigator and post-dose (study eye). Post-dose evaluation must be performed immediately after injection.

*Fundus Photography (FP) and Fluorescein Angiography (FA)* -The anatomical state of the retinal vasculature of the study eye will be evaluated by FP and FA. The treating investigator may perform additional FA/FP at other times during the study based on his/her medical judgment and standard of care. Photographers must be masked to treatment assignment and must be certified by the reading center to ensure consistency and quality in image acquisition. FP and FA images will be read by the investigator for individual treatment decisions and sent to an independent reading center where images will be read by masked readers. The participants' eligibility to participate in the study in terms of FA will be confirmed by the central reading center before randomization. The same FA/FP imaging system used at screening and Day 1 must be used at all subsequent visits in each participant. Images will be taken in both eyes before dosing (active or sham injection).

*Spectral Domain Optical Coherence Tomography (SD-OCT) -*Retinal and lesion characteristics will be evaluated using SD-OCT. For all visits where the SD-OCT procedure is scheduled, images will be captured and read by the technician and investigator for individual treatment decisions and sent to an independent reading center. The participants' eligibility to take part in the study in terms of SD-OCT will be confirmed by the central reading center before randomization. The same SD-OCT imaging system used at screening and Day 1 must be used at all follow-up visits in each participant. Images will be taken in both eyes before dosing (active or sham injection).

*Indocyanine Green Angiography (ICGA)* - ICGA will be optional, performed at sites with the appropriate equipment. ICGA will be used to diagnose and characterize the PCV subtype of nAMD. The same imaging modality used at screening must be used at all follow-up visits in each participant. Images will be taken in both eyes before dosing (active or sham injection).

*Optical Coherence Tomography Angiography (OCT-A)* - Optical coherence tomography angiography (OCT-A) will be optional, performed at sites with the relevant equipment. The same imaging modality used at screening must be used at all follow-up visits in each participant. Images will be taken in both eyes before dosing (active or sham injection).

*National Eye Institute Visual Functioning Questionnaire-25 (NEI-VFQ-25)* - Visionrelated quality of life (QoL) will be assessed using the NEI-VFQ-25 questionnaire (3) in the interviewer-administered format. It is a reliable and valid 25-item version of the 51-item NEI-VFQ.

### Dose Regimen Modification (DRM)

For masking purposes, assessments for dose regimen modifications (DRM) will be performed in all participants at all visits starting from Week 16. Based on these assessments, participants in the HD groups may have their treatment intervals shortened or extended. The minimum interval between injections will be 8 weeks, which is considered a rescue regimen for participants randomized to HD aflibercept who are unable to tolerate a dosing interval greater than every 8 weeks. Participants in the aflibercept 2 mg group will remain on fixed q8 dosing throughout the study (i.e., will not have modifications of their treatment intervals regardless of the outcomes of the DRM assessments).

Baseline to Week 48 - Beginning at Week 16, participants in the HD groups will have the dosing interval shortened (at the visits described below) if BOTH the following DRM criteria are met:
1. BCVA loss >5 letters from Week 12, AND
2. >25 µm increase in central retinal thickness (CRT) from Week 12 OR new foveal hemorrhage OR new foveal neovascularization If a participant in the HDq12 group or the HDq16 group meets both criteria at Week 16 or Week 20, the participant will be dosed with 8 mg aflibercept at that visit and will continue on rescue regimen (aflibercept 8 mg, every 8 weeks).

If a participant in the HDq16 group who has not met the criteria at Week 16 or Week 20 meets both criteria at Week 24, the participant will be dosed with 8 mg aflibercept at that visit and will continue on q12 dosing.

For participants whose interval was not shortened to q8 dosing at or before Week 24, the interval will be shortened if the DRM criteria are met at subsequent visits with active injection. Participants in the HDq12 group who meet the criteria will receive the planned dose at that visit and will then continue on rescue regimen (aflibercept 8 mg, every 8 weeks). Participants in the HDq16 group who meet these criteria will receive the planned dose at that visit and will then continue to be dosed every 12 weeks if they were on a 16-week interval, or switch to the rescue regimen (aflibercept 8 mg, every 8 weeks) if they were on a 12-week interval. Therefore, a participant randomized to HDq16 whose injection interval has been shortened to q12 will have their injection interval further shortened to q8 if these criteria are met at any subsequent assessment.

Week 52 to Week 96 (End of Study) - From Week 52 through the end of study (Year 2), all participants in the HD groups will continue to have the interval shortened in 4-week intervals (to a minimum of q8) if the DRM criteria for shortening are met at visits with active injection, using the criteria described above for Year 1.

In addition to shortening of the interval, all participants in the HD groups (including HD group participants whose interval was shortened during Year 1) may be eligible for interval extension (by 4-week increments) (if the following DRM criteria are met at visits with active injection in Year 2:
1. BCVA loss <5 letters from Week 12, AND
2. No fluid at the central subfield on OCT, AND
3. No new onset foveal hemorrhage or foveal neovascularization

For participants who do not meet the criteria for shortening or extension of the interval, the dosing interval will be maintained.

As in Year 1, all participants in all treatment groups (including the 2q8 group) will be evaluated against both DRM criteria at all visits. However, changes to dosing schedule will only be implemented as described above. No changes to the dosing schedule will be made to the 2q8 treatment group at any time. All anatomic criteria will be based on the site evaluations/OCT assessments, not on the reading center assessments.

Intervention After the End of the Study Intervention will not be supplied after the end of the study. Participants will not be restricted with regard to pursuing available approved treatments for nAMD.

### Treatment group descriptions

**2q8**: Aflibercept 2 mg administered every 8 weeks, after 3 initial injections at 4-week intervals.

**HDq12**: High dose aflibercept 8 mg administered every 12 weeks, after 3 initial injections at 4-week intervals.

**HDq16**: High dose aflibercept 8 mg administered every 16 weeks, after 3 initial injections at 4-week intervals.

**All HD**: Pooled high dose aflibercept 8 mg administered every 12 weeks or every 16 weeks, after 3 initial injections at 4-week intervals.

### Results at Week 48

### Visual Outcomes

The primary analysis of the change from baseline in BCVA resulted in LSₘₑₐₙ changes from baseline to Week 48 (*i.e.*, estimated, adjusted mean changes) of 7.03, 6.06 and 5.89 letters for the 2q8, HDq12 and HDq16 groups, respectively (Table 1-5).

The estimated difference in LSₘₑₐₙₛ changes from baseline to Week 48 in BCVA (with corresponding 95% CI) of HDq12 vs. 2q8 was -0.97 (-2.87, 0.92) letters and of HDq16 vs. 2q8 was -1.14 (-2.97, 0.69) letters (Table 1-5). The p-values for the non-inferiority test at a margin of 4 letters were 0.0009 for HDq12 vs. 2q8, and 0.0011 for HDq16 vs. 2q8; p-values for a superiority test were 0.8437 for HDq12 vs. 2q8 and of 0.8884 for HDq16 vs. 2q8.

The arithmetic mean (SD) changes from baseline in BCVA to Week 48 (*i.e.*, observed, unadjusted mean changes) were 7.6 (12.2), 6.7 (12.6), and 6.2 (11.7) letters for the 285, 299, and 289 participants with Week 48 data, *i.e.*, excluding data after an ICE as handled by the hypothetical strategy, in the 2q8, HDq12, and HDq16 groups, respectively (Table 1-5).

**Table 1-5. Change From Baseline in BCVA Measured by the ETDRS Letter Score at Week 48 and Week 60 in the Study Eye, MMRM (Full Analysis Set)**

| | **2q8** N = 336 | **H̅D̅q̅1̅2̅** N = 335 | **HDq16** N = 338 |
|---|---|---|---|
| **Week 48 (primary endpoint)** | | | |
| Baseline mean (a) | 58.9 | 59.9 | 60.0 |
| Number of subjects with Week 48 data | 285 | 299 | 289 |
| Arithmetic mean (SD) change from baseline (a) | 7.6 (12.2) | 6.7 (12.6) | 6.2 (11.7) |
| LS mean (SE) change from baseline | 7.03 (0.74) | 6.06 (0.77) | 5.89 (0.72) |
| DF | / | 622.1 | 647.7 |
| Contrast (b) | / | HDq12 - 2q8 | HDq16 - 2q8 |
| t-value | / | 3.14 | 3.07 |
| p-value of one-sided test for non-inferiority at a margin of 4 letters | / | 0.0009 | 0.0011 |
| Estimate for Contrast and two-sided 95% CI (c) | / | -0.97 (-2.87,0.92) | -1.14 (-2.97,0.69) |

| **Week 60 (key secondary endpoint)** | | | |
|---|---|---|---|
| Baseline mean (a) | 58.9 | 59.9 | 60.0 |
| Number of subjects with Week 60 data | 268 | 283 | 282 |
| Arithmetic mean (SD) change from baseline (a) | 7.8 (12.6) | 6.6 (13.6) | 6.6 (11.7) |
| LS mean (SE) change from baseline | 7.23 (0.68) | 6.37 (0.74) | 6.31 (0.66) |
| DF | / | 896.3 | 928.7 |
| Contrast (b) | / | HDq12 - 2q8 | HDq16 - 2q8 |
| t-value | / | 3.61 | 3.81 |
| p-value of one-sided test for non-inferiority at a margin of 4 letters | / | 0.0002 | <0.0001 |
| Estimate for Contrast and two-sided 95% CI (c) | / | -0.86 (-2.57,0.84) | -0.92 (-2.51,0.66) |

| | | | |
|---|---|---|---|
| BCVA = best corrected visual acuity, CI = Confidence interval, DF = Degrees of freedom, ETDRS = Early Treatment Diabetic Retinopathy Study, LS = Least Square, SAP = statistical analysis plan, SD = Standard deviation, SE = Standard error | | | |

A mixed model for repeated measurements (MMRM) was used with baseline BCVA measurement as a covariate, treatment group, visit and the stratification variables (geographic region [Japan vs. Rest of World]; baseline BCVA [< 60 vs. ≥ 60]) as fixed factors, and terms for the interaction between baseline BCVA and visit and the interaction between treatment and visit.

A Kenward-Roger approximation was used for the denominator degrees of freedom. In order to model the within-subject error the following covariance structure was used: unstructured (for Week 48) and Toeplitz with heterogeneity (for Week 60).

Intercurrent events (ICE) were handled according to primary estimand strategy for continuous endpoints.
(a): Based on observed assessments.
(b): The contrast also includes the interaction term for treatment x visit
(c): Estimate based on the MMRM model, was computed for the differences of HDq12 minus 2q8 and HDq16 minus 2q8, respectively, with two-sided 95% CIs.

The proportions of participants gaining at least 15 letters in BCVA from baseline at Week 48, using LOCF in the FAS, were similar across the 3 treatment groups; the small numerical differences across the treatment groups were not clinically meaningful (Table 1-6). The proportions and between-treatment differences obtained for the corresponding analysis based on OC prior to ICE were consistent with the results using LOCF.

**Table 1-6. Proportion of Participants who Gained at Least 15 Letters in BCVA from Baseline at Week 48, LOCF (Full Analysis Set)**

| | **2q8 N=336** | **HDq12 N=335** | **HDq16 N=338** |
|---|---|---|---|
| Week 48 (additional secondary efficacy variable) | | | |
| Subjects who gained ≥ 15 letters, Num/Den (%) | 74/335 (22.1%) | 69/334 (20.7%) | 73/337 (21.7%) |
| Contrast | / | HDq12 - 2q8 | HDq16 - 2q8 |
| Difference (a) % (two-sided 95% CI) | / | -1.748 (-7.784, 4.287) | -0.939 (-6.997, 5.119) |
| CMH test (b) p-value | / | 0.5704 | 0.7611 |

| | | | |
|---|---|---|---|
| *BCVA* ≥ *69* | | | |

The proportions of participants achieving an ETDRS letter score of at least 69 (approximate 20/40 Snellen equivalent) at Week 48 using LOCF in the FAS were similar across the 3 treatment groups; the small numerical differences between the treatment groups were not clinically meaningful. The proportions and between-treatment differences obtained for the corresponding analysis based on OC prior to ICE were consistent with the results using LOCF.

**Table 1-7. Proportion of Participants who Achieved an ETDRS Letter Score of at Least 69 at Week 48, LOCF (Full Analysis Set)**

| | **2q8** | **HDq12** | **HDq16** |
|---|---|---|---|
| | N = 336 | N = 335 | N = 338 |
| Subjects who achieved ≥ 69 letters, | 194/335 (57.9%) | 190/334 (56.9%) | 183/337 (54.3%) |
| Num/Den (%) | | | |
| Contrast | / | HDq12 - 2q8 | HDq16 - 2q8 |
| Difference (a) % (two-sided 95% CI) | / | -0.182 (-6.565, 6.200) | -2.221 (-8.435, 3.994) |
| CMH test (b) p-value | / | 0.9554 | 0.4834 |

| | | | |
|---|---|---|---|
| BCVA = best corrected visual acuity, CI = Confidence interval, ETDRS = Early Treatment Of Diabetic Retinopathy Study, LOCF = Last observation carried forward, Num/Den = numerator/denominator, SAP = statistical analysis plan; LOCF method for the last available observed value prior to ICE was carried forward to impute missing data; Intercurrent events (ICE) were handled according to primary estimand strategy for continuous endpoints. (a) Difference is HD groups minus 2q8 and CI was calculated using Mantel-Haenszel weighting scheme adjusted by geographical region and baseline BCVA (< 60 vs. ≥ 60) and is displayed with two-sided 95% CIs. (b) Nominal p-value for the two-sided Cochran-Mantel-Haenszel (CMH) test. | | | |

### Gaining at least 15 Letters

The proportions of participants gaining at least 15 letters in BCVA from baseline at Week 48, using LOCF in the FAS, were similar across the 3 treatment groups; the small numerical differences between the treatment groups were not clinically meaningful (see Table 1-15 below). The proportions and between-treatment differences obtained for the corresponding analysis based on OC prior to ICE were consistent with the results using LOCF.

### Compliance with Study Treatment

79% of patients in the HDq12 group and 77% of patients in the HDq16 group and 83% of combined patients in the HDq12 and HDq16 groups (≥ 12 weeks) were maintained in these groups through week 48 of the study. Treatment compliance in the safety analysis set is summarized in Table 1-8; see also Table 1-46.

**Table 1-8. Compliance with Study Treatment: Through Week 48 (Safety Analysis Set)**

| | | **2q8** | **HDq12** | **HDq16** | **All HD** |
|---|---|---|---|---|---|
| | | N = 336 (100%) | N = 335 (100%) | N = 338 (100%) | N = 673 (100%) |
| Number of subjects receiving 100% planned injections within 48-week period | | 275 (81.8%) | 287 (85.7%) | 284 (84.0%) | 571 (84.8%) |

| Treatment compliance (%) | | | | | |
|---|---|---|---|---|---|
| | n | 336 | 335 | 337 | 672 |
| | Mean (SD) | 97.69 (5.80) | 98.03 (5.48) | 98.02 (5.16) | 98.03 (5.32) |
| | Median | 100.00 | 100.00 | 100.00 | 100.00 |
| | Min, Max | 63.6,100 | 63.6,100 | 63.6,100 | 63.6,100 |

| Compliance categories, n (%) | | | | | |
|---|---|---|---|---|---|
| | > 90 to ≤ 100% | 321 (95.5%) | 317 (94.6%) | 321 (95.0%) | 638 (94.8%) |
| | > 80 to ≤ 90% | 7 (2.1%) | 12 (3.6%) | 12 (3.6%) | 24 (3.6%) |
| | ≤ 80% | 8 (2.4%) | 6 (1.8%) | 4 (1.2%) | 10 (1.5%) |

| | | | | | |
|---|---|---|---|---|---|
| SD = standard deviation Compliance = (Number of actual study interventions received during period before Week 48 or up to premature discontinuation)/ (Number of planned study interventions during period before Week 48 or up to premature discontinuation) x 100 | | | | | |

### Retinal Fluid

The proportion of participants with no retinal fluid (no IRF and no SRF) in the center subfield at Week 48 was numerically higher in the HDq12 and HDq16 groups (71.1% and 66.8%, respectively) compared to the 2q8 treatment group 59.4%, based on LOCF in the FAS. The pair-wise differences (95% CI) for the 2-sided tests, using Mantel-Haenszel weighting scheme adjusted by geographical region and baseline BCVA (< 60 vs. ≥ 60), of 11.725% points (4.527%, 18.923%) for HDq12 vs. 2q8 and 7.451% points (0.142%, 14.760%) for HDq16 vs. 2q8 were both in favor of HD treatment.

Even larger differences in favor of HD treatment were obtained using OC prior to ICE for the pair-wise comparisons in the FAS, providing differences of 15.417% points (7.664%, 23.170%) for HDq12 vs. 2q8 and 11.397% points (3.452%, 19.343%) for HDq12 vs. 2q8. See Table 1-9.

**Table 1-9. Proportion of Participants with No IRF and No SRF in the Central Subfield at Week 48, LOCF (Full Analysis Set)**

| | **2q8** | **HDq12** | **HDq16** |
|---|---|---|---|
| | **N = 336** | **N = 335** | **N = 338** |
| Subjects who had no IRF and no SRF, Num/Den (%) | 199/335 (59.4%) | 236/332 (71.1%) | 223/334 (66.8%) |
| Contrast | / | HDq12 - 2q8 | HDq16 - 2q8 |
| Difference (a) % (two-sided 95% CI) | / | 11.725 (4.527, 18.923) | 7.451 (0.142, 14.760) |
| CMH test (b) p-value | / | 0.0015 | 0.0458 |

| | | | |
|---|---|---|---|
| BCVA = best corrected visual acuity, CI = Confidence interval, CMH = Cochran-Mantel-Haenszel, IRF = Intraretinal fluid, LOCF = Last observation carried forward, Num/Den = numerator/denominator, SAP = statistical analysis plan, SRF = Subretinal fluid LOCF method for the last available observed value prior to ICE were carried forward to impute missing data. | | | |

Intercurrent events (ICE) were handled according to primary estimand strategy for binary endpoints.
(a) Difference is HD groups minus 2q8 and CI was calculated using Mantel-Haenszel weighting scheme adjusted by geographical region and baseline BCVA (< 60 vs. ≥ 60) and is displayed with two-sided 95% CIs.
(b) Nominal p-value for the two-sided Cochran-Mantel-Haenszel (CMH) test.

### Retinal Thickness

The mean values of CST at baseline were similar, ranging from 367.1 to 370.7 µm across the 3 treatment groups. Mean decreases from baseline were observed in all treatment groups at Week 48, which were higher in the HD groups than in the 2q8 group. The estimated contrasts (95% CIs) for the 2-sided tests, using the MMRM in the FAS, of -11.12 (-21.06,-1.18) µm for HDq12 vs. 2q8 and of -10.51 (-20.12,-0.90) µm for HDq16 vs. 2q8 were both numerically in favor of HD treatment (Table 1-10).

The corresponding analysis using an ANCOVA with LOCF in the FAS provided mean changes from baseline to Week 48 and estimated contrasts (95% CIs) for the 2-sided tests between the HD groups and the 2q8 group that were numerically also in favor of HD treatment and thus consistent with the results from the analysis using MMRM.

**Table 1-10. Change from Baseline in CST (µm) at Week 48, MMRM (full analysis set)**

| | **2q8** | **HDq12** | **HDq16** |
|---|---|---|---|
| | **N = 336** | **N = 335** | **N = 338** |
| LS mean (SE) change from baseline | -136.25 (4.24) | -147.37 (4.01) | -146.76 (3.76) |
| Arithmetic mean (SD) change from baseline (a) | -126.3 (124.3) | -141.9 (120.1) | -147.1 (131.2) |
| Baseline mean (a) | 367.1 | 370.3 | 370.7 |
| Number of subjects with Week 48 data | 273 | 289 | 282 |
| DF | / | 626.1 | 608.6 |
| Contrast (b) | / | HDq12-2q8 | HDq16-2q8 |
| t-value | / | -2.20 | -2.15 |
| P-value(c) | / | 0.0283 | 0.0321 |
| Estimate for Contrast and two-sided 95% CI (d) | / | -11.12 (-21.06,-1.18) | -10.51 (-20.12,-0.90) |

| | | | |
|---|---|---|---|
| BCVA = best corrected visual acuity, CI = confidence interval, CST = central subfield retinal thickness, DF = degrees of freedom, LS = least square, SAP = statistical analysis plan, SD = standard deviation, SE = standard error | | | |

A mixed model for repeated measurements (MMRM) was used with baseline CST as a covariate, treatment group, visit and the stratification variables (geographic region [Japan vs. Rest of World]; baseline BCVA [< 60 vs. ≥ 60]) as fixed factors, and terms for the interaction between baseline CST and visit and the interaction between treatment and visit.

A Kenward-Roger approximation was used for the denominator degrees of freedom. In order to model the within-subject error the following covariance structure was used: unstructured.

Intercurrent events (ICE) were handled according to primary estimand strategy for continuous endpoints.
(a) Based on observed assessments.
(b) The contrast also includes the interaction term for treatment x visit.
(c) P-value for the two-sided test.
(d) Estimate based on the MMRM model, was computed for the differences of HDq12 minus 2q8 and HDq16 minus 2q8, respectively with two-sided 95% CIs.

### Patient Reported Outcomes

The mean values of the NEI-VFQ-25 total score at baseline were similar across the 3 treatment groups, ranging from 76.4 to 77.8. Mean increases from baseline were observed in all groups at Week 48, which were numerically lower in the HD groups than in the 2q8 group. The estimated contrasts (95% CIs) for the 2-sided tests using the MMRM in the FAS were small and not clinically meaningful for both comparisons, HDq12 vs. 2q8 and HDq16 vs. 2q8 (Table 1-11).

The corresponding analysis using an ANCOVA with LOCF in the FAS provided mean changes from baseline to Week 48 and estimated contrasts (95% CIs) for the 2-sided tests between the HD groups and the 2q8 group that were similar to those based on MMRM and thus also not clinically meaningful.

**Table 1-11. Change from Baseline in NEI-VFQ-25 Total Score at Week 48, MMRM (Full Analysis Set)**

| | **2q8** | **H̅D̅q̅1̅2̅** | **HDq16** |
|---|---|---|---|
| | N = 336 | N = 335 | N = 338 |
| Baseline mean (a) | 77.8 | 76.4 | 77.7 |
| Number of subjects with Week 48 data | 266 | 285 | 266 |
| Arithmetic mean (SD) change from baseline (a) | 4.6 (11.0) | 4.1 (10.4) | 3.4 (10.8) |
| LS mean (SE) change from baseline | 4.22 (0.70) | 3.50 (0.70) | 3.35 (0.72) |
| DF | / | 571.7 | 540.3 |
| Contrast (b) | / | HDq12-2q8 | HDq16-2q8 |
| t-value | / | -0.88 | -1.02 |
| P-value (c) | / | 0.3817 | 0.3070 |
| Estimate for Contrast and two-sided 95% CI (d) | / | -0.72 (-2.35,0.90) | -0.87 (-2.55,0.80) |

| | | | |
|---|---|---|---|
| CI = Confidence interval, DF = Degrees of freedom, LS = Least Square, NEI-VFQ-25 = National Eye Institute Visual Functioning Questionnaire-25, SAP = statistical analysis plan, SD = Standard deviation, SE = Standard error | | | |

A mixed model for repeated measurements (MMRM) was used with baseline total lesion area as a covariate, treatment group, visit and the stratification variables (geographic region [Japan vs. Rest of World]; baseline BCVA [< 60 vs. ≥ 60]) as fixed factors, and terms for the interaction between baseline NEI-VFQ-25 total score and visit and the interaction between treatment and visit.

A Kenward-Roger approximation was used for the denominator degrees of freedom. In order to model the within-subject error the following covariance structure was used: unstructured.

Intercurrent events (ICE) were handled according to primary estimand strategy for continuous endpoints.
(a) Based on observed assessments.
(b) The contrast also includes the interaction term for treatment x visit.
(c) Nominal p-value for the two-sided test.
(d) Estimate based on the MMRM model, was computed for the differences of HDq12 minus 2q8 and HDq16 minus 2q8, respectively with two-sided 95% CIs.

### CNV Size

The mean CNV size at baseline was similar ranging from 6.0 to 6.5 mm² across the 3 treatment groups. Mean changes from baseline at Week 48 showed mean decreases in the HD groups and the 2q8 group. The estimated contrasts (95% CI) for the 2-sided test, using the MMRM in the FAS, of -1.22 (-1.94, -0.51) mm² for HDq12 vs. 2q8 and of -0.48 (-1.22, 0.27) mm² for HDq16 vs. 2q8 were both numerically in favor of HD treatment (Table 1-12).

The corresponding analysis using an ANCOVA with LOCF in the FAS provided mean changes from baseline to Week 48 and estimated contrasts (95% CIs) for the 2-sided tests between the HD groups and the 2q8 group that were numerically also in favor of HD treatment and thus consistent with the results from the analysis using MMRM.

**Table 1-12. Change from baseline in CNV size (mm²) at Week 48, MMRM (Full Analysis Set)**

| | **2q8** | **HDq12** | **HDq16** |
|---|---|---|---|
| | **N = 336** | **N = 335** | **N = 338** |
| LS mean (SE) change from baseline | -2.43 (0.31) | -3.65 (0.28) | -2.91 (0.29) |
| Arithmetic mean (SD) change from baseline (a) | -2.4 (5.3) | -3.5 (5.0) | -2.9 (5.3) |
| Baseline mean (a) | 6.4 | 6.0 | 6.5 |
| Number of subjects with Week 48 data | 276 | 285 | 274 |
| DF | / | 614.0 | 609.2 |
| Contrast (b) | / | HDq12-2q8 | HDq16-2q8 |
| t-value | / | -3.35 | -1.26 |
| P-value(c) | / | 0.0009 | 0.2076 |
| Estimate for Contrast and two-sided 95% CI (d) | / | -1.22 (-1.94,-0.51) | -0.48 (-1.22,0.27) |

| | | | |
|---|---|---|---|
| BCVA = best corrected visual acuity, CI = Confidence interval, CNV = Choroidal neovascularization, DF = Degrees of freedom, LS = Least Square, SAP = statistical analysis plan, SD = Standard deviation, SE = Standard error | | | |

A mixed model for repeated measurements (MMRM) was used with baseline CNV measurement as a covariate, treatment group, visit and the stratification variables (geographic region [Japan vs. Rest of World]; baseline BCVA [< 60 vs. ≥ 60]) as fixed factors, and terms for the interaction between baseline CNV and visit and the interaction between treatment and visit. A Kenward-Roger approximation was used for the denominator degrees of freedom. In order to model the within-subject error the following covariance structure was used: unstructured.

Intercurrent events (ICE) were handled according to primary estimand strategy for continuous endpoints.
(a) Based on observed assessments.
(b) The contrast also includes the interaction term for treatment x visit.
(c) p-value for the two-sided test.
(d) Estimate based on the MMRM model, was computed for the differences of HDq12 minus 2q8 and HDq16 minus 2q8, respectively with two-sided 95% CIs.

### Total Lesion Area

The mean total lesion area at baseline was similar across the 3 treatment groups, ranging from 6.4 to 6.9 mm². Mean changes from baseline at Week 48 showed mean decreases in the HD groups but a mean increase in the 2q8 group. The estimated contrasts (95% CI) for the 2-sided test, using the MMRM in the FAS, of -0.55 (-1.04, -0.06) mm² for HDq12 vs. 2q8 and and of -0.44 (-0.94,0.06) mm² for HDq16 vs. 2q8 were numerically in favor of HD treatment (Table 1-13).

The corresponding analysis using an ANCOVA with LOCF in the FAS provided mean changes from baseline to Week 48 and estimated contrasts (95% CIs) for the 2-sided tests between the HD groups and the 2q8 group that were numerically also in favor of HD treatment and thus consistent with the results from the analysis using MMRM.

**Table 1-13. Change in total lesion area (mm²) from baseline to Week 48, MMRM (full analysis set)**

| | **2q8** | **HDq12** | **HDq16** |
|---|---|---|---|
| | N = 336 | N = 335 | N = 338 |
| Baseline mean (a) | 6.9 | 6.4 | 6.9 |
| Number of subjects with Week 48 data | 277 | 285 | 273 |
| Arithmetic mean (SD) change from baseline (a) | 0.1 (3.6) | -0.4 (2.9) | -0.2 (3.1) |
| LS mean (SE) change from baseline | 0.09 (0.22) | -0.46 (0.19) | -0.35 (0.20) |
| DF | / | 631.4 | 640.4 |
| Contrast (b) | / | HDq12-2q8 | HDq16-2q8 |
| t-value | / | -2.19 | -1.71 |
| P-value (c) | / | 0.0287 | 0.0870 |
| Estimate for Contrast and two-sided 95% CI (d) | / | -0.55 (-1.04,-0.06) | -0.44 (-0.94,0.06) |

| | | | |
|---|---|---|---|
| BCVA = best corrected visual acuity, CI = Confidence interval, DF = Degrees of freedom, LS = Least Square, SAP = statistical analysis plan, SD = Standard deviation, SE = Standard error A mixed model for repeated measurements (MMRM) was used with baseline total lesion area as a covariate, treatment group, visit and the stratification variables (geographic region [Japan vs. Rest of World]; baseline BCVA [< 60 vs. ≥ 60]) as fixed factors, and terms for the interaction between baseline total lesion area and visit and the interaction between treatment and visit. | | | |

A Kenward-Roger approximation was used for the denominator degrees of freedom. In order to model the within-subject error the following covariance structure was used: unstructured. Intercurrent events (ICE) were handled according to primary estimand strategy for continuous endpoints
(a) Based on observed assessments.
(b) The contrast also includes the interaction term for treatment x visit
(c) Nominal p-value for the two-sided test.
(d) Estimate based on the MMRM model, was computed for the differences of HDq12 minus 2q8 and HDq16 minus 2q8, respectively with two-sided 95% CIs.

### Safety

Ocular and non-ocular safety for patients receiving the 8 mg doses of aflibercept was similar to that of patients receiving aflibercept intravitreally dosed at 2 mg approximately every 4 weeks for the first 5 injections followed by 2 mg approximately once every 8 weeks or once every 2 months.

### Summary

At 48 weeks, PULSAR met the primary endpoints of non-inferiority of aflibercept 8 mg to EYLEA, with BCVA improvements from baseline demonstrated across dosing groups (all p=<0.003). The EYLEA outcomes in wet AMD were consistent with previous clinical trial experience. In the every 16-week dosing regimen groups, 77% of wet AMD patients in PULSAR maintained this dosing interval with an average of 5 injections in the first year. In the every 12-week dosing regimen groups, 79% of wet AMD patients in PULSAR maintained this dosing interval with an average of 6 injections in the first year. In a pooled analysis of aflibercept 8 mg dosing groups, 83% of wet AMD patients in PULSAR maintained 12-week dosing or longer. These data demonstrated that a remarkably high percentage of patients can be maintained on 12- and 16-week dosing intervals.

Key efficacy findings at 48 weeks are set forth in Table 1-14.

**Table 1-14. Key 48 Week Efficacy Findings**

| | **High-dose aflibercept 12-week regimen** | **High-dose aflibercept 16-week regimen** | **EYLEA 8-week regimen** |
|---|---|---|---|
| **PULSAR (wet AMD)** | **n=335** | **n=338** | **n=336** |
| Mean BCVA improvement, primary endpoint | 6.7 letters | 6.2 letters | 7.6 letters |
| Non-inferiority p-value | 0.0009 | 0.0011 | N/A |
| Absolute BCVA | 66.9 letters | 66.3 letters | 66.5 letters |
| Patients maintained on dosing interval | 79% | 77% | N/A |
| Patients with no fluid in the central subfield at 16 weeks, key secondary endpoint | 63% (one-sided superiority p=0.0002) | | 52% |

| | | | |
|---|---|---|---|
| DRSS: diabetic retinopathy severity scale; N/A: not applicable | | | |

Mean changes from BL in BCVA at Week 48 were numerically larger in patients with lower BL BCVA (≤54 letters), and smaller in those with higher BL BCVA (≥74 letters). Within the BL subgroups, mean changes and absolute BCVA letter scores at Week 48 were similar in the HDq12, HDq16 and 2q8 treatment groups. Mean increases from BL in BCVA with HDq12, HDq16 and 2q8 were also similar, with overlapping CIs, in patients with BL central subfield retinal thickness (CRT) <400 µm and ≥400 µm, again resulting in similar absolute BCVA letter scores at Week 48 irrespective of treatment group. The same trends were also observed in the subgroup of patients with minimally classic, occult, and predominantly classic disease. Data will also be presented for additional patient subgroups, including by race. In patients with nAMD, BCVA gains from baseline at Week 48 were seen in all subgroups based on baseline BCVA, CRT, and lesion type, with comparable BCVA letter scores at Week 48 achieved with aflibercept 8 mg and 2 mg. See Table 1-15.

**Table 1-15. Week 48 Mean Change in BCVA Letter Scores According to BL BCVA, CRT or Lesion Type.**

| | | N | Mean±SD absolute BL BCVA | Mean (95% CI) Wk48 change from BL | Mean±SD absolute Wk48 BCVA |
|---|---|---|---|---|---|
| **Baseline BCVA ≤54** | | | | | |
| | 8q12 | 97 | 42.6±9.3 | +10.2 (7.2, 13.2) | 52.8±16.5 |
| | 8q16 | 99 | 44.3±8.1 | +7.1 (3.9, 10.3) | 51.4±16.5 |
| | 2q8 | 106 | 41.4±9.2 | +11.1 (82, 14.0) | 52.4±16.5 |

| **Baseline BCVA 55-73** | | | | | |
|---|---|---|---|---|---|
| | 8q12 | 196 | 65.0±5.7 | +5.1 (3.4, 6.9) | 70.1±13.0 |
| | 8q16 | 191 | 64.3±5.3 | +6.1 (4.7, 7.5) | 70.4±10.4 |
| | 2q8 | 181 | 64.7±5.1 | +6.9 (5.3, 8.4) | 71.6±11.5 |

| **Baseline BCVA ≥74** | | | | | |
|---|---|---|---|---|---|
| | 8q12 | 42 | 75.9±1.5 | +1.5 (-1.8, 4.7) | 77.4±10.4 |
| | 8q16 | 48 | 75.6±1.5 | +2.9 (0.8, 4.9) | 78.5±7.6 |
| | 2q8 | 49 | 75.5±1.5 | +2.3 (0.2, 4.4) | 77.9±7.9 |

| **Baseline CRT <400 µm** | | | | | |
|---|---|---|---|---|---|
| | 8q12 | 228 | 63.4±11.5 | +4.6 (3.1, 6.2) | 68.1±15.5 |
| | 8q16 | 226 | 63.4±10.9 | +6.3 (4.9, 7.6) | 69.7±12.9 |
| | 2q8 | 231 | 62.9±11.6 | +6.2 (4.8, 7.7) | 69.2±15.1 |

| **Baseline CRT ≥400 µm** | | | | | |
|---|---|---|---|---|---|
| | 8q12 | 107 | 52.2±13.7 | +9.4 (6.4, 12.3) | 61.6±17.3 |
| | 8q16 | 110 | 53.0±12.4 | +5.2 (2.5, 8.0) | 58.1±17.9 |
| | 2q8 | 104 | 49.9±14.7 | +10.3 (7.7, 12.91 | 60.2±16.7 |

| **Minimally classic CNV** | | | | | |
|---|---|---|---|---|---|
| | 8q12 | 56 | 57.9±12.9 | +2.6 (-2.2, 7.4) | 60.5±20.3 |
| | 8q16 | 68 | 56.3±11.7 | +5.9 (2.5, 9.4) | 62.2±15.9 |
| | 2q8 | 61 | 54.4±13.7 | +6.4 (2.6, 10.2) | 60.8±17.0 |

| **Occult only CNV** | | | | | |
|---|---|---|---|---|---|
| | 8q12 | 197 | 62.9±12.5 | +5.3 (3.8, 6.9) | 68.3±15.2 |
| | 8q16 | 186 | 63.7±11.3 | +5.1 (3.7, 6.5) | 68.8±14.1 |
| | 2q8 | 192 | 63.6±11.8 | +7.2 (5.7, 8.6) | 70.8±13.7 |

| **Predominantly classic CNV** | | | | | |
|---|---|---|---|---|---|
| | 8q12 | 71 | 53.5±12.8 | +10.4 (7.2, 13.61 | 53.9±15.3 |
| | 8q16 | 67 | 53.8±12.5 | +6.6 (3.1, 10.1) | 60.2±18.4 |
| | 2q8 | 71 | 50.9±15.0 | +9.2 (5.9, 12.5) | 60.1±17.9 |

| | | | | | |
|---|---|---|---|---|---|
| BCVA, best corrected visual acuity; BL, baseline; CNV, choroidal neovascularization; CRT, central subfield retinal thickness; Wk, week. | | | | | |

The safety of high-dose (HD) aflibercept was similar to EYLEA and consistent with the safety profile of EYLEA. There were no new safety signals for high-dose aflibercept and EYLEA, and no cases of retinal vasculitis, occlusive retinitis or endophthalmitis. Comparing pooled data for the 12- and 16-week high-dose aflibercept groups to the EYLEA groups, the following rates were observed:
- Serious ocular adverse events (AE): 1.6% versus 0.6% in PULSAR
- Intraocular inflammation: 0.7% versus 0.6% in PULSAR
- Patients meeting intraocular pressure criteria: 1.3% versus 2.1% in PULSAR
- Serious non-ocular AEs: 9.8% versus 13.7% in PULSAR

### Results at Week 60

There were 1395 enrolled participants at 251 sites in 27 countries countries/regions (Europe, North America, Latin America, Australia, and Asia Pacific), of whom 383 participants did not complete screening; one participant was randomized in error although he/she did not complete screening and had withdrawn consent. Therefore, this participant was not considered as randomized in the Week 48 datasets and thus 1011 participants at 223 sites were randomized.

### Disposition of Subjects

With the exception of 2 participants who did not receive any study treatment, all other randomized participants were included in the FAS and the SAF (N=1009). Of these, 937 participants completed study treatment phase through Week 48 and 925 participants through Week 60. At the time of the last participant last Week 48 and last Week 60 visits, 66 and 80 participants, respectively, did not complete study treatment, with no notable differences between the treatment groups with regard to the reasons for premature discontinuation. For 6 and 4 participants it was unknown as to whether they had completed study treatment through Week 48 and Week 60, respectively. See Table 1-16.

**Table 1-16. Disposition in overall study: Week 48 and Week 60 (all enrolled participants)**

| Number of subjects | | | | **2q8** | **HDq12** | **HDq16** | **All HD** | **Total** |
|---|---|---|---|---|---|---|---|---|
| **Week 48** | | | | | | | | |
| Enrolled, n | | | | | | | | 1395 |
| Randomized, n (%) | | | | 337 (100%) | 336 (100%) | 338 (100%) | 674 (100%) | 1011 (100%) |
| Treated, n (%) | | | | 336 (99.7%) | 335 (99.7%) | 338 (100%) | 673 (99.9%) | 1009 (99.8%) |
| Completed study until Week 48, n (%) | | | | 309 (91.7%) | 316 (94.0%) | 312 (92.3%) | 628 (93.2%) | 937 (92.7%) |
| Unknown if completed study until Week 48^{a}, n (%) | | | | 3 (0.9%) | 2 (0.6%) | 1 (0.3%) | 3 (0.4%) | 6 (0.6%) |
| Did not complete study until Week 48, n (%) | | | | 25 (7.4%) | 18 (5.4%) | 25 (7.4%) | 43 (6.4%) | 68 (6.7%) |
| | Primary reason ^{b} | | | | | | | |
| | | Adverse event | | 5 (1.5%) | 1 (0.3%) | 5 (1.5%) | 6 (0.9%) | 11 (1.1%) |
| | | Physician decision | | 1 (0.3%) | 3 (0.9%) | 2 (0.6%) | 5 (0.7%) | 6 (0.6%) |
| | | Non-compliance with study treatment | | 0 | 0 | 0 | 0 | 0 |
| | | Pregnancy | | 0 | 0 | 0 | 0 | 0 |
| | | Protocol deviation | | 0 | 1 (0.3%) | 1 (0.3%) | 2 (0.3%) | 2 (0.2%) |
| | | Lost to follow-up | | 1 (0.3%) | 1 (0.3%) | 0 | 1 (0.1%) | 2 (0.2%) |
| | | Study terminated by sponsor | | 0 | 0 | 0 | 0 | 0 |
| | | Lack of efficacy | | 2 (0.6%) | 0 | 0 | 0 | 2 (0.2%) |
| | | Technical problems | | 0 | 0 | 0 | 0 | 0 |
| | | Logistical problems | | 0 | 0 | 0 | 0 | 0 |
| | | Withdrawal by subject | | 5 (1.5%) | 5 (1.5%) | 12 (3.6%) | 17 (2.5%) | 22 (2.2%) |
| | | Wish for pregnancy | | 0 | 0 | 0 | 0 | 0 |
| | | Death | | 5 (1.5%) | 3 (0.9%) | 1 (0.3%) | 4 (0.6%) | 9 (0.9%) |
| | | Other | | 4 (1.2%) | 2 (0.6%) | 2 (0.6%) | 4 (0.6%) | 8 (0.8%) |
| | | COVID-19 pandemic | | 2 (0.6%) | 2 (0.6%) | 2 (0.6%) | 4 (0.6%) | 6 (0.6%) |
| | | | Subject decision: COVID-19 pandemic related | 2 (0.6%) | 2 (0.6%) | 2 (0.6%) | 4 (0.6%) | 6 (0.6%) |
| | | | Physician decision: COVID-19 pandemic related | 0 | 0 | 0 | 0 | 0 |
| | | | Logistical reason: COVID-19 pandemic related | 0 | 0 | 0 | 0 | 0 |
| | | | Other: COVID-19 pandemic related | 0 | 0 | 0 | 0 | 0 |

| **Week 60** | | | | | | | | |
|---|---|---|---|---|---|---|---|---|
| Enrolled, n | | | | | | | | 1395 |
| Randomized, n (%) | | | | 337 (100%) | 336 (100%) | 338 (100%) | 674 (100%) | 1011 (100%) |
| Treated, n (%) | | | | 336 (99.7%) | 335 (99.7%) | 338 (100%) | 673 (99.9%) | 1009 (99.8%) |
| Completed study until Week 60, n (%) | | | | 305 (90.5%) | 310 (92.3%) | 308 (91.1%) | 618 (91.7%) | 923 (91.3%) |
| Unknown if completed study until Week 60^{c}, n (%) | | | | 3 (0.9%) | 3 (0.9%) | 1 (0.3%) | 4 (0.6%) | 7 (0.7%) |
| Did not complete study until Week 60, n (%) | | | | 29 (8.6%) | 23 (6.8%) | 29 (8.6%) | 52 (7.7%) | 81 (8.0%) |
| | Primary reason^{d} | | | | | | | |
| | | Adverse event | | 6 (1.8%) | 2 (0.6%) | 5 (1.5%) | 7 (1.0%) | 13 (1.3%) |
| | | Physician decision | | 1 (0.3%) | 4 (1.2%) | 1 (0.3%) | 5 (0.7%) | 6 (0.6%) |
| | | Non-compliance with study treatment | | 0 | 0 | 0 | 0 | 0 |
| | | Pregnancy | | 0 | 0 | 0 | 0 | 0 |
| | | Protocol deviation | | 0 | 1 (0.3%) | 1 (0.3%) | 2 (0.3%) | 2 (0.2%) |
| | | Lost to follow-up | | 1 (0.3%) | 1 (0.3%) | 2 (0.6%) | 3 (0.4%) | 4 (0.4%) |
| | | Study terminated by sponsor | | 0 | 0 | 0 | 0 | 0 |
| | | Lack of efficacy | | 2 (0.6%) | 0 | 0 | 0 | 2 (0.2%) |
| | | Technical problems | | 0 | 0 | 0 | 0 | 0 |
| | | Logistical problems | | 0 | 0 | 0 | 0 | 0 |
| | | Withdrawal by subject | | 6 (1.8%) | 8 (2.4%) | 14 (4.1%) | 22 (3.3%) | 28 (2.8%) |
| | | Wish for pregnancy | | 0 | 0 | 0 | 0 | 0 |
| | | Death | | 5 (1.5%) | 3 (0.9%) | 2 (0.6%) | 5 (0.7%) | 10 (1.0%) |
| | | Other | | 6 (1.8%) | 2 (0.6%) | 2 (0.6%) | 4 (0.6%) | 10 (1.0%) |
| | | COVID-19 pandemic | | 2 (0.6%) | 2 (0.6%) | 2 (0.6%) | 4 (0.6%) | 6 (0.6%) |
| | | | Subject decision: COVID-19 pandemic related | 2 (0.6%) | 2 (0.6%) | 2 (0.6%) | 4 (0.6%) | 6 (0.6%) |
| | | | Physician decision: COVID-19 pandemic related | 0 | 0 | 0 | 0 | 0 |
| Number of subjects | | | | **2q8** | **HDq12** | **HDq16** | **All HD** | **Total** |
| | | | Logistical reason: COVID-19 pandemic related | 0 | 0 | 0 | 0 | 0 |
| | | | Other: COVID-19 pandemic related | 0 | 0 | 0 | 0 | 0 |

| | | | | | | | | |
|---|---|---|---|---|---|---|---|---|
| COVID-19 = Coronavirus Disease 2019. a 6 participants who had missing Week 48 information (i.e. they neither discontinued during Week 48 time frame, nor had Week 48 visit performed or marked as not done) were summarized as Unknown if completed study until Week 48. b For some participants the reason for premature discontinuation of study was inconsistently reported. c 7 participants who had missing Week 60 information (i.e., they neither discontinued during Week 60 time frame, nor had Week 60 visit performed or marked as not done) were summarized as Unknown if completed study until Week 60. d For some participants the reason for premature discontinuation of study was inconsistently reported. | | | | | | | | |

Definition of completed study until Week 60 = did not answer NO to the question "Did the subject complete the study?" on the "End of study" form prior to Week 60 visit.

Number of subjects enrolled is the number of subjects who signed informed consent.

### Protocol Deviations

The frequency of participants with important protocol deviations through Week 60 was similar across the treatment groups (Table 1-17).

Overall, 355 (35.1%) participants reported important protocol deviations. The most frequent (≥ 5%) important protocol deviations were related to the categories "procedure deviations", "treatment deviations", "time schedule deviations" and "informed consent".

Among the total of 59 participants with important protocol deviations related to informed consent, there were 57 screen failure participants who did not provide sufficient authorization for use of their data but were included in the database. These deviations and those regarding the 4 randomized and treated participants with similar deviations who were excluded from the database as well as the preventive and corrective actions taken because of that are described in more detail in a Note to File.

The most frequent important deviation of the category "inclusion/exclusion criteria not met but subject entered treatment" was related to Exclusion criterion 4 (participant had uncontrolled blood pressure [defined as systolic > 160 mmHg or diastolic > 95 mmHg]), which was reported for 20 (2.0%) participants. All other important protocol deviations for this category were reported for 1 or 2 participants.

Of note, 3 additional protocol deviations in 3 participants who met exclusion criteria were reported late and were thus not part of the Week 48 database and not included in the analyses for Week 48. Two of these additional protocol deviations were judged as being important, and one of them should have resulted in exclusion of the participant from the PPS for the Week 48 analysis. This participant met the exclusion criterion "Subject has subretinal hemorrhage that is at least 50% of the total lesion area, or if the blood under the fovea is 1 or more disc areas in size in the study eye" and was excluded from the Week 60 PPS analysis. Therefore, the PPS in the Week 48 database, which was used for supplemental analyses of the primary and key secondary efficacy endpoints (Change from baseline in BCVA at Week 48 and Proportion of subjects with no IRF and no SRF in central subfield at Week 16, respectively) included a total of 970 (95.9%) participants, whereas the PPS in the Week 60 database, which was used for a supplemental analysis of the key secondary endpoint at Week 60 (Change from baseline in BCVA at Week 60) included a total of 969 (95.8%) participants. The other deviation considered to be important but not included in the Week 48 database was deleted as it was entered by mistake. The third protocol deviation, judged not important, was still not included in the Week 60 database and analyses. This third participant was included in the Week 48 and Week 60 PPS; the deviation, judged non- important, would not have affected inclusion in the PPS.

In addition, there were 5 protocol deviations related to "time schedule deviations" for missing Visit 15, which were not included in the Week 48 database. Four of these protocol deviations were resolved or included in the Week 60 database and analyses, whereas the remaining 1 protocol deviation was still queried at the site and thus not included in the Week 60 database.

**Table 1-17. Number of participants with important protocol deviations through Week 60 (all randomized participants)**

| | **Protocol Deviation category** | **2q8** N = 337 (100%) | **HDq12** N = 336 (100%) | **HDq16** N = 338 (100%) | **All HD** N = 674 (100%) | **Total** N = 1011 (100%) |
|---|---|---|---|---|---|---|
| Excluded concomitant medication treatment | | 1 (0.3%) | 1 (0.3%) | 1 (0.3%) | 2 (0.3%) | 3 (0.3%) |
| | Subject received any standard or investigational agents for treatment of their nAMD in the study eye other than IVT aflibercept as specified in this protocol | 1 (0.3%) | 1 (0.3%) | 1 (0.3%) | 2 (0.3%) | 3 (0.3%) |
| Inclusion/exclusion criteria not met but subject entered treatment^{a} | | 8 (2.4%) | 9 (2.7%) | 11 (3.3%) | 20 (3.0%) | 28 (2.8%) |
| | Exclusion Criteria: Subject has subretinal hemorrhage that is at least 50% of the total lesion area. or if the blood under the fovea is 1 or more disc areas in size in the study eye ^{b} | 0 | 1 (0.3%) | 0 | 1 (0.1%) | 1 (0.1%) |
| | Exclusion Criteria: Subject has a history or clinical evidence of diabetic retinopathy. diabetic macular edema. or any retinal vascular disease other than nAMD in either eye. | 1 (0.3%) | 1 (0.3%) | 0 | 1 (0.1%) | 2 (0.2%) |
| | Exclusion Criteria: Subject has known cardiac arrhythmia. based on medical history and/or outcome of ECG at screening. (Dense PK Substudy) | 1 (0.3%) | 1 (0.3%) | 0 | 1 (0.1%) | 2 (0.2%) |
| | Exclusion Criteria: Subject has uncontrolled blood pressure (defined as systolic >160 mmHg or diastolic >95 mmHg). | 5 (1.5%) | 6 (1.8%) | 9 (2.7%) | 15 (2.2%) | 20 (2.0%) |
| | Inclusion Criteria: Subject does not have BCVA ETDRS letter score of 78 to 24 at Baseline (corresponding to a Snellen equivalent of approximately 20/32 to 20/320) in the study eye (Left Eye). | 1 (0.3%) | 0 | 0 | 0 | 1 (0.1%) |
| | Inclusion Criteria: Subject does not have BCVA ETDRS letter score of 78 to 24 at Baseline (corresponding to a Snellen equivalent of approximately 20/32 to 20/320) in the study eye (Right Eye). | 0 | 0 | 1 (0.3%) | 1 (0.1%) | 1 (0.1%) |
| | Inclusion Criteria: The patient does not have evidence of IRF and/or SRF affecting the central subfield of the study eye on OCT | 0 | 0 | 1 (0.3%) | 1 (0.1%) | 1 (0.1%) |
| Informed consent | | 22 (6.5%) | 20 (6.0%) | 17 (5.0%) | 37 (5.5%) | 59 (5.8%) |
| | Informed consent process not followed properly | 0 | 0 | 1 (0.3%) | 1 (0.1%) | 1 (0.1%) |
| | The Informed Consent is incomplete. The subject's signature/sign date are missing or partially signed or incorrect etc. | 21 (6.2%) | 20 (6.0%) | 16 (4.7%) | 36 (5.3%) | 57 (5.6%) |
| | The subject signed the ICF after starting his/her participation on the study (The ICF date is after the assessment date). | 1 (0.3%) | 0 | 0 | 0 | 1 (0.1%) |
| Other protocol deviations ^{c} | | 13 (3.9%) | 14 (4.2%) | 11 (3.3%) | 25 (3.7%) | 38 (3.8%) |
| Procedure deviations ^{c} | | 38 (11.3%) | 55 (16.4%) | 48 (14.2%) | 103 (15.3%) | 141 (13.9%) |
| Time schedule deviations ^{c, d} | | 23 (6.8%) | 20 (6.0%) | 29 (8.6%) | 49 (7.3%) | 72 (7.1%) |
| Treatment deviations | | 44 (13.1%) | 20 (6.0%) | 25 (7.4%) | 45 (6.7%) | 89 (8.8%) |
| | Expired study drug administered to patient | 8 (2.4%) | 0 | 0 | 0 | 8 (0.8%) |
| | Incorrect study drug kit administered to patient | 0 | 1 (0.3%) | 4 (1.2%) | 5 (0.7%) | 5 (0.5%) |
| | Patient was randomized to the wrong stratum | 1 (0.3%) | 1 (0.3%) | 0 | 1 (0.1%) | 2 (0.2%) |

| Protocol deviation category | | **2q8** | **HDq12** | **HDq16** | **All HD** | **Total** |
|---|---|---|---|---|---|---|
| | | N = 337 (100%) | N = 336 (100%) | N = 338 (100%) | N = 674 (100%) | N = 1011 (100%) |
| | Study drug not administrated for reason other than documented medical issue. | 16 (4.7%) | 6 (1.8%) | 3 (0.9%) | 9 (1.3%) | 25 (2.5%) |
| | Received wrong dose treatment (high dose instead of low dose or vice versa; sham injection instead of active injection or vice versa) | 19 (5.6%) | 12 (3.6%) | 13 (3.8%) | 25 (3.7%) | 44 (4.4%) |
| | Regional Crisis Study drug not administered | 2 (0.6%) | 0 | 6 (1.8%) | 6 (0.9%) | 8 (0.8%) |
| | Subject was given incorrect study treatment | 0 | 1 (0.3%) | 2 (0.6%) | 3 (0.4%) | 3 (0.3%) |

| | | | | | | |
|---|---|---|---|---|---|---|
| BCVA = best corrected visual acuity, ECG = electrocardiogram, ETDRS = Early Treatment Diabetic Retinopathy Study, ICF = informed consent form, IRF = intraretinal fluid, nAMD = neovascular (wet) age-related macular degeneration, IVT = intravitreal, OCT = optical coherence tomography, PK = pharmacokinetics, PPS = per protocol set, SRF = subretinal fluid Subjects could have more than one protocol deviation but are only counted once within each deviation category. a A protocol deviation for 1 participant who was randomized and completed Day 1 assessments but did not receive study drug and was later found to meet Exclusion criterion 13 was not included in the Week 60 database. b This protocol deviation, which was not included in the Week 48 analysis but was included in the Week 60 analysis, resulted in exclusion of the participant from the PPS. c Subcategories are provided in source table. d There was 1 protocol deviation related to "time schedule deviations" for missing Visit 15 related to the COVID-19 pandemic, which was not included in the analyses for Week 60 | | | | | | |

Mean treatment compliance through Week 60 was > 97% in each of the 3 treatment groups (Table 1-18).

**Table 1-18. Compliance with study treatment: through Week 60 (safety analysis set)**

| | | **2q8** | **HDq12** | **HDq16** | **All HD** |
|---|---|---|---|---|---|
| | | N = 336 (100%) | N = 335 (100%) | N = 338 (100%) | N = 673 (100%) |
| Number of subjects receiving 100% planned injections within 60-week period | | | | | |
| Treatment compliance (%) | | | | | |
| | n | 336 | 335 | 337 | 672 |
| | Mean (SD) | 97.24 (5.99) | 97.60 (5.80) | 97.93 (4.89) | 97.77 (5.36) |
| | Median | 100.00 | 100.00 | 100.00 | 100.00 |
| | Min, Max | 64.3,100 | 57.1,100 | 66.7,100 | 57.1,100 |

| Compliance categories, n (%) | | | | | |
|---|---|---|---|---|---|
| | > 90 to ≤ 100% | 306 (91.1%) | 308 (91.9%) | 314 (92.9%) | 622 (92.4%) |
| | > 80 to ≤ 90% | 15 (4.5%) | 16 (4.8%) | 15 (4.4%) | 31 (4.6%) |
| | ≤ 80% | 15 (4.5%) | 11 (3.3%) | 8 (2.4%) | 19 (2.8%) |

| | | | | | |
|---|---|---|---|---|---|
| Max = maximum, Min = minimum, SD = standard deviation Compliance = (Number of actual study interventions received during period before Week 60 or up to premature discontinuation)/ (Number of planned study interventions during period before Week 60 or up to premature discontinuation) x 100 | | | | | |

### Visual Outcomes

The primary analysis of the change from baseline in BCVA resulted in LSₘₑₐₙ changes from baseline to Week 48 (*i.e*., estimated, adjusted mean changes) of 7.03, 6.06 and 5.89 letters for the 2q8, HDq12 and HDq16 groups, respectively (Table 1-19).

The estimated difference in LSₘₑₐₙₛ changes from baseline to Week 48 in BCVA (with corresponding 95% CI) of HDq12 vs. 2q8 was -0.97 (-2.87, 0.92) letters and of HDq16 vs. 2q8 was -1.14 (-2.97, 0.69) letters (Table 1-19). The p-values for the non-inferiority test at a margin of 4 letters were 0.0009 for HDq12 vs. 2q8, and 0.0011 for HDq16 vs. 2q8; p-values for a superiority test were 0.8437 for HDq12 vs. 2q8 and of 0.8884 for HDq16 vs. 2q8.

The arithmetic mean (SD) changes from baseline in BCVA to Week 48 (*i.e.,* observed, unadjusted mean changes) were 7.6 (12.2), 6.7 (12.6), and 6.2 (11.7) letters for the 285, 299, and 289 participants with Week 48 data, *i.e.,* excluding data after an ICE as handled by the hypothetical strategy, in the 2q8, HDq12, and HDq16 groups, respectively (Table 1-19).

The analysis of the key secondary efficacy variable (Change from baseline in BCVA measured by the ETDRS letter score at Week 60) resulted in LSₘₑₐₙ changes from baseline to Week 60 (*i.e.,* estimated, adjusted mean changes) of 7.23, 6.37 and 6.31 letters for the 2q8, HDq12 and HDq16 groups, respectively (Table 1-19).

The estimated difference in LSₘₑₐₙₛ changes from baseline to Week 60 in BCVA (with corresponding 95% CI) of HDq12 vs. 2q8 was -0.86 (-2.57, 0.84) letters and of HDq16 vs. 2q8 was -0.92 (-2.51, 0.66) letters (Table 1-19). The p-values for the non-inferiority test at a margin of 4 letters were 0.0002 for HDq12 vs. 2q8, and < 0.0001 for HDq16 vs. 2q8; p-values for a superiority test were 0.8393 for HDq12 vs. 2q8 and of 0.8731 for HDq16 vs. 2q8.

The arithmetic mean (SD) changes from baseline in BCVA to Week 60 (*i.e.,* observed, unadjusted mean changes) were 7.8 (12.6), 6.6 (13.6), and 6.6 (11.7) letters for the 268, 283, and 282 participants with Week 60 data, *i.e.,* excluding data after an ICE as handled by the hypothetical strategy, in the 2q8, HDq12, and HDq16 groups, respectively (Table 1-19).

**Table 1-19. Change from baseline in BCVA measured by the ETDRS letter score at Week 48 and Week 60 in the study eye, MMRM (full analysis set) (see Table 1-5)**

| | **2q8** | **HDq12** | **HDq16** |
|---|---|---|---|
| | N = 336 | N = 335 | N = 338 |
| **Week 48 (primary endpoint)** | | | |
| Baseline mean (a) | 58.9 | 59.9 | 60.0 |
| Number of subjects with Week 48 data | 285 | 299 | 289 |
| Arithmetic mean (SD) change from baseline (a) | 7.6 (12.2) | 6.7 (12.6) | 6.2 (11.7) |
| LS mean (SE) change from baseline | 7.03 (0.74) | 6.06 (0.77) | 5.89 (0.72) |
| DF | / | 622.1 | 647.7 |
| Contrast (b) | / | HDq12 - 2q8 | HDq16 - 2q8 |
| t-value | / | 3.14 | 3.07 |
| p-value of one-sided test for non-inferiority at a margin of 4 letters | / | 0.0009 | 0.0011 |
| Estimate for Contrast and two-sided 95% CI (c) | / | -0.97 (-2.87,0.92) | -1.14 (-2.97,0.69) |

| **Week 60 (key secondary endpoint, according to EP-SAP)** | | | |
|---|---|---|---|
| Baseline mean (a) | 58.9 | 59.9 | 60.0 |
| Number of subjects with Week 60 data | 268 | 283 | 282 |
| Arithmetic mean (SD) change from baseline (a) | 7.8 (12.6) | 6.6 (13.6) | 6.6 (11.7) |
| LS mean (SE) change from baseline | 7.23 (0.68) | 6.37 (0.74) | 6.31 (0.66) |
| DF | / | 896.3 | 928.7 |
| Contrast (b) | / | HDq12 - 2q8 | HDq16 - 2q8 |
| t-value | / | 3.61 | 3.81 |
| p-value of one-sided test for non-inferiority at a margin of 4 letters | / | 0.0002 | <0.0001 |
| Estimate for Contrast and two-sided 95% CI (c) | / | -0.86 (-2.57,0.84) | -0.92 (-2.51,0.66) |

| | | | |
|---|---|---|---|
| BCVA = best corrected visual acuity, CI = confidence interval, DF = degrees of freedom, ETDRS = Early Treatment Diabetic Retinopathy Study, LS = least squares, SAP = statistical analysis plan, SD = standard deviation, SE = standard error | | | |

A mixed model for repeated measurements (MMRM) was used with baseline BCVA measurement as a covariate, treatment group, visit and the stratification variables (geographic region [Japan vs. Rest of World]; baseline BCVA [< 60 vs. ≥ 60]) as fixed factors, and terms for the interaction between baseline BCVA and visit and the interaction between treatment and visit.

A Kenward-Roger approximation was used for the denominator degrees of freedom. In order to model the within-subject error the following covariance structure was used: unstructured (for Week 48) and Toeplitz with heterogeneity (for Week 60).

Intercurrent events (ICE) were handled according to primary estimand strategy for continuous endpoints.
(a) : Based on observed assessments.
(b) : The contrast also includes the interaction term for treatment x visit (at Week 48 or Week 60).
(c) : Estimate based on the MMRM model, was computed for the differences of HDq12 minus 2q8 and HDq16 minus 2q8, respectively, with two-sided 95% CIs.

Mean (SD) values in BCVA were similar among treatment groups in the FAS at baseline across all treatment groups. The observed mean (SD) changes from baseline in BCVA averaged over the period from Week 36 to Week 48 and from Week 48 to Week 60 were similar to those for the primary endpoint (Table 1-20). Similar mean changes from baseline averaged over the period from Week 36 to Week 48 and from Week 48 to Week 60 were also observed using LOCF in the FAS.

**Table 1-20. Summary Statistics for Averaged BCVA in ETDRS Letter Score, OC Prior to ICE (Full Analysis Set)**

| **Averaged value for period** | | | | **Change from baseline** | | | | | |
|---|---|---|---|---|---|---|---|---|---|
| Treatment | Visit / Period | n | Mean (SD) | Median | Min, Max | n | Mean (SD) | Median | Min, Max |
| 2q8 (N = 336) | Baseline | 336 | 58.94 (14.02) | 62.00 | 24.00, 78.00 | / | / | / | / |
| | Average BCVA over the period from Week 36 to Week 48 | 299 | 66.88 (15.59) | 72.25 | 10.50, 92.00 | 299 | 7.78 (11.42) | 8.25 | -44.75, 47.50 |
| | Average BCVA over the period from Week 48 to Week 60 | 300 | 66.93 (15.60) | 72.25 | 10.50, 92.00 | 300 | 7.88 (11.53) | 8.25 | -44.75, 47.50 |
| HDq12 (N = 335) | Baseline | 335 | 59.85 (13.37) | 62.00 | 24.00, 78.00 | / | / | / | / |
| | Average BCVA over the period from Week 36 to Week 48 | 313 | 66.87 (15.02) | 71.50 | 13.25, 91.00 | 313 | 6.85 (11.58) | 6.25 | -58.75, 46.25 |
| | Average BCVA over the period from Week 48 to Week 60 | 313 | 66.87 (15.02) | 71.50 | 13.25, 91.00 | 313 | 6.85 (11.58) | 6.25 | -58.75, 46.25 |
| HDq16 (N = 338) | Baseline | 338 | 60.04 (12.38) | 61.00 | 24.00, 78.00 | / | / | / | / |
| | Average BCVA over the period from Week 36 to Week 48 | 308 | 66.21 (14.85) | 69.75 | 6.75, 94.00 | 308 | 6.21 (11.14) | 6.75 | -43.25, 39.50 |
| | Average BCVA over the period from Week 48 to Week 60 | 308 | 66.20 (14.85) | 69.75 | 6.75, 94.00 | 308 | 6.20 (11.15) | 6.75 | -43.25, 39.50 |

| | | | | | | | | | |
|---|---|---|---|---|---|---|---|---|---|
| BCVA = best corrected visual acuity, ETDRS = Early Treatment Diabetic Retinopathy Study, Max = maximum, Min = minimum, SAP = statistical analysis plan, SD = standard deviation OC (observed cases) prior to ICE: observations after an intercurrent event (ICE) defined for the primary estimand excluded as described in the SAP. Intercurrent events (ICE) were handled according to primary estimand strategy for continuous endpoints. | | | | | | | | | |

Overall, the proportions of participants gaining or losing at least 5 or 10 letters in BCVA from baseline at Week 48 were similar across the treatment groups, with minor numerical differences in favor of the 2q8 group, as can be seen from Table 1-21. This is consistent with the primary endpoint data, which showed that the overall changes in BCVA through Week 48 and Week 60 in the HDq12 and HDq16 groups were non-inferior to that in the 2q8 group.

The proportion of participants gaining at least 10 letters or at least 5 letters in BCVA from baseline at Week 48 were numerically higher in the 2q8 group than in the HDq12 and HDq16 treatment groups, based on LOCF in the FAS. In contrast, the proportion of participants who showed any gain (> 0 letters) in BCVA from baseline was similar in the HDq16 and 2q8 groups and lower in the HDq12 group. Similar results for the proportions of participants gaining at least 10 letters, at least 5 letters, or any gain (> 0 letters) in BCVA from baseline were observed at Week 60.

The numerical differences in the proportion of participants who lost at least 5 or 10 letters across the treatment groups were generally small, with the lowest proportions observed in the 2q8 group at Week 48 as well as at Week 60.

The results of the analysis for the same endpoint using OC prior to ICE at Week 48 and at Week 60 were in line with those based on LOCF in the FAS.

**Table 1-21. Proportion of Participants who Gained or Lost at Least 5, 10 or 15 Letters in BCVA from Baseline at Week 48 and Week 60, LOCF (Full Analysis Set)**

| Response category | Treatment | **Subjects with response** | **category, Num/Den (%)** |
|---|---|---|---|
| | | **Week 48** | **Week 60** |
| Gained ≥ 15 letters | 2q8 (N = 336) | 74/335 (22.1%) | 78/335 (23.3%) |
| | HDq12 (N = 335) | 69/334 (20.7%) | 79/334 (23.7%) |
| | HDq16 (N = 338) | 73/337 (21.7%) | 78/337 (23.1%) |
| Gained ≥ 10 letters | 2q8 (N = 336) | 142/335 (42.4%) | 143/335 (42.7%) |
| | HDq12 (N = 335) | 130/334 (38.9%) | 137/334 (41.0%) |
| | HDq16 (N = 338) | 130/337 (38.6%) | 126/337 (37.4%) |
| Gained ≥ 5 letters | 2q8 (N = 336) | 213/335 (63.6%) | 216/335 (64.5%) |
| | HDq12 (N = 335) | 186/334 (55.7%) | 191/334 (57.2%) |
| | HDq16 (N = 338) | 196/337 (58.2%) | 204/337 (60.5%) |
| Gained > 0 letters (any gain) | 2q8 (N = 336) | 255/335 (76.1%) | 266/335 (79.4%) |
| | HDq12 (N = 335) | 240/334 (71.9%) | 233/334 (69.8%) |
| | HDq16 (N = 338) | 258/337 (76.6%) | 253/337 (75.1%) |
| Lost ≥ 5 letters | 2q8 (N = 336) | 37/335 (11.0%) | 35/335 (10.4%) |
| | HDq12 (N = 335) | 44/334 (13.2%) | 45/334 (13.5%) |
| | HDq16 (N = 338) | 48/337 (14.2%) | 50/337 (14.8%) |
| Lost ≥ 10 letters | 2q8 (N = 336) | 21/335 (6.3%) | 26/335 (7.8%) |
| | HDq12 (N = 335) | 27/334 (8.1%) | 30/334 (9.0%) |
| | HDq16 (N = 338) | 31/337 (9.2%) | 30/337 (8.9%) |
| Lost ≥ 15 letters | 2q8 (N = 336) | 14/335 (4.2%) | 14/335 (4.2%) |
| | HDq12 (N = 335) | 18/334 (5.4%) | 22/334 (6.6%) |
| | HDq16 (N = 338) | 18/337 (5.3%) | 17/337 (5.0%) |

| | | | |
|---|---|---|---|
| BCVA = best corrected visual acuity, Num/Den = numerator/denominator, SAP = statistical analysis plan; LOCF (last observation carried forward): last available observed value prior to ICE was used to impute missing data. Intercurrent events (ICE) were handled according to sensitivity estimand strategy for continuous endpoints. | | | |

The proportion of participants who lost at least 15 letters in BCVA from baseline was < 6.0% at Week 48 and < 7.0% at Week 60 in all 3 treatment groups, based on LOCF in the FAS, with only small numerical differences across the treatment groups.

The analysis of the same endpoint using OC prior to ICE in the FAS provided proportions of participants who lost at least 15 letters in BCVA from baseline at Week 48 of 4.2%, 4.3% and 4.8% in the 2q8, HDq12, and HDq16 group, respectively. Similar proportions of 4.1%, 6.0% and 4.3% in the 2q8, HDq12, and HDq16 group, respectively, were observed at Week 60. This was largely in line with the results based on LOCF in the FAS.

### BCVA≥69 ETDRS Letter Score

The proportions of participants achieving an ETDRS letter score of at least 69 increased from values of 29.5% (2q8), 34.0% (HDq12), and 28.4% (HDq16) at baseline to values > 50% at Week 8 (2q8), Week 12 (HDq12), or Week 16 (HDq16) and remained > 50% with similar values in all 3 treatment groups at Week 48 (54.3% to 57.9%) and at Week 60 (54.6% to 58.2%).

### Retinal Fluid

This key secondary efficacy endpoint, proportion of participants with no IRF and no SRF in central subfield at Week 16, describes the proportion of all participants with no IRF and no SRF in central subfield at Week 16 as assessed by the reading center.

As both HD groups and the 2 mg group were all treated identically with 3 initial monthly doses prior to Week 16, the pooled HDq12 and HDq16 were compared to the 2q8 group for this endpoint. At Week 16, 63.3% of participants in the pooled HD groups had no retinal fluid (no IRF and no SRF) compared to 51.6% in the 2q8 treatment group. The difference (95% CI) between pooled HD groups vs. 2q8 treatment was 11.733% points (5.263%, 18.204%) superiority. The p-value of the 1-sided Cochran-Mantel-Haenszel test for superiority was 0.0002. See Table 1-22A.

Of note, the observation that 3.6% of the participants in the 2q8 and the pooled HD groups, respectively, in the FAS had no IRF and no SRF in central subfield at screening with similar proportions at baseline, although Inclusion criterion 6 required the presence of IRF and/or SRF, can be explained by the fact that the eligibility criteria were assessed by the investigators at screening based on preliminary data, whereas the above observations of no retinal fluid (no IRF and no SRF) in some participants were based on updated reading center data. The reading center provided eligibility assessment for all participants based on imaging exams performed at screening, while the investigator confirmed eligibility based on imaging exams performed at randomization. The imaging exams performed at screening, baseline and every other visit subsequently underwent detailed grading by the reading center, independently from the eligibility check. Based on this detailed grading, a very small number of discrepancies were noted in the assessment of fluid in screening OCTs. These do not represent a protocol deviation since the initial eligibility check was positive in all cases.

This observation did not appear to have a major impact on the results: The analysis of this key secondary endpoint was repeated on the PPS as supplementary analysis, in which participants with no IRF and no SRF in central subfield at baseline were excluded, and the results were consistent with those obtained in the FAS.

At Week 16, 62.5% of participants in the pooled HD groups had no retinal fluid (no IRF and no SRF) compared to 50.3% in the 2q8 treatment group. The difference (95% CI) between the pooled HD groups and the 2q8 group, using Mantel-Haenszel weighting scheme adjusted by geographical region and baseline BCVA (< 60 vs. ≥ 60), was 12.327% points (5.726%, 18.929%).

**Table 1-22A. Proportion of Participants with no IRF and no SRF in Central Subfield at Week 16, LOCF (Full Analysis Set)**

| | **2q8** | **HDq12** | **HDq16** | **All HD** |
|---|---|---|---|---|
| | N = 336 | N = 335 | N = 338 | N = 673 |
| Subjects who had no IRF and no SRF, Num/Den (%) | 173/335 (51.6%) | 205/333 (61.6%) | 217/334 (65.0%) | 422/667 (63.3%) |
| Contrast | / | / | / | All HD - 2q8 |
| Difference (a) % (two-sided 95% CI) | / | / | / | 11.733 (5.263, 18.204) |
| CMH test (b) p-value | / | / | / | 0.0002 |

Summary statistics for the proportion of participants with no IRF and no SRF in central subfield at baseline, Week 16, Week 48, and Week 60, using LOCF for the FAS, are presented in Table 1-22B. As can be seen from this table, the proportions of participants with no retinal fluid were > 50% at both Week 16 and Week 48 and numerically higher at Week 48 than at Week 16 in all 3 treatment groups and the pooled HD groups. At Week 60, the proportions of participants with no retinal fluid were > 70% and similar in all 3 treatment groups and the pooled HD groups.

**Table 1-22B. Summary Statistics for Proportion of Participants with No IRF and No SRF in Central Subfield by Visit through Week 60, LOCF (Full Analysis Set)**

| | | **2q8** | **HDq12** | **HDq16** | **All HD** |
|---|---|---|---|---|---|
| | | N = 336 | N = 335 | N = 338 | N = 673 |
| Visit | Fluid status | Num/Den(%) | Num/Den(%) | Num/Den(%) | Num/Den(%) |
| Baseline | Dry ^{a} | 13/336 (3.9%) | 8/335 (2.4%) | 9/336 (2.7%) | 17/671 (2.5%) |
| | Not dry ^{b} | 323/336 (96.1%) | 327/335 (97.6%) | 327/336 (97.3%) | 654/671 (97.5%) |
| | Missing or undetermined | 0 | 0 | 2 | 2 |
| Week 16 | Dry ^{a} | 173/335 (51.6%) | 205/333 (61.6%) | 217/334 (65.0%) | 422/667 (63.3%) |
| | Not dry ^{b} | 162/335 (48.4%) | 128/333 (38.4%) | 117/334 (35.0%) | 245/667 (36.7%) |
| | Missing or undetermined | 1 | 2 | 4 | 6 |
| Week 48 | Dry ^{a} | 199/335 (59.4%) | 236/332 (71.1%) | 223/334 (66.8%) | 459/666 (68.9%) |
| | Not dry ^{b} | 136/335 (40.6%) | 96/332 (28.9%) | 111/334 (33.2%) | 207/666 (31.1%) |
| | Missing or undetermined | 1 | 3 | 4 | 7 |
| Week 60 | Dry ^{a} | 249/334 (74.6%) | 247/331 (74.6%) | 242/335 (72.2%) | 489/666 (73.4%) |
| | Not dry ^{b} | 85/334 (25.4%) | 84/331 (25.4%) | 93/335 (27.8%) | 177/666 (26.6%) |
| | Missing or undetermined | 2 | 4 | 3 | 7 |

| | | | | | |
|---|---|---|---|---|---|
| ICE = intercurrent events, IRF = intraretinal fluid, LOCF = last observation carried forward, Num/Den = numerator/denominator, SAP = statistical analysis plan, SRF = subretinal fluid LOCF method for the last available observed value prior to ICE was carried forward to impute missing data. Intercurrent events (ICE) were handled according to primary estimand strategy for binary endpoints a Dry = defined as no IRF nor SRF detected b Not dry = defined as IRF and/or SRF detected | | | | | |

There were no clinically meaningful pairwise differences between the HD treatment groups and the 2q8 group in the median time to fluid-free retina (no IRF and no SRF), median time to IRF-free retina, or median time to SRF-free retina over 48 weeks in the FAS.

There were also no clinically meaningful pairwise differences between the HD treatment groups and the 2q8 group in the median time to fluid-free retina (no IRF and no SRF), median time to IRF-free retina, or median time to SRF-free retina over 60 weeks in the FAS.

There were no clinically meaningful pairwise differences between the HD treatment groups and the 2q8 group in the median time to sustained fluid-free retina (no IRF and no SRF), median time to IRF-free retina, or median time to SRF-free retina over 48 weeks in the FAS

There were also no clinically meaningful pairwise differences between the HD treatment groups and the 2q8 group in the median time to sustained fluid-free retina (no IRF and no SRF), median time to IRF-free retina, or median time to SRF-free retina over 60 weeks in the FAS.

The proportion of participants without subRPE fluid in central subfield at Week 48 using LOCF in the FAS increased to values > 90% in both HD treatment groups and 86.2% in the 2q8 group. At Week 60, the proportion of participants without subRPE fluid in central subfield increased to values > 90% in all treatment groups (Table 1-23).

The proportion of participants with both no subRPE fluid and no retinal fluid (no IRF and no SRF) in central subfield increased from approximately 2% in each treatment group at baseline to proportions > 60% in both HD treatment groups and of 54.6% in the 2q8 group at Week 48. At Week 60, the proportion of participants with both no subRPE fluid and no retinal fluid in central subfield increased further to values of approximately 69% to 71% in all treatment groups (Table 1-23).

**Table 1-23. Proportion of Participants without Retinal Fluid and Subretinal Pigment Epithelium Fluid in Central Subfield by Visit, LOCF (Full Analysis Set)**

| | | | **2q8** | **HDq12** | **HDq16** |
|---|---|---|---|---|---|
| | | | N = 336 | N = 335 | N = 338 |
| Visit | Fluid status | | Num/Den(%) | Num/Den(%) | Num/Den(%) |
| Baseline | No SubRPE fluid | | 237/335 (70.7%) | 225/334 (67.4%) | 236/336 (70.2%) |
| | | Dry | 7/335 (2.1%) | 5/334 (1.5%) | 6/336 (1.8%) |
| | | Not dry (IRF and/or SRF) | 230/335 (68.7%) | 220/334 (65.9%) | 230/336 (68.5%) |
| | | Both IRF and SRF missing or undetermined | 0/335 | 0/334 | 0/336 |
| | SubRPE fluid present | | 98/335 (29.3%) | 109/334 (32.6%) | 100/336 (29.8%) |
| | | Dry | 6/335 (1.8%) | 3/334 (0.9%) | 3/336 (0.9%) |
| | | Not dry (IRF and/or SRF) | 92/335 (27.5%) | 106/334 (31.7%) | 97/336 (28.9%) |
| | | Both IRF and SRF missing or undetermined | 0/335 | 0/334 | 0/336 |
| | SubRPE missing or undetermined | | 1 | 1 | 2 |
| Week 48 | No SubRPE fluid | | 281/326 (86.2%) | 298/325 (91.7%) | 308/330 (93.3%) |
| | | Dry | 178/326 (54.6%) | 216/325 (66.5%) | 207/330 (62.7%) |
| | | Not dry (IRF and/or SRF) | 103/326 (31.6%) | 82/325 (25.2%) | 100/330 (30.3%) |
| | | Both IRF and SRF missing or undetermined | 0/326 | 0/325 | 0/330 |
| | SubRPE fluid present | | 45/326 (13.8%) | 27/325 (8.3%) | 22/330 (6.7%) |
| | | Dry | 17/326 (5.2%) | 16/325 (4.9%) | 14/330 (4.2%) |
| | | Not dry (IRF and/or SRF) | 28/326 (8.6%) | 11/325 (3.4%) | 8/330 (2.4%) |
| | | Both IRF and SRF missing or undetermined | 0/326 | 0/325 | 0/330 |
| | SubRPE missing or undetermined | | 10 | 10 | 8 |
| Week 60 | No SubRPE fluid | | 296/326 (90.8%) | 305/328 (93.0%) | 309/331 (93.4%) |
| | | Dry | 226/326 (69.3%) | 232/328 (70.7%) | 228/331 (68.9%) |
| | | Not dry (IRF and/or SRF) | 70/326 (21.5%) | 72/328 (22.0%) | 81/331 (24.5%) |
| | | Both IRF and SRF missing or undetermined | 0/326 | 0/328 | 0/331 |
| | SubRPE fluid present | | 30/326 (9.2%) | 23/328 (7.0%) | 22/331 (6.6%) |
| | | Dry | 18/326 (5.5%) | 12/328 (3.7%) | 13/331 (3.9%) |
| | | Not dry (IRF and/or SRF) | 12/326 (3.7%) | 11/328 (3.4%) | 9/331 (2.7%) |
| | | Both IRF and SRF missing or undetermined | 0/326 | 0/328 | 0/331 |
| | SubRPE missing or undetermined | | 10 | 7 | 7 |

| | | | | | |
|---|---|---|---|---|---|
| IRF = Intraretinal fluid, LOCF = Last observation carried forward, Num/Den = numerator/denominator, SAP = statistical analysis plan, SRF = Subretinal fluid, subRPE = subretinal pigment epithelium fluid; LOCF: last available observed value prior to ICE was used to impute missing data. Intercurrent events (ICE) were handled according to primary estimand strategy for binary endpoints. Dry = defined as no IRF nor SRF in central subfield detected; Not dry = defined as IRF and/or SRF in central subfield detected. | | | | | |

### Fluid Leakage

The proportion of participants without leakage on FA increased in all groups over time reaching values of > 40% in the HDq16 and the 2q8 groups and > 60% in the HDq12 group at Week 48. At Week 60, the proportion of participants without leakage on FA increased further, reaching values of > 50% in the HDq16 and the 2q8 groups and > 60% in the HDq12 group. The number of participants with an undetermined leakage status was generally small and similar across the treatment groups over time (Table 1-24).

The analysis for the same endpoint based on OC in the FAS provided results that were consistent with the results using LOCF.

**Table 1-24. Proportion of Participants without Leakage on FA by Visit, LOCF (Full Analysis Set)**

| Visit | Leakage status | **2q8** | **HDq12** | **HDq16** |
|---|---|---|---|---|
| | | N = 336 | N = 335 | N = 338 |
| | | Num/Den(%) | Num/Den(%) | Num/Den(%) |
| Baseline | No leakage | 0/336 | 0/335 | 1/337 (0.3%) |
| | Any leakage | 336/336 (100%) | 335/335 (100%) | 336/337 (99.7%) |
| | Undetermined | 0 | 0 | 1 |
| Week 12 | No leakage | 61/308 (19.8%) | 70/305 (23.0%) | 67/307 (21.8%) |
| | Any leakage | 247/308 (80.2%) | 235/305 (77.0%) | 240/307 (78.2%) |
| | Undetermined | 9 | 8 | 7 |
| Week 48 | No leakage | 136/322 (42.2%) | 193/319 (60.5%) | 140/319 (43.9%) |
| | Any leakage | 186/322 (57.8%) | 126/319 (39.5%) | 179/319 (56.1%) |
| | Undetermined | 8 | 9 | 8 |
| Week 60 | No leakage | 178/320 (55.6%) | 195/318 (61.3%) | 169/316 (53.5%) |
| | Any leakage | 142/320 (44.4%) | 123/318 (38.7%) | 147/316 (46.5%) |
| | Undetermined | 10 | 10 | 10 |

| | | | | |
|---|---|---|---|---|
| ICE = Intercurrent events, FA = fluorescein angiography, LOCF = Last observation carried forward, Num/Den = numerator/denominator, SAP = statistical analysis plan LOCF: last available observed value prior to ICE was used to impute missing data. ICE were handled according to primary estimand strategy for binary endpoints | | | | |

### Choroidal Neovascularization

Summary statistics for the CNV size at baseline, Week 12, Week 48, and Week 60 based on OC prior to ICE in the FAS, are presented in Table 1-25.

The mean (SD) CNV size at baseline ranged from 5.9768 (4.8306) mm² to 6.5459 (5.5315) mm² across the 3 treatment groups. Numerical mean and median decreases from baseline were observed in all 3 treatment groups at Week 12, Week 48, and Week 60. At Week 60, the mean (SD) decreases in CNV size from baseline were of similar extent in all 3 treatment groups ranging from -3.6610 (5.6624) mm² to -3.8795 (5.4295) mm².

**Table 1-25. Summary Statistics for Choroidal Neovascularization Size (mm²) by Visit, OC prior to ICE (Full Analysis Set)**

| **Value at Visit** | | | | | **Change from Baseline** | | | | |
|---|---|---|---|---|---|---|---|---|---|
| **Treatment** | **Visit** | **n** | **Mean (SD)** | **Median** | **Min, Max** | **n** | **Mean (SD)** | **Median** | **Min, Max** |
| 2q8 (N = 336) | Baseline | 336 | 6.3593 (5.0394) | 4.9970 | 0.148, 24.129 | / | / | / | / |
| | Week 12 | 314 | 5.2107 (5.4069) | 3.7455 | 0.000, 29.362 | 314 | -1.1702 (3.4604) | -0.4930 | -22.149, 11.671 |
| | Week 48 | 280 | 4.1366 (5.5680) | 1.6195 | 0.000, 27.675 | 280 | -2.3934 (5.2421) | -1.3125 | -24.129, 16.636 |
| | Week 60 | 250 | 2.7613 (4.5855) | 0.0000 | 0.000, 24.233 | 250 | -3.8795 (5.4295) | -2.5440 | -24.129, 12.664 |
| HDq12 (N = 335) | Baseline | 335 | 5.9768 (4.8306) | 4.8990 | 0.115, 30.023 | / | / | / | / |
| | Week 12 | 312 | 4.3936 (4.6561) | 3.0690 | 0.000, 30.212 | 312 | -1.4886 (3.6500) | -0.5530 | -21.998, 11.890 |
| | Week 48 | 287 | 2.4733 (4.6964) | 0.0000 | 0.000, 27.034 | 287 | -3.5530 (5.0074) | -2.5760 | -21.998, 15.501 |
| | Week 60 | 249 | 2.1483 (4.2820) | 0.0000 | 0.000, 26.051 | 249 | -3.8080 (4.9944) | -2.7740 | -21.998, 12.783 |
| HDq16 (N = 338) | Baseline | 337 | 6.5459 (5.5315) | 4.6980 | 0.000, 28.650 | / | / | / | / |
| | Week 12 | 313 | 4.8923 (5.1756) | 3.3660 | 0.000, 27.081 | 313 | -1.5729 (4.2200) | -0.4950 | -25.133, 16.628 |
| | Week 48 | 279 | 3.5367 (5.1716) | 0.7110 | 0.000, 28.936 | 279 | -2.9790 (5.3189) | -1.4060 | -25.354, 15.869 |
| | Week 60 | 258 | 2.6576 (4.7255) | 0.0000 | 0.000, 30.991 | 258 | -3.6610 (5.6624) | -2.1700 | -26.231, 16.769 |

| | | | | | | | | | |
|---|---|---|---|---|---|---|---|---|---|
| Max = maximum, Min = minimum, SAP = statistical analysis plan, SD = standard deviation OC (observed cases) prior to ICE: observations after an intercurrent event (ICE) defined for the primary estimand excluded as described in the SAP. Intercurrent events (ICE) were handled according to primary estimand strategy for continuous endpoints. | | | | | | | | | |

### Total Lesion Area

Summary statistics for the total lesion area at baseline, Week 12, Week 48, and Week 60 based on OC prior to ICE in the FAS, are presented in Table 1-26. The mean (SD) total lesion area at baseline ranged from 6.3820 (5.0664) mm² to 6.8814 (5.6514) mm² across the 3 treatment groups.

Numerical mean and median decreases in total lesion area from baseline were observed in all 3 treatment groups from Week 12 to Week 60, except for a numerical mean increase in the 2q8 group at Week 48. At Week 60, the mean (SD) decreases in total lesion area from baseline were of similar extent in all 3 treatment groups ranging from -0.3095 (3.1708) mm² to -0.5199 (2.8399) mm².

**Table 1-26. Summary Statistics for Total Lesion Area (mm²) by Visit, OC prior to ICE (Full Analysis Set)**

| | **Value at Visit** | | | | | **Change from Baseline** | | | |
|---|---|---|---|---|---|---|---|---|---|
| Treatment 2q8 (N = 336) | Visit | n | Mean (SD) | Median | Min, Max | n | Mean (SD) | Median | Min, Max |
| | Baseline | 336 | 6.8647 (5.4145) | 5.4120 | 0.148, 27.409 | / | / | / | / |
| | Week 12 | 314 | 6.6722 (5.4651) | 4.8480 | 0.271, 29.362 | 314 | -0.2130 (2.4653) | -0.1510 | -11.233, 13.520 |
| | Week 48 | 281 | 7.2282 (6.1106) | 5.5800 | 0.271, 35.332 | 281 | 0.1110 (3.5498) | -0.2690 | -11.242, 24.641 |
| | Week 60 | 250 | 6.8963 (5.7963) | 5.1360 | 0.379, 30.953 | 250 | -0.3095 (3.1708) | -0.3715 | -11.494, 15.885 |
| HDq12 (N = 335) | Baseline | 335 | 6.3820 (5.0664) | 5.0260 | 0.185, 30.023 | / | / | / | / |
| | Week 12 | 312 | 5.8133 (4.7055) | 4.9645 | 0.154, 30.212 | 312 | -0.4475 (2.2635) | -0.2345 | -8.654, 10.872 |
| | Week 48 | 287 | 6.0700 (5.2298) | 4.9800 | 0.110, 30.259 | 287 | -0.3628 (2.8917) | -0.2550 | -8.719, 13.190 |
| | Week 60 | 249 | 5.8150 (5.2904) | 4.4550 | 0.138, 31.583 | 249 | -0.5199 (2.8399) | -0.3180 | -11.011, 14.006 |
| HDq16 (N = 338) | Baseline | 336 | 6.8814 (5.6514) | 5.0685 | 0.180, 28.650 | / | / | / | / |
| | Week 12 | 312 | 6.3994 (5.2627) | 5.0060 | 0.180, 27.081 | 312 | -0.3923 (2.6320) | -0.1355 | -11.856, 16.628 |
| | Week 48 | 278 | 6.5391 (5.5705) | 4.9190 | 0.137, 28.936 | 278 | -0.2856 (3.1628) | -0.0460 | -13.105, 15.869 |
| | Week 60 | 257 | 6.2872 (5.6288) | 4.5190 | 0.134, 34.294 | 257 | -0.3530 (3.2158) | -0.1350 | -12.755, 16.769 |

| | | | | | | | | | |
|---|---|---|---|---|---|---|---|---|---|
| Max = maximum, Min = minimum, SAP = statistical analysis plan, SD = standard deviation OC (observed cases) prior to ICE: observations after an intercurrent event (ICE) defined for the primary estimand excluded as described in the SAP. Intercurrent events (ICE) were handled according to primary estimand strategy for continuous endpoints | | | | | | | | | |

### Central Retinal Thickness

The mean and LSₘₑₐₙ decreases from baseline in CST over time, based on OC prior to ICE, were similar across all groups through Week 48 with generally minor numerical differences between the treatment groups that were not considered clinically meaningful. The mean (SD) decreases from baseline were maintained through Week 60 where they reached values between -143.0 (120.9) µm in the 2q8 group and -153.4 (134.1) µm in the HDq16 group.

Mean changes from baseline in CST (µm) by visit through Week 60, based on OC prior to ICE in the FAS, are graphically displayed in post-hoc Figure 29 (B); the LSₘₑₐₙ (95% CIs) changes from baseline in CST (µm) by visit through Week 48, based on MMRM in the FAS, are graphically displayed in post-hoc Figure 29 (C).

### Patient-Reported Outcomes

The mean NEI-VFQ-25 total score at baseline was similar across the 3 treatment groups, ranging from 76.36 to 77.81. The mean changes from baseline in NEI-VFQ-25 total score over time, based on OC prior to ICE, were all mean increases, which were numerically lower in the HD groups than in the 2q8 group at Week 24, Week 48, and Week 60. The mean (SD) increases from baseline at Week 60, which ranged from 3.65 (12.08) in the HDq12 group to 5.10 (11.38) in the 2q8 group, were similar to those at Week 48 and the minor differences across the treatment groups were not clinically meaningful.

### Pharmacokinetic evaluation

The PKS was used for the descriptive statistics of the general (sparse) PK assessment and included 934 (92.4%) participants in total and 641 (63.4%) participants with unilateral treatment. A subset of the PKS was used for the analysis of the PK sub-study (DPKS) with dense sampling and included 19 (1.9%) participants with unilateral treatment assessed after the first administration of aflibercept up to Week 48. Data for the PK sub-study were analyzed using non-compartmental analysis (NCA).

Summary of free aflibercept concentrations for participants in the DPKS are presented by treatment in Table 1-27. After initial IVT administration of 2 mg or 8 mg (HDq12 pooled with HDq16) aflibercept, the concentration-time profiles of free aflibercept were characterized by an initial phase of increasing concentrations reflecting initial absorption from the ocular space and initial distribution into the systemic circulation from the ocular space into systemic circulation followed by a mono-exponential elimination phase.

For participants with unilateral treatment up to Week 48 enrolled in the dense PK substudy and receiving aflibercept 2 mg (N = 6), plasma concentrations of free aflibercept were detectable in 4 participants on Day 8 but in only 1 single participant on Day 15 with values only twice the LLOQ. For the aflibercept 8 mg treatment (N = 13), free aflibercept concentrations were detectable in 38% the participants (N = 5) at the end of dense PK sampling on Day 29 (Table 1-27). Most of the participants in the 2q8 DPKS had concentrations of free aflibercept < 0.04 mg/L on Day 2 which corresponds to the expected maximum concentration. However, there was 1 participant in the DPKS (2q8) with implausibly high concentrations (up to 15 times higher than the rest of the participants in this group). These high values in a single participant influence the arithmetic mean considerably. These values appeared pharmacokinetically implausible but were left in this data presentation, since an analytical artifact was not proven. Therefore, the median was more meaningful and was used for comparison, although arithmetic means remained the general base for data presentation.

**Table 1-27. Summary of Concentration of Free Aflibercept (mg/L) in Plasma by Time and Treatment in Participants with nAMD With Unilateral Treatment Up To Day 29 - Dense PK Substudy (DPKS)**

| | | | | **Free aflibercept** | | |
|---|---|---|---|---|---|---|
| **Treatment** | **Time** | **n total** | **n ≥ LLOQ** | **Geom. Mean ± SD** | **Arithm. Mean ± SD** | **Median** |
| 2q8 (N = 6) | Day 1, Pre-dose | 4 | 0 | N.C | 0.00 (0.00) | 0.00 |
| 2q8 (N = 6) | Day 1, 4 Hours Post-dose | 6 | 3 | N.C | 0.0182 (0.0210) | 0.0127 |
| 2q8 (N = 6) | Day 1, 8 Hours Post-dose | 5 | 4 | 0.03 (3.31) | 0.0549 (0.0742) | 0.0282 |
| 2q8 (N = 6) | Day 2 | 6 | 6 | 0.04 | 0.0734 (0.105) | 0.0357 |
| 2q8 (N = 6) | Day 3 | 6 | 6 | 0.04 (2.49) | 0.0622 (0.0835) | 0.0320 |
| 2q8 (N = 6) | Day 5 | 6 | 5 | (2.68) 0.03 (2.67) | 0.0451 (0.0580) | 0.0283 |
| 2q8 (N = 6) | Day 8 | 5 | 4 | 0.02 (1.66) | 0.0187 (0.0110) | 0.0212 |
| 2q8 (N = 6) | Day 15 | 5 | 1 | N.C | 0.00624 (0.0140) | 0.00 |
| 2q8 (N = 6) | Day 22 | 5 | 0 | N.C | 0.00 (0.00) | 0.00 |
| 2q8 (N = 6) | Day 29 | 5 | 1 | N.C | 0.00330 (0.00738) | 0.00 |
| HDq12 +HDq16 (N = 13) | Day 1, Pre-dose | 12 | 0 | N.C | 0.00 (0.00) | 0.00 |
| HDq12 +HDq16 (N = 13) | Day 1, 4 Hours Post-dose | 13 | 6 | N.C | 0.0409 (0.0605) | 0.00 |
| HDq12 +HDq16 (N = 13) | Day 1, 8 Hours Post-dose | 12 | 9 | 0.05 (3.78) | 0.0973 (0.102) | 0.0672 |
| HDq12 +HDq16 (N = 13) | Day 2 | 12 | 12 | 0.11 (2.21) | 0.146 (0.110) | 0.0903 |
| HDq12 +HDq16 (N = 13) | Day 3 | 12 | 12 | 0.11 (2.06) | 0.137 (0.0947) | 0.112 |
| HDq12 +HDq16 (N = 13) | Day 5 | 11 | 11 | 0.08 (1.86) | 0.0933 (0.0481) | 0.0854 |
| HDq12 +HDq16 (N = 13) | Day 8 | 13 | 13 | 0.07 (1.75) | 0.0794 (0.0413) | 0.0682 |
| HDq12 +HDq16 (N = 13) | Day 15 | 13 | 12 | 0.04 (1.76) | 0.0435 (0.0199) | 0.0385 |
| HDq12 +HDq16 (N = 13) | Day 22 | 11 | 9 | 0.02 (1.76) | 0.0213 (0.0148) | 0.0232 |
| HDq12 +HDq16 (N = 13) | Day 29 | 12 | 5 | N.C | 0.00766 (0.00958) | 0.00 |

| | | | | | | |
|---|---|---|---|---|---|---|
| Arithm. = arithmetic, DPKS = dense pharmacokinetic analysis set, LLOQ = lower level of quantification, geom. = geometric, N = Number of participants with unilateral treatment up to Week 48, n = Number of observations, nAMD = neovascular (wet) age-related macular degeneration, PK = pharmacokinetic, SD = standard deviation; N.C.: not calculated (less than 2/3 of values per time point are ≥ LLOQ for geometric mean calculation). Values below LLOQ (0.0156 mg/L) were substituted by 1/2 LLOQ for the calculation of geometric statistics and 0 for arithmetic statistics and median. Minimum and Maximum have been rounded to 4 decimal places. | | | | | | |

Summaries of PK parameters for free aflibercept for participants in the DPKS are presented by treatment in Table 1-28 for non-Japanese (rest of world) participants and in Table 1-29 for Japanese participants.

After the initial monthly aflibercept dose of 2 mg (2q8) or 8 mg (HDq12 pooled with HDq16) in non-Japanese participants, free aflibercept median time to peak concentration (tₘₐₓ) was 1.05 and 1.93 days for the aflibercept 2 mg and 8 mg treatments, respectively. As the IVT dose of aflibercept increased from 2 mg to 8 mg (4-fold ratio), the median peak concentration (Cₘₐₓ) for free aflibercept increased in a slightly less than dose-proportional manner (about 3-fold) and in a greater than dose-proportional manner (about 7-fold) for median area under the plasma concentration-time curve from time zero to the time of the last measurable concentration (AUCₗₐₛₜ).

Following the third initial monthly IVT dose of aflibercept, based on the ratio of aflibercept concentration at Week 12 to Week 4 (C_{Week12}/C_{Week4}), the accumulation ratio of free aflibercept was 1.17 for HDq12 + HDq16 (Table 1-28). The accumulation ratio of free aflibercept could not be determined for 2q8 since all aflibercept concentration values at Week 12 were below LLOQ.

In general, concentrations of free aflibercept as well as PK parameters (Cₘₐₓ, AUCₗₐₛₜ) in a single Japanese participant (in the HDq12 + HDq16 group) were in the same range of values seen in non-Japanese participants after administration of aflibercept 8 mg.

**Table 1-28. Summary Statistics of Free Aflibercept Pharmacokinetic Parameters in Plasma in Participant with Unilateral Treatment from Pooled Region: Rest Of The World - Dense PK Substudy (DPKS)**

| **Pooled region** | **PK parameter (unit)** | **Treatment** | **n** | **Geom. mean (SD)** | **Geom. Mean 95% CI** | **Geom. CV (%)** | **Arithm. mean (SD)** | **Arithm. CV (%)** | **Median** | **Min, Max** |
|---|---|---|---|---|---|---|---|---|---|---|
| Rest of the World | Cmax (mg/L) | 2q8 (N = 6) | 6 | 0.0469 (2.51) | 0.02, 0.12 | 116 | 0.0758 (0.103) | 136 | 0.0357 | 0.0223, 0.286 |
| | | HDq12+HDq16 (N = 11) | 11 | 0.115 (1.95) | 0.06, 0.22 | 74.8 | 0.137 (0.0755) | 55.0 | 0.111 | 0.0288, 0.231 |
| | Cmax/Dose (mg/L/mg) | 2q8 (N = 6) | 6 | 0.0235 (2.51) | 0.01, 0.06 | 116 | 0.0379 (0.0517) | 136 | 0.0179 | 0.0112, 0.143 |
| | | HDq12+HDq16 (N = 11) | 11 | 0.0144 (1.95) | 0.01, 0.03 | 74.8 | 0.0171 (0.00943) | 55.0 | 0.0139 | 0.0036, 0.0289 |
| | Clast (mg/L) | 2q8 (N = 6) | 6 | 0.0202 (1.12) | 0.02, 0.02 | 11.5 | 0.0203 (0.00226) | 11.1 | 0.0202 | 0.0165, 0.0235 |
| | | HDq12+HDq16 (N = 11) | 11 | 0.0217 (1.44) | 0.02, 0.03 | 37.6 | 0.0233 (0.0113) | 48.7 | 0.0183 | 0.0164, 0.0549 |
| | Ctrough (mg/L) | 2q8 | 5 | 0.0165 | NE | NE | 0.00330 | 224 | 0.00 | 0.00, |
| | Day 29 | (N = 6) | | (NE) | | | (0.00738) | | | 0.0165 |
| | | HDq12+HDq16 (N = 11) | 10 | 0.0186 (1.14) | 0.02, 0.02 | 13.3 | 0.00750 (0.00980) | 131 | 0.00 | 0.00, 0.0226 |
| | AUClast (day*mg/L) | 2q8 (N = 6) | 6 | 0.188 (3.17) | 0.06, 0.60 | 167 | 0.362 (0.538) | 149 | 0.18 | 0.0532, 1.45 |
| | | HDq12+HDq16 (N = 11) | 11 | 1.12 (1.99) | 0.56, 2.23 | 78.0 | 1.28 (0.476) | 37.1 | 1.31 | 0.156, 1.85 |
| | AUCinf (day*mg/L) | 2q8 (N = 6) | 0 | / | / | / | / | / | / | / |
| | | HDq12+HDq16 (N = 11) | 7 | 1.73 (1.19) | 1.46, 2.05 | 17.2 | 1.749 (0.26879) | 15.4 | 1.83 | 1.22, 1.98 |
| | AUCinf/Dose (day*mg/L/mg) | 2q8 (N = 6) | 0 | / | / | / | / | / | / | / |
| | | HDq12+HDq16 (N = 11) | 7 | 0.216 (1.19) | 0.18, 0.26 | 17.2 | 0.219 (0.0336) | 15.4 | 0.229 | 0.153, 1.98 |
| | AUC(0-28d) Norm by *WT* | 2q8 (N = 6) | 3 | 0.0059 (2.26) | 0.00, 0.01 | 96.9 | 0.00753 (0.00656) | 87.1 | 0.00413 | 0.0034, 0.0151 |
| | (day*mg/L/kg) | HDq12+HDq16 (N = 11) | 9 | 0.0172 (1.32) | 0.01, 0.02 | 27.9 | 0.0177 (0.00441) | 24.9 | 0.0161 | 0.0098, 0.0232 |
| | Accumulation Ratio (C_{Week12/}C_{Week4}) | 2q8 (N = 6) | 1 | NE (NE) | NE | NE | 0.00 (NE) | NE | 0.00 | 0.00, 0.00 |
| | | HDq12+HDq16 (N = 11) | 4 | 1.16 (1.11) | 1.05, 1.29 | 10.2 | 1.165 (0.118) | 10.1 | 1.17 | 1.04, 1.28 |
| | t1/2 (day) | 2q8 (N = 6) | 2 | 5.93 (1.04) | 5.72, 6.16 | 3.71 | 5.94 (0.220) | 3.71 | 5.94 | 5.78, 6.09 |
| | | HDq12+HDq16 (N = 11) | 9 | 10.3 (1.95) | 5.30, 20.1 | 74.7 | 13.3 (13.1) | 98.6 | 10.6 | 5.21, 47.3 |
| | tmax (day) | 2q8 (N = 6) | 6 | / | / | / | / | / | 1.05 | 0.333, 1.95 |
| | | HDq12+HDq16 (N = 11) | 11 | / | / | / | / | / | 1.93 | 0.333, 4.03 |
| | tlast (day) | 2q8 (N = 6) | 6 | / | / | / | / | / | 6.98 | 2.00, 26.9 |
| | | HDq12+HDq16 (N = 11) | 11 | / | / | / | / | / | 21.1 | 7.05, 28.1 |

| | | | | | | | | | | |
|---|---|---|---|---|---|---|---|---|---|---|
| Arithm. = arithmetic, AUC_{inf} = Area under the curve from time zero to infinity, AUCₗₐₛₜ = Area under the curve to the last quantifiable concentration, CI = confidence interval, Cₗₐₛₜ = Last concentration, Cₘₐₓ = Maximum concentration, C_{trough} = trough concentration, CV = coefficient of variation, geom. = geometric, DPKS = dense pharmacokinetic analysis set, LLOQ = Lower level of quantification, Max = Maximum, Min = Minimum, N = Number of participants with unilateral treatment up to Week 48, n = Number of observations, NE = Not Evaluable, PK = pharmacokinetic, SD = Standard deviation, t_{1/2} = half-life, tₗₐₛₜ = time to last concentration, tₘₐₓ = time to maximum concentration, WT = weight. / indicates categories that do not apply. | | | | | | | | | | |

For Cₗₐₛₜ, values below LLOQ were substituted by 1/2 LLOQ for the calculation of geometric statistics and 0 for arithmetic statistics.

**Table 1-29. Summary Statistics of Free Aflibercept Pharmacokinetic Parameters in Plasma in Participants with Unilateral Treatment from Pooled Region: Japan - Dense PK Substudy (DPKS)**

| **Pooled region** | **PK parameter (unit)** | **Treatment** | **n** | **Geom. mean (SD)** | **Geom. Mean 95% CI** | **Geom. CV (%)** | **Arithm. mean (SD)** | **Arthm. CV (%)** | **Median** | **Min, Max** |
|---|---|---|---|---|---|---|---|---|---|---|
| Japan | Cmax (mg/L) | 2q8 | 0 | / | / | / | / | / | / | / |
| | | (N = 0) | | | | | | | | |
| | | HDq12+HDq16 (N = 2) | 1 | 0.393 (NE) | NE | NE | 0.393 (NE) | NE | 0.39300 | 0.393, 0.393 |
| | Cmax/Dose (mg/L/mg) | 2q8 (N = 0) | 0 | / | / | / | / | / | / | / |
| | | HDq12+HDq16 (N = 2) | 1 | 0.0491 (NE) | NE | NE | 0.0491 (NE) | NE | 0.0491 | 0.0491, 0.0491 |
| | Clast (mg/L) | 2q8 (N = 0) | 0 | / | / | / | / | / | / | / |
| | | HDq12+HDq16 (N = 2) | 1 | 0.0169 (NE) | NE | NE | 0.0169 (NE) | NE | 0.01690 | 0.0169, 0.0169 |
| | Ctrough (mg/L) | 2q8 (N = 0) | 0 | / | / | / | / | / | / | / |
| | | HDq12+HDq16 (N = 2) | 1 | 0.0169 (NE) | NE | NE | 0.0169 (NE) | NE | 0.01690 | 0.0169, 0.0169 |
| | AUClast (day*mg/L) | 2q8 (N = 0) | 0 | / | / | / | / | / | / | / |
| | | HDq12+HDq16 (N = 2) | 1 | 3.51 (NE) | NE | NE | 3.51 (NE) | NE | 3.51 | 3.51, 3.51 |
| | AUCinf (day*mg/L) | 2q8 (N = 0) | 0 | / | / | / | / | / | / | / |
| | | HDq12+HDq16 (N = 2) | 1 | 3.66 (NE) | NE | NE | 3.66 (NE) | NE | 3.66 | 3.66, 3.66 |
| | AUCinf/Dose (day*mg/L/mg) | 2q8 (N = 0) | 0 | / | / | / | / | / | / | / |
| | | HDq12+HDq16 (N = 2) | 1 | 0.457 (NE) | NE | NE | 0.457 (NE) | NE | 0.457 | 0.457, 0.457 |
| | AUC(0-28d) Norm by WT | 2q8 (N = 0) | 0 | / | / | / | / | / | / | / |
| | (day*mg/L/kg) | HDq12+HDq16 (N = 2) | 1 | 0.0807 (NE) | NE | NE | 0.0807 (NE) | NE | 0.0807 | 0.0807, 0.0807 |
| | Accumulation Ratio (C_{Week12}/C_{Week4}) | 2q8 (N = 0) | 0 | / | / | / | / | / | / | / |
| | | HDq12+HDq16 (N = 2) | 1 | 1.37 (NE) | NE | NE | 1.37 (NE) | NE | 1.37 | 1.37, 1.37 |
| | t1/2 (day) | 2q8 (N = 0) | 0 | / | / | / | / | / | / | / |
| | | HDq12+HDq16 (N = 2) | 1 | 6.03 (NE) | NE | NE | 6.03 (NE) | NE | 6.03 | 6.03, 6.03 |
| | tmax (day) | 2q8 (N = 0) | 0 | / | / | / | / | / | / | / |
| | | HDq12+HDq16 (N = 1) | 1 | / | / | / | / | / | 1.02 | 1.02, 1.02 |
| | tlast (day) | 2q8 (N = 0) | 0 | / | / | / | / | / | / | / |
| | | HDq12+HDq16 (N = 1) | 1 | / | / | / | / | / | 27.9 | 27.9, 27.9 |

| | | | | | | | | | | |
|---|---|---|---|---|---|---|---|---|---|---|
| Arithm. = arithmetic, AUC_{inf} = Area under the curve from time zero to infinity, AUCₗₐₛₜ = Area under the curve to the last quantifiable concentration, CI = confidence interval, Cₗₐₛₜ = Last concentration, Cₘₐₓ = Maximum concentration, C_{trough} = trough concentration, CV = coefficient of variation, geom. = geometric, DPKS = dense pharmacokinetic analysis set, geom. = geometric, LLOQ = lower level of quantification, Max = maximum, Min = minimum, N = Number of participants with unilateral treatment up to Week 48, n = Number of observations, NE = Not Evaluable, PK = pharmacokinetic, SD = standard deviation, t_{1/2} = half-life, tₗₐₛₜ = time to last concentration, tₘₐₓ = time to maximum concentration, WT = weight. | | | | | | | | | | |

/ indicates categories that do not apply. For Cₗₐₛₜ, values below LLOQ were substituted by 1/2 LLOQ for the calculation of geometric statistics and 0 for arithmetic statistics.

Summary of adjusted bound aflibercept concentrations for participants in the DPKS are presented by treatment in Table 1-30. After the initial IVT administration of aflibercept of 2 mg or 8 mg (HDq12 pooled with HDq16), the concentration-time profiles of adjusted bound aflibercept were characterized by a slower attainment of peak concentration compared to free aflibercept. Following attainment of Cₘₐₓ, a slight decrease of the concentration-time profiles was observed until the end of the dosing interval of 4 weeks for both dose groups.

For unilaterally treated participants enrolled in the dense PK substudy who received aflibercept 2 mg (N = 6), concentrations of adjusted bound aflibercept were detectable in almost all participants until the end of the dense PK sampling. For the aflibercept 8 mg treatment (N = 13), adjusted bound aflibercept concentrations were detectable in almost all participants until the end of the dense PK sampling at Day 29 (Table 1-30).

**Table 1-30. Concentration of Adjusted Bound Aflibercept (mg/L) in Plasma by Time and Treatment in Participants with Unilateral Treatment Group up to Day 29 - Dense PK substudy (DPKS)**

| **Treatment** | **Time** | **n total** | **n ≥ LLOQ** | **Geom. Mean ± SD** | **Arithm. Mean ± SD** |
|---|---|---|---|---|---|
| 2q8 (N = 6) | Day 1, Pre-dose | 4 | 0 | N.C | 0.00 (0.00) |
| 2q8 (N = 6) | Day 1, 4 Hours Post-dose | 6 | 0 | N.C | 0.00 (0.00) |
| 2q8 (N = 6) | Day 1, 8 Hours Post-dose | 5 | 0 | N.C | 0.00 (0.00) |
| 2q8 (N = 6) | Day 2 | 6 | 4 | 0.02 (1.91) | 0.0249 (0.0216) |
| 2q8 (N = 6) | Day 3 | 6 | 6 | 0.06 (1.57) | 0.0638 (0.0343) |
| 2q8 (N = 6) | Day 5 | 6 | 6 | 0.10 (1.52) | 0.104 (0.0518) |
| 2q8 (N = 6) | Day 8 | 5 | 5 | 0.16 (1.55) | 0.179 (0.0990) |
| 2q8 (N = 6) | Day 15 | 5 | 5 | 0.16 (1.65) | 0.181 (0.108) |
| 2q8 (N = 6) | Day 22 | 5 | 5 | 0.16 (1.53) | 0.169 (0.0773) |
| 2q8 (N = 6) | Day 29 | 5 | 5 | 0.12 (1.90) | 0.149 (0.124) |
| HDq12 +HDq16 (N = 13) | Day 1, Pre-dose | 12 | 0 | N.C | 0.00 (0.00) |
| HDq12 +HDq16 (N = 13) | Day 1, 4 Hours Post-dose | 13 | 0 | N.C | 0.00 (0.00) |
| HDq12 +HDq16 (N = 13) | Day 1, 8 Hours Post-dose | 12 | 0 | N.C | 0.00 (0.00) |
| HDq12 +HDq16 (N = 13) | Day 2 | 12 | 10 | 0.06 (3.50) | 0.124 (0.186) |
| HDq12 +HDq16 (N = 13) | Day 3 | 12 | 12 | 0.13 (2.07) | 0.173 (0.155) |
| HDq12 +HDq16 (N = 13) | Day 5 | 11 | 11 | 0.18 (1.88) | 0.223 (0.157) |
| HDq12 +HDq16 (N = 13) | Day 8 | 13 | 13 | 0.31 (1.56) | 0.334 (0.135) |
| HDq12 +HDq16 (N = 13) | Day 15 | 13 | 13 | 0.37 (1.50) | 0.393 (0.130) |
| HDq12 +HDq16 (N = 13) | Day 22 | 11 | 10 | 0.25 (3.00) | 0.335 (0.155) |
| HDq12 +HDq16 (N = 13) | Day 29 | 12 | 12 | 0.32 (1.39) | 0.331 (0.0953) |

| | | | | | |
|---|---|---|---|---|---|
| Arithm. = arithmetic, DPKS = dense pharmacokinetic analysis set, geom. = geometric, LLOQ = Lower level of quantification, N = Number of participants with unilateral treatment up to Week 48, n = Number of observations, PK = pharmacokinetic, SD = standard deviation N.C.: not calculated (less than 2/3 of values per time point are ≥ LLOQ for geometric mean calculation). Values below LLOQ (0.022442 mg/L) were substituted by 1/2 LLOQ for the calculation of geometric statistics and 0 for arithmetic statistics and median. | | | | | |

Summaries of PK parameters for adjusted bound aflibercept for participants in the DPKS are presented by treatment in Table 1-31 for non-Japanese participants and in Table 1-32 for Japanese participants.

After the initial monthly aflibercept dose of 2 mg (2q8) or 8 mg (HDq12 pooled with HDq16), adjusted bound aflibercept median tmax was 14 days for the aflibercept 2 mg and 8 mg treatments. As the IVT dose of aflibercept increased from 2 mg to 8 mg (4-fold dose), the mean Cₘₐₓ and mean AUCₗₐₛₜ for adjusted bound aflibercept increased in a less than dose-proportional manner (about 2 to 2.5-fold) (Table 1-31).

Following the third initial monthly IVT dose of aflibercept, based on the ratio of aflibercept concentration at Week 12 to Week 4 (C_{Week12}/C_{Week4}), the accumulation ratio of adjusted bound aflibercept was 1.83 and 1.72 for 2q8, and HDq12+HDq16, respectively (Table 1-31).

In general, concentrations of adjusted bound aflibercept as well as PK parameters (Cₘₐₓ, AUCₗₐₛₜ) in the 2 Japanese participants (one each in the HDq12 + HDq16 groups, with only one of them providing data for PK parameters) were in the same range of values seen in non- Japanese participants after administration of aflibercept 8 mg.

**Table 1-31. Summary statistics of adjusted bound aflibercept pharmacokinetic parameters in plasma in participants with unilateral treatment for pooled region: Rest of the World - Dense PK substudy (DPKS)**

| **Pooled region** | **PK parameter (unit)** | **Treatment** | **n** | **Geom. mean (SD)** | **Geom. Mean 95% CI** | **Geom. CV (%)** | **Arithm. mean (SD)** | **Arthm. CV (%)** | **Median** | **Min, Max** |
|---|---|---|---|---|---|---|---|---|---|---|
| Rest of the World | Cmax (mg/L) | 2q8 (N = 6) | 6 | 0.164 (1.54) | 0.11, 0.25 | 44.9 | 0.179 (0.0950) | 53.0 | 0.158 | 0.108, 0.368 |
| | | HDq12+HDq16 (N = 11) | 11 | 0.415 (1.50) | 0.28, 0.62 | 42.6 | 0.441 (0.138) | 31.3 | 0.424 | 0.143, 0.611 |
| | Cmax/Dose (mg/L/mg) | 2q8 (N = 6) | 6 | 0.0821 (1.54) | 0.05, 0.13 | 44.9 | 0.0897 (0.0475) | 53.0 | 0.0789 | 0.0541, 0.184 |
| | | HDq12+HDq16 (N = 11) | 11 | 0.0519 (1.50) | 0.03, 0.08 | 42.6 | 0.0552 (0.0173) | 31.3 | 0.0531 | 0.0178, 0.0764 |
| | Clast (mg/L) | 2q8 (N = 6) | 6 | 0.120 (1.78) | 0.07, 0.21 | 63.1 | 0.142 (0.112) | 78.5 | 0.106 | 0.0739, 0.368 |
| | | HDq12+HDq16 | 11 | 0.325 | 0.23, | 34.8 | 0.341 | 28.4 | 0.338 | 0.143, |
| | | (N = 11) | | (1.40) | 0.46 | | (0.0966) | | | 0.512 |
| | Ctrough (mg/L) Day 29 | 2q8 (N = 6) | 5 | 0.122 (1.90) | 0.06, 0.23 | 71.8 | 0.149 (0.124) | 82.7 | 0.105 | 0.0739, 0.368 |
| | | HDq12+HDq16 (N = 11) | 10 | 0.317 (1.41) | 0.22, 0.45 | 35.4 | 0.332 (0.0970) | 29.3 | 0.336 | 0.143, 0.512 |
| | AUClast (day*mg/L) | 2q8 (N = 6) | 6 | 3.42 (1.66) | 2.06, 5.68 | 54.2 | 3.84 (2.21) | 57.6 | 3.34 | 1.77, 8.08 |
| | | HDq12+HDq16 (N = 11) | 11 | 7.98 (1.47) | 5.43, 11.7 | 39.8 | 8.45 (2.64) | 31.3 | 8.00 | 3.12, 12.3 |
| | AUCinf (day*mg/L) | 2q8 (N = 6) | 0 | / | / | / | / | / | / | / |
| | | HDq12+HDq16 (N = 11) | 0 | / | / | / | / | / | / | / |
| | AUCinf/Dose (day*mg/L/mg) | 2q8 (N = 6) | 0 | / | / | / | / | / | / | / |
| | | HDq12+HDq16 (N = 11) | 0 | / | / | / | / | / | / | / |
| | AUC(0-28d) Norm by *WT* | 2q8 (N = 6) | 2 | 0.0379 (1.19) | 0.03, 0.05 | 17.9 | 0.0382 (0.00673) | 17.6 | 0.0382 | 0.0334, 0.0429 |
| | (day*mg/L/kg) | HDq12+HDq16 (N = 11) | 5 | 0.118 (1.55) | 0.08, 0.18 | 46.2 | 0.127 (0.0514) | 40.3 | 0.140 | 0.069, 0.190 |
| | Accumulation Ratio (C_{Week12}/C_{Week4}) | 2q8 (N = 6) | 5 | 1.68 (1.63) | 1.04, 2.74 | 51.6 | 1.83 (0.766) | 41.8 | 1.66 | 0.790, 2.75 |
| | | HDq12+HDq16 (N = 11) | 10 | 1.59 (1.53) | 1.04, 2.42 | 44.2 | 1.72 (0.774) | 45.0 | 1.58 | 0.707, 3.55 |
| | t1/2 (day) | 2q8 (N = 6) | 1 | 25.3 (NE) | NE | NE | 25.3 (NE) | NE | 25.3 | 25.3, 25.3 |
| | | HDq12+HDq16 (N = 11) | 1 | 281 (NE) | NE | NE | 281 (NE) | NE | 281 | 281, 281 |
| | tmax (day) | 2q8 (N = 6) | 6 | / | / | / | / | / | 14.0 | 7.05, 21.0 |
| | | HDq12+HDq16 (N = 11) | 11 | / | / | / | / | / | 14.1 | 1.03, 28.1 |
| | tlast (day) | 2q8 (N = 6) | 6 | / | / | / | / | / | 28.0 | 21.0, 29.2 |
| | | HDq12+HDq16 (N = 11) | 11 | / | / | / | / | / | 27.9 | 21.0, 32.0 |

| | | | | | | | | | | |
|---|---|---|---|---|---|---|---|---|---|---|
| Arithm. = arithmetic, AUC_{inf} = Area under the curve from time zero to infinity, AUCₗₐₛₜ = Area under the curve to the last quantifiable concentration, CI = confidence interval, Cₗₐₛₜ = Last concentration, Cₘₐₓ = Maximum concentration, C_{trough} = trough concentration, CV = coefficient of variation, geom. = geometric, DPKS = dense pharmacokinetic analysis set, LLOQ = Lower level of quantification, Max = maximum, Min = minimum, N = Number of participants with unilateral treatment up to Week 48, n = Number of observations, NE = Not Evaluable, PK = pharmacokinetic, SD = standard deviation, t1/2 = half-life, tₗₐₛₜ = time to last concentration, tₘₐₓ = time to maximum concentration, WT = weight. / indicates categories that do not apply. For Cₗₐₛₜ, values below LLOQ were substituted by 1/2 LLOQ for the calculation of geometric statistics and 0 for arithmetic statistics | | | | | | | | | | |

**Table 1-32. Summary Statistics of Adjusted Bound Aflibercept Pharmacokinetic Parameters in Plasma in Participants with Unilateral Treatment for Pooled Region: Japan - Dense PK Substudy (DPKS)**

| **Pooled region** | **PK parameter (unit)** | **Treatment** | **n** | **Geom. mean (SD)** | **Geom. Mean 95% CI** | **Geom. CV (%)** | **Arithm. mean (SD)** | **Arthm. CV (%)** | **Median** | **Min, Max** |
|---|---|---|---|---|---|---|---|---|---|---|
| Japan | Cmax (mg/L) | 2q8 (N = 0) | 0 | / | / | / | / | / | / | / |
| | | HDq12+HDq16 (N = 2) | 1 | 0.567 (NE) | NE | NE | 0.567 (NE) | NE | 0.567 | 0.567, 0.567 |
| | Cmax/Dose (mg/L/mg) | 2q8 (N = 0) | 0 | / | / | / | / | / | / | / |
| | | HDq12+HDq16 (N = 2) | 1 | 0.0709 (NE) | NE | NE | 0.0709 (NE) | NE | 0.0709 | 0.0709, 0.0709 |
| | Clast (mg/L) | 2q8 (N = 0) | 0 | / | / | / | / | / | / | / |
| | | HDq12+HDq16 (N = 2) | 1 | 0.412 (NE) | NE | NE | 0.412 (NE) | NE | 0.412 | 0.412, 0.412 |
| | Ctrough (mg/L) Day 29 | 2q8 (N = 0) | 0 | / | / | / | / | / | / | / |
| | | HDq12+HDq16 (N = 2) | 1 | 0.412 (NE) | NE | NE | 0.412 (NE) | NE | 0.412 | 0.412, 0.412 |
| | AUClast (day*mg/L) | 2q8 (N = 0) | 0 | / | / | / | / | / | / | / |
| | | HDq12+HDq16 (N = 2) | 1 | 11.7 (NE) | NE | NE | 11.7 (NE) | NE | 11.7 | 11.7, 11.7 |
| | AUCinf (day*mg/L) | 2q8 (N = 0) | 0 | / | / | / | / | / | / | / |
| | | HDq12+HDq16 (N = 2) | 0 | / | / | / | / | / | / | / |
| | AUCinf/Dose (day*mg/L/mg) | 2q8 (N = 0) | 0 | / | / | / | / | / | / | / |
| | | HDq12+HDq16 (N = 2) | 0 | / | / | / | / | / | / | / |
| | AUC(0-28d) | 2q8 (N = 0) | 0 | / | / | / | / | / | / | / |
| | Norm by WT (day*mg/L/kg) | HDq12+HDq16 (N = 2) | 0 | / | / | / | / | / | / | / |
| | Accumulation | 2q8 (N = 0) | 0 | / | / | / | / | / | / | / |
| | Ratio (C_{Week12}/C_{Week4}) | HDq12+HDq16 (N = 2) | 1 | 1.47 (NE) | NE | NE | 1.47 (NE) | NE | 1.47 | 1.47, 1.47 |
| | 11/2 (day) | 2q8 (N = 0) | 0 | / | / | / | / | / | / | / |
| | | HDq12+HDq16 (N = 2) | 0 | / | / | / | / | / | / | / |
| | tmax (day) | 2q8 (N = 0) | 0 | / | / | / | / | / | / | / |
| | | HDq12+HDq16 (N = 2) | 1 | / | / | / | / | / | 14.0 | 14.0 |
| | | | | | | | | | | 14.0 |
| | tlast (day) | 2q8 (N = 0) | 0 | / | / | / | / | / | / | / |
| | | HDq12+HDq16 (N = 2) | 1 | / | / | / | / | / | 27.9 | 27.9 |
| | | | | | | | | | | 27.9 |

| | | | | | | | | | | |
|---|---|---|---|---|---|---|---|---|---|---|
| Arithm. = arithmetic, AUC_{inf} = Area under the curve from time zero to infinity, AUCₗₐₛₜ = Area under the curve to the last quantifiable concentration, CI = confidence interval, Cₗₐₛₜ = Last concentration, Cₘₐₓ = Maximum concentration, C_{trough} = trough concentration, CV = coefficient of variation, geom. = geometric, DPKS = dense pharmacokinetic analysis set, LLOQ = Lower level of quantification, Max = Maximum, Min = Minimum, N = Number of participants with unilateral treatment up to Week 48, n = Number of observations, NE = Not Evaluable, PK = pharmacokinetic, SD = standard deviation, t_{1/2} = half-life, tₗₐₛₜ = time to last concentration, tₘₐₓ = time to maximum concentration, WT = weight. / indicates categories that do not apply. For Cₗₐₛₜ, values below LLOQ were substituted by 1/2 LLOQ for the calculation of geometric statistics and 0 for arithmetic statistics. | | | | | | | | | | |

Table 1-33 shows an overview of sampling time points for the sparse sampling in the 3 different dosing groups. Table 1-34 summarizes the plasma concentration-time data for free aflibercept in participants with unilateral treatment (sparse PK sampling, PKS) after IVT administration of aflibercept in the 2q8, HDq12, and HDq16 regimens, respectively.

Concentrations of free aflibercept concentration in plasma were, on average, higher for the HDq12 and HDq16 treatment groups than the 2q8 treatment group. Mean free aflibercept concentrations increased from baseline to Visit 5 (60-64 days after first administration). Thereafter, mean concentrations of free aflibercept declined in all 3 dose groups. In the 2q8 treatment group, mean concentrations of free aflibercept declined to values close to or below LLOQ in almost all participants 4 weeks after treatment, in the HD groups 8 weeks after treatment (Week 28 for HDq12, Week 48 for HDq16) (Table 1-34).

**Table 1-33. Overview of Sampling Time Points for the Different Dosing Groups (PKS)**

| **Sampling time point** | **2q8** | **HDq12** | **HDq16** |
|---|---|---|---|
| Baseline | Prior to first administration | | |
| Week 4 | 4 weeks after first administration | | |
| Visit 5 | 4-7 days after third monthly administration | | |
| Week 12 | 4 weeks after third montly administration | | |
| Week 28 | 4 weeks after Week 24 administration | 8 weeks after Week 20 administration | 4 weeks after Week 24 administration |
| Week 48 | 4 weeks after Week 40 administration | 4 weeks after Week 44 administration | 8 weeks after Week 40 administration |

Comparison of mean concentrations of free aflibercept at Visit 5 which could be considered a time point around an expected Cₘₐₓ rather than a trough value, showed that concentrations increased about 6-fold as the IVT dose of aflibercept increased from 2 mg to 8 mg (4-fold dose). Based on the ratio of aflibercept concentration at Week 12 to Day 29 (C_{Week12}/C_{Day29}), the accumulation ratio of free aflibercept was 1.06, 1.69, and 1.92 for 2q8, HDq12, and HDq16, respectively.

Exploratory analysis of subgroups with respect to age, body mass index (BMI), medical history of renal impairment (as determined by creatinine clearance), medial history of hepatic impairment, ethnicity (Latino/Hispanic vs not Latino/Hispanic), race (White vs. Asian), and treatment-emergent antibody status did not reveal any meaningful differences for free aflibercept concentrations.

**Table 1-34. Summary of Concentration of Free Aflibercept (mg/L) in Plasma by Time and Treatment in Participants with Unilateral Treatment and without DRMs upto Week 48 - General PK (PKS)**

| | | | | **Free aflibercept** | |
|---|---|---|---|---|---|
| **Time** | **Treatment** | **n total** | **n ≥ LLOQ** | **Geom. Mean ± SD** | **Arithm. Mean ± SD** |
| Baseline (Visit 2) | 2q8 (N = 237) | 228 | 3 | N.C | 0.00033 (0.00303) |
| | HDq12 (N = 203) | 189 | 0 | N.C | 0.00 (0.00) |
| | HDq16 (N = 193) | 178 | 0 | N.C | 0.00 (0.00) |
| Week 4 (Visit 3) | 2q8 (N = 237) | 219 | 4 | N.C | 0.00044 (0.00371) |
| | HDq12 (N = 203) | 192 | 121 | N.C | 0.0172 (0.0157) |
| | HDq16 (N = 193) | 182 | 117 | N.C | 0.0170 (0.0163) |
| Visit 5 | 2q8 (N = 237) | 203 | 167 | 0.02 (1.83) | 0.0256 (0.0165) |
| | HDq12 (N = 203) | 176 | 173 | 0.13 (1.97) | 0.157 (0.101) |
| | HDq16 (N = 193) | 165 | 161 | 0.13 (2.02) | 0.151 (0.0810) |
| Week 12 (Visit 6) | 2q8 (N = 237) | 221 | 11 | N.C | 0.00133 (0.00654) |
| | HDq12 (N = 203) | 191 | 155 | 0.02 (1.98) | 0.0284 (0.0227) |
| | HDq16 (N = 193) | 188 | 149 | 0.02 (2.02) | 0.0293 (0.0226) |
| Week 28 (Visit 10) | 2q8 (N = 237) | 203 | 4 | N.C | 0.00068 (0.00601) |
| | HDq12 (N = 203) | 187 | 6 | N.C | 0.00088 (0.00525) |
| | HDq16 (N = 193) | 179 | 101 | N.C | 0.0152 (0.0160) |
| Week 48 (Visit 15) | 2q8 (N = 237) | 202 | 2 | N.C | 0.00087 (0.0100) |
| | HDq12 (N = 203) | 180 | 87 | N.C | 0.0136 (0.0179) |
| | HDq16 (N = 193) | 165 | 4 | N.C | 0.00056 (0.00375) |

| | | | | | |
|---|---|---|---|---|---|
| Arithm. = arithmetic, DRM = dose regimen modification, geom. = geometric, LLOQ = Lower level of quantification, N = Number of participants with unilateral treatment and without DRMs up to Week 48, n = Number of observations, PKS = pharmacokinetic analysis set, SD = standard deviation; N.C.: not calculated (less than 2/3 of values per time point are ≥ LLOQ for geometric mean calculation). Values below LLOQ (0.0156 mg/L) were substituted by 1/2 LLOQ for the calculation of geometric statistics and 0 for arithmetic statistics and median. | | | | | |

Table 1-35 summarizes the plasma concentration-time data for adjusted bound aflibercept in all participants (sparse PK sampling, PKS) after IVT administration of aflibercept in the 2q8, HDq12, and HDq16 regimens, respectively. Concentrations of adjusted bound aflibercept in plasma were, on average, higher for the HDq12 and HDq16 treatment groups than the 2q8 treatment group. Mean adjusted bound aflibercept concentrations increased from baseline to Visit 5 (60-64 days after first administration). Thereafter, a slight decrease of the concentration-time profiles was observed until the end of the observation period (Week 48).

Evaluation of mean concentrations of adjusted bound aflibercept at Visit 5 which could be considered a time point around an expected Cₘₐₓ rather than a trough value, showed that concentrations increased about 3-fold as the IVT dose of aflibercept increased from 2 mg to 8 mg (4-fold dose). Based on the ratio of aflibercept concentration at Week 12 to Day 29 (C_{Week12}/C_{Day29}), the accumulation ratio of adjusted bound aflibercept was 1.83, 2.03, and 2.22 for 2q8, HDq12, and HDq16, respectively.

Exploratory analysis of subgroups with respect to age, BMI, medical history of renal impairment (as determined by creatinine clearance), medial history of hepatic impairment, ethnicity (Latino/Hispanic vs not Latino/Hispanic), race (White vs. Asian), and treatment-emergent antibody status did not reveal any meaningful differences for adjusted bound aflibercept concentrations.

**Table 1-35. Summary of Concentration of Adjusted Bound Aflibercept (mg/L) in Plasma by Time and Treatment in Participants with Unilateral Treatment and Without DRMs upto Week 48 - General PK (PKS)**

| **Adjusted Bound Aflibercept** | | | | | |
|---|---|---|---|---|---|
| **Time** | **Treatment** | **n total** | **n ≥ LLOQ** | **Geom. Mean ± SD** | **Arithm. Mean ± SD** |
| Baseline (Visit 2) | 2q8 (N = 237) | 228 | 7 | N.C | 0.0109 (0.0915) |
| | HDq12 (N = 203) | 189 | 1 | N.C | 0.00029 (0.00404) |
| | HDq16 (N = 193) | 178 | 2 | N.C | 0.00081 (0.00923) |
| Week 4 (Visit 3) | 2q8 (N = 237) | 219 | 215 | 0.12 (1.67) | 0.130 (0.0839) |
| | HDq12 (N = 203) | 192 | 191 | 0.34 (1.65) | 0.375 (0.132) |
| | HDq16 (N = 193) | 182 | 181 | 0.33 (1.72) | 0.362 (0.141) |
| Visit 5 | 2q8 (N = 237) | 203 | 201 | 0.24 (1.59) | 0.255 (0.0920) |
| | HDq12 (N = 203) | 176 | 174 | 0.65 (1.77) | 0.710 (0.249) |
| | HDq16 (N = 193) | 165 | 164 | 0.64 (1.63) | 0.687 (0.222) |
| Week 12 (Visit 6) | 2q8 (N = 237) | 221 | 219 | 0.18 (1.72) | 0.208 (0.119) |
| | HDq12 (N = 203) | 191 | 191 | 0.58 (1.46) | 0.615 (0.216) |
| | HDq16 (N = 193) | 188 | 187 | 0.56 (1.63) | 0.610 (0.220) |
| Week 28 (Visit 10) | 2q8 (N = 237) | 203 | 203 | 0.15 (1.57) | 0.168 (0.107) |
| | HDq12 (N = 203) | 187 | 185 | 0.19 (1.81) | 0.222 (0.119) |
| | HDq16 (N = 193) | 179 | 174 | 0.32 (2.04) | 0.372 (0.145) |
| Week 48 (Visit 15) | 2q8 (N = 237) | 202 | 184 | 0.06 (2.10) | 0.0775 (0.0703) |
| | HDq12 (N = 203) | 180 | 171 | 0.30 (2.44) | 0.375 (0.178) |
| | HDq16 (N = 193) | 165 | 161 | 0.17 (1.97) | 0.204 (0.107) |

| | | | | | |
|---|---|---|---|---|---|
| Arithm. = arithmetic, DRM = dose regimen modification, geom. = geometric, LLOQ = Lower level of quantification, N = Number of participants with unilateral treatment and without DRMs up to Week 48, n = Number of observations, PKS = pharmacokinetic analysis set, SD = standard deviation; N.C.: not calculated (less than 2/3 of values per time point are ≥ LLOQ for geometric mean calculation). Values below LLOQ (0.022442 mg/L) were substituted by 1/2 LLOQ for the calculation of geometric statistics and 0 for arithmetic statistics and median. | | | | | |

### Expanded Population PK Analysis (referred to as the Population PK model, or PopPK).

With availability of the free and adjusted bound aflibercept concentration data from the CANDELA, PULSAR, and PHOTON along PK data from the other studies listed herein, a comprehensive PopPK model was developed, In this latter PopPK model, the PK of free and adjusted bound aflibercept following IV, SC, or IVT administration was adequately described by a 3-compartment PopPK model with the binding of free aflibercept from the central compartment to VEGF described by Michaelis-Menten kinetics. An additional tissue compartment that could represent platelets (Sobolewska et al., Human Platelets Take up Anti-VEGF Agents. J Ophthalmol 2021; 2021:8811672) was added where the rate of elimination from the central compartment of free aflibercept to the platelet compartment was dependent on the number of platelets that were able to uptake anti-VEGF agents such as ranibizumab, bevacizumab, and aflibercept (Figure 33).

Although PK parameters for free and adjusted bound aflibercept in plasma were determined by noncompartmental analysis (NCA) and reported at the level of the individual study reports, the PK parameters determined by population PK analysis are considered to be the more accurate estimate and therefore the definitive PK parameters are those assessed by the population PK model.

Across all 3 studies (CANDELA, PULSAR, and PHOTON), the pharmacokinetic analysis set (PKAS) includes all treated participants who received any amount of study drug (aflibercept or HD aflibercept) and had at least 1 non-missing aflibercept or adjusted bound aflibercept measurement following the first dose of study drug. The PKAS is based on the actual treatment received (as treated), rather than as randomized. The PKAS-dense (PK-dense) analysis set is a subset of the PKAS and includes participants who had dense blood sample collection for systemic drug concentrations.

CANDELA, PULSAR, and PHOTON each included a PK substudy where drug concentration data were collected using dense blood sample collection schedules during the first dosing interval and sparse PK sampling thereafter in up to approximately 30 participants. Drug concentration data were also collected in each study for all participants using a sparse sampling schedule throughout the 44 weeks (CANDELA) or 48 weeks (PHOTON, PULSAR) of treatment.

Pharmacokinetic parameters for individual studies were calculated by non-compartmental analysis for free and adjusted bound aflibercept concentration data collected from participants with dense sampling schedules in these 3 studies.

Additionally, all concentration data from these 3 studies were incorporated into the Population PK data set.

The concentration time profiles of free and adjusted bound aflibercept in plasma after the initial dose of HD aflibercept by IVT administration were consistent between all studies in participants with nAMD or DME. The consistency of the concentration-time profiles for free and adjusted bound aflibercept in plasma between the nAMD and DME populations is further supported by population PK analysis (Figure 35 and Figure 36)**.**

Population PK estimated post-hoc concentration-time profiles and PK parameters for combined nAMD and DME populations following single IVT administration of 2 mg aflibercept or HD aflibercept are provided in Figure 35 and Figure 36 and in Table 1-36 and Table 1-39.

Following single IVT administration of aflibercept 2 mg or HD aflibercept, the concentration-time profiles of free and adjusted bound aflibercept in plasma in participants who underwent dense sample collection for systemic drug concentrations (dense PK substudy) after the initial dosing of aflibercept 2 mg or HD aflibercept, respectively, were consistent between the 3 studies in participants with nAMD or DME (Figure 34). Notably, there was one excluded participant in the PULSAR dense-sampling PK substudy that had free aflibercept concentrations over time that were approximately 10-fold higher than the mean concentration-time profiles in that study.

The consistency of the concentration-time profiles for free and adjusted bound aflibercept between the nAMD and DME populations is further supported by Population PK analysis (Figure 35). Population PK estimated post-hoc concentration-time profiles and PK parameters for combined nAMD and DME populations following single IVT administration of 2 mg aflibercept or HD aflibercept are provided below in Figure 35 and in Table 1-37 and Table 1-38.

The corresponding observed and Population PK estimated post-hoc concentration-time profiles and PK parameters for participants with nAMD and DME are provided in Figure 38, Figure 39, and Figure 40 and Table 1-39, Table 1-40.

Following single IVT administration of 2 mg aflibercept or HD aflibercept, the concentration-time profiles of free aflibercept are characterized by an initial phase of increasing concentrations, as the drug moved from the ocular space into systemic circulation, followed by a mono-exponential elimination phase. The concentration time profiles of adjusted bound aflibercept in plasma are characterized by a slower attainment of Cmax compared to free aflibercept. Following attainment of Cₘₐₓ, a sustained plateau of the concentration-time profiles of adjusted bound aflibercept in plasma was observed until approximately the end of the first dosing interval (Figure 34, Figure 35).

For participants who underwent dense blood sample collection for systemic drug concentrations across the CANDELA, PULSAR, and PHOTON studies, after the initial dosing of 2 mg IVT aflibercept (n = 34), observed concentrations of free aflibercept were detectable in 15 (44.1%) participants by week 1 and in 3 (8.8%) participants by week 2.

For participants who underwent dense blood sample collection for systemic drug concentrations after the initial dosing of 8 mg IVT aflibercept (n = 54), observed concentrations of free aflibercept were detectable in 46 (85.2%) participants by week 1 and in 44 (77.8%) participants by week 2. The observed and Population PK simulated free and adjusted bound aflibercept concentrations in plasma for up to 48 weeks are presented for the combined nAMD and DME population (Figure 36), and the nAMD (Figure 41) and DME (Figure 42) populations. Based on the Population PK analysis, the median time for free aflibercept concentrations to reach LLOQ in plasma following HDq12 or HDq16 was more than double (3.50 weeks versus 1.5 weeks) the median time needed to reach LLOQ following aflibercept 2q8 (Table 1-41).

The longer duration of systemic exposure to free aflibercept following HDq12 and HDq16 compared to the 2 mg aflibercept is attributed to not only a higher administered dose and nonlinear systemic target-mediated elimination, but also to a 34% slower ocular clearance of free aflibercept. The slower ocular clearance of free aflibercept for HD aflibercept is attributed to a HD drug product effect which was identified as a statistically significant covariate in the Population PK model.

Population PK analysis confirmed no relevant differences in PK between the nAMD and DME populations, and therefore all subsequent analyses are presented for the combined nAMD and DME population.

The pharmacokinetic (PK) data set forth above summarize the observed systemic concentration-time profiles and associated PK parameters for free and adjusted bound aflibercept for each individual study. The analyses utilized to estimate the PK parameters in each individual study were performed by non-compartmental analysis. While the individual PHOTON study results describe the observed systemic concentration-time profiles and associated PK parameters of free and adjusted bound aflibercept in plasma, they do not specifically identify PK characteristics of the HD 8 mg aflibercept drug product contributing to the unexpected pharmacodynamic (PD) and efficacy results for HD aflibercept observed in the CANDELA (NCT04126317), PULSAR (NCT04423718), and PHOTON (European Clinical Trials Database (EudraCT): 2019-003851-12) studies.

An expanded PopPK analysis that utilized free and adjusted bound concentration in plasma data from the HD clinical studies, as well as 13 prior studies:

### DME population

- VGFT-OD-0307: A double-blind, randomised, dose-escalating study evaluating safety, tolerability and bio-effect after intravenous (IV) administration of VEGF Trap in subjects with DME. Subjects were planned to receive 4 IV infusions of VEGF Trap, once every 2 weeks (day 1, day 15, day 29, and day 43), at dose levels of 0.3 mg/kg, 1 mg/kg, or 3 mg/kg, or placebo. However, dosing was stopped before the planned sequential dose escalation when dose-limiting toxicities (grade 2 proteinuria in a single subject and grade 4 treatment-related malignant hypertension in a single subject) were observed in another phase 1 dose-escalation study in subjects with AMD (VGFT-OD-0305). The dose limiting toxicities observed in study VGFT-OD-0305 occurred at the 3 mg/kg IV dose. Therefore, only the lowest dose (0.3 mg/kg) of study drug was investigated. Nine subjects were randomised and treated (3 placebo, 6 VEGF Trap 0.3 mg/kg). Concentrations of free and bound VEGF Trap were determined at selected time intervals following dose administration (screening, day 1[pre-dose], day 8, day 15, day 29, day 43, day 57, day 71, day 85, and day 133 [3 months post-last dose]);
- VGFT-OD-0512: An open-label, proof-of-concept study evaluating safety, tolerability and bio-effect of VEGF Trap IVT administration in subjects with DME. Five (5) subjects with DME were enrolled. Subjects received a single IVT injection of 4 mg VEGF Trap into the study eye. During the first 6 weeks after the injection, vital signs as well as ocular and systemic adverse events (AEs) were recorded. Blood samples for analysis of free and bound VEGF Trap concentrations in plasma were collected at screening, day 1 (pre-dose), day 3, day 8, day 15, day 29, day 43 and day 155 following the single IVT administration;
- VGFT-OD-0706.PK (PK sub-study of VGFT-OD- 0706): DME And VEGF Trap-Eye [Intravitreal Aflibercept Injection (IAI;EYLEA^{®};BAY86-5321)] INvestigation of Clinical Impact (DA VINCI)-Clinicaltrials.gov Identifier NCT00789477; and
- 91745: Intravitreal Aflibercept Injection in Vision Impairment Due to DME (VIVID-DME)-ClinicalTrials.gov Identifier NCT01331681;

### AMD population

- VGFT-OD-0305: A double-masked, placebo controlled, sequential group, dose escalating, (0.3 mg/kg, 1 mg/kg, 3 mg/kg, 5 mg/kg, 7 mg/kg, and 10 mg/kg) study of safety and bioeffect. The study included subjects with a diagnosis of visual impairment associated with neovascular AMD. Subjects were required to have visual loss due to subfoveal choroidal neovascularization (CNV) secondary to AMD, be 50 years of age or older, with no history of Type I or Type II diabetes, without significant cardiac, liver or kidney disease, or congestive heart failure (CHF); and without confounding ophthalmic issues. The study treatments were: 1. VEGF Trap 0.3 mg/kg. 2. VEGF Trap 1 mg/kg, and 3. VEGF Trap 3 mg/kg;
- VGFT-OD-0306: An open label, long term safety and tolerability extension study of intravenous VEGF Trap in subjects with neovascular AMD who had been included in Study VEGF-OD-0305. The study treatments were VEGF Trap at the same dose level the subjects had been treated with in Study VEGF-OD-0305: either 0.3 mg/kg or 1 mg/kg, by intravenous administration every 2 weeks. Placebo patients from Study VGFT-OD-0305 were assigned to VEGF Trap at the dose level at which they were enrolled in Study VGFT-OD-0305. The efficacy outcome measures were: Visual acuity (ETDRS), Retinal thickness (OCT), Funduscopic examination, Fundus photography, and FA. The safety outcome measures were: AEs, Clinical laboratory tests, and Ophthalmic exam. Treatment duration was for up to 106 days. There were seven subjects: four subjects treated with 0.3 mg/kg, 3 subjects treated with 1 mg/kg. There were five females, two males and the age range was 68 to 84 years. Six of the seven subjects had a slight reduction in ERT in the study eye and six of seven subjects had slight reductions in macular volume in the study eye;

- VGFT-OD-0502 Part A: Safety and Tolerability Study of Intravitreal VEGF-Trap Administration in Patients With Neovascular AMD-ClinicalTrials.gov Identifier: NCT00320775;
- VGFT-OD-0502 Part C: ClinicalTrials.gov Identifier: NCT00320775;
- VGFT-OD-0603: Safety and Tolerability of Intravitreal VEGF Trap Formulations in Subjects With Neovacular AMD-ClinicalTrials.gov Identifier: NCT00383370;
- VGFT-OD-0702.PK (PK sub-study of VGFT-OD-0702): Randomized, Single-Masked, Long-Term, Safety and Tolerability Study of VEGF Trap-Eye in AMD-ClinicalTrials.gov Identifier: NCT00527423; and
- 311523 (VIEW2): A multicentre, double masked, randomised, active controlled, parallel group, non-inferiority efficacy and safety study. The study was almost identical in design to Study VGFT-OD-0605/14393 (VIEW 1). The submission contained the report of the first 52 weeks of the study. The study was conducted at 186 centres in 26 countries.

The inclusion criteria, exclusion criteria and study treatments were identical to Study VGFT-OD-0605/14393 (VIEW 1). The efficacy outcome measures were the same, except for the additional outcome measure: change in scores of the EQ-5D questionnaire from screening at Week 52;

### Oncology population

- VGFT-ST-0103, (also known as TED6113): VEGF Trap in Treating Patients With Relapsed or Refractory Solid Tumors or Non-Hodgkin's Lymphoma-ClinicalTrials.gov Identifier: NCT00036946;

### Healthy participant population

- PDY6655: A Phase I, single centre, randomised, single dose, crossover, pharmacokinetic (PK) study in healthy volunteers to compare the pharmacokinetics and pharmacodynamic (PD) of intravenous and subcutaneous administration of aflibercept. The study included 40 healthy male subjects aged 18 to 45 years. The study treatments were: aflibercept 2.0 mg/kg as an intravenous infusion over 1 hour, and as a subcutaneous injection. The aflibercept was presented as 4 mL of 25 mg/mL solution. The treatments were administered as single doses followed by 6 week observation period. The treatment periods were separated by 1 to 2 weeks. The PK outcome measures were: Cmax, AUC, apparent volume of distribution at steady state (Vss), clearance and half life (t½). The PD outcome measures were: systolic blood pressure, diastolic blood pressure, heart rate, mean arterial pressure, plasma renin activity, angiotensin I, aldosterone, and free endogenous VEGF. The safety outcome measures were: AEs, clinical laboratory test, injection site reactions, and anti-aflibercept antibodies. AUC and Cmax were slightly higher for Period 2, indicating some carry over. For Period 1, for free aflibercept mean (co-variance (CV%)) AUC was 177 (33) µg•day/mL and peak plasma concentration (Cmax) was 44.4 (36) µg/mL for intravenous and AUC was 84.9 (30) µg•day/mL and Cmax was 7.76 (39) µg/mL for subcutaneous. For Period 1, for bound aflibercept mean (CV%) AUC was 57.7 (19) µg•day/mL and Cmax was 1.84 (22) µg/mL for intravenous and AUC was 47.3 (27) µg•day/mL and Cmax was 1.60 (27) µg/mL for subcutaneous. The mean (90% CI) ratio for AUC, subcutaneous/ intravenous, was 0.51 (0.46 to 0.56) [(range)], and
- PDY6656: A single centre, Phase I, randomised, double blind, placebo controlled, sequential ascending dose study of intravenous aflibercept. The study included healthy male subjects 18 to 45 years of age; non-smoker; 18≤ body mass index (BMI) ≤28 kg/m²; with normal vital signs and no symptomatic hypotension. The study treatments were aflibercept 1 mg/kg, 2 mg/kg and 4 mg/kg, and placebo. There were three cohorts of 16 subjects: twelve treated with aflibercept and four treated with placebo. The treatments were administered as a single dose by intravenous infusion over 1 hour. The pharmacodynamic outcome measures were: systolic blood pressure (SBP), diastolic blood pressure (DBP), mean arterial pressure (MAP), plasma active renin, aldosterone and angiotensin I; markers of endothelium dysfunction (plasma endothelin-1, E-selectin, cyclic guanosine 3'5' monophosphate (cGMP), and urine nitrites/nitrates); renal function; and VEGF. The safety outcome measures were: AEs and laboratory tests. The study included 48 subjects: 12 treated with 1 mg/kg, 12 with 2 mg/kg, 12 with 4 mg/kg and 12 with placebo. The age range was 21 to 45 years. For free aflibercept mean (CV%) Cmax was 18.2 (18) µg/mL for the 1 mg/kg dose, 39.7 (27) µg/mL for the 2 mg/kg dose and 78.6 (15) µg/mL for the 4 mg/kg dose; and mean (CV%) AUC was 64.8 (20) µg.day/mL for the 1 mg/kg dose, 180 (20) for the 2 mg/kg dose and 419 (21) for the 4 mg/kg dose. Bound aflibercept concentrations were not dose dependent and the proportion of bound aflibercept decreased with increasing dose. However, Cmax and AUC for total aflibercept were dose proportional [(range)];(See Australian Public Assessment Report for Afibercept, AusPAR Eylea Aflibercept Bayer Australia Ltd; PM-2010-03802-3-5 Final 30 July 2012; and Assessment Report, Eylea, Committee for Medicinal Products for Human Use (CHMP) 26 June 2014 EMA/430291/2014; FDA, Center for Drug Evaluation and Research, Approval Package for: APPLICATION NUMBER: 125387Orig1s048, Eylea, March 25, 2015) of aflibercept 2 mg in the DME and nAMD populations, healthy participants, and participants with oncology diseases after intravenous (IV), subcutaneous (SC), or intravitreal (IVT) administration was performed to: characterize the concentration-time profiles of free and adjusted bound aflibercept in plasma; estimate population and individual PK parameters of aflibercept in patients with nAMD and DME; investigate the effects of relevant covariates which may explain variability in aflibercept PK parameters; and derive *post-hoc* estimates of individual exposure metrics in the nAMD and DME patients from the final PopPK model that formed the basis for pharmacokinetic/pharmacodynamic (PK/PD) analyses.

A key finding from this expanded PopPK analysis is that clearance of free aflibercept from the ocular compartment (ocular clearance) is 34.3% slower for HD drug product than for 2 mg IVT aflibercept reference drug product, and is attributed to an "HD aflibercept drug product effect". Ultimately, it is this HD drug product effect on slowing the ocular clearance that resulted in a longer than expected ocular residence time, and the greater than expected proportion of patients able to be maintained on the longer dosing intervals of q12 and q16.

The consequences of the slower ocular clearance for HD (8 mg) aflibercept, as identified in the PopPK analysis, were further evaluated via PopPK model-based simulations to predict the time-course of free aflibercept in the eye (ocular compartment) under different dosing scenarios, and via exposure-response analyses to assess whether PopPK estimates of ocular clearance are predictive of the time required for dose regimen modification (DRM).

Efficacy data from the phase 3 PULSAR study in the nAMD population confirmed that the HDq12 and HDq16 regimens provide durable efficacy over the 48-week treatment period, as both regimens met the primary endpoint for efficacy of non-inferior change from baseline in BCVA at week 48 compared to 2q8. A majority of participants randomized to HDq12 or HDq16 maintained their 12-week (79%) and 16-week (77%) dosing intervals, without the need for DRM, through 48 weeks.

Results from the phase 2/3 PHOTON study also confirmed efficacy of the HDq12 and HDq16 regimens in participants with DME and DR as both met the primary endpoint for efficacy of noninferior change from baseline in BCVA at week 48 compared to 2q8, with a majority of participants maintaining their HDq12 (91%) and HDq16 (89%) regimens, without the need for DRM, through the end of the 48-week treatment period.

As the vast majority of participants enrolled in the PHOTON study had underlying DR, they were also assessed for efficacy endpoints associated with the improvement of their underlying retinopathy. The HDq12 regimen met the key secondary efficacy endpoint of noninferiority for the proportion of participants with a ≥2-step improvement in DRSS score compared to 2q8 at the prespecified margin of 15%. Additionally, noninferiority was demonstrated using the FDA recommended 10% margin. Non-inferiority was not established for HDq16 at the 15% margin. The HDq16 group had more participants with mild to moderate disease than both the HDq12 and the 2q8 group, which may have contributed to these findings.

Regarding safety, similar ocular and systemic safety profiles for HDq12 and HDq16 compared to 2q8 aflibercept were observed in all 3 studies, with no new safety signals identified for HD aflibercept.

Residual variability was modeled separately for free and adjusted bound aflibercept using an additive + proportional error model. Estimated bioavailability for free aflibercept was 71.9% following IVT administration (Table 1-36). Parameter estimates for the Population PK model are presented in Table 1-36.

**Table 1-36. Population Pharmacokinetic Parameter Estimates for the Final Model for Aflibercept**

| **Parameter** | **Estimate** | **C.I.95** | **RSE %** | **CV %** |
|---|---|---|---|---|
| K20 (1/day) [run431 Estimate] | 0.0807 | 0.0438 - 0.136 | 29.5 | - |
| V2 V4 (L) [run431 Estimate] | 4.99 | 4.71 - 5.25 | 2.79 | - |
| V3 (L) [run431 Estimate] | 1.08 | 0.816 - 1.58 | 17 | - |
| QF1 (L/day) [run431 Estimate] | 0.849 | 0.435 - 1.33 | 29.2 | - |
| V8 (L) [run431 Estimate] | 1.18 | 0.281 - 0.541 | 16.8 | - |
| QF2 (L/day) [run431 Estimate] | 0.0763 | 0.147 - 0.186 | 5.93 | - |
| KM (mg/L) [run431 Estimate] | 0.411 | 0.293 - 0.442 | 10.5 | - |
| VMK24 (mg/day/L) [run431 Estimate] | 0.167 | 0.482 - 0.593 | - | - |
| K40 (1/day) [run463 Estimate] | 0.035 | - | - | - |
| F1 & F5 | 0.719 | 0.706 - 0.731 | - | - |
| QE (L/day) | 0.000624 | 0.000577 - 0.000674 | 3.97 | - |
| K62 (1/day) [run431 Estimate] | 0.368 | 0.0165 - 0.0632 | 35.3 | - |
| F6 [run431 Estimate] | 0.536 | 0.00584 - 0.149 | 99 | - |
| VMK27 (mg/day/L) [run431 Estimate] | 0.031 | 4.17 - 340 | 159 | - |
| K70 (1/day) [run431 Estimate] | 0.0265 | 0.632 - 2.84 | 39.8 | - |
| KMK27 (mg) [run431 Estimate] | 42.7 | 0.0481 - 0.12 | 23.7 | - |
| TWGT V2+V4 [run431 Estimate] | 0.872 | 0.55 - 1.22 | 19.3 | - |
| TWGT V3 [run431 Estimate] | 1.08 | -0.00762 - 2.45 | 51.3 | - |
| TWGT V8 [run431 Estimate] | 1.16 | -2.97 - 6.58 | 135 | - |
| TWGT K20 [run431 Estimate] | -0.192 | -1.28 - 0.819 | 234 | - |
| TWGT K40 [run463 Estimate] | -0.153 | - | - | - |
| HD QE | 0.657 | 0.607 - 0.712 | 4.08 | - |
| AGE QE | -1.53 | -1.76 - -1.3 | 7.68 | - |
| TALB K40 [run463 Estimate] | -0.767 | - | - | - |
| IIV K20 [run431 Estimate] | 0.207 | -0.0147 - 0.508 | 54.1 | 48 |
| IIV covariance(K20, V2 & V4) [run431 Estimate] | -0.0727 | -0.136 - -0.0183 | 38.9 | - |
| IIV V2 & V4 [run431 Estimate] | 0.0618 | 0.0198 - 0.105 | 34.7 | 25.3 |
| IIV VMK24 [run431 Estimate] | 0.305 | -0.083 - 0.67 | 65.4 | 59.8 |
| IIV K40 [run463 Estimate] | 0.0452 | - | - | 21.5 |
| IIV QE | 0.297 | 0.257 - 0.336 | 6.86 | 58.8 |
| IIV K62 [run431 Estimate] | 0.852 | 0.124 - 1.45 | 43 | 116 |
| IIV F6 [run431 Estimate] | 0.629 | 0.288 - 0.905 | 26.4 | 93.6 |
| SD ADD LLOQ 0.0313 (Free,IV+SC) [run463 Estimate] | 0.025 | - | - | - |
| SD PROP LLOQ 0.0313 (Free,IV+SC) [run463 Estimate] | 0.403 | - | - | - |
| SD ADD LLOQ 0.0156 (Free) | 0.00779 | 0.00624 - 0.00973 | 11.4 | - |
| SD PROP LLOQ 0.0156 (Free) | 0.433 | 0.418 - 0.448 | 1.72 | - |
| SD ADD LLOQ 0.0315 (Adj.Bound) | 0.0216 | 0.0177 - 0.0264 | 10.2 | - |
| SD PROP LLOQ 0.0315 (Adj.Bound) | 0.159 | 0.12 - 0.197 | 12.4 | - |
| SD ADD LLOQ 0.0224 (Adj.Bound) | 0.0291 | 0.0202 - 0.0419 | 18.8 | - |
| SD PROP LLOQ 0.0224 (Adj.Bound) | 0.214 | 0.194 - 0.234 | 4.83 | |

| | | | | |
|---|---|---|---|---|
| ADD = additive, age = baseline age, C.I.95 = 95% confidence intervals, CV = coefficient of variation, F1 and F5 = bioavailability of intravitreal injections in ocular compartments, F6 = bioavailability of subcutaneous injections, HD = high dose (8 mg IVT cohorts), IIV = inter-participant variability, IV = intravenous, K20 = elimination rate of free aflibercept, K40 = elimination rate constant for adjusted bound aflibercept, K62 = rate of absorption from subcutaneous injection dosing compartment, K70 = elimination rate from tissue (platelet) compartment, KM = concentration of free aflibercept at half of maximum binding capacity with VEGF; KMK27 = concentration of free aflibercept at half of maximum binding capacity to platelets, LLOQ = lower limit of quantification, PROP = proportional, QE = inter-compartmental clearance between ocular compartment and central compartment of free aflibercept, QF1 and QF2 = inter-compartmental clearances of free aflibercept, RSE% = percent relative standard error, SC = subcutaneous, TALB = time varying albumin, TWGT = time varying body weight, V2 = central volume of free aflibercept in plasma, V3 and V8 = peripheral volumes of free aflibercept in tissues, V4 = central volume of adjusted bound aflibercept in plasma, VMK24 = maximum binding rate of free aflibercept to VEGF, VMK27 = maximum binding rate of aflibercept to platelets Estimates of fixed-effect parameters are presented in the natural scale; IIV are reported as variances around the log of the parameters or the logit of F6. Residual errors of IV and SC data not presented in the table for LLOQ=0.0156 (*σ* additive=0.00786, σproportional= 0.357) and LLOQ=0.0315 (*σ*additive=0.0206, *σ*proportional=0.167) were fixed in the final model to estimates from run463. C.I.95 and %RSE% for run431 were calculated from bootstrap. *η*-shrinkage: *η*K20= 54.2%, *η*V2,V4= 15.2%, *η*VMK24= 42.2%, *η*K40= 39.1%, *η*QE= 31.6%, *η*K62= 1e-10%, *η*F6= 17.7%. | | | | |

Concentrations of free and bound aflibercept in plasma were measured using validated enzyme-linked immunosorbent assay (ELISA) methods. The assay for bound aflibercept is calibrated using the VEGF:aflibercept standards, and the results are reported for bound aflibercept as weight per volume (*e.g.*, ng/mL or mg/L) of the VEGF:aflibercept complex. Therefore, to account for the difference in molecular weight and normalize the relative concentrations between free and bound aflibercept, the concentration of the bound aflibercept complex is adjusted by multiplying the bound aflibercept concentration by 0.717. This is to account for the presence of VEGF in the bound complex and report the complex in terms of mg/L (i.e., mass/volume) that are corrected for, and consistent with, the molar concentrations (referred to as adjusted bound aflibercept in this module). Herein, concentrations of aflibercept:VEGF complex are limited to the adjusted bound concentrations.

The concentration of bound aflibercept was normalized to determine the amount of aflibercept present in the bound aflibercept complex. The bound aflibercept complex consisted of 71.7% aflibercept and 28.3% human VEGF₁₆₅ based on the molecular weight of each component. Therefore, the concentration of the bound aflibercept complex was multiplied by 0.717 to yield the concentration of adjusted bound aflibercept (Equation 1). Total aflibercept was calculated by summing the plasma concentrations of free and adjusted bound aflibercept (Equation 2). $Adjusted bound aflibercept \left(mg/L\right) = Bound aflibercept \left(mg/L\right) \times 0.717$ $\begin{matrix}Total aflibercept \left(mg/L\right) = Sum of adjusted bound aflibercept \left(mg/L\right) + free \\ aflibercept \left(mg/L\right)\end{matrix}$ Time-varying body weight was a predictor of the central volumes for free and adjusted bound aflibercept (V2=V4), the peripheral volumes of free aflibercept in tissues (V3, and V8), and elimination rate of free aflibercept (K20) and adjusted bound aflibercept (K40). The effect of time-varying albumin was also a predictor of elimination rate of adjusted bound aflibercept (K40). Age and the effect of HD drug product versus aflibercept groups with doses ≤4 mg presented as the reference drug product were predictors of clearance from the ocular compartment (QE). The clearance of free aflibercept from the ocular compartment slowed with age, with an estimated exponent in the relationship of -1.53, resulting in clearance from the ocular compartment being approximately 25% slower for an 86 year-old (95^{th} percentile of age in the analysis population) participant than a 71 year-old (median age in analysis population) participant.

Following IVT administration, HD drug product was estimated to have 34.3% slower clearance from the ocular compartment compared to the reference IVT aflibercept drug product for doses ≤4 mg. This slower ocular clearance resulted in a longer duration of ocular exposure to free aflibercept in the ocular compartment for the HD drug product. Through PopPK covariate analysis, the 34% slower ocular clearance (QE) and longer duration of free aflibercept ocular exposure for HD drug product is statistically attributed to an "HD aflibercept drug product effect". The exact nature or attributes of the HD drug product responsible for the attenuated ocular clearance cannot be fully explained by increasing the dose alone.

Exposure-Response Analyses. An exposure-response analysis was conducted using the time to dose regimen modification (TTDRM). A KM (Kaplan-Meier) plot of TTDRM stratified by indication showed a statistically significant (p < 0.00001) difference in TTDRM between participants with AMD and participants with DME, per the logrank test. KM plots of TTDRM, stratified by quartiles of ocular clearance (QE) within indication, showed rank ordering of longer TTDRM by lower ocular clearance percentile. A Cox proportional hazard model that included indication, baseline CRT, and ocular clearance as predictors of DRM showed that the rate of DRM due to the HD drug product effect is 20.6% lower than would have been expected if the HD drug product had the same ocular clearance as the 2 mg aflibercept presented as the reference drug product.

The need for DRM is determined by the clinician objective measurements obtained during an office visit, at which time a participant's dosing regimen can be shortened due to suboptimal efficacy. Faster transit of aflibercept from the eye into the systemic circulation leads to earlier depletion of the drug from the ocular space and therefore a more rapid loss of efficacy. While there may be other factors affecting efficacy, such as disease progression, comorbidities, or variability in response, this analysis shows a statistically significant relationship between an independently determined PK parameter (ocular clearance) that describes the transit of aflibercept from the eye and a reduction in efficacy as indicated by an earlier retreatment (DRM) than anticipated based on clinical assessment via BCVA and CRT.

For those participants requiring a DRM, Cox proportional hazard modeling was performed to evaluate factors that may contribute to the need for a reduction in the dosing interval. The results of these analyses estimate a 260% higher rate for DRMs for participants with nAMD compared to participants with DME and DR. After accounting for indication (nAMD or DME and DR), ocular clearance of free aflibercept and baseline CRT were identified as significant covariates contributing to the need for DRM. Within an indication (nAMD or DME and DR), for participants with the same ocular clearance of free aflibercept, a 52.8% higher rate of DRM is predicted for participants at the 75^{th} percentile vs 25^{th} percentile of baseline CRT. Similarly, for participants with the same baseline CRT, a 32.9% higher rate of DRM is predicted for participants at the 75^{th} vs 25^{th} percentile of ocular clearance of free aflibercept. The results of these analyses also estimate that the lower ocular clearance for HD drug product resulted in a 20.6% lower rate of DRM than would have been expected if the HD drug product had the same ocular clearance as 2 mg aflibercept.

Comparison of Pharmacokinetics Across Studies in Participants with Neovascular Age-Related Macular Degeneration or Diabetic Macular Edema. In the clinical development of HD aflibercept for treatment of AMD and DME, a dosage regimen of 8 mg IVT (3 initial monthly doses followed by q12w or q16w IVT dosing) was evaluated and compared to an aflibercept 2

mg IVT dosage regimen (3 or 5 initial monthly doses followed by q8w or q12w IVT dosing) in the clinical studies CANDELA, PULSAR, and PHOTON. This allowed for a direct comparison of the systemic exposures of free and adjusted bound aflibercept across the 3 studies. CANDELA and PULSAR studies included participants with nAMD while PHOTON study included participants with DME and DR.

Following single IVT administration of aflibercept 2 mg or HD aflibercept, the concentration-time profiles of free and adjusted bound aflibercept in plasma in participants who underwent dense sample collection for systemic drug concentrations (dense PK sub-study) after the initial dosing of aflibercept 2 mg or HD aflibercept presented as the HD drug product, respectively, were consistent between the 3 studies in participants with nAMD or DME (Figure 34).

The consistency of the concentration-time profiles for free and adjusted bound aflibercept between the nAMD and DME populations is further supported by Population PK analysis (Figure 35). Population PK estimated *post-hoc* concentration-time profiles and PK parameters for combined nAMD and DME populations following single IVT administration of 2 mg aflibercept or HD aflibercept are provided in Figure 35 and in Table 1-37 and Table 1-38.

**Table 1-37. Summary of Post-hoc Simulated Pharmacokinetic Parameters for Free Aflibercept in Plasma after Single Dose IVT Administration in the Combined nAMD and DME Population Treated Only in the Study Eye and Without Study Eye Dosing Modifications in the Dense PK Sub-studies (DPKS)**

| | **Aflibercept** | | | **HD Aflibercept** | |
|---|---|---|---|---|---|
| | **2 mg IVT (N = 31)** | | | **8 mg IVT (N = 50)** | |
| **PK Parameters** | **Unit** | **Mean (SD)** | **Median** | **Mean (SD)** | **Median** |
| **AUC₀₋₂₈** | **mg x day/L** | 0.282 (0.189) | 0.238 | 2.55 (2.31) | 2 |
| **Cₘₐₓ** | **mg/L** | 0.0394 (0.0391) | 0.0251 | 0.304 (0.267) | 0.222 |
| **C_{trough},₂₈** | **mg/L** | < LLOQ (0) | < LLOQ | < LLOQ (0.00853) | < LLOQ |
| **tₘₐₓ** | **day** | 2.26 (0.783) | 2.16 | 2.8 (1.08) | 2.89 |

| | | | | | |
|---|---|---|---|---|---|
| AUC = area under the concentration-time curve, Cₘₐₓ = maximum (peak) concentration for a 28-day interval following dosing, C_{trough} = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, PK = pharmacokinetics, SD = Standard deviation, tₘₐₓ = median time to peak concentration; Note: Participants who had fellow eye treatment before day 28 are excluded. | | | | | |

**Table 1-38. Summary of Post-hoc Simulated Pharmacokinetic Parameters for Adjusted Bound Aflibercept in Plasma after Single Dose IVT administration in the Combined nAMD and DME Population Treated Only in the Study Eye and Without Study Eye Dosing Modifications in the Dense PK Sub-studies (DPKS)**

| | | **Aflibercept** | | **HD Aflibercept** | |
|---|---|---|---|---|---|
| | | **2 mg IVT (N = 31)** | | **8 mg IVT (N = 50)** | |
| **PK Parameters** | **Unit** | **Mean (SD)** | **Median** | **Mean (SD)** | **Median** |
| **AUC₀₋₂₈** | **mg x day/L** | 3.07 (1.31) | 3.05 | 10.8 (6.14) | 9.03 |
| **Cₘₐₓ** | **mg/L** | 0.142 (0.0616) | 0.139 | 0.507 (0.282) | 0.434 |
| **C_{trough},₂₈** | **mg/L** | 0.105 (0.0393) | 0.0994 | 0.386 (0.21) | 0.338 |
| **tₘₐₓ** | **day** | 14.8 (5.65) | 13.7 | 15.5 (5.22) | 15.8 |

| | | | | | |
|---|---|---|---|---|---|
| AUC = area under the concentration-time curve, Cₘₐₓ = maximum (peak) concentration for a 28-day interval following dosing, C_{trough} = trough concentration, DME = diabetic macular edema, DPKS = dense pharmacokinetic sub-studies, IVT = intravitreally, nAMD = neovascular age-related macular degeneration, PK = pharmacokinetics , SD = standard deviation, tₘₐₓ = median time to peak concentration; Note: Participants who had fellow eye treatment before day 28 are excluded. | | | | | |

The corresponding observed and Population PK estimated *post-hoc* concentration-time profiles and PK parameters for participants with nAMD or DME are provided in Figure 38, Figure 39, Figure 40, Table 1-39 and Table 1-40.

**Table 1-39. Summary of Simulated Pharmacokinetic Parameters for Free Aflibercept in Plasma after Single Dose IVT Administration in Participants with nAMD or DME Treated Only in the Study Eye and Without Study Eye Dosing Modifications in the Dense PK Sub-studies (DPKS)**

| | | | **2 mg IVT** | | **8 mg IVT** | | |
|---|---|---|---|---|---|---|---|
| **PK Parameters** | **Unit** | **N** | **Mean (SD)** | **Median** | **N** | **Mean (SD)** | **Median** |
| **nAMD participants** | | | | | | | |
| **AUC₀₋₂₈** | mg x day/L | 21 | 0.302 (0.223) | 0.258 | 29 | 2.77 (2.77) | 1.95 |
| **Cₘₐₓ** | mg/L | | 0.0419 (0.0439) | 0.0258 | | 0.306 (0.302) | 0.172 |
| **C_{trough},₂₈** | mg/L | | < LLOQ (0) | < LLOQ | | < LLOQ (0.0094) | < LLOQ |
| **tₘₐₓ** | day | | 2.19 (0.606) | 2.16 | | 2.97 (1.08) | 3.05 |

| **DME participants** | | | | | | | |
|---|---|---|---|---|---|---|---|
| **AUC₀₋₂₈** | mg x day/L | 10 | 0.238 (0.0732) | 0.236 | 21 | 2.25 (1.45) | 2.17 |
| **Cₘₐₓ** | mg/L | | 0.0343 (0.0275) | 0.0212 | | 0.302 (0.216) | 0.265 |
| **C_{trough},₂₈** | mg/L | | < LLOQ (0) | < LLOQ | | < LLOQ (0.00732) | < LLOQ |
| **tₘₐₓ** | day | | 2.41 (1.09) | 2.23 | | 2.56 (1.06) | 2.36 |

| | | | | | | | |
|---|---|---|---|---|---|---|---|
| AUC₀₋₂₈ = area under the concentration-time curve, Cₘₐₓ = maximum (peak) concentration for a 28-day interval following dosing, C_{trough},₂₈ = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, PK = pharmacokinetic, SD = Standard deviation, tₘₐₓ = median time to peak concentration. Note: Participants who had fellow eye treatment before day 28 are excluded. | | | | | | | |

**Table 1-40. Summary of Simulated Pharmacokinetic Parameters for Adjusted Bound Aflibercept in Plasma after Single Dose IVT administration in Participants with nAMD or DME Treated Only in the Study Eye and Without Study Eye Dosing Modifications in the Dense PK Sub-studies (DPKS)**

| | | **Adjusted Bound Aflibercept** | | | | | |
|---|---|---|---|---|---|---|---|
| | | **2 mg IVT** | | | **8 mg IVT** | | |
| **PK Parameters** | **Unit** | **N** | **Mean (SD)** | **Median** | **N** | **Mean (SD)** | **Median** |
| **nAMD participants** | | | | | | | |
| **AUC₀₋₂₈** | mg x day/L | 21 | 3.35 (1.44) | 3.16 | 29 | 11.8 (7.17) | 11 |
| **Cₘₐₓ** | mg/L | | 0.155 (0.0686) | 0.144 | | 0.558 (0.329) | 0.51 |
| **C_{trough},₂₈** | mg/L | | 0.113 (0.0418) | 0.113 | | 0.439 (0.23) | 0.415 |
| **tₘₐₓ** | day | | 14.4 (4.89) | 13.1 | | 16.9 (5.26) | 17.2 |
| **DME participants** | | | | | | | |
| **AUC₀₋₂₈** | mg x day/L | 10 | 2.46 (0.726) | 2.43 | 21 | 9.33 (4.06) | 8.39 |
| **Cₘₐₓ** | mg/L | | 0.115 (0.0317) | 0.117 | | 0.438 (0.187) | 0.4 |
| **C_{trough},₂₈** | mg/L | | 0.088 (0.0281) | 0.09 | | 0.314 (0.156) | 0.25 |
| **tₘₐₓ** | day | | 15.6 (7.21) | 15.4 | | 13.7 (4.65) | 12.9 |

| | | | | | | | |
|---|---|---|---|---|---|---|---|
| AUC₀₋₂₈ = area under the concentration-time curve, Cₘₐₓ = maximum (peak) concentration for a 28-day interval following dosing, C_{trough},₂₈ = trough concentration, DME = diabetic macular edema, DPKS = dense pharmacokinetic sub-studies, IVT = intravitreally, LLOQ = lower limit of quantitation, n = number of participants, nAMD = neovascular age-related macular degeneration, SD = standard deviation, tₘₐₓ = median time to peak concentration; Note: Participants who had fellow eye treatment before day 28 are excluded. | | | | | | | |

Following single IVT administration of 2 mg aflibercept or HD aflibercept presented as HD drug product, the concentration-time profiles of free aflibercept are characterized by an initial phase of increasing concentrations, as the drug moved from the ocular space into systemic circulation, followed by a mono-exponential elimination phase. The concentration time profiles of adjusted bound aflibercept in plasma are characterized by a slower attainment of Cₘₐₓ compared to free aflibercept. Following attainment of Cₘₐₓ, a sustained plateau of the concentration-time profiles of adjusted bound aflibercept in plasma was observed until approximately the end of the first dosing interval (Figure 34, Figure 35).

For participants who underwent dense blood sample collection for systemic drug concentrations across the CANDELA, PULSAR, and PHOTON studies, after the initial dosing of 2 mg IVT aflibercept (n = 34), observed concentrations of free aflibercept were detectable in 15 (44.1%) participants by week 1 and in 3 (8.8%) participants by week 2. For participants who underwent dense blood sample collection for systemic drug concentrations after the initial dosing of 8 mg IVT aflibercept (n = 54), observed concentrations of free aflibercept were detectable in 46 (85.2%) participants by week 1 and in 44 (77.8%) participants by week 2.

The observed and Population PK simulated free and adjusted bound aflibercept concentrations in plasma for up to 48 weeks are presented for the combined nAMD and DME population (Figure 36), and the nAMD (Figure 41) and DME (Figure 42) populations. Based on the Population PK analysis, the median time for free aflibercept concentrations to reach LLOQ following HDq12 or HDq16 was 3.5 weeks, which is more than double the median time needed to reach LLOQ (1.5 weeks) following aflibercept 2q8 (Table 1-41).

**Table 1-41. Summary of Model-Predicted Time to LLOQ of Free Aflibercept in Plasma Following IVT for Participants With nAMD and DME, Combined**

| **Regimen** | **Mean (SD) Week** | **Median (90% PI) Week** |
|---|---|---|
| **2q8** | 1.58 (0.712) | 1.5 (0.524, 2.82) |
| **HDq12** | 3.81 (1.61) | 3.51 (1.83, 6.81) |
| **HDq16** | 3.79 (1.58) | 3.50 (1.83, 6.73) |

| | | |
|---|---|---|
| Model-predicted time = time after a single IVT dose of the 2q8, HDq12 or HDq16 regimens. 2q8 = aflibercept 2 mg administered every 8 weeks, after 3 initial injections at 4-week intervals, DME = diabetic macular edema, HDq12 = aflibercept 8 mg administered every 12 weeks following 3 initial monthly injections, HDq16 = aflibercept 8 mg administered every 16 weeks following 3 initial monthly injections, IVT = intravitreally, LLOQ = lower limit of quantification, nAMD = neovascular age related macular degeneration, PI = prediction interval, SD = standard deviation | | |

The longer duration of systemic exposure to free aflibercept following HDq12 and HDq16 compared to the 2 mg aflibercept is attributed to not only a higher administered dose and nonlinear systemic target-mediated elimination, but also to a 34% slower ocular clearance of free aflibercept. The 34% slower ocular clearance of free aflibercept for HD aflibercept is attributed to a HD drug product effect which was identified as a statistically significant covariate in the Population PK model.

Ocular Elimination. Based on the Population PK analysis, HD aflibercept, presented as the HD drug product, was estimated to have a 34% slower clearance from the ocular compartment compared to the lower IVT doses of aflibercept (≤ 4 mg doses) that was presented as the standard, or reference drug product. The median time for the amount of free aflibercept to reach the adjusted LLOQ [the adjusted LLOQ imputes the LLOQ of free aflibercept in from the assay in plasma (that is, 0.0156 mg/L) times the assumed volume of the study eye compartment in the PK model (that is, 4 mL)] in the ocular compartment was estimated using Population PK simulation analyses, after a single 2 mg or 8 mg IVT dose. In the combined nAMD and DME population, the median time for the amount of free aflibercept to reach the adjusted LLOQ in the ocular compartment increased from 8.71 weeks after a 2 mg IVT dose to 15 weeks after an 8 mg IVT dose (i.e., the duration of free aflibercept ocular exposure following HD drug product is extended by approximately 6 weeks relative to 2 mg drug product). The slower ocular clearance and longer duration of free aflibercept ocular exposure for HD aflibercept are attributed to an HD aflibercept drug product effect. Assuming no HD aflibercept drug product effect (*i.e.*, that the 8 mg IVT dose has the same ocular clearance as the 2 mg IVT dose), the Population PK simulated median time for the amount of free aflibercept to reach the adjusted LLOQ in the ocular compartment was only 10 weeks for 8 mg aflibercept, which is only 1.3 weeks longer than that for 2 mg aflibercept (Figure 37).

As the PULSAR and PHOTON studies were designed to assess non-inferiority of the HDq12 and HDq16 regimens versus the 2q8 regimen, it was of interest to estimate how long it takes for the amount of free aflibercept in the ocular compartment for the HDq12 and HDq16 regimens to reach the same amount of free aflibercept remaining in the ocular compartment for the 2q8 regimen at the end of an 8-week dosing interval (2q8 target). Using a modified approach, using Population PK simulation analyses in the combined nAMD and DME population, the median time for HDq12 and HDq16 regimens to reach the 2q8 target in the ocular compartment after single IVT administration was 14 weeks, suggesting that the HD aflibercept regimens may provide a 6-week longer duration of efficacy than the 2q8 regimen. In contrast, if there were no HD aflibercept drug product effect, the Population PK simulated median time for the amount of free aflibercept to reach the 2q8 target in the ocular compartment would be only 9.21 weeks for an 8 mg dose, representing an extension of only 1.21 weeks relative to the 2q8 regimen, and is consistent with the prior example.

High-Dose Aflibercept Drug Product. The totality of the composition of the HD drug product used to deliver the 8 mg dose is different from that for the 2 mg aflibercept IVT dose. Based on Population PK analysis, the HD aflibercept drug product is a statistically significant predictor of ocular clearance of free aflibercept that results in a slower ocular clearance for the HD aflibercept versus 2 mg aflibercept when administered by the IVT route. (Table 1-42). The slower ocular clearance and higher molar dose for the HD aflibercept drug product results in a longer duration of ocular exposure to free aflibercept compared to the 2 mg IVT dose. The slower ocular clearance of the HD aflibercept drug product is predicted to provide a 6-week longer duration of efficacy compared to 2q8, as the time to achieve the free aflibercept amount in the ocular compartment for the 2q8 regimen at the end of an 8-week dosing interval occurs 6 weeks later for the HD aflibercept drug product. Consistent with these predictions, the HDq12 and HDq16 regimens demonstrated noninferiority to the 2q8 regimen in the PHOTON (for DME only) and PULSAR studies. Correspondingly, a slower ocular clearance for the HD aflibercept drug product contributes in part to a longer duration of systemic exposure to free aflibercept for HD aflibercept versus the 2 mg IVT dose. The slower ocular clearance for HD aflibercept is attributed to a difference in the HD aflibercept drug product, not just an increase in the IVT dose from 2 mg to 8 mg. These results were further confirmed by a sensitivity analysis conducted in the final model.

**Table 1-42. Comparison of Clearance from the Ocular Compartment (QE) (Mean [95% Cl]) of Aflibercept for HD Aflibercept and 2 mg Aflibercept**

| **Dose Group** | **Clearance from the Ocular Compartment (QE)** | **k = QE/0.004 Mean (95% CI (day-1)** |
|---|---|---|
| | **Mean (95% CI)** | |
| | **(mL/day)** | |
| **2 mg Aflibercept** | 0.624 (0.577 - 0.674) | 0.156 (0.144 - 0.169) |
| **HD 8 mg Aflibercept** | 0.41 (0.367 - 0.458) | 0.102 (0.0916 - 0.115) |

| | | |
|---|---|---|
| QE = inter-compartmental clearance between ocular compartment and central compartment of free aflibercept, 95% CI of parameters are provided. | | |

Pharmacokinetic Conclusions. The concentration time profiles of free and adjusted bound aflibercept in plasma after the initial dose of HD aflibercept by IVT administration were consistent between all studies in participants with nAMD or DME. Population PK analysis confirmed no relevant differences in PK between the nAMD and DME populations, and therefore all subsequent analyses are presented for the combined nAMD and DME population.

Following the initial monthly IVT dose, the observed concentration-time profile of free aflibercept in plasma is characterized by an initial phase of increasing concentrations as the drug is absorbed from the ocular space into the systemic circulation, followed by a mono-exponential elimination phase. The longer duration of systemic exposure to free aflibercept for HD aflibercept is attributed to not only a higher administered dose and non-linear systemic target mediated elimination but also to a 34% slower ocular clearance of free aflibercept, which is statistically attributed to the HD drug product as a covariate in the expanded PopPK model. This slower than expected ocular clearance of free aflibercept when presented as the HD aflibercept drug product is simulated to provide a 6-week longer duration of efficacy compared to 2q8, as the time to achieve the free aflibercept amount in the ocular compartment for the 2q8 regimen at the end of an 8-week dosing interval occurs 6 weeks later for the HD aflibercept drug product. Consistent with these simulations for the 8mg presented as the HD drug product, the HDq12 and HDq16 regimens demonstrated noninferiority (at a longer treatment interval) to the 2q8 regimen presented as the reference drug product in the predefined statistical analysis plan for both the PHOTON (for DME only) and PULSAR phase 3 studies.

Based on expanded population PK analysis, following single IVT doses of 2 mg aflibercept and HD aflibercept, systemic exposures of free aflibercept (AUC₀₋₂₈ and Cₘₐₓ) in the combined nAMD and DME population increase in a greater than dose-proportional manner (approximately 9.0-fold and 7.7-fold). These results demonstrate and are consistent with the known nonlinear PK for free aflibercept. Bioavailability of free aflibercept following IVT administration is estimated to be approximately 72%, and the total volume of distribution of free aflibercept after IV administration is estimated to be approximately 7 L.

Following 3 initial monthly HD aflibercept doses, the population PK simulated mean accumulation ratio of free and adjusted bound aflibercept in plasma based on AUC was 1.16 and 2.28 in the combined DME and nAMD population. After the 3 initial monthly doses of HD aflibercept (presented as the HD drug product), no further accumulation of either free or adjusted bound aflibercept in plasma occurs as the dosing interval is extended from every 4 weeks to every 12 weeks or 16 weeks resulting in a decline in systemic concentrations of both free and adjusted bound aflibercept.

Amongst the covariates evaluated in the Population PK analysis, body weight was the covariate with the greatest impact on systemic exposures to free and adjusted bound aflibercept. For participants in the lowest quintile of body weight (38.1 kg to 64.5 kg), the predicted impact on systemic exposures (Cₘₐₓ and AUCₜₐᵤ) was modest, with 27% to 39% higher exposures to free aflibercept and 25% to 27% higher exposures to adjusted bound aflibercept when compared to the reference body weight range (73.5 to 83.5 kg). The effects of other covariates (age, albumin, disease population, and race, which included evaluation of Japanese race) on systemic exposures (Cₘₐₓ, AUCₜₐᵤ) to free and adjusted bound aflibercept were small (<25% increase in exposure for covariate subgroups relative to the reference group), with several of these other covariate effects correlating with a consistent trend in body weight. All of these covariates were independent of the HD drug product effect on ocular clearance and did not confound the interpretation of the HD drug product effect on the ocular clearance. No dosage adjustments of HD aflibercept are warranted based on the assessed covariates.

Mild to severe renal impairment also had a small impact on free aflibercept systemic exposures, as the increase in free aflibercept Cₘₐₓ and AUCₜₐᵤ in these participants was less than approximately 28% compared to participants with normal renal function. Adjusted bound aflibercept systemic exposures in participants with mild to severe renal impairment ranged from 13% to 39% higher compared to participants with normal renal function. Here too, the perceived impact of renal impairment is best explained by the associated decrease in body weight with increasing renal impairment. Mild hepatic impairment had no effect on systemic exposures to free and adjusted bound aflibercept. No dosage adjustments of aflibercept are warranted for these populations.

Model-Based Exposure-Response Analysis for Proportion of Participants Requiring Dose Regimen Modification Cox proportional hazard modeling was performed to evaluate factors that may contribute to the need for a reduction in the dosing interval. Within any one specific patient population, nAMD, DME (with and without DR), ocular clearance of free aflibercept and baseline CRT were identified as significant predictors of time to DRM. Within an indication (nAMD or DME (with and without DR)), for participants with the same ocular clearance of free aflibercept, a 52.8% higher rate of DRM is modeled for participants at the 75^{th} vs 25^{th} percentile of baseline CRT. Similarly, for participants with the same baseline CRT, a 32.9% higher rate of DRM is modeled for participants at the 75^{th} vs 25^{th} percentile of ocular clearance of free aflibercept, corresponding to those participants who are predicted to have the lowest aflibercept concentration in the eye. These results are shown in Table 1-43. The outcomes of these analyses also estimate that the slower ocular clearance for HD aflibercept, attributable to a HD drug product effect, results in a 20.6% lower rate of DRM than would have been expected if the HD drug product had the same ocular clearance as 2 mg aflibercept presented as the reference drug product.

**Table 1-43. Hazard Ratio Contrasts for Time to DRM Model**

| **Effect** | **Hazard Ratio** |
|---|---|
| Baseline CRT | 1.53 (1.34 - 1.75) |
| Ocular Clearance (QE) | 1.33 (1.18 - 1.49) |
| Indication AMD Participants: DME Participants | 3.6 (2.56-5.06) |

| | |
|---|---|
| AMD = age-related macular degeneration, CRT = central retinal thickness, DME = diabetic macular edema, DRM = dose regimen modification, QE = ocular clearance | |

Dose-Response and Exposure-Response Conclusions. As the IVT dose increased from 2 mg of aflibercept to 8 mg of HD aflibercept, no further increase in PD effect (decrease in CRT) was observed 4 weeks after each initial q4w dose through 12 weeks, in either the nAMD or DME population. Despite 2 mg of aflibercept (as reference drug product) and 8 mg of HD aflibercept (as HD drug product) having similar PD effect during the initial 3 x q4w dosing period, the 8 mg HD drug product provided a longer duration of pharmacological effect in the maintenance phase compared to 2 mg aflibercept. In nAMD participants, the small fluctuations in CRT or CST during a maintenance dosing interval attenuated over time for all dosing regimens, with only minor numerical differences observed between treatment groups. For DME participants, a greater reduction in CRT was observed from weeks 16 to 20 for 2q8 compared to both HD aflibercept regimens (HDq12 and HDq16). This is attributable to a difference in the number of doses administered during this time period, with the 2q8 regimen receiving 2 additional initial q4w doses at weeks 12 and 16 compared to the HD aflibercept regimens which received their last initial q4w dose at week 8. These differences in CRT did not translate into any meaningful difference in mean BCVA response. The fluctuations in CRT response over the course of a maintenance dosing interval attenuated over time for all dosing regimens. For participants with nAMD or DME, the HDq12 and HDq16 regimens provided rapid and durable response in CRT and BCVA over 48 weeks of treatment, with the majority of participants maintaining their randomized HDq12 (79% nAMD; 91% DME) and HDq16 (77% nAMD; 89% DME) treatment regimens, without the need for DRM. Ocular clearance of free aflibercept and baseline CRT were identified as significant covariates contributing to the need for DRM. Higher ocular clearance of free aflibercept and higher baseline CRT (indicative of more severe disease) were associated with an increased rate of DRM. The slower ocular clearance for HD aflibercept, attributable to a HD drug product effect, is estimated to result in a 20.6% lower rate of DRM compared to HD aflibercept if the same ocular clearance was observed as the 2 mg aflibercept when presented as the reference drug product.

Overall Clinical Pharmacology Conclusions. Consistent with the known target-mediated kinetic properties exhibited at low plasma concentrations of aflibercept, free aflibercept exhibited nonlinear systemic PK over the 2 mg to 8 mg IVT dose range. Following the initial IVT dose, the concentration-time profile for free aflibercept in plasma is characterized by an initial absorption phase as drug moves from the ocular space into the systemic circulation. This absorption phase is followed by a mono-exponential elimination phase. The concentration time profile of adjusted bound aflibercept in plasma following the initial IVT dose is characterized by a slower attainment of Cₘₐₓ (tₘₐₓ) compared to free aflibercept, after which the concentrations are sustained or slightly decrease until the end of the dosing interval.

Analyses of observed PK by cross-study comparison and by Population PK analyses suggested similar systemic PK in the nAMD and DME populations. Following IVT administration, Population PK methods estimate the bioavailability of free aflibercept at 72%, a median tₘₐₓ of 2.89 days, and mean Cₘₐₓ of 0.304 mg/L for the 8 mg dose of HD aflibercept. As the aflibercept IVT dose increased from 2 mg to 8 mg and the treatment changes from 2 mg aflibercept (presented as the reference drug product) to 8 mg HD aflibercept (presented as the HD drug product), consistent with the known target-mediated related nonlinear PK of free aflibercept mean AUC₀₋₂₈ and Cₘₐₓ for free aflibercept increased in a greater than dose-proportional manner. After IV administration, free aflibercept has a low total volume of distribution of 7 L, indicating distribution largely in the vascular compartment. Following 3 initial monthly HD aflibercept IVT doses, the mean accumulation ratio of free and adjusted bound aflibercept in plasma based on AUC is 1.16 and 2.28. After the 3 initial monthly doses of HD drug product, no further accumulation of either free or adjusted bound aflibercept in plasma occurred as the dosing interval is extended from every 4 weeks to every 12 weeks or 16 weeks resulting in an expected decline in systemic concentrations of both free and adjusted bound aflibercept.

The longer duration of systemic exposure to free aflibercept for HD aflibercept is attributed to not only a higher administered dose and nonlinear systemic target-mediated elimination, but also to a 34% slower ocular clearance of free aflibercept. This 34% slower ocular clearance of free aflibercept for HD aflibercept is attributed to a HD drug product effect, which was identified as a statistically significant covariate in the Population PK model. Based on the extended PopPK model, the slower ocular clearance of the HD aflibercept drug product provides a 6-week longer duration of efficacy compared to 2q8 when presented as the reference drug product. Resulting from this unexpected and non-obvious slower ocular clearance, was a longer than expected ocular residence time, leading to a greater than expected proportion of patients able to be maintained on the longer dosing intervals of q12 and q16 with HD drug product. Consistent with these predictions, the HDq12 and HDq16 regimens demonstrated non-inferiority to the 2q8 regimen in the PHOTON and PULSAR studies.

Body weight was the covariate with the greatest impact on systemic exposures to free and adjusted bound aflibercept. For participants in the lowest quintile of body weight (38.1 to 64.5 kg), the predicted impact on free aflibercept Cₘₐₓ and AUCₜₐᵤ was modest, with 27% to 39% higher exposures and 25% to 27% higher for adjusted bound aflibercept when compared the reference body weight range (73.5 to 83.5 kg). The effects of other covariates (age, albumin, disease population. and race, which included evaluation of Japanese race) on systemic exposures (Cₘₐₓ, AUCₜₐᵤ) to free and adjusted bound aflibercept were small (<25% increase in exposure for covariate subgroups relative to the reference group). These other covariates did not confound the assessment of the effect of HD drug product on ocular clearance. No dosage adjustments of aflibercept are warranted based on the above findings.

No formal studies were conducted in special populations (*e.g.*, participants with renal or hepatic impairment) because like most therapeutic proteins, the large molecular weight of aflibercept (approximately 115 kDa) is expected to preclude elimination via the kidney, and its metabolism is expected to be limited to proteolytic catabolism to small peptides and individual amino acids. Mild to severe renal impairment had a small impact on free aflibercept systemic exposures, as the increase in free aflibercept Cₘₐₓ and AUCₜₐᵤ in these participants was less than approximately 28% compared to participants with normal renal function. Adjusted bound aflibercept systemic exposures in participants with mild to severe renal impairment ranged from 13% to 39% higher compared to participants with normal renal function. The perceived impact of renal impairment is explained by the associated decrease in body weight with increasing renal impairment. Mild hepatic impairment had no effect on systemic exposures to free and adjusted bound aflibercept. No dosage adjustments of aflibercept are warranted in these populations.

Dose-response analyses of CRT performed in the CANDELA, PULSAR, and PHOTON studies indicated no further increase in PD effect for 2 mg aflibercept and HD aflibercept IVT 4 weeks after each initial q4W dose through 12 weeks. Despite the 2 mg aflibercept and HD aflibercept having similar PD effect during the initial q4w dosing period, the HD aflibercept drug product provided a longer duration (up to 16 weeks) of pharmacological effect in the maintenance phase than the 2 mg dose presented as the reference drug product (up to 8 weeks).

For participants with nAMD or DME, the HDq12 and HDq16 regimens provided rapid and durable response in CRT and BCVA over 48 weeks of treatment, with the majority of participants maintaining their randomized HDq12 (79% nAMD; 91% DME) and HDq16 (77% nAMD; 89% DME) treatment regimens, without the need for DRM.

Ocular clearance of free aflibercept and baseline CRT were identified as significant covariates contributing to the need for DRM. Higher ocular clearance and higher baseline CRT (indicative of more severe disease) were associated with an increased rate of DRM. For HD aflibercept, the slower ocular clearance and longer duration of ocular exposure to free aflibercept, attributable to the HD drug product effect, have been identified in an exposure-response analysis to result in a reduction of DRM of 20.6%.

Immunogenicity of HD aflibercept administered IVT was low across all treatment groups for both nAMD and DME participants. During the 48-week treatment with aflibercept administered IVT, the incidence of ADA in the combined 8 mg HD aflibercept treatment group was 2.7% (25/937 participants with nAMD or DME). None of the TE ADA positive samples were found to be positive in the NAb assay. Based on the lack of impact of ADA on concentrations of aflibercept in plasma, no effect on efficacy is anticipated. Positive responses in the ADA assays were not associated with significant AEs.

Overall, the clinical pharmacology data support the proposed aflibercept dosing regimens of 8 mg every 8 to 16 weeks after 3 initial monthly doses for the treatment of adults with nAMD, DME (with and without DR).

### Immunogenicity

Samples for anti-drug antibody (ADA) examinations were taken at baseline and subsequently at Week 48 and the results are presented based on the Week 60 database. The samples were analyzed using a validated, electrochemiluminescence bridging assay to detect the presence of ADA.

Out of the 833 participants in the ADA analysis set (AAS), a total of 43 participants had positive samples in the ADA assay at any time (including baseline); 11 participants in the 2q8 group, 19 participants in the HDq12 group, and 13 participants in the HDq16 group (Table 1-44).

A total of 24 participants participating in this study exhibited a treatment-emergent ADA response; 4 participants in the 2q8 group, 11 participants in the HDq12 group, and 9 participants in the HDq16 group. The incidence of treatment-emergent immunogenicity in the 2q8, HDq12 and HDq16 groups was approximately 1.5%, 3.9%, and 3.2%, respectively. No treatment-boosted ADA was observed, and all treatment-emergent responses were low titer (< 1000). None of the samples that were positive in the ADA assay demonstrated neutralizing activity (Table 1-44).

**Table 1-44. Summary of ADA status, ADA category, maximum titer category, and NAb status through Week 60**

| Visit | Category | | | **2q8** | **HDq12** | **HDq16** | **All HD** |
|---|---|---|---|---|---|---|---|
| | | | | N = 273 (100%) | N = 283 (100%) | N = 277 (100%) | N = 560 (100%) |
| Week 48 | Total ADA subjects, n (%) | | | 273 (100%) | 283 (100%) | 277 (100%) | 560 (100%) |
| | | Negative (a) | | 262 (96.0%) | 263 (92.9%) | 263 (94.9%) | 526 (93.9%) |
| | | | Pre-existing immunoreactivity (b) | 7 (2.6%) | 8 (2.8%) | 4 (1.4%) | 12 (2.1%) |
| | | Treatment-boosted (c) | | 0 | 0 | 0 | 0 |
| | | | Treatment-emergent positive (d) | 4 (1.5%) | 11 (3.9%) | 9 (3.2%) | 20 (3.6%) |
| | | Missing | | 0 | 1 (0.4%) | 1 (0.4%) | 2 (0.4%) |
| | Treatment-emergent positive or Treatment-boosted | | | | | | |
| | | Low (< 1000) | | 4 | 11 | 9 | 20 |
| | | Moderate (1000-10000) | | 0 | 0 | 0 | 0 |
| | | High (> 10000) | | 0 | 0 | 0 | 0 |

| | | | | | | | |
|---|---|---|---|---|---|---|---|
| ADA = anti-drug antibody, NAb = neutralizing antibody (a) ADA Negative: negative response in the ADA assay at all time points and those that exhibited a pre-existing response, as defined in (b), regardless of any missing samples. (b) Pre-existing immunoreactivity: either a positive response in the ADA assay at baseline with all post first dose ADA results negative OR a positive response at baseline with all post first dose ADA responses less than 4-fold of baseline titer levels. (c) Treatment-boosted ADA response: positive response post first dose that is greater than or equal to 4-fold over baseline titer level, when baseline results were positive. (d) Treatment-emergent positive: ADA-positive response post first dose when baseline results were negative or missing. (e) Samples that tested negative for ADA were not assayed in the NAb assay and the corresponding NAb result was imputed as negative and included as such in the NAb analysis set. Participants in the NAbAS with multiple post-dose ADA results which consisted of both imputed NAb-negative result(s) for ADA negative samples and only missing NAb results for all ADA-positive result(s), were set to NAb negative. Participants in the NAbAS that have at least one post-dose positive NAb analysis result were set to NAb positive even if other NAb results were missing. | | | | | | | |

Overall, the low level of immunogenicity was not considered clinically relevant. In participants with treatment-emergent ADA, one participant in the HDq12 group had an AE of mild iritis which was not considered to be related to study treatment by the investigator.

### Treatment Exposure

A summary of exposure to study treatment and duration of treatment in the SAF is presented in Table 1-45.

The mean number of active injections in the SAF population through Week 60 was 8.5, 6.9 and 6.0 in the 2q8, HDq12 and HDq16 treatment groups, respectively (Table 1-45). For the 925 participants in the SAF considered as completers of 60 weeks of study treatment (i.e., SAF completers), the mean number of active injections was 8.8, 7.1 and 6.2 in the 2q8, HDq12 and HDq16 treatment groups, respectively. The observed decrease in the mean and median number of active injections and the corresponding increase in the number of sham injections from the 2q8 group to the HDq12 and HDq16 group reflects the protocol-driven increase in treatment intervals across these groups.

**Table 1-45. Exposure to Study Treatment: Through Week 48 and Week 60 (Safety Analysis Set)**

| | | | **2q8** | **HDq12** | **HDq16** | **All HD** |
|---|---|---|---|---|---|---|
| | | | N = 336 (100%) | N = 335 (100%) | N = 338 (100%) | N = 673 (100%) |
| **Week48** | | | | | | |
| | | Total number of active injections, n | 2267 | 1986 | 1703 | 3689 |
| | | Total number of sham injections, n | 1212 | 1515 | 1793 | 3308 |

| Number of active injections, n (%) | | | | | | |
|---|---|---|---|---|---|---|
| | 1 | | 1 (0.3%) | 2 (0.6%) | 2 (0.6%) | 4 (0.6%) |
| | 2 | | 1 (0.3%) | 2 (0.6%) | 1 (0.3%) | 3 (0.4%) |
| | 3 | | 4 (1.2%) | 3 (0.9%) | 9 (2.7%) | 12 (1.8%) |
| | 4 | | 6 (1.8%) | 7 (2.1%) | 22 (6.5%) | 29 (4.3%) |
| | 5 | | 6 (1.8%) | 22 (6.6%) | 263 (77.8%) | 285 (42.3%) |
| | 6 | | 29 (8.6%) | 260 (77.6%) | 11 (3.3%) | 271 (40.3%) |
| | 7 | | 288 (85.7%) | 39 (11.6%) | 29 (8.6%) | 68 (10.1%) |

| | 8 | | 1 (0.3%) | 0 | 0 | 0 |
|---|---|---|---|---|---|---|
| Number of active injections n | | | | | | |
| | | | 336 | 335 | 337 | 672 |
| | Mean (SD) | | 6.7 (0.8) | 5.9 (0.8) | 5.1 (0.8) | 5.5 (0.9) |
| | Median | | 7.0 | 6.0 | 5.0 | 6.0 |
| | Min, Max | | 1,8 | 1,7 | 1,7 | 1,7 |

| Number of sham injections, n (%) 1 | | | | | | |
|---|---|---|---|---|---|---|
| | | | 7 (2.1%) | 4 (1.2%) | 3 (0.9%) | 7 (1.0%) |
| | 2 | | 15 (4.5%) | 4 (1.2%) | 8 (2.4%) | 12 (1.8%) |
| | 3 | | 46 (13.7%) | 17 (5.1%) | 5 (1.5%) | 22 (3.3%) |
| | 4 | | 258 (76.8%) | 63 (18.8%) | 40 (11.8%) | 103 (15.3%) |
| | 5 | | 1 (0.3%) | 240 (71.6%) | 45 (13.3%) | 285 (42.3%) |
| | 6 | | 0 | 0 | 229 (67.8%) | 229 (34.0%) |

| Number of sham injections n | | | | | | |
|---|---|---|---|---|---|---|
| | | | 327 | 328 | 330 | 658 |
| | | Mean (SD) | 3.7 (0.7) | 4.6 (0.7) | 5.4 (1.0) | 5.0 (1.0) |
| | | Median | 4.0 | 5.0 | 6.0 | 5.0 |
| | | Min, Max | 1,5 | 1,5 | 1,6 | 1,6 |

| Duration of treatment (weeks) | | | | | | |
|---|---|---|---|---|---|---|
| | n | | 336 | 335 | 337 | 672 |
| | Mean (SD) | | 46.28 (6.62) | 46.54 (6.56) | 46.18 (6.97) | 46.36 (6.77) |
| | | | **2q8** | **HDq12** | **HDq16** | **All HD** |
| | | | N = 336 (100%) | N = 335 (100%) | N = 338 (100%) | N = 673 (100%) |
| | | Median | 48.00 | 48.00 | 48.00 | 48.00 |
| | | Min, Max | 4,50.9 | 4,52 | 4,53.3 | 4,53.3 |

| **Week 60** | | | | | | |
|---|---|---|---|---|---|---|
| Total number of active injections, n | | | 2854 | 2324 | 2018 | 4342 |
| Total number of sham injections, n | | | 1502 | 2080 | 2380 | 4460 |

| | | Number of active injections, n (%) | | | | |
|---|---|---|---|---|---|---|
| | 1 | | 1 (0.3%) | 2 (0.6%) | 2 (0.6%) | 4 (0.6%) |
| | 2 | | 1 (0.3%) | 2 (0.6%) | 1 (0.3%) | 3 (0.4%) |
| | 3 | | 4 (1.2%) | 3 (0.9%) | 9 (2.7%) | 12 (1.8%) |
| | 4 | | 6 (1.8%) | 7 (2.1%) | 10 (3.0%) | 17 (2.5%) |
| | 5 | | 5 (1.5%) | 5 (1.5%) | 20 (5.9%) | 25 (3.7%) |
| | 6 | | 9 (2.7%) | 24 (7.2%) | 255 (75.4%) | 279 (41.5%) |
| | 7 | | 6 (1.8%) | 239 (71.3%) | 11 (3.3%) | 250 (37.1%) |
| | 8 | | 43 (12.8%) | 38 (11.3%) | 21 (6.2%) | 59 (8.8%) |
| | 9 | | 260 (77.4%) | 15 (4.5%) | 8 (2.4%) | 23 (3.4%) |
| | 10 | | 1 (0.3%) | 0 | 0 | 0 |

| Number of active injections n | | | | | | |
|---|---|---|---|---|---|---|
| | | | 336 | 335 | 337 | 672 |
| | Mean (SD) | | 8.5 (1.3) | 6.9 (1.1) | 6.0 (1.1) | 6.5 (1.2) |
| | Median | | 9.0 | 7.0 | 6.0 | 6.0 |
| | Min, Max | | 1,10 | 1,9 | 1,9 | 1,9 |

| Number of sham injections, n (%) 1 | | | | | | |
|---|---|---|---|---|---|---|
| | | | 5 (1.5%) | 4 (1.2%) | 3 (0.9%) | 7 (1.0%) |
| | 2 | | 12 (3.6%) | 2 (0.6%) | 8 (2.4%) | 10 (1.5%) |
| | 3 | | 15 (4.5%) | 5 (1.5%) | 2 (0.6%) | 7 (1.0%) |
| | 4 | | 48 (14.3%) | 12 (3.6%) | 5 (1.5%) | 17 (2.5%) |
| | 5 | | 246 (73.2%) | 34 (10.1%) | 19 (5.6%) | 53 (7.9%) |
| | 6 | | 1 (0.3%) | 58 (17.3%) | 31 (9.2%) | 89 (13.2%) |
| | 7 | | 0 | 213 (63.6%) | 42 (12.4%) | 255 (37.9%) |
| | 8 | | 0 | 0 | 220 (65.1%) | 220 (32.7%) |

| Number of sham injections n | | | | | | |
|---|---|---|---|---|---|---|
| | | | 327 | 328 | 330 | 658 |
| | Mean (SD) | | 4.6 (0.9) | 6.3 (1.2) | 7.2 (1.5) | 6.8 (1.4) |
| | Median | | 5.0 | 7.0 | 8.0 | 7.0 |
| | Min, Max | | 1,6 | 1,7 | 1,8 | 1,8 |

| | Duration of treatment (weeks) | | | | | |
|---|---|---|---|---|---|---|
| | | n | 336 | 335 | 337 | 672 |
| | | Mean (SD) | 57.23 (9.56) | 57.74 (9.12) | 57.44 (9.80) | 57.59 (9.46) |
| | | Median | 60.00 | 60.00 | 60.00 | 60.00 |
| | | Min, Max | 4,64.7 | 4,63.3 | 4,63.6 | 4,63.6 |

| | | | | | | |
|---|---|---|---|---|---|---|
| Max = maximum, Min = minimum, SD = standard deviation Duration (weeks) = [(date of last study treatment) - (date of first study treatment) +28]/7; 28 days were added because of the minimum 4 week dosing interval in the study. Study interventions given at Week 60 or beyond are not included in this table. | | | | | | |

The results of the exploratory endpoints, proportions of participants with a q16 or longer treatment interval through Week 48 and Week 60 in the HDq16 group, with a q12 or longer interval through Week 48 and Week 60 in the HDq12 and HDq16 groups, and with a q12 or q16 or longer treatment interval as the last intended interval at Week 48 and Week 60 in the HDq12 and HDq16 groups, respectively, in the SAF, are presented Table 1-46. In addition, the proportions of participants with q20 treatment interval as the last intended interval at Week 60 in the HDq16 group and the proportion of participants who shortened treatment intervals in the HDq12 and HDq16 groups are presented in this table.

Overall, the target treatment intervals of either q12 or q16 were maintained in more than 3 quarters of all participants in the HD groups through Week 48 and in approximately 3 quarters of all participants in the HD groups through Week 60.

Overall, the target treatment intervals of either q12 or q16 were maintained in more than 3 quarters of all participants in the HD groups through Week 48 and in approximately 3 quarters of all participants in the HD groups through Week 60.

**Table 1-46. Exposure to study treatment through Week 48 and Week 60 - Dosing intervals (safety analysis set, only participants considered as completers for Week 48)**

| **Through Week 48 (a)** | | | | | |
|---|---|---|---|---|---|
| | | **2q8** | **HDq12** | **HDq16** | **All HD** |
| | | N = 309 (100%) | N = 316 (100%) | N = 312 (100%) | N = 628 (100%) |
| Subjects with q12 or longer dosing interval (b), n (%) | | / | 251 (79.4%) | 272 (87.2%) | 523 (83.3%) |
| Subjects with q16 dosing interval (c), n (%) | | / | / | 239 (76.6%) | / |
| Subjects with q12 or longer dosing interval as the last intended dosing interval (d), n (%) | | / | 251 (79.4%) | 271 (86.9%) | 522 (83.1%) |
| Subjects with q16 dosing interval as the last intended dosing interval (d), n (%) | | / | / | 239 (76.6%) | / |
| Subjects shortened to q8 dosing interval at Week 16, n (%) | | / | 17 (5.4%) | 10 (3.2%) | 27 (4.3%) |
| Subjects shortened to q8 dosing interval at Week 20, n (%) | | / | 25 (7.9%) | 21 (6.7%) | 46 (7.3%) |
| Subjects shortened anytime, n (%) | | / | 65 (20.6%) | 73 (23.4%) | 138 (22.0%) |
| | Subjects shortened to q8 dosing interval anytime, n (%) | / | 65 (20.6%) | 40 (12.8%) | 105 (16.7%) |
| | Subjects shortened to q12 dosing interval anytime, n (%) (without shortening to q8) | / | / | 33 (10.6%) | / |

| **Through Week 60 (e)** | | | | | |
|---|---|---|---|---|---|
| | | **2q8** | **HDq12** | **HDq16** | **All HD** |
| | | N = 305 (100%) | N = 311 (100%) | N = 309 (100%) | N = 620 (100%) |
| | | | | | |
| Subjects maintained with q12 or longer dosing interval (f), n (%) | | / | 242 (77.8%) | 264 (85.4%) | 506 (81.6%) |
| Subjects maintained with q16 or longer dosing interval (g), n (%) | | / | / | 229 (74.1%) | / |
| Subjects with q12 or longer dosing interval as the last intended dosing interval (h), n (%) | | / | 263 (84.6%) | 278 (90.0%) | 541 (87.3%) |
| Subjects with q16 or longer dosing interval as the last intended dosing interval (h), n (%) | | / | 134 (43.1%) | 239 (77.3%) | 373 (60.2%) |
| Subjects with q20 dosing interval as the last intended dosing interval (h), n (%) | | / | / | 119 (38.5%) | / |
| Subjects shortened to q8 dosing interval at Week 16, n (%) | | / | 17 (5.5%) | 10 (3.2%) | 27 (4.4%) |
| Subjects shortened to q8 dosing interval at Week 20, n (%) | | / | 25 (8.0%) | 20 (6.5%) | 45 (7.3%) |
| Subjects shortened anytime, n (%) | | / | 69 (22.2%) | 80 (25.9%) | 149 (24.0%) |
| | Subjects shortened to q8 dosing interval anytime, n (%) | / | 69 (22.2%) | 45 (14.6%) | 114 (18.4%) |
| | Subjects shortened to q12 dosing interval anytime (without shortening to q8), n (%) | / | / | 35 (11.3%) | / |
| Subjects never extended dosing interval, n (%) (i) | | / | 159 (51.1%) | 174 (56.3%) | 333 (53.7%) |
| Subjects extended dosing interval anytime, n (%) (j) | | / | 152 (48.9%) | 135 (43.7%) | 287 (46.3%) |

| | | | | | |
|---|---|---|---|---|---|
| DRM = dose regimen modification / indicates categories that do not apply. a Study interventions given at Week 48 or beyond are not included in this table. b All subjects on q12 or q16 interval for whom it was not planned to have their interval shortened to q8 interval [according to DRM criteria until Week 44] prior to Week 48. c All subjects on q16 interval for whom it was not planned to have their interval shortened to q12 or q8 interval [according to DRM criteria until Week 44] prior to Week 48. d Based on DRM criteria assessed at the last visit on or before Week 48. e Study interventions given at Week 60 or beyond are not included in this table. f All subjects on q12 or q16 interval for whom it was not planned to have their interval shortened to q8 interval [according to DRM criteria until Week 56] prior to Week 60. g All subjects on q16 interval for whom it was not planned to have their interval shortened to q12 or q8 interval [according to DRM criteria until Week 56] prior to Week 60. h Based on DRM criteria assessed at the last visit on or before Week 60. i All subjects on q12 or q16 interval for whom it was not planned to have their interval extended [according to DRM criteria until Week 56] prior to Week 60. j All subjects on q12 or q16 interval for whom it was planned to have their interval extended [according to DRM criteria until Week 56] prior to Week 60. | | | | | |

### Polypoidal Choroidal Vasculopathy (PCV):

Overall, 1,009 patients were randomized and treated, and the primary endpoint was met with aflibercept 8 mg (p=0.0009 and p=0.0011 for HDq12 and HDq16, respectively) vs 2q8. Observed mean±SD change from BL BCVA was +6.7±12.6 (HDq12), +6.2±11.7 (HDq16), and +7.6±12.2 (2q8) letters (BL: 59.9±13.4, 60.0±12.4, and 58.9±14.0, respectively). PCV was present in 141 patients (2q8: n=54; HDq12: n=45; HDq16: n=42) and absent in 153 (unknown in 715) based on ICGA (indocyanine green angiography (ICGA)). In patients with PCV, observed mean±SD change from BL in BCVA at Week 48 was +9.3±13.2, +8.5±7.8, and +9.5±11.7 letters with HDq12, HDq16, and 2q8, respectively (BL BCVA: 56.7±13.4, 60.1±11.3, and 57.6±15.4, respectively). In the PCV subgroup, 67.8% of patients in the pooled aflibercept 8 mg groups had no central subfield IRF/SRF at Week16 versus 63.0% in the 2q8 group. At Week 48, 33/42 (78.6%) of patients with PCV randomized to HDq12 maintained 12-week treatment intervals, and 33/38 (86.8%) randomized to HDq16 maintained 16-week intervals; 69/80 (86.3%) of patients with PCV receiving 8 mg were maintained on ≥12-week treatment intervals. The safety profile of aflibercept was similar in patients with PCV and the overall PULSAR population. In patients with PCV, aflibercept 8 mg provides similar improvements in BCVA at Week 48 compared to 2q8, with an extended injection interval, and comparable safety profile.

### Conclusions

This is an ongoing Phase 3, multi-center, randomized, double-masked, active-controlled study investigating the efficacy, safety, and tolerability of IVT administration of HD aflibercept versus aflibercept 2 mg in participants with treatment-naïve nAMD. Presented herein are the results of the pre-planned Week 48 and Week 60 analyses of the data for the primary and the key secondary endpoints, and for the additional secondary efficacy, PK, and safety endpoints. Study participants, the masked study team, central reading center, and Steering Committee members are remaining masked until the end of the masked part of the study (up to Week 96).

A total of 1011 participants recruited at 223 sites in 27 countries/regions (Europe, North America, Latin America, Australia, and Asia Pacific) were randomized in nearly equal numbers to 1 of the 3 treatment groups, of whom 1009 participants received at least one IVT injection. All of these treated participants were included in the safety analysis (SAF).

Compliance with the treatment schedule was high in all groups with a mean treatment compliance through Week 48 and through Week 60 of > 97% in all groups. The analysis of efficacy was based on the data of the FAS (n=1009), which was identical to the SAF, and the PPS (n=970 in Week 48 analysis, n=969 in Week 60 analysis), which showed group sizes of ≥ 95% in all treatment groups. The analysis of general PK assessments was based on the data of the PKS (n=934), and the analysis of the Dense PK study on the data of the DPKS (n=23).

The FAS (and SAF) consisted of 459 (45.5%) male and 550 (54.5%) female participants aged from 50 to 96 years (median: 75 years) overall. Most participants were White (75.8%) or Asian (23.2%). The mean (SD) visual acuity score BCVA at baseline was 59.6 (13.3) letters. All lesion types, *i.e.*, occult, minimally classic, and predominantly classic lesions, were represented. Overall, the 3 treatment groups were well balanced with regard to demographic and disease characteristics. Minor numerical imbalances in some comparisons were considered not to be of relevance for the evaluation of the study objectives.

The primary endpoint, change from baseline in BCVA measured by the ETDRS letter score at Week 48, and the key secondary endpoints, change from baseline in BCVA measured by the ETDRS letter score at Week 60 and proportion of participants with no IRF and no SRF in central subfield at Week 16, were assessed together using a hierarchical testing procedure as per the EP-SAP based on the FAS.

The primary analysis endpoint was met: treatment with HDq12 and HDq16 demonstrated non-inferiority to 2q8 using the margin of 4 letters, with LSₘₑₐₙ changes from baseline in BCVA from baseline to Week 48 of 6.06 letters (HDq12) and 5.89 letters (HDq16), respectively, versus 7.03 letters in the 2q8 group. Treatment differences in LSₘₑₐₙₛ (95% CI) were -0.97 (-2.87, 0.92) letters and -1.14 (-2.97, 0.69) letters for HDq12 and HDq16, respectively, compared to 2q8. The corresponding key secondary endpoint at Week 60 was also met: treatment with HDq12 and HDq16 demonstrated non-inferiority to 2q8 using the margin of 4 letters, with LSₘₑₐₙ changes from baseline in BCVA from baseline to Week 60 of 6.37 letters (HDq12) and 6.31 letters (HDq16), respectively, versus 7.23 letters in the 2q8 group. Treatment differences in LSₘₑₐₙₛ (95% CI) were -0.86 (-2.57, 0.84) letters and -0.92 (-2.51, 0.66) letters for HDq12 and HDq16, respectively, compared to 2q8. The robustness of these results for the primary endpoint and the corresponding key secondary endpoint was supported by supplementary analyses in the PPS as well as by sensitivity analyses in the FAS.

The non-inferiority in the mean change in BCVA at Week 48 and Week 60 were achieved in participants treated at extended intervals in the HD groups compared to the 2q8 group. Moreover, 79.4% and 77.8% of completers in the HDq12 group and 76.6% and 74.1% of completers in the HDq16 group maintained their randomized treatment interval through Week 48 and Week 60, respectively. This resulted in numerically lower mean numbers of active injections through Week 60 of 6.9 in the HDq12 group and 6.0 in the HDq16 group compared to 8.5 in the 2q8 group. Overall, 82% of participants in the pooled HD groups were able to be maintained on a dosing interval of 12 weeks or longer with HD aflibercept treatment through Week 60 and, thus, the remaining proportion of 18% of HD participants did require shortening of the dosing interval to every 8 weeks.

For the key secondary endpoint of proportion of participants with no IRF and no SRF in central subfield at Week 16, superiority in the pooled HD groups versus the comparator 2q8 was demonstrated. This analysis showed that no retinal fluid status (no IRF and no SRF) at Week 16 was reached in 63.3% of the participants in the pooled HD groups compared with 51.6% in the 2q8 group. This resulted in a difference (95% CI) between the pooled HD groups and the 2q8 group of 11.73% (5.26%, 18.20%) with an associated p-value for the one- sided test for superiority of 0.0002.

The subsequent test for superiority in the primary endpoint of HDq12 vs. 2q8 treatment was not statistically significant (p = 0.8437) so that the hierarchical testing strategy was stopped at this point.

Subgroup analyses for the primary and key secondary endpoints, which were performed on a descriptive level by age, sex, geographic region, ethnicity, race, baseline BCVA letters, and baseline PCV, did not show clinically meaningful differences between the subgroup population and the total population.

Descriptive analyses of the additional secondary endpoints at Week 48, change in CNV size from baseline, change in total lesion area from baseline, change from baseline in CST, and proportion of participants with no IRF and no SRF in the center subfield, provided differences across the treatment groups that were in favor of HD vs. 2q8 treatment. The exploratory descriptive analyses of the same endpoints at Week 60 suggested similar outcomes across all treatment groups through Week 60.

The estimated contrasts for change in CNV size from baseline at Week 48 suggested greater reductions of -1.22 mm² in the HDq12 group and of -0.48 mm² in the HDq16 group in comparison with 2q8 treatment. The corresponding estimated contrasts for change in total lesion area from baseline to Week 48 were -0.55 mm² and 0.44 mm², respectively. The corresponding contrasts for change from baseline in CST at Week 48 were -11.12 µm and - 10.51 µm, respectively, while the mean decreases in CST over time were similar across all groups. Moreover, the proportion of participants with no IRF and no SRF in the center subfield was 11.725% higher in the HDq12 and 7.451% higher in the HDq16 groups in comparison with 2q8 treatment.

The descriptive analyses of the other additional secondary endpoints evaluated at Week 48, which were evaluated as exploratory endpoints at Week 60, proportion of participants who gained at least 15 letters in BCVA from baseline, proportion of participants achieving an ETDRS letter score of at least 69 (approximate 20/40 Snellen equivalent), and change from baseline in NEI-VFQ-25 total score, provided similar results in the HD groups and the 2q8 group at Week 48 and Week 60.

The mean number of active injections in the SAF population was 8.5, 6.9 and 6.0 in the 2q8, HDq12 and HDq16 treatment groups, respectively. For the 925 participants in the SAF considered as completers of 60 weeks of study treatment (*i.e.*, SAF completers), the mean number of active injections was 8.8, 7.1 and 6.2 in the 2q8, HDq12 and HDq16 treatment groups, respectively. The observed decrease in the mean and median number of active injections and the corresponding increase in the number of sham injections from the 2q8 group to the HDq12 and HDq16 group reflects the protocol-driven increase in treatment intervals across these groups.

The safety profile of the HD treatments was similar to that of the comparator treatment (2 mg). The overall rates of ocular and non-ocular TEAEs and SAEs reported through Week 60 were similar among the treatment groups. Most of the reported TEAEs were evaluated as mild and resolved within the observation period without permanent discontinuation of the study drug. Ocular TEAEs in the study eye that resulted in discontinuation of the study drug affected few participants: 8 (1.2%) participants in the pooled HD groups and 2 (0.6%) participants in the 2q8 group. Similarly, non-ocular TEAEs resulted in discontinuation of the study drug in 3 (0.4%) participants in the pooled HD groups and 6 (1.8%) participants in the 2q8 group.

A total of 10 deaths were reported during the study through Week 60, 5 (0.7%) in the pooled HD groups and 5 (1.5%) in the 2q8 group. None of these deaths were considered related to the study drug, to fellow-eye treatment, the injection procedure, or protocol-required procedures and were consistent with concurrent medical conditions and the complications of these conditions associated with an older population.

No dose-response relationship in the incidence or the types of TEAEs was apparent between participants in the HD groups and the 2q8 group. The results of the subgroup analyses of the TEAEs were similar to those in the entire study population and did not suggest medically relevant differences across the treatment groups.

The analyses of laboratory data, vital signs, and ECG data (including QT interval) did not show any remarkable mean changes over time within the HD groups and the 2q8 group or differences between the groups.

No clinically meaningful trends in mean or median changes from baseline to pre-dose IOP in the study eye were observed in any treatment group through Week 60. The proportion of participants meeting pre-defined IOP criteria was generally low and similar across the treatment groups. Other technical ophthalmologic examinations (slit lamp) did also not point to any noticeable trends towards differences among the treatment groups or relevant changes within treatment groups from baseline through Week 60.

After the initial aflibercept dose of 2 mg (2q8) or 8 mg (HDq12 pooled with HDq16) aflibercept in the dense PK group, the concentration-time profiles of free aflibercept were characterized by an initial phase of increasing concentrations reflecting initial absorption from the ocular space and initial distribution into the systemic circulation from the ocular space into systemic circulation followed by a mono-exponential elimination phase. The concentration-time profiles of adjusted bound aflibercept were characterized by a slower attainment of Cₘₐₓ compared to free aflibercept. Following attainment of Cₘₐₓ, a slight decrease of the concentration-time profile was observed until approximately the end of the dosing interval (Day 29).

As the IVT dose of aflibercept increased from 2 mg to 8 mg (4-fold dose), the median Cₘₐₓ and AUCₗₐₛₜ for free aflibercept increased in a slightly less than dose-proportional manner (about 3-fold) for Cₘₐₓ and a greater than dose-proportional manner for AUCₗₐₛₜ (about 7-fold). This larger increase in AUCₗₐₛₜ is unexpected and difficult to explain based on dose alone but it is consistent with known nonlinear target-mediated kinetics of aflibercept. Mean Cₘₐₓ and AUCₗₐₛₜ for adjusted bound aflibercept increased in a less than dose-proportional manner (approximately 2- to 2.5-fold) which is also consistent with the known nonlinear kinetics of aflibercept.

There was no accumulation seen after the 2 mg dose which is consistent with historical data. Accumulation of free aflibercept for the 8-mg treatments was 1.17. For adjusted bound aflibercept, accumulation ranged from 1.83 to 1.72 for the 2 mg and 8 mg treatments, respectively.

In general, PK in Japanese participants were in the same range as seen in non-Japanese participants. However, this should be interpreted with caution as concentrations and PK parameter were based on single participants.

In the general (sparse) PK assessment of mainly trough concentrations (except Visit 5 which was 4-8 days after the third administration), IVT administration of mean free aflibercept concentrations increased from baseline to Visit 5 (60-64 days after first administration).

Thereafter, mean concentrations of free aflibercept declined in all 3 dose groups. In the 2q8 treatment group mean concentrations of free aflibercept decline to values close to or below LLOQ in almost all participants 4 weeks after treatment, in the HD groups 8 weeks after treatment (Week 28 for HDq12, Week 48 for HDq16). Comparison of mean concentrations of free aflibercept at Visit 5 showed that concentrations increased about 6-fold as the IVT dose of aflibercept increased from 2 mg to 8 mg (4-fold dose).

Mean adjusted bound aflibercept concentrations increased from baseline to Visit 5. Following attainment of Cₘₐₓ, a slight decrease of the concentration-time profiles was observed until approximately the end of the observation period for both dose groups. Comparison of mean concentrations of adjusted bound aflibercept at Visit 5 showed that concentrations increased close to dose-proportional (3-fold) as the IVT dose of aflibercept increased from 2 mg to 8 mg (4-fold dose).

lmmunogenicity was low across all treatment groups. Out of the 833 participants included in the ADA analysis set, the incidence of treatment-emergent ADA during the 48-week of treatment with aflibercept administered IVT in the 2q8, HDq12, and HDq16 treatment groups was 4/273 (1.5%), 11/283 (3.9%), and 9/277 (3.2%), respectively; all of these responses were of low maximum titer. None of the ADA-positive samples were found to be positive in the NAb assay. The immunogenicity observed in this study was consistent with that historically observed at the 2 mg dose suggesting no increase in immunogenicity at this higher dose.

### Overall conclusions

- Treatment with HD aflibercept at intervals of 12 or 16 weeks provided non-inferior increases in BCVA from baseline at Week 48 and at Week 60 compared to treatment with aflibercept 2 mg every 8 weeks.
- Treatment with HD aflibercept was superior to treatment with aflibercept 2 mg in that 11.7% more participants in the pooled HD groups than in the 2q8 group had no IRF and no SRF in central subfield at Week 16.
- The non-inferior visual acuity outcomes in the HDq12 and HDq16 groups compared to 2q8 were achieved with the majority of participants remaining on their randomized treatment interval through Week 48 (79% and 77%, respectively) and through Week 60 (78% and 74%, respectively). Based on the dosing schedule, the Week 60 time point represents four 12-week intervals for the HDq12 group and three 16-week intervals for the HDq16 group following the 3 initial monthly doses. This led to a clinically meaningful reduction in the number of injections over 60 weeks compared to treatment with aflibercept 2 mg every 8 weeks.
- Overall, 82% of participants in the pooled HD groups were able to be maintained on a dosing interval of 12 weeks or longer with HD aflibercept treatment through Week 60, while 18% of participants did require shortening of the dosing interval to q8.
- Overall, the efficacy results obtained at Week 60 were consistent with those from Week 48.
- Immunogenicity was low across all treatment groups. None of the ADA-positive samples were found to be positive in the NAb assay.
- Review of the safety data did not reveal any new safety signals or adverse trends in the HD aflibercept groups compared to the 2 mg group. The ocular and systemic safety profile of aflibercept HD was consistent with the established safety profile of aflibercept 2 mg.

All references cited herein are incorporated by reference to the same extent as if each individual publication, database entry (e.g., Genbank sequences or GenelD entries), patent application, or patent, was specifically and individually indicated to be incorporated by reference. This statement of incorporation by reference is intended by Applicants to relate to each and every individual publication, database entry (e.g., Genbank sequences or GenelD entries), patent application, or patent, each of which is clearly identified in even if such citation is not immediately adjacent to a dedicated statement of incorporation by reference. The inclusion of dedicated statements of incorporation by reference, if any, within the specification does not in any way weaken this general statement of incorporation by reference. Citation of the references herein is not intended as an admission that the reference is pertinent prior art, nor does it constitute any admission as to the contents or date of these publications or documents.

### Embodiments of the description:

1. A method for treating or preventing neovascular age related macular degeneration (nAMD) in a subject in need thereof comprising administering one or more doses of aflibercept at an interval and quantity whereby
   - the clearance of free aflibercept from the ocular compartment is about 0.3, 0.4, 0.41 or 0.37-0.46 mL/day after an intravitreal injection of aflibercept,
   - the time for the amount for free aflibercept to reach the lower limit of quantitation (LLOQ) in the ocular compartment of a subject after said intravitreal injection of aflibercept is about 15 weeks; and/or
   - the time for free aflibercept to reach the lower limit of quantitation (LLOQ) in the plasma of the subject after said intravitreal injection of aflibercept is about 3.8, 3.5 or 3.5-3.8 weeks.
2. A method for slowing the clearance of free aflibercept from the ocular compartment after an intravitreal injection relative to the rate of clearance of aflibercept from the ocular compartment after an intravitreal injection of 2 mg or <4 mg aflibercept comprising
   intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
3. The method of any one of embodiments 1-2 wherein the clearance of free aflibercept from the ocular compartment is about 34% slower than that from the ocular compartment after an intravitreal injection of 2 or < 4 mg aflibercept.
4. The method of any one of embodiments 1-3 wherein the clearance of free aflibercept from the ocular compartment is about 0.37-0.46 mL/day or 0.41 mL/day after an intravitreal injection of > 8 mg aflibercept.
5. A method for increasing the duration of efficacy and/or the time for the amount of free aflibercept to reach the lower limit of quantitation (LLOQ) in the ocular compartment of a subject after an intravitreal injection of aflibercept, relative to the time to reach LLOQ of the amount of free aflibercept in the ocular compartment of a subject after an intravitreal injection of about 2 mg or <4 mg aflibercept, comprising intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
6. The method of embodiment 5 wherein the duration of efficacy and/or the time for the amount of free aflibercept to reach the lower limit of quantitation (LLOQ) in the ocular compartment of a subject after said intravitreal injection of aflibercept is increased by about 5 or 6 weeks, relative to the time to reach LLOQ of the amount of free aflibercept in the ocular compartment of a subject after an intravitreal injection of about 2 or <4 mg aflibercept.
7. The method of any one of embodiments 5-6 wherein the time for the amount of free aflibercept to reach the lower limit of quantitation (LLOQ) in the ocular compartment of a subject after said intravitreal injection of aflibercept is increased by more than about 1, 2, 1.2 or 1.3 weeks relative to the time to reach LLOQ of free aflibercept in the ocular compartment of a subject after an intravitreal injection of about 2 or <4 mg aflibercept.
8. The method of any one of embodiments 5-7 wherein the time for the amount for free aflibercept to reach the lower limit of quantitation (LLOQ) in the ocular compartment of a subject after said intravitreal injection of > 8 mg aflibercept is about 15 weeks.
9. The method of any one of embodiments 5-8 wherein the time for the amount of free aflibercept to reach the lower limit of quantitation (LLOQ) in the ocular compartment of a subject after said intravitreal injection of > 8 mg aflibercept is greater than about 8, 8.7, 8.71, 9, 9.2, 9.21 or 10 weeks.
10. A method for increasing the time for free aflibercept to reach the lower limit of quantitation (LLOQ) in the plasma of a subject after an intravitreal injection of aflibercept relative to the time to reach LLOQ of free aflibercept in the plasma of a subject after an intravitreal injection of about 2 or <4 mg aflibercept, comprising intravitreally injecting into an eye of a subject in need thereof,
   a single initial dose of about 8 mg or more of aflibercept, followed by
   one or more secondary doses of about 8 mg or more of the aflibercept, followed by
   one or more tertiary doses of about 8 mg or more of the aflibercept;
   wherein each secondary dose is administered about 2 to 4 weeks after the immediately preceding dose; and
   wherein each tertiary dose is administered about 12-20 weeks after the immediately preceding dose.
11. The method of embodiment 10 wherein said LLOQ of free aflibercept measured in plasma is about 0.0156 mg/L.
12. The method of any one of embodiments 10-11 wherein the time for free aflibercept to reach the lower limit of quantitation (LLOQ) in the plasma of a subject after said intravitreal injection of aflibercept is increased by about 2 weeks relative to the time to reach LLOQ of free aflibercept in the plasma of a subject after an intravitreal injection of about 2 mg aflibercept.
13. The method of any one of embodiments 10-12 wherein the time for free aflibercept to reach the lower limit of quantitation (LLOQ) in the plasma of a subject after said intravitreal injection of > 8 mg aflibercept is about 3, 3.5, 3.8 or 4 weeks.
14. The method of any one of embodiments 10-13 wherein the time for free aflibercept to reach the lower limit of quantitation (LLOQ) in the plasma of a subject after said intravitreal injection of >8 mg aflibercept is greater than about 1.5 or 1.6 weeks.
15. The method of any one of embodiments 1-14 wherein < 4mg is about 2 mg or 2-4 mg.
16. The method of any one of embodiments 1-15 wherein the subject suffers from neovascular age related macular degeneration (nAMD).
17. The method of any one of embodiments 1-16 wherein the >8 mg aflibercept is in an aqueous pharmaceutical formulation comprising histidine-based buffer.
18. The method of any one of embodiments 1-17 wherein the >8 mg aflibercept is in an aqueous pharmaceutical formulation comprising arginine.
19.The method of any one of embodiments 1-18 wherein the >8 mg aflibercept is in an aqueous pharmaceutical formulation having a pH of about 5.8.
20.The method of any one of embodiments 1-19 wherein the >8 mg aflibercept is in an aqueous pharmaceutical formulation comprising a sugar or polyol.
21.The method of any one of embodiments 1-20 wherein the >8 mg aflibercept is in an aqueous pharmaceutical formulation comprising a sucrose.
22.The method of any one of embodiments 1-21 wherein the >8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8°C.
23. The method of any one of embodiments 1-22 wherein the >8 mg aflibercept is in an aqueous pharmaceutical formulation comprising an aqueous pharmaceutical formulation comprising: at least about 100 mg/ml of a VEGF receptor fusion protein comprising two polypeptides that each comprises an immunoglobin-like (Ig) domain 2 of VEGFR1, an Ig domain 3 of VEGFR2, and a multimerizing component; about 10-100 mM L-arginine; sucrose; a histidine-based buffer; and a surfactant; wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.
24. A method
   - for treating or preventing neovascular age related macular degeneration (nAMD), in a subject in need thereof,
   - for improving best corrected visual acuity in a subject in need thereof with nAMD; or
   - for promoting retinal drying in a subject with nAMD in need thereof;
   comprising administering to an eye of the subject, one or more doses of about 8 mg or more of VEGF receptor fusion protein once every 12, 13, 14, 15, 16, 17, 18, 19 or 20 or 12-20 or 12-16 or 16-20 weeks.
25. The method of any one of embodiments 1-24 for treating or preventing neovascular age-related macular degeneration (nAMD), in a subject in need thereof, comprising administering to an eye of the subject,
   a single initial dose of about 8 mg or more of a VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12-20 weeks after the immediately preceding dose.
26. The method of any one of embodiments 1-24 for treating or preventing neovascular age-related macular degeneration (nAMD), in a subject in need thereof, comprising administering to an eye of the subject,
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12 weeks after the immediately preceding dose.
27. The method of any one of embodiments 1-24 for treating or preventing neovascular age-related macular degeneration (nAMD), in a subject in need thereof, comprising administering to an eye of the subject, a single initial dose of about 8 mg or more of VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 16 weeks after the immediately preceding dose.
28. The method of any one of embodiments 1-24 for treating or preventing neovascular age-related macular degeneration (nAMD), in a subject in need thereof, comprising administering to an eye of the subject,
   a single initial dose of about 8 mg or more of VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 20 weeks after the immediately preceding dose.
29. A method for treating or preventing neovascular age-related macular degeneration (nAMD), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg aflibercept (0.07 mL) via intravitreal injection once every 8 - 16 weeks (2 - 4 months, +/- 7 days).
30. A method for treating or preventing neovascular age-related macular degeneration (nAMD), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 12 weeks (2 - 4 months, +/- 7 days).
31. A method for treating or preventing neovascular age-related macular degeneration (nAMD), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 16 weeks (2 - 4 months, +/- 7 days).
32. A method for treating or preventing neovascular age-related macular degeneration (nAMD), in a subject in need thereof comprising administering 8 mg VEGF receptor fusion protein (0.07 mL or 70 microliters) administered by intravitreal injection every 4 weeks (approximately every 28 days +/- 7 days, monthly) for the first three doses, followed by 8 mg VEGF receptor fusion protein (0.07 mL) via intravitreal injection once every 20 weeks (+/- 7 days).
33. A method for treating or preventing neovascular age-related macular degeneration (nAMD) in a subject in need thereof at a dose of 8 mg aflibercept (equivalent to 70 microliters solution for injection) comprising administering 1 injection per month (every 4 weeks) for 3 consecutive doses of said 8 mg aflibercept wherein said injection intervals may then be extended up to every 16 weeks based on the physician's judgement of visual and/or anatomic outcomes.
34. A method for treating or preventing neovascular age related macular degeneration (nAMD), in a subject in need thereof, wherein:
   (1) the subject has received an initial 2 mg dose of VEGF receptor fusion protein then the method comprises, after 1 month, administering to the subject the initial 8 mg dose of VEGF receptor fusion protein and, 1 month thereafter, the 1^{st} 8 mg secondary dose of VEGF receptor fusion protein; and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (2) the subject has received an initial 2 mg dose of VEGF receptor fusion protein, then the method comprises, after 1 month, administering to the subject, the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (3) the subject has received an initial 2 mg dose of VEGF receptor fusion protein, then the method comprises, after 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (4) the subject has received an initial 2 mg dose of VEGF receptor fusion protein, then the method comprises, after 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   (5) the subject has received an initial 2 mg dose of VEGF receptor fusion protein and a 1^{st} 2 mg secondary dose of VEGF receptor fusion protein after 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (6) the subject has received an initial 2 mg dose of VEGF receptor fusion protein and a 1^{st} 2 mg secondary dose of VEGF receptor fusion protein after 1 month, then the method comprises, after another 1 month, administering to the subject a first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (7) the subject has received an initial 2 mg dose of VEGF receptor fusion protein and a 1^{st} 2 mg secondary dose of VEGF receptor fusion protein after 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (8) the subject has received an initial 2 mg dose of VEGF receptor fusion protein and a 1^{st} 2 mg secondary dose of VEGF receptor fusion protein after 1 month, then the method comprises, after another 1 month, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   (9) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the initial 8 mg dose of VEGF receptor fusion protein and, 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (10) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the first 8 mg secondary dose of VEGF receptor fusion protein and, 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (11) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after another 1 month, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (12) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month, then the method comprises, after 2 months, administering to the subject the 1^{st} 8 mg maintenance dose of VEGF receptor fusion protein and, all further 8 mg maintenance doses of VEGF receptor fusion protein every 12 or 16 or 20 weeks according to the HDq12 or HDq16 or HDq20 dosing regimen;
   (13) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and a 1^{st} 2 mg maintenance dose of VEGF receptor fusion protein after 8 weeks, then the method comprises, up to 2 months after the last dose of VEGF receptor fusion protein, administering to the subject the initial 8 mg dose of VEGF receptor fusion protein and 1 month thereafter, the 1^{st} 8 mg secondary dose of VEGF receptor fusion protein; and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (14) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the first 8 mg secondary dose of VEGF receptor fusion protein and 1 month thereafter, the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (15) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 2^{nd} 8 mg secondary dose of VEGF receptor fusion protein and then, every 12 or 16 or 20 weeks thereafter, one or more 8 mg maintenance doses of VEGF receptor fusion protein according to the HDq12 or HDq16 or HDq20 dosing regimen;
   (16) the subject has received an initial 2 mg dose of VEGF receptor fusion protein and a 1^{st} 2 mg secondary dose of VEGF receptor fusion protein after 1 month and a 2^{nd} 2 mg secondary dose of VEGF receptor fusion protein after another 1 month and 1 or more 2 mg maintenance doses of VEGF receptor fusion protein after 8 weeks, then the method comprises up to 2 months after the last dose of VEGF receptor fusion protein, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
   wherein,
   (i) said HDq12 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 12 weeks after the immediately preceding dose;
      (ii) said HDq16 dosing regimen comprises:
         a single initial dose of about 8 mg or more of VEGF receptor fusion protein, followed by one or more secondary doses of about 8 mg or more of the VEGF receptor fusion protein, followed by
         one or more tertiary doses of about 8 mg or more of the VEGF receptor fusion protein;
         wherein each secondary dose is administered about 2 to 4 weeks after the immediately preceding dose; and
         wherein each tertiary dose is administered about 16 weeks after the immediately preceding dose;
         and
      (iii) said HDq20 dosing regimen comprises:
         a single initial dose of about 8 mg or more of VEGF receptor fusion protein, followed by one or more secondary doses of about 8 mg or more of the VEGF receptor fusion protein, followed by
         one or more tertiary doses of about 8 mg or more of the VEGF receptor fusion protein;
         wherein each secondary dose is administered about 2 to 4 weeks after the immediately preceding dose; and
         wherein each tertiary dose is administered about 20 weeks after the immediately preceding dose.
35. A method for treating or preventing neovascular age related macular degeneration (nAMD), in a subject in need thereof, wherein:
   (a) the subject has received an initial 8 mg dose of VEGF receptor fusion protein; then the method comprises after 1 month administering to the subject the first 8 mg secondary dose of VEGF receptor fusion protein and 1 month thereafter, administering the 2^{nd} 8 mg secondary dose of VEGF receptor fusion protein; and then, every 12 or 16 or 20 weeks thereafter, administering one or more 8 mg maintenance doses of VEGF receptor fusion protein according to the HDq12 or HDq16 or HDq20 dosing regimen;
   (b) the subject has received an initial 8 mg dose of VEGF receptor fusion protein & a 1^{st} 8 mg secondary dose of VEGF receptor fusion protein after 1 month, then the method comprises, after another 1 month, administering to the subject the 2^{nd} 8 mg secondary dose of VEGF receptor fusion protein; and then, every 12 or 16 or 20 weeks thereafter, one or more 8 mg maintenance doses of VEGF receptor fusion protein according to the HDq12 or HDq16 or HDq20 dosing regimen;
   (c) the subject has received an initial 8 mg dose of VEGF receptor fusion protein & a 1^{st} 8 mg secondary dose of VEGF receptor fusion protein after 1 month & a 2^{nd} 8 mg secondary dose of VEGF receptor fusion protein after another month; then the method comprises, after 12 or 16 or 20 weeks, administering to the subject the 1^{st} 8 mg maintenance dose of VEGF receptor fusion protein and all further 8 mg maintenance doses of VEGF receptor fusion protein every 12 or 16 or 20 weeks according to the HDq12 or HDq16 or HDq20 dosing regimen;
      or
   (d) the subject has received an initial 8 mg dose of VEGF receptor fusion protein & a 1^{st} 8 mg secondary dose of VEGF receptor fusion protein after 1 month & a 2^{nd} 8 mg secondary dose of VEGF receptor fusion protein after another month, then, every 12 or 16 or 20 weeks thereafter, the subject has received one or more 8 mg maintenance doses of VEGF receptor fusion protein; then the method comprises after 12 or 16 or 20 weeks from the last maintenance dose of VEGF receptor fusion protein, administering to the subject one or more 8 mg maintenance doses of VEGF receptor fusion protein and all further 8 mg maintenance doses of VEGF receptor fusion protein every 12 or 16 or 20 weeks according to the HDq12 or HDq16 or HDq20 dosing regimen;
   wherein,
   (i) said HDq12 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 12 weeks after the immediately preceding dose;
   (ii) said HDq16 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 16 weeks after the immediately preceding dose;
      and
   (iii) said HDq20 dosing regimen comprises:
      a single initial dose of about 8 mg or more of VEGF receptor fusion protein, followed by one or more secondary doses of about 8 mg or more of the VEGF receptor fusion protein, followed by
      one or more tertiary doses of about 8 mg or more of the VEGF receptor fusion protein;
      wherein each secondary dose is administered about 2 to 4 weeks after the immediately preceding dose; and
      wherein each tertiary dose is administered about 20 weeks after the immediately preceding dose.
36. A method for treating or preventing neovascular age related macular degeneration (nAMD), in a subject in need thereof who has been on a dosing regimen for treating or preventing the nAMD calling for
   a single initial dose of about 2 mg of VEGF receptor fusion protein, followed by one or more secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose; and wherein the subject is at any phase of the 2 mg VEGF receptor fusion protein dosing regimen,
   comprising administering to an eye of the subject,
   an 8 mg dose of VEGF receptor fusion protein,
   evaluating the subject in about 8 or 10 or 12 weeks after said administering and, if, in the judgment of the treating physician dosing every 12 weeks or every 16 weeks is appropriate, then continuing to dose the subject every 12 weeks or 16 weeks with 8 mg VEGF receptor fusion protein; or
   evaluating the subject in about 8 or 10 or 12 weeks after said administering and, if, in the judgment of the treating physician dosing every 12 weeks is appropriate, then administering another 8 mg dose of VEGF receptor fusion protein, re-evaluating the subject in about 12 weeks and if in the judgment of the treating physician, dosing every 16 weeks is appropriate, then continuing to dose the subject every 16 weeks with 8 mg VEGF receptor fusion protein.
37. The method of any one of embodiments 1-36 wherein the subject has been on a dosing regimen for treating or preventing neovascular age related macular degeneration of:
   a single initial dose of about 2 mg of VEGF receptor fusion protein, followed by 2 secondary doses of about 2 mg of the VEGF receptor fusion protein, followed by one or more tertiary doses of about 2 mg of the VEGF receptor fusion protein;
   wherein each secondary dose is administered about 4 weeks after the immediately preceding dose; and
   wherein each tertiary dose is administered about 8 weeks after the immediately preceding dose.
38. A method of any one of embodiments 1-37 for treating or preventing neovascular age-related macular degeneration (nAMD), in a subject in need thereof, comprising administering to an eye of the subject,
   a single initial dose of about 8 mg or more of VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and wherein each tertiary dose is administered about 12 or 16 weeks after the immediately preceding dose;
   further comprising, after receiving one or more of said tertiary doses about 12 or 16 after the immediately preceding dose, lengthening the tertiary dose interval from
      - 12 weeks to 16 weeks;
      - 12 weeks to 20 weeks; or
      - 16 weeks to 20 weeks,
   after the immediately preceding dose.
39. The method of embodiment 38 wherein said tertiary dose interval is adjusted about 48 or 60 weeks after treatment initiation.
40. The method of any one of embodiments 38-39 wherein, prior to said lengthening, the subject exhibits
   (a) <5 letter loss in BCVA; and/or
   (b) CRT <300 or 320 µm.
41. The method of any one of embodiments 38-40 further comprising evaluating BVCA and/or CRT in the subject and, if the subject exhibits
   (a) <5 letter loss in BCVA; and/or
   (b) CRT <300 or 320 µm.
   lengthening the tertiary dose interval.
42. A method of any one of embodiments 1-41 for treating or preventing nAMD, in a subject in need thereof, comprising administering to an eye of the subject,
   a single initial dose of about 8 mg or more of a VEGF receptor fusion protein, followed by one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12 or 16 or 20 weeks after the immediately preceding dose;
   further comprising, after receiving one or more of said tertiary doses about 12 or 16 or 20 weeks after the immediately preceding dose, shortening the tertiary dose interval from
      - 12 weeks to 8 weeks;
      - 16 weeks to 12 weeks;
      - 16 weeks to 8 weeks,
      - 20 weeks to 8 weeks,
      - 20 weeks to 12 weeks, or
      - 20 weeks to 16 weeks.
43. The method of embodiment 42 wherein, prior to said shortening, the subject exhibits
   (a) > 10 letter loss in BCVA relative to baseline; and/or
   (b) > 50 µm increase in CRT relative to baseline.
44. The method of any one of embodiments 42-43 further comprising evaluating BVCA and/or CRT in the subject and, if the subject exhibits
   (a) > 10 letter loss in BCVA relative to baseline; and/or
   (b) > 50 µm increase in CRT relative to baseline,
   shortening the tertiary dose interval.
45. The method of any one of embodiments 1-44 wherein if
   (a) greater than 5 letters are lost in BCVA (ETDRS), relative to the BCVA observed at about 12 weeks after treatment initiation;
   (b) a greater than 25 micrometers increase in CRT is observed relative to the CRT observed at about 12 weeks after treatment initiation; and/or
   (c) there is a new onset foveal neovascularization or foveal hemorrhage;

   at week 16 or week 20 after treatment initiation,
   then, the interval between tertiary doses is shortened from 12 weeks or 16 weeks to 8 weeks; or
      (a) greater than 5 letters are lost in BCVA (ETDRS), relative to the BCVA observed at about 12 weeks after treatment initiation;
      (b) a greater than 25 micrometers increase in CRT is observed relative to the CRT observed at about 12 weeks after treatment initiation; and/or
      (c) there is a new onset foveal neovascularization or foveal hemorrhage;
   at week 24 after treatment initiation
   then, the interval between tertiary doses is shortened from 16 weeks to 12 weeks.
46. The method of any one of embodiments 1-45 for treating or preventing neovascular age related macular degeneration, in a subject in need thereof, comprising administering to an eye of the subject 3 doses of about 8 mg VEGF receptor fusion protein in a formulation that comprises about 114.3 mg/ml VEGF receptor fusion protein at an interval of once every 4 weeks; and, after said 3 doses, administering one or more doses of the VEGF receptor fusion protein at an interval which is lengthened up to 12, 16 or 20 weeks.
47. A method for treating or preventing nAMD, in a subject in need thereof, comprising administering to an eye of the subject,
   a single initial dose of about 8 mg or more of VEGF receptor fusion protein, followed by 2 secondary doses of about 8 mg or more of the VEGF receptor fusion protein,
   wherein each secondary dose is administered about 2 to 4 weeks after the immediately preceding dose; and, after said doses,
      a) determining if the subject meets at least one criterion for reducing or lengthening one or more intervals, of 2 weeks, 3 weeks, 4 weeks or 2-4 weeks, between doses of the VEGF receptor fusion protein; and
      b) if said determination is made, administering further doses of the VEGF receptor fusion protein at said reduced or lengthening intervals between doses
   wherein criteria for reducing the interval include:
      1. BCVA loss >5 letters;
      2. >25 micrometers increase in central retinal thickness (CRT);
      3. new foveal hemorrhage; and/or
      4. new foveal neovascularization.
   wherein criteria for lengthening the interval include:
      1. BCVA loss <5 letters,
      2. No fluid at the central subfield;
      3. No new onset foveal hemorrhage; and/or
      4. No foveal neovascularization
48. The method of embodiment 47 wherein criteria for lengthening the interval include:
   1. BCVA loss <5 letters from Week 12;
   2. No fluid at the central subfield on OCT, and
   3. No new onset foveal hemorrhage or foveal neovascularization
   and/or wherein criteria for reducing the interval include both:
   1. BCVA loss >5 letters from Week 12, and
   2. >25 micrometers increase in central retinal thickness (CRT) from Week 12 or new foveal hemorrhage or new foveal neovascularization.
49. The method of embodiment 48 wherein if said criteria are met, said interval is lengthened to 12, 16 or 20 weeks.
50. The method of any one of embodiments 1-49 for treating or preventing neovascular age-related macular degeneration (nAMD), in a subject in need thereof that has been pre-treated with one or more 2 mg doses of VEGF receptor fusion protein, comprising administering to an eye of the subject,
   a single initial dose of about 8 mg or more of a VEGF receptor fusion protein, followed by
   one or more secondary doses of about 8 mg or more of the VEGF receptor fusion protein, followed by
   one or more tertiary doses of about 8 mg or more of the VEGF receptor fusion protein;
   wherein each secondary dose is administered about 2 to 4 weeks after the immediately preceding dose; and
   wherein each tertiary dose is administered about 12-20 weeks after the immediately preceding dose.
51. A method for treating or preventing an angiogenic eye disorder, in a subject in need thereof, comprising administering to an eye of the subject,
   (1) a single initial dose of about 8 mg or more of a VEGF receptor fusion protein, followed by one or more secondary doses of about 8 mg or more of the VEGF receptor fusion protein, followed by one or more tertiary doses of about 8 mg or more of the VEGF receptor fusion protein; wherein each secondary dose is administered about 2 to 4 weeks after the immediately preceding dose; and wherein each tertiary dose is administered about 8 weeks after the immediately preceding dose; or
   (2) one or more doses of 8 mg or more of VEGF receptor fusion protein about every 4 weeks.
52. The method of embodiment 51 wherein the angiogenic eye disorder is nAMD.
53. The method of any one of embodiments 51-52 wherein one or more secondary doses is 2, 3 or 4 secondary doses and/or about 2-4 weeks is about 4 weeks.
54. The method of any one of embodiments 1-53 wherein a subject having any one or more of ocular or periocular infection;
   active intraocular inflammation; and/or
   hypersensitivity;
   is excluded from treatment or prevention.
55. The method of any one of embodiments 1-54 further comprising a step of evaluating the subject for:
   ocular or periocular infection;
   active intraocular inflammation; and/or
   hypersensitivity;
   and excluding the subject treatment or prevention if any one or more if found in the subject.
56. The method of any one of embodiments 1-55 further comprising monitoring the subject during said treatment or prevention for conjunctival hemorrhage, cataract, vitreous detachment, vitreous floaters, corneal epithelium defect and/or increased intraocular pressure.
57. The method of any one of embodiments 1-56 comprising, prior to any administration, providing
   - one single-dose glass vial having a protective plastic cap and a stopper containing an aqueous formulation comprising 8 mg VEGF receptor fusion protein in about 70 microliters;
   - one 18-gauge x 1½-inch, 5-micron, filter needle that includes a bevel;
   - one 30-gauge x ½-inch injection needle; and
   - one 1-mL Luer lock syringe having a graduation marking for 70 microliters of volume packaged together; then
      (1) visually inspecting the aqueous formulation in which the VEGF receptor fusion protein is provided and, if particulates, cloudiness, or discoloration are visible, then using another vial of aqueous formulation;
      (2) removing the protective plastic cap from the vial; and
      (3) Clean the top of the vial with an alcohol wipe; then
   using aseptic technique:
   (4) removing the 18-gauge x 1½-inch, 5-micron, filter needle and the 1 mL syringe from their packaging
   (5) attaching the filter needle to the syringe by twisting it onto the Luer lock syringe tip
   (6) Pushing the filter needle into the center of the vial stopper until the needle is completely inserted into the vial and the tip touches the bottom or a bottom edge of the vial;
   (7) Withdrawing all of the VEGF receptor fusion protein vial contents into the syringe, keeping the vial in an upright position, slightly inclined, while ensuring the bevel of the filter needle is submerged into the liquid;
   (8) Continuing to tilt the vial during withdrawal keeping the bevel of the filter needle submerged in the formulation;
   (9) Drawing the plunger rod sufficiently back when emptying the vial in order to completely empty the filter needle;
   (10) Removing the filter needle from the syringe and disposing of the filter needle;
   (11) Removing the 30-gauge x ½-inch injection needle from its packaging and attaching the injection needle to the syringe by firmly twisting the injection needle onto the Luer lock syringe tip;
   (12) Holding the syringe with the needle pointing up, and checking the syringe for bubbles, wherein if there are bubbles, gently taping the syringe with a finger until the bubbles rise to the top; and
   (13) Slowly depressing the plunger so that the plunger tip aligns with the line that marks 70 microliters on the syringe.
58. The method of any one of embodiments 1-57 wherein VEGF inhibitor is VEGF receptor fusion protein and injection of VEGF receptor fusion protein is performed under controlled aseptic conditions, which comprise surgical hand disinfection and the use of sterile gloves, a sterile drape, and a sterile eyelid speculum (or equivalent) and anesthesia and a topical broad-spectrum microbicide are administered prior to the injection.
59. The method of any one of embodiments 1-58 wherein the subject has been receiving a dosing regimen for treating or preventing nAMD calling for:
   a single initial dose of about 2 mg of VEGF receptor fusion protein, followed by 2 secondary doses of about 2 mg of the VEGF receptor fusion protein, followed by one or more tertiary doses of about 2 mg of the VEGF receptor fusion protein; wherein each secondary dose is administered about 4 weeks after the immediately preceding dose; and wherein each tertiary dose is administered about 8 weeks after the immediately preceding dose;
   wherein the subject is at any phase (initial dose, secondary dose or tertiary dose) of the 2 mg VEGF receptor fusion protein dosing regimen.
60. The method of any one of embodiments 1-59, wherein
   - one or more secondary doses is 2 secondary doses;
   - 2 to 4 weeks is about 4 weeks;
   - 12-20 weeks is about 12 weeks;
   - 12-20 weeks is about 16 weeks;
   - 12-20 weeks is about 20 weeks;
   - 12-20 weeks is about 12-16 weeks;
   - 8-16 weeks is about 12 weeks;
   - 8-16 weeks is about 16 weeks;
   - 8-16 weeks is about 12-16 weeks;
   - 2 to 4 weeks is about 4 weeks and one or more secondary doses is 2 secondary doses;
   - 12-20 weeks is about 12 weeks and one or more secondary doses is 2 secondary doses;
   - 12-20 weeks is about 16 weeks and one or more secondary doses is 2 secondary doses;
   - 12-20 weeks is about 20 weeks and one or more secondary doses is 2 secondary doses;
   - 12-20 weeks is about 12-16 weeks and one or more secondary doses is 2 secondary doses;
   - 2 to 4 weeks is about 4 weeks and one or more secondary doses is 2 secondary doses and a VEGF receptor fusion protein is aflibercept;
   - 12-20 weeks is about 12 weeks and one or more secondary doses is 2 secondary doses and a VEGF receptor fusion protein is aflibercept;
   - 12-20 weeks is about 16 weeks and one or more secondary doses is 2 secondary doses and a VEGF receptor fusion protein is aflibercept;
   - 12-20 weeks is about 20 weeks and one or more secondary doses is 2 secondary doses and a VEGF receptor fusion protein is aflibercept; and/or
   - 12-20 weeks is about 12-16 weeks and one or more secondary doses is 2 secondary doses and a VEGF receptor fusion protein is aflibercept.
61. The method of any one of embodiments 1-60, wherein the VEGF receptor fusion protein:
   (i) comprises two polypeptides that comprise (1) a VEGFR1 component comprising amino acids 27 to 129 of SEQ ID NO: 2; (2) a VEGFR2 component comprising amino acids 130-231 of SEQ ID NO: 2; and (3) a multimerization component comprising amino acids 232-457 of SEQ ID NO: 2;
   (ii) comprises two polypeptides that comprise an immunoglobin-like (Ig) domain 2 of VEGFR1, an Ig domain 3 of a VEGFR2, and a multimerizing component;
   (iii) comprises two polypeptides that comprise an immunoglobin-like (Ig) domain 2 of VEGFR1, an Ig domain 3 of VEGFR2, an Ig domain 4 of VEGFR2 and a multimerizing component;
   (iv) comprises two VEGFR1R2-FcΔC1(a) polypeptides encoded by the nucleic acid sequence of SEQ ID NO: 1; or
   (v) is selected from the group consisting of: aflibercept and conbercept
62. The method of embodiment 61, wherein the VEGF receptor fusion protein comprises amino acids 27-457 of the amino acid sequence set forth in SEQ ID NO: 2.
63. The method of any one of embodiments 1-62, wherein the 8 mg of VEGF receptor fusion protein is in an aqueous pharmaceutical formulation selected from the group consisting of A-KKKK.
64. The method of any one of embodiments 1-63 wherein 8 mg of VEGF receptor fusion protein is administered in an aqueous pharmaceutical formulation comprising about 114.3 mg/ml VEGF receptor fusion protein.
65. The method of any one of embodiments 1-64, wherein VEGF receptor fusion protein is intravitreally administered from a syringe or pre-filled syringe.
66. The method of embodiment 65, wherein the syringe or pre-filled syringe is glass or plastic, and/or sterile.
67. The method of any one of embodiments 1-66 wherein VEGF receptor fusion protein is intravitreally injected with a 30 gauge × ½-inch sterile injection needle.
68. The method of any one of embodiments 1-67 wherein the subject has previously received one or more doses of 2 mg VEGF receptor fusion protein.
69. The method of any one of embodiments 1-68 wherein one or more further doses are administered.
70. The method of any one of embodiments 1-69 wherein 2 mg VEGF receptor fusion protein is in an aqueous pharmaceutical formulation comprising 40 mg/ml VEGF receptor fusion protein.
71. The method of embodiment 70 wherein 2 mg of VEGF receptor fusion protein is in a pharmaceutical formulation comprising:
   40 mg/ml VEGF receptor fusion protein, 10 mM sodium phosphate, 40 mM NaCl, 0.03% polysorbate 20 and 5% sucrose, with a pH of 6.2.
72. The method of any one of embodiments 1-71 wherein 8 mg of VEGF receptor fusion protein is in an aqueous pharmaceutical formulation that comprises a sugar or polyol.
73. The method of any one of embodiments 1-72 wherein 8 mg VEGF receptor fusion protein in an aqueous pharmaceutical formulation that comprises sucrose.
74. The method of any one of embodiments 1-73 wherein 8 mg of a VEGF receptor fusion protein is in an aqueous pharmaceutical formulation that has a pH of about 5.8.
75. The method of any one of embodiments 1-74 wherein 8 mg of a VEGF receptor fusion protein is in an aqueous pharmaceutical formulation comprising about 103-126 mg/ml VEGF receptor fusion protein, histidine-based buffer and arginine.
76. The method of any one of embodiments 1-75 wherein 8 mg of a VEGF receptor fusion protein is an aqueous pharmaceutical formulation comprising about 114.3 mg/ml VEGF receptor fusion protein, histidine-based buffer and arginine.
77. The method of any one of embodiments 1-76 wherein the ≥8 mg aflibercept is in an aqueous pharmaceutical formulation wherein the aflibercept has less than about 3.5% high molecular weight species immediately after manufacture and purification and/or less than or equal to about 6% high molecular weight species after storage for about 24 months at about 2-8° C.
78. The method of any one of embodiments 1-77 wherein the ≥8 mg VEGF receptor fusion protein is in an aqueous pharmaceutical formulation comprising:
   at least about 100 mg/ml of a VEGF receptor fusion protein;
   about 10-100 mM L-arginine;
   sucrose;
   a histidine-based buffer; and
   a surfactant;
   wherein the formulation has a pH of about 5.0 to about 6.8; wherein the VEGF receptor fusion protein has less than about 3.5% high molecular weight species immediately after manufacture and purification and/or less than or equal to about 6% high molecular weight species after storage for about 24 months at about 2-8°C.
79. The method of embodiment 1-78 wherein 8 mg of VEGF receptor fusion protein is in an aqueous pharmaceutical formulation comprising
   - ≥100 mg/ml VEGF receptor fusion protein, histidine-based buffer and L-arginine;
   - 140 mg/ml aflibercept; 20 mM histidine-based buffer; 5 % sucrose; 0.03 % polysorbate 20; 10 mM L-arginine; pH 5.8;
   - 150 ± 15 mg/ml aflibercept, 10 mM phosphate-based buffer, 8 ± 0.8% (w/v) sucrose, 0.02-0.04% (w/v) polysorbate 20 and 50 mM L-arginine, pH 5.9-6.5;
   - 103-126 mg/ml aflibercept, 10 ± 1 mM histidine-based buffer, 5 + 0.5% (w/v) sucrose, 0.02-0.04% (w/v) polysorbate 20, and 50 ± 5 mM L-arginine, pH 5.5-6.1;
   - 140 mg/ml aflibercept, 10 mM histidine-based buffer, 2.5 % (w/v) sucrose, 2.0 % (w/v) proline, 0.03 % (w/v) polysorbate 20 and 50 mM L-arginine, pH 5.8;
   - 114.3 mg/ml aflibercept, 10 mM histidine-based buffer, 5% (w/v) sucrose, 0.03% (w/v) polysorbate 20 and 50 mM L-arginine, pH 5.8;
   - >100 mg/ml aflibercept, histidine-based buffer and L-arginine;
   - >100 mg/ml aflibercept at about pH 5.8, wherein the formulation forms <3% HMW aggregates after incubation at 5°C for 2 months;
   - About 114.3 mg/mL aflibercept; 10 mM - 50 mM histidine-based buffer, sugar, non-ionic surfactant, L-Arginine, pH 5.8; or
   - About 114.3 mg/mL aflibercept; 10 mM His/His-HCl-based buffer, 5% sucrose, 0.03% polysorbate-20, 50 mM L-Arginine, pH 5.8.
80. The method of any one of embodiments 1-79 wherein 8 mg of VEGF receptor fusion protein is administered in about 100 µl or less, about 75 µl or less; about 70 µl or less; or about 50 µl; 51 µl; 52 µl; 53 µl; 54 µl; 55 µl; 56 µl; 57 µl; 58 µl; 59 µl; 60 µl; 61 µl; 62 µl; 63 µl; 64 µl; 65 µl; 66 µl; 67 µl; 68 µl; 69 µl; 70 µl; 71 µl; 72 µl; 73 µl; 74 µl; 75 µl; 76 µl; 77 µl; 78 µl; 79 µl; 80 µl; 81 µl; 82 µl; 83 µl; 84 µl; 85 µl; 86 µl; 87 µl; 88 µl; 89 µl; 90 µl; 91 µl; 92 µl; 93 µl; 94 µl; 95 µl; 96 µl; 97 µl; 98 µl; 99 µl; or 100 µl.
81. The method of embodiment 80 wherein said VEGF receptor fusion protein is administer in about 70 ± 4 or 5 microliters.
82. The method of any one of embodiments 1-81 comprising administering the VEGF receptor fusion protein to both eyes of the subject.
83. The method of any one of embodiments 1-82, wherein the subject achieves and/or maintains one or more of:
   - Increase in best corrected visual acuity (BCVA) by ≥5, ≥10, ≥15, or ≥20 letters;
   - No decrease in best corrected visual acuity (BCVA);
   - Elimination of retinal fluid;
   - Elimination of intraretinal fluid (IRF) and/or subretinal fluid;
   - Decrease in total lesion choroidal neovascularization (CNV) area;
   - Loss of or decrease in intraretinal fluid;
   - Loss of or decrease in subretinal fluid;
   - Decrease in central subfield retinal thickness (CST);
   - Increase in vision-related quality of life;
   - Lack of treatment-emergent adverse events (AEs) and/or serious AEs (SAEs);
   - ETDRS letter score of at least 69 (approximate 20/40 Snellen equivalent);
   - Increase in BCVA as measured by the Early Treatment Diabetic Retinopathy Study (ETDRS) visual acuity chart by ≥5, ≥10, ≥15, or ≥20 letters, or lack of loss thereof during the course of treatment;
   - Increase in BCVA as averaged over a period of 12 weeks;
   - No intraretinal fluid (IRF) and no subretinal fluid;
   - Decrease in choroidal neovascularization (CNV) size;
   - Decrease in total lesion CNV area from baseline;
   - Loss of IRF and/or SRF;
   - Decrease in central subfield retinal thickness (CST);
   - Increase in vision-related quality of life as measured by the National Eye Institute Visual Functioning Questionnaire-25 (NEI-VFQ-25);
   - Lack of treatment-emergent adverse events (AEs) and/or serious AEs (SAEs);
   - Efficacy and/or safety, in a subject suffering from nAMD, similar to that of aflibercept which is intravitreally dosed at 2 mg approximately every 4 weeks for the first 3 months, followed by 2 mg approximately once every 8 weeks or once every 2 months wherein efficacy is measured as increase in BCVA and/or reduction in central retinal thickness, and wherein safety is as measured as the incidence of adverse events such as blood pressure increase, intraocular pressure increase, visual impairment, vitreous floaters, vitreous detachment, iris neovascularization and/or vitreous hemorrhage;
   - No detectable anti-drug antibody during receipt of treatment;
   - Improvement in best corrected visual acuity (BVCA) by week 4, week 8, week 12, week 16, week 20, week 24, week 28, week 32, week 36, week 40, week 44, week 48, week 52, week 56 or week 60 from start of treatment (baseline);
   - Increase in BCVA as measured by the Early Treatment Diabetic Retinopathy Study (ETDRS) visual acuity chart or Snellen equivalent by ≥2 letters, ≥3 letters, ≥4 letters, ≥5 letters, ≥6 letters or ≥7 letters;
   - Improvement in BCVA, by 4 weeks after initiation of treatment, of about 2 or 3 letters (ETDRS or Snellen equivalent) when on HDq12 regimen; or of about 3 letters (ETDRS or Snellen equivalent) when on HDq16 regimen;
   - Improvement in BCVA, by 8 weeks after initiation of treatment, of about 5 letters (ETDRS or Snellen equivalent) when on HDq12 regimen; or of about 4 or 5 letters (ETDRS or Snellen equivalent) when on HDq16 regimen;
   - Improvement in BCVA, by 12 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
   - Improvement in BCVA, by 16 weeks after initiation of treatment, of about 6 letters (ETDRS or Snellen equivalent) when on HDq12 regimen; or of about 6 or 7 letters (ETDRS or Snellen equivalent) when on HDq16 regimen;
   - Improvement in BCVA, by 20 weeks after initiation of treatment, of about 6 letters (ETDRS or Snellen equivalent) when on HDq12 regimen; or of about 6 letters (ETDRS or Snellen equivalent) when on HDq16 regimen;
   - Improvement in BCVA, by 24 weeks after initiation of treatment, of about 5 or 6 letters (ETDRS or Snellen equivalent) when on HDq12 regimen; or of about 5 or 6 letters (ETDRS or Snellen equivalent) when on HDq16 regimen;
   - Improvement in BCVA, by 28 weeks after initiation of treatment, of about 7 letters (ETDRS or Snellen equivalent) when on HDq12 regimen; or of about 6 letters (ETDRS or Snellen equivalent) when on HDq16 regimen;
   - Improvement in BCVA, by 32 weeks after initiation of treatment, of about 6 or 7 letters (ETDRS or Snellen equivalent) when on HDq12 regimen; or of about 7 letters (ETDRS or Snellen equivalent) when on HDq16 regimen;
   - Improvement in BCVA, by 36 weeks after initiation of treatment, of about 6 or 7 letters (ETDRS or Snellen equivalent) when on HDq12 regimen; or of about 6 letters (ETDRS or Snellen equivalent) when on HDq16 regimen;
   - Improvement in BCVA, by 40 weeks after initiation of treatment, of about 6 or 7 letters (ETDRS or Snellen equivalent) when on HDq12 regimen; or of about 5 or 6 letters (ETDRS or Snellen equivalent) when on HDq16 regimen;
   - Improvement in BCVA, by 44 weeks after initiation of treatment, of about 7 letters (ETDRS or Snellen equivalent) when on HDq12 regimen; or of about 5 or 6 letters (ETDRS or Snellen equivalent) when on HDq16 regimen;
   - Improvement in BCVA, by 48 weeks after initiation of treatment, of about 6 or 7 letters (ETDRS or Snellen equivalent) when on HDq12 regimen; or of about 6 letters (ETDRS or Snellen equivalent) when on HDq16 regimen;
   - Improvement in BCVA, by 52 weeks after initiation of treatment, of about 7 or 8 letters (ETDRS or Snellen equivalent) when on HDq12 regimen; or of about 6 or 7 letters (ETDRS or Snellen equivalent) when on HDq16 regimen;
   - Improvement in BCVA, by 56 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
   - Improvement in BCVA, by 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
   - Improvement in BCVA, from about 48 to about 60 weeks after initiation of treatment, of about 6 or 7 letters (ETDRS or Snellen equivalent) when on HDq12 regimen; or of about 6 or 7 letters (ETDRS or Snellen equivalent) when on HDq16 regimen;
   - Improvement in BCVA, by 60 weeks after initiation of treatment, of about 5, 10 or 15 letters (ETDRS or Snellen equivalent) when on the HDq12 regimen; or the HDq16 regimen;
   - An improvement in BCVA by about week 8, 9, 10, 11 or 12 after initiation of treatment which is maintained (within about ±1 or ±2 ETDRS letters or Snellen equivalent) thereafter during the treatment regimen;
   - A BCVA by 4 weeks after initiation of treatment of about 63 letters (ETDRS or Snellen equivalent) when on the HDq12 regimen; or a BCVA of about 63 letters (ETDRS or Snellen equivalent) when on the HDq16 regimen;
   - A BCVA by 8 weeks after initiation of treatment of about 65 letters (ETDRS or Snellen equivalent) when on the HDq12 regimen; or a BCVA of about 65 letters (ETDRS or Snellen equivalent) when on the HDq16 regimen;
   - A BCVA by12 weeks after initiation of treatment of about 66 letters (ETDRS or Snellen equivalent) when on the HDq12 regimen; or a BCVA of about 66 letters (ETDRS or Snellen equivalent) when on the HDq16 regimen;
   - A BCVA by 16 weeks after initiation of treatment of about 66 letters (ETDRS or Snellen equivalent) when on the HDq12 regimen; or a BCVA of about 66 letters (ETDRS or Snellen equivalent) when on the HDq16 regimen;
   - A BCVA by 20 weeks after initiation of treatment of about 66 letters (ETDRS or Snellen equivalent) when on the HDq12 regimen; or a BCVA of about 66 letters (ETDRS or Snellen equivalent) when on the HDq16 regimen;
   - A BCVA by 24 weeks after initiation of treatment of about 66 letters (ETDRS or Snellen equivalent) when on the HDq12 regimen; or a BCVA of about 66 letters (ETDRS or Snellen equivalent) when on the HDq16 regimen;
   - A BCVA by 28 weeks after initiation of treatment of about 67 letters (ETDRS or Snellen equivalent) when on the HDq12 regimen; or a BCVA of about 66letters (ETDRS or Snellen equivalent) when on the HDq16 regimen;
   - A BCVA by 32 weeks after initiation of treatment of about 67 letters (ETDRS or Snellen equivalent) when on the HDq12 regimen; or a BCVA of about 67 letters (ETDRS or Snellen equivalent) when on the HDq16 regimen;
   - A BCVA by 36 weeks after initiation of treatment of about 67 letters (ETDRS or Snellen equivalent) when on the HDq12 regimen; or a BCVA of about 66 letters (ETDRS or Snellen equivalent) when on the HDq16 regimen;
   - A BCVA by 40 weeks after initiation of treatment of about 67 letters (ETDRS or Snellen equivalent) when on the HDq12 regimen; or a BCVA of about 66 letters (ETDRS or Snellen equivalent) when on the HDq16 regimen;
   - A BCVA by 44 weeks after initiation of treatment of about 68 letters (ETDRS or Snellen equivalent) when on the HDq12 regimen; or a BCVA of about 66 letters (ETDRS or Snellen equivalent) when on the HDq16 regimen;
   - A BCVA by 48 weeks after initiation of treatment of about 67 letters (ETDRS or Snellen equivalent) when on the HDq12 regimen; or a BCVA of about 66 letters (ETDRS or Snellen equivalent) when on the HDq16 regimen;
   - A BCVA by 52 weeks after initiation of treatment of about 67 or 68 letters (ETDRS or Snellen equivalent) when on the HDq12 regimen; or a BCVA of about 66 or 67 letters (ETDRS or Snellen equivalent) when on the HDq16 regimen;
   - A BCVA by 56 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
   - A BCVA by 60 weeks after initiation of treatment of about 66 or 67 letters (ETDRS or Snellen equivalent) when on the HDq12 regimen; or a BCVA of about 66 or 67 letters (ETDRS or Snellen equivalent) when on the HDq16 regimen;
   - A BCVA between about week 48 and about 60 week after initiation of treatment of about 66 to about 72 letters (ETDRS or Snellen equivalent) when on the HDq12 regimen; or a BCVA of about 66 to about 70 letters (ETDRS or Snellen equivalent) when on the HDq16 regimen;
   - Retina without fluid (total fluid, intraretinal fluid [IRF] and/or subretinal fluid [SRF]) in center subfield;
   - No subretinal pigment epithelium fluid;
   - Lack of fluid leakage on fluorescein angiography (FA);
   - Decrease in central retinal thickness (CRT) by at least about 100, 125, 130, 135, 140, 141, 142, 143, 144, 145, 146, 147, 148, 149 or 150 micrometers;
   - A change in central retinal thickness, by 4 weeks after initiation of treatment of about - 120 or -121 or -122 or -122.4 or -120.2 micrometers (± about 10, 11 or 12 micrometers) when on the HDq12 regimen; or of about -126 or -127 or-126.6 or -126.3 micrometers (+ about 10, 11 or 12 micrometers) when on the HDq16 regimen;
   - A change in central retinal thickness, by 8 weeks after initiation of treatment of about - 132, -133, -134, -135 or -136 or -136.2 or -132.8 micrometers (± about 10, 11 or 12 micrometers) when on the HDq12 regimen; or of about -139 or -140 or -139.5 or -139.6 micrometers (± about 10, 11 or 12 micrometers) when on the HDq16 regimen;
   - A change in central retinal thickness, by 12 weeks after initiation of treatment of about - 136, -137, -138, -139, -140 or -141 or -140.9 or -136.6 micrometers (± about 10, 11 or 12 micrometers) when on the HDq12 regimen; or of about -144 or -143 or -143.5 micrometers (± about 10, 11 or 12 micrometers) when on the HDq16 regimen
   - A change in central retinal thickness, by 16 weeks after initiation of treatment of about - 120 or -121 or -122 or -123 or -124 or -123.4 or -120.1 micrometers (± about 10, 11 or 12 micrometers) when on the HDq12 regimen; or of about -132 or -133 or -132.1 or - 133.1 micrometers (± about 10, 11 or 12 micrometers) when on the HDq16 regimen;
   - A change in central retinal thickness, by 20 weeks after initiation of treatment of about - 110 or -111 or -112 or -113 or -114 or -113.6 or -110.9 micrometers (± about 10, 11 or 12 micrometers) when on the HDq12 regimen; or of about -115 or -116 or -117 or -118 or -115.8 or -117.7 micrometers (± about 10, 11 or 12 micrometers) when on the HDq16 regimen;
   - A change in central retinal thickness, by 24 weeks after initiation of treatment of about - 134, -135, -136 or -137 or -138 or -137.6 or -134.9 micrometers (± about 10, 11 or 12 micrometers) when on the HDq12 regimen; or of about -105 or -106 or -107 or -108 or - 105.3 or -107.8 micrometers (± about 10, 11 or 12 micrometers) when on the HDq16 regimen;
   - A change in central retinal thickness, by 28 weeks after initiation of treatment of about - 130, -131 or -132 or -133 or -134 or -133.7 or -130.7 micrometers (± about 10, 11 or 12 micrometers) when on the HDq12 regimen; or of about -144 or -145 or -146 or -147 or - 148 or -144.7 or -147.2 micrometers (± about 10, 11 or 12 micrometers) when on the HDq16 regimen;
   - A change in central retinal thickness, by 32 weeks after initiation of treatment of about - 118 or -19 or -120 or -121 or -120.4 or -118.1 micrometers (± about 10, 11 or 12 micrometers) when on the HDq12 regimen; or of about -141 or -142 or -143 or -144 or - 141.5 or -144 micrometers (± about 10, 11 or 12 micrometers) when on the HDq16 regimen;
   - A change in central retinal thickness, by 36 weeks after initiation of treatment of about - 142 or -143 or -144 or -144.2 or -142.2 micrometers (± about 10, 11 or 12 micrometers) when on the HDq12 regimen; or of about -126 or -127, -128 or -129 or -130 or -131 or - 126.4 or -130.5 micrometers (± about 10, 11 or 12 micrometers) when on the HDq16 regimen;
   - A change in central retinal thickness, by 40 weeks after initiation of treatment of about - 131, -132, -133 or -134 or -133.8 or -131.2 micrometers (± about 10, 11 or 12 micrometers) when on the HDq12 regimen; or of about -127, -128 or -127.5 micrometers (± about 10, 11 or 12 micrometers) when on the HDq16 regimen;
   - A change in central retinal thickness, by 44 weeks after initiation of treatment of about - 120 or -121 or -122 or -123 or -124 or -125 or -124.7 or -120.3 micrometers (± about 10, 11 or 12 micrometers) when on the HDq12 regimen; or of about -143, -144 or -145 or - 144.8 micrometers (± about 10, 11 or 12 micrometers) when on the HDq16 regimen;
   - A change in central retinal thickness, by 48 weeks after initiation of treatment of about - 142 or -143 or -144 or -144.4 or -142.3 micrometers (± about 10, 11 or 12 micrometers) when on the HDq12 regimen; or of about -143 or -144 or -145 or -146 or -147 or -148 or -143.8 or -147.1 micrometers (± about 10, 11 or 12 micrometers) when on the HDq16 regimen;
   - A change in central retinal thickness, by 52 weeks after initiation of treatment of about - 143.2 micrometers (± about 10, 11 or 12 micrometers) when on the HDq12 regimen; or of about -139.6 micrometers (± about 10, 11 or 12 micrometers) when on the HDq16 regimen;
   - A change in central retinal thickness, by 56 weeks after initiation of treatment of about - 136.3 micrometers (± about 10, 11 or 12 micrometers) when on the HDq12 regimen; or of about -137.5 micrometers (± about 10, 11 or 12 micrometers) when on the HDq16 regimen;
   - A change in central retinal thickness, by 60 weeks after initiation of treatment of about - 151.8 micrometers (± about 10, 11 or 12 micrometers) when on the HDq12 regimen; or of about -148.8 micrometers (± about 10, 11 or 12 micrometers) when on the HDq16 regimen;
   - A central retinal thickness by week 4 after initiation of treatment of about 248.2 micrometers when on the HDq12 regimen; or of about 244.1 micrometers when on the HDq16 regimen;
   - A central retinal thickness by week 8 after initiation of treatment of about 234.4 micrometers when on the HDq12 regimen; or of about 231.2 micrometers when on the HDq16 regimen;
   - A central retinal thickness by week 12 after initiation of treatment of about 229.7 micrometers when on the HDq12 regimen; or of about 226.7 micrometers when on the HDq16 regimen;
   - A central retinal thickness by week 16 after initiation of treatment of about 247.2 micrometers when on the HDq12 regimen; or of about 238.6 micrometers when on the HDq16 regimen;
   - A central retinal thickness by week 20 after initiation of treatment of about 257 micrometers when on the HDq12 regimen; or of about 254.9 micrometers when on the HDq16 regimen;
   - A central retinal thickness by week 24 after initiation of treatment of about 233 micrometers when on the HDq12 regimen; or of about 265.4 micrometers when on the HDq16 regimen;
   - A central retinal thickness by week 28 after initiation of treatment of about 236.9 micrometers when on the HDq12 regimen; or of about 226 micrometers when on the HDq16 regimen;
   - A central retinal thickness by week 32 after initiation of treatment of about 250.2 micrometers when on the HDq12 regimen; or of about 229.2 micrometers when on the HDq16 regimen;
   - A central retinal thickness by week 36 after initiation of treatment of about 226.4 micrometers when on the HDq12 regimen; or of about 244.3 micrometers when on the HDq16 regimen;
   - A central retinal thickness by week 40 after initiation of treatment of about 236.8 micrometers when on the HDq12 regimen; or of about 243.7 micrometers when on the HDq16 regimen;
   - A central retinal thickness by week 44 after initiation of treatment of about 245.9 micrometers when on the HDq12 regimen; or of about 227.7 micrometers when on the HDq16 regimen;
   - A central retinal thickness by week 48 after initiation of treatment of about 226.2 micrometers when on the HDq12 regimen; or of about 226.9 micrometers when on the HDq16 regimen;
   - A central retinal thickness by week 52 after initiation of treatment of about 227.4 micrometers when on the HDq12 regimen; or of about 231.1 micrometers when on the HDq16 regimen;
   - A central retinal thickness by week 56 after initiation of treatment of about 234.3 micrometers when on the HDq12 regimen; or of about 233.2 micrometers when on the HDq16 regimen;
   - A central retinal thickness by week 60 after initiation of treatment of about 218.8 micrometers when on the HDq12 regimen; or of about 221.9 micrometers when on the HDq16 regimen;
   - A CRT by about week 4, 5, 6, 7 or 8 after initiation of treatment or a reduction in CRT by week 4, 5, 6, 7 or 8 after initiation of treatment which is maintained (within about ±10, ±11 or ±12 micrometers) thereafter during the treatment regimen;
   - At about 4 hours after treatment initiation of HDq12 or HDq16, a free aflibercept concentration in plasma of about 0.0409 (±0.0605) or 0 mg/L (or <0.0156 mg/L);
   - At about 8 hours after treatment initiation of HDq12 or HDq16, a free aflibercept concentration in plasma of about 0.05 (±3.78), 0.0973 (±0.102) or 0.0672 mg/L;
   - At about day 2 after treatment initiation of HDq12 or HDq16, a free aflibercept concentration in plasma of about 0.11 (±2.21), 0.146 (±0.110) or 0.0903 mg/L;
   - At about day 3 after treatment initiation of HDq12 or HDq16, a free aflibercept concentration in plasma of about 0.11 (±2.06), 0.137 (±0.0947) or 0.112 mg/L;
   - At about day 5 after treatment initiation of HDq12 or HDq16, a free aflibercept concentration in plasma of about 0.08 (±1.86), 0.0933 (±0.0481) or 0.0854 mg/L;
   - At about day 8 after treatment initiation of HDq12 or HDq16, a free aflibercept concentration in plasma of about 0.07 (±1.75), 0.0794 (±0.0413) or 0.0682 mg/L ;
   - At about day 15 after treatment initiation of HDq12 or HDq16, a free aflibercept concentration in plasma of about 0.04 (±1.76), 0.0435 (±0.0199) or 0.0385 mg/L ;
   - At about day 22 after treatment initiation of HDq12 or HDq16, a free aflibercept concentration in plasma of about 0.02 (±1.76), 0.0213 (±0.0148) or 0.0232 mg/L;
   - At about day 29 after treatment initiation of HDq12 or HDq16, a free aflibercept concentration in plasma of about 0.00766 (±0.00958) or 0 mg/L (or <0.0156 mg/L);
   - At about 4 hours post-dose after treatment initiation of HDq12 or HDq16, an adjusted bound aflibercept concentration in plasma of about 0.00 mg/L;
   - At about 8 hours post-dose after treatment initiation of HDq12 or HDq16, an adjusted bound aflibercept concentration in plasma of about 0.00 mg/L;
   - At about day 2 after treatment initiation of HDq12 or HDq16, an adjusted bound aflibercept concentration in plasma of about 0.06 (±3.50) or 0.124 (±0.186) mg/L;
   - At about day 3 after treatment initiation of HDq12 or HDq16, an adjusted bound aflibercept concentration in plasma of about 0.13 (±2.07) or 0.173 (±0.155) mg/L;
   - At about day 5 after treatment initiation of HDq12 or HDq16, an adjusted bound aflibercept concentration in plasma of about 0.18 (±1.88) or 0.223 (±0.157) mg/L;
   - At about day 8 after treatment initiation of HDq12 or HDq16, an adjusted bound aflibercept concentration in plasma of about 0.31 (±1.56) or 0.334 (±0.135) mg/L;
   - At about day 15 after treatment initiation of HDq12 or HDq16, an adjusted bound aflibercept concentration in plasma of about 0.37 (±1.50) or 0.393 (±0.130) mg/L;
   - At about day 22 after treatment initiation of HDq12 or HDq16, an adjusted bound aflibercept concentration in plasma of about 0.25 (±3.00) or 0.335 (±0.155) mg/L;
   - At about day 29 after treatment initiation of HDq12 or HDq16, an adjusted bound aflibercept concentration in plasma of about 0.32 (±1.39) or 0.331 (±0.0953) mg/L;
   - Non-inferior BVCA compared to that of aflibercept which is intravitreally dosed at 2 mg approximately every 4 weeks for the first 5 injections followed by 2 mg approximately once every 8 weeks or once every 2 months;
   - Ocular and non-ocular safety or death rate, in a subject suffering from DME, similar to that of aflibercept which is intravitreally dosed at 2 mg approximately every 4 weeks for the first 3 or 4 or 5 injections followed by 2 mg approximately once every 8 weeks or once every 2 months;
   - An improvement in best corrected visual acuity by 4, 8, 12, 16, 20, 24, 28, 32, 36, 40, 44 or 48 weeks from start of treatment;
   - An increase in best corrected visual acuity, as measured by Early Treatment Diabetic Retinopathy Study (ETDRS) visual acuity chart or Snellen equivalent of ≥ 2 letters, ≥ 3 letters, ≥ 4 letters, ≥ 5 letters, ≥ 6 letters or ≥ 7 letters by 4, 8, 12, 16, 20, 24, 28, 32, 36, 40, 44, 48, 52, 56 or 60 weeks from start of treatment;
   - A retina without fluid (total fluid, intraretinal fluid [IRF] and/or subretinal fluid [SRF]) in center subfield by 4, 8, 12, 16, 20, 24, 28, 32, 36, 40, 44, 48, 52, 56 or 60 weeks from start of treatment; and/or
   - a decrease in central retinal thickness (CRT) of at least 100, 125, 130, 135, 140, 141, 142, 143, 144, 145, 146, 147, 148, 149 or 150 micrometers by 4, 8, 12, 16, 20, 24, 28, 32, 36, 40, 44, 48, 52, 56 or 60 weeks from start of treatment.
84. The method of embodiment 83, wherein a dry retina lacks intraretinal fluid and/or subretinal fluid.
85. The method of embodiment 83, wherein dry retina is characterized by no intraretinal fluid (IRF) and no subretinal fluid (SRF) in the eye of the subject.
86. The method of embodiment 83, wherein dry retina is characterized by no intraretinal fluid (IRF) and no subretinal fluid (SRF) in the eye of the subject, after the subject has received three monthly doses of VEGF receptor fusion protein.
87. The method of any one of embodiments 1-86 wherein
   - 1 initial dose, 2 secondary doses and 3 tertiary doses of said ≥ 8mg VEGF receptor fusion protein are administered to the subject in the first year;
   - 1 initial dose, 2 secondary doses and 2 tertiary doses of said ≥ 8mg VEGF receptor fusion protein are administered to the subject in the first year; or
   - 1 initial dose, 2 secondary doses and 3 tertiary doses of said ≥ 8mg VEGF receptor fusion protein are administered to the subject in the first year followed by 2 -4 tertiary doses in the second year.
88. The method of any one of embodiments 1-87 wherein the interval between doses are adjusted (increased/maintained/reduced) based on visual and/or anatomic outcomes.
89. The method of any one of embodiments 1-88, wherein 12-20 weeks is 12, 13, 14, 15, 16, 17, 18, 19 or 20 weeks; and 2-4 weeks is 2, 3, 4 or 5 weeks.
90. A method for treating or preventing a neovascular age-related macular degeneration (nAMD), in a subject in need thereof, comprising
   administering to an eye of the subject, a single initial dose of about ≥8 mg or more of a VEGF receptor fusion protein,
   followed by one or more secondary doses of about 8 mg or more of the VEGF receptor fusion protein,
   followed by one or more tertiary doses of about 8 mg or more of the VEGF receptor fusion protein;
   wherein each secondary dose is administered about 2 to 4 weeks after the immediately preceding dose; and
   wherein each tertiary dose is administered about 12-16 weeks after the immediately preceding dose and
   wherein the VEGF receptor fusion protein is aflibercept and
   wherein the vitreal half-life of aflibercept is increased compared to the intravitreal half-life of aflibercept after intravitreal application of 40 mg/mL aflibercept, 10 mM phosphate buffer, 5% Sucrose, 0.03% polysorbate 20, 40 mM NaCl, pH 6.2 - 6.3.
91. The method of any one of embodiments 1-90 wherein the ≥8 mg aflibercept is in an aqueous pharmaceutical formulation and forms 3 - 20% high molecular weight (HMW) aggregates after incubation at 37°C for 28 days and forms <3% HMW aggregates after incubation at 5°C for 2 month.
92. The method of any one of embodiments 1-91 further including one or more periods of *pro re nata* (PRN), capped PRN or treat and extend (T&E) dosing.
93. The method of any one of embodiments 1-92, wherein VEGF receptor fusion protein is aflibercept.
94. A kit comprising
   - a container comprising VEGF receptor fusion protein; and
   - Instruction for use of VEGF receptor fusion protein,

   wherein the container is a vial or a pre-filled syringe,
   wherein the container comprises ≥ 100 mg/mL VEGF receptor fusion protein, or wherein the container comprises ≥ 114.3 mg/mL VEGF receptor fusion protein,
   wherein the instruction comprises instruction for the administration of VEGF receptor fusion protein to nAMD patients,
   wherein the instruction comprises instruction that VEGF receptor fusion protein 8 mg treatment is initiated with 1 injection per month (every 4 weeks) for 3 consecutive doses,
   wherein the instruction comprises instruction that after the initial 3 consecutive doses the injection interval may be lengthened up to every 16 weeks or every 20 weeks, and
   wherein the instruction comprises instruction that the treatment interval may be adjusted based on the physician's judgement of visual and/or anatomic outcomes.

## Claims

1. Aflibercept for use a method of treating neovascular age related macular degeneration (nAMD) in a subject which was pre-treated with 2 mg aflibercept, the method comprising administering to an eye of the subject a single initial dose of about 8 mg aflibercept by intravitreal injection, followed by one or more secondary doses of about 8 mg of aflibercept administered by intravitreal injection, followed by one or more tertiary doses of about 8 mg aflibercept administered by intravitreal injection, wherein each secondary dose is administered about 4 weeks or about every 28 days ± 5 days or about every month after the immediately preceding dose and wherein each tertiary dose is administered about 8, 10, 12, 14, 16, 18 or 20 weeks after the immediately preceding dose.

2. Aflibercept for use according to claim 1, wherein the method comprises administering the one or more tertiary doses according to a treat and extend dosing regimen.

3. Aflibercept for use according to claim 1 or 2, wherein the method comprises administering the one or more tertiary doses every 16 weeks or about every 4 months or about every 112 days ± 5 days.

4. Aflibercept for use according to claim 1 or 2, wherein the method comprises administering the one or more tertiary doses every 12 weeks, about every 3 months, about every 90 days, 84 days, 84 + 5 days or 90 + 5 days.

5. Aflibercept for use of any one of the preceding claims wherein two secondary doses are administered.

6. Aflibercept for use according to any one of the preceding claims, wherein the subject previously received aflibercept according to a 2q8 dosing regimen comprising 3 initial monthly doses followed by one or more maintenance doses every 8 weeks.

7. Aflibercept for use according to any one of the preceding claims, wherein the 8 mg aflibercept is administered to both eyes of the subject.

8. Aflibercept for use according to any one of the preceding claims, wherein the 8 mg aflibercept is administered from a syringe or pre-filled syringe.

9. Aflibercept for use according to any one of the preceding claims, wherein the 8 mg aflibercept is in an aqueous pharmaceutical formulation comprising 114.3 mg/mL aflibercept.

10. Aflibercept for use according to any one of the preceding claims, wherein the 8 mg aflibercept is administered in a volume of about 70 ± 4 or 5 microliters.

11. Aflibercept for use according to any one of the preceding claims, further comprising monitoring the subject during said treatment for conjunctival hemorrhage, cataract, vitreous detachment, vitreous floaters, corneal epithelium defect and/or increased intraocular pressure.
